(19) Europäisches Patentamt
European Patent Office
Office européen des brevets

(11) EP 3 822 368 A1

(12) EUROPEAN PATENT APPLICATION

(43) Date of publication:
**19.05.2021 Bulletin 2021/20**

(51) Int Cl.:
***C12Q 1/6886*** *(2018.01)*

(21) Application number: **19209115.5**

(22) Date of filing: **14.11.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
- **ALVES DE INDA, Márcia**
  **5656 AE Eindhoven (NL)**
- **VAN DE STOLPE, Anja**
  **5656 AE Eindhoven (NL)**
- **VAN OOIJEN, Hendrik Jan**
  **5656 AE Eindhoven (NL)**

(74) Representative: **Philips Intellectual Property & Standards**
**High Tech Campus 5**
**5656 AE Eindhoven (NL)**

# (54) ASSESSMENT OF PR CELLULAR SIGNALING PATHWAY ACTIVITY USING MATHEMATICAL MODELLING OF TARGET GENE EXPRESSION

(57) The present invention relates to a computer-implemented method for inferring activity of a PR cellular signaling pathway in a subject based on expression levels of three or more target genes of the PR cellular signaling pathway measured in a sample of the subject. The present invention further relates to an apparatus, to a non-transitory storage medium, and to a computer program for inferring activity of a PR cellular signaling pathway in a subject. The present invention further relates to a kit for measuring expression levels of three or more target genes of the PR cellular signaling pathway in a sample of a subject, to a kit for inferring activity of a PR cellular signaling pathway in a subject, and to the use of such kits in performing the method.

EP 3 822 368 A1

SEQUENCE LISTING

<110> Koninklijke Philips N.V.

<120> Assessment of PR cellular signaling pathway activity using mathematical modelling of target gene expression

<130> 2018PF00756

<160> 38

<170> PatentIn version 3.5

<210> 1
<211> 4206
<212> DNA
<213> Homo sapiens

<400> 1
agtgcccgcc actcccactg agattgagag acgcggcaag gaggcagcct gtggaggaac 60

tgggtaggat ttaggaacgc accgtgcaca tgcttggtgg tcttgttaag tggaaactgc 120

tgctttagag tttgtttgga aggtccgggt gactcatccc aacatttaca tccttaattg 180

ttaaagcgct gcctccgagc gcacgcatcc tgagatcctg agcctttggt taagaccgag 240

ctctattaag ctgaaaagat aaaaactctc cagatgtctt ccagtaatgt cgaagttttt 300

atcccagtgt cacaaggaaa caccaatggc ttccccgcga cagcttccaa tgacctgaag 360

gcatttactg aaggagctgt gttaagtttt cataacatct gctatcgagt aaaactgaag 420

agtggctttc taccttgtcg aaaaccagtt gagaaagaaa tattatcgaa tatcaatggg 480

atcatgaaac ctggtctcaa cgccatcctg ggacccacag gtggaggcaa atcttcgtta 540

ttagatgtct tagctgcaag gaaagatcca agtggattat ctggagatgt tctgataaat 600

ggagcaccgc gacctgccaa tttcaaatgt aattcaggtt acgtggtaca agatgatgtt 660

gtgatgggca ctctgacggt gagagaaaac ttacagttct cagcagctct tcggcttgca 720

acaactatga cgaatcatga aaaaaacgaa cggattaaca gggtcattca agagttaggt 780

ctggataaag tggcagactc caaggttgga actcagttta tccgtggtgt gtctggagga 840

gaaagaaaaa ggactagtat aggaatggag cttatcactg atccttccat cttgttcttg 900

gatgagccta caactggctt agactcaagc acagcaaatg ctgtcctttt gctcctgaaa 960

aggatgtcta agcagggacg aacaatcatc ttctccattc atcagcctcg atattccatc 1020

ttcaagttgt ttgatagcct caccttattg gcctcaggaa gacttatgtt ccacgggcct 1080

gctcaggagg ccttgggata ctttgaatca gctggttatc actgtgaggc ctataataac 1140

cctgcagact tcttcttgga catcattaat ggagattcca ctgctgtggc attaaacaga 1200

gaagaagact ttaaagccac agagatcata gagccttcca agcaggataa gccactcata 1260

gaaaaattag cggagattta tgtcaactcc tccttctaca aagagacaaa agctgaatta 1320

catcaacttt ccgggggtga gaagaagaag aagatcacag tcttcaagga gatcagctac 1380

accacctcct tctgtcatca actcagatgg gtttccaagc gttcattcaa aaacttgctg 1440

ggtaatcccc aggcctctat agctcagatc attgtcacag tcgtactggg actggttata 1500

ggtgccattt actttgggct aaaaaatgat tctactggaa tccagaacag agctggggtt 1560

ctcttcttcc tgacgaccaa ccagtgtttc agcagtgttt cagccgtgga actctttgtg 1620

gtagagaaga agctcttcat acatgaatac atcagcggat actacagagt gtcatcttat 1680

ttccttggaa aactgttatc tgatttatta cccatgagga tgttaccaag tattatattt 1740

acctgtatag tgtacttcat gttaggattg aagccaaagg cagatgcctt cttcgttatg 1800

atgtttaccc ttatgatggt ggcttattca gccagttcca tggcactggc catagcagca 1860

ggtcagagtg tggtttctgt agcaacactt ctcatgacca tctgttttgt gtttatgatg 1920

atttttttcag gtctgttggt caatctcaca accattgcat cttggctgtc atggcttcag 1980

tacttcagca ttccacgata tggatttacg gctttgcagc ataatgaatt tttgggacaa 2040

aacttctgcc caggactcaa tgcaacagga aacaatcctt gtaactatgc aacatgtact 2100

ggcgaagaat atttggtaaa gcagggcatc gatctctcac cctggggctt gtggaagaat 2160

cacgtggcct tggcttgtat gattgttatt ttcctcacaa ttgcctacct gaaattgtta 2220

tttcttaaaa aatattctta aatttcccct taattcagta tgatttatcc tcacataaaa 2280

aagaagcact ttgattgaag tattcaatca agtttttttg ttgttttctg ttcccttgcc 2340

atcacactgt tgcacagcag caattgtttt aaagagatac atttttagaa atcacaacaa 2400

actgaattaa acatgaaaga acccaagaca tcatgtatcg catattagtt aatctcctca 2460

gacagtaacc atggggaaga aatctggtct aatttattaa tctaaaaaag gagaattgaa 2520

ttctggaaac tcctgacaag ttattactgt ctctggcatt tgtttcctca tctttaaaat 2580

gaataggtag gttagtagcc cttcagtctt aatactttat gatgctatgg tttgccatta 2640

tttaataaat gacaaatgta ttaatgctat actggaaatg taaaattgaa aatatgttgg 2700

aaaaaagatt ctgtcttata gggtaaaaaa agccaccgtg atagaaaaaa aatctttttg 2760

ataagcacat taaagttaat agaacttact gatattcctg tctagtggta taatatctca 2820

ggaatcttgg ctgagggttt ggaactgtgg gtagagtaga gggccaggag tccagtaata 2880

gaattcttgc accatttctg gaacattcta gctctgggag gtcacgtaac cttcttgggg 2940

tagttcagtg gtttagtggt ttataatcca ggtgtgcgtc agaatcatct gaggaacttt 3000

gctaaaatac aaaaatctgg cctaagtagc tccagatcta ccttcataaa ggaatctgac 3060

cactcctgga tttggtaatt tccaagttct gaaaatttta cttaggattt aataactatt 3120

aacatctgtc cctacatagg ttttctttcc tacttatata ccttatgttc tcttcattct 3180

aaccttcatc agtaataggg aaatgtttta atttatttt tttagttgaa gggtaatgta 3240

ccaaaaaata tagttcagtg aattaaaatg aacacacatg tgcaaccatc aattcaggtc 3300

aagaaataga agattgtagc acacaaaagc ctactcagcc attctcccag tcactacttc 3360

cttccttacc cctgggttat ttttgaaatg acacttgatg tatttccctc tgttgctgtt 3420

atgagaacat tgctacagcc aagtgttgtg tttctgtgtg cataggttga tacttaatta 3480

tctccccact ttttaataaa cttttaattt ggaaataatt ttagattgac agaaaagttg 3540

caaagatagt gaggaaagtt cctgtctact ctttgctcag cttcccttaa tgttaacatt 3600

ttatatagca agatgcattt gtcaaagcta acaagttaac attggtacaa tcactgttaa 3660

ttaaactgca cacaatattc agatttcacc acttttccac taatattctt tcattgttct 3720

aggattcaat tcaggagacc acatttcatc tagccctctt ttttaaaagt aaatactttt 3780

cagcacttac aggagttaac tgagctgggg catcatggtg tatagacgcc ctgacactgg 3840

tcatcttgga attcatttag tttgtcagtg ggtgccctga cattctgtca caacatcaat 3900

ttgggaacat ggcattatat ttttatcttt gaactttttt ctttttggat gacatttgat 3960

taatgcgtca tcttggaaca cattatcttt tttcttggtt atgtgatcag gaagattaat 4020

cagtttttcc tgttcttggt ataattcctg cttttcacat acctgtccct tacagttctc 4080

tatatatacc cttcccttat tacacagaga gaaatatcta tctatacttt ttacacaaaa 4140

tatacttcaa aagaaacaaa acagccacaa ttattaactt tttaaataaa tgagaattta 4200

attata 4206

<210> 2
<211> 3632
<212> DNA
<213> Homo sapiens

<400> 2
actctgggag cacagagagc tcaggtagcc tgcctagatg gcggcgcgca ccctgggccg 60

cggcgtcggg aggctgctgg gcagcctgcg agggctctcg gggcagcccg cgcggccgcc 120

gtgcggggtg agcgcgccgc gcagggcggc ctcgggaccc tcgggcagcg ctcccgcagt 180

tgcagcagca gcagcacagc caggctcgta tcccgcgctg agtgcacagg cagcccggga 240

gccggccgcc ttctgggggc ctctggcgcg ggacactctc gtgtgggaca ccccctacca 300

caccgtctgg gactgcgact tcagcactgg caagatcggc tggttcctgg gaggccagtt 360

aaatgtctct gtcaactgct tggaccagca tgttcggaag tcccccgaga gcgttgcttt 420

gatctgggag cgcgatgagc ctggaacgga agtgaggatc acctacaggg aactactgga 480

gaccacgtgc cgcctggcca acacgctgaa gaggcatgga gtccaccgtg gggaccgtgt 540

tgccatctac atgcccgtgt ccccattggc tgtggcagca atgctggcct gtgccaggat 600

cggagctgtc cacacagtca tctttgctgg cttcagtgca gagtccttgg ctgggaggat 660

caatgatgcc aagtgcaagg tggttatcac cttcaaccaa ggactccggg gtgggcgcgt 720
ggtggagctg aagaaaatag tggatgaggc tgtgaagcac tgccccaccg tgcagcatgt 780
cctggtggct cacaggacag acaacaaggt ccacatgggg gatctggacg tcccgctgga 840
gcaggaaatg gccaaggagg accctgtttg cgccccagag agcatgggca gtgaggacat 900
gctcttcatg ctgtacacct cagggagcac cggaatgccc aagggcatcg tccataccca 960
ggcaggctac ctgctctatg ccgccctgac tcacaagctt gtgtttgacc accagccagg 1020
tgacatcttt ggctgtgtgg ccgacatcgg ttggattaca ggacacagct acgtggtgta 1080
tgggcctctc tgcaatggtg ccaccagcgt cctttttgag agcaccccag tttatcccaa 1140
tgctggtcgg tactgggaga cagtagagag gttgaagatc aatcagttct atggcgcccc 1200
aacggctgtc cggctgttgc tgaaatacgg tgatgcctgg gtgaagaagt atgatcgctc 1260
ctccctgcgg accctggggt cagtgggaga gcccatcaac tgtgaggcct gggagtggct 1320
tcacagggtg gtgggggaca gcaggtgcac gctggtggac acctggtggc agacagaaac 1380
aggtggcatc tgcatcgcac cacggccctc ggaagaaggg gcggaaatcc tccctgccat 1440
ggcgatgagg cccttctttg gcatcgtccc cgtcctcatg gatgagaagg gcagcgtcgt 1500
ggagggcagc aacgtctccg gggccctgtg catctcccag gcctggccgg gcatggccag 1560
gaccatctat ggcgaccacc agcgatttgt ggacgcctac ttcaaggcct acccaggcta 1620
ttacttcact ggagacgggg cttaccgaac tgagggcggc tattaccaga tcacagggcg 1680
gatggatgat gtcatcaaca tcagtggcca ccggctgggg accgcagaga ttgaggacgc 1740
catcgccgac caccctgcag taccagaaag tgctgtcatt ggctaccccc acgacatcaa 1800
aggagaagct gcctttgcct tcattgtggt gaaagatagt gcgggtgact cagatgtggt 1860
ggtgcaggag ctcaagtcca tggtggccac caagatcgcc aaatatgctg tgcctgatga 1920
gatcctggtg gtgaaacgtc ttccaaaaac caggtctggg aaggtcatgc ggcggctcct 1980
gaggaagatc atcactagtg aggcccagga gctgggagac actaccacct tggaggaccc 2040
cagcatcatc gcagagatcc tgagtgtcta ccagaagtgc aaggacaagc aggctgctgc 2100
taagtgagct ggcaccttgt ggggctcttg ggatgggcgg gcacccaagc cctggcttgt 2160
ccttcccaga aggtacccct gaggttggcg tcttcctacg tcccagaagc agcccccacc 2220
ccacacatga cccacaccgc cctcacgtga agctgggctg agagcccttt ctcccatcca 2280
ttggaggtcc caggagtgtc acccatggag aggctatgcg acatggctag ggctggttct 2340
gccatctgag tttggtttcc tggaatgaaa aggcattgcc atctccattc ctctgccctc 2400
ttgagccagc acaggaaggt gaggccctgg gatagcgcgc ctgctcagat aacacagagc 2460
tagttagcta gtagcaaccg tgttttctcc agatctgtct agatacaaag gtcagaaatc 2520

ttatttttat acttttatat tgtggaagaa cagcatgcaa cactcacatg tagtgtgtgg 2580

atttacttga acatgttctt tttaacatgt agttatgaaa atctcctttt ttgcctctac 2640

tggtgaggaa acatgaggat cagaggccac atttttaatt attgttagtg tatttggaag 2700

tctgaattgg agatgtttgt acctctgtct aaacagttcc cttgagaact tccaagcctc 2760

cggcatcttt tcctggtgag tgtttctcct gtgcttggtt gtgtataatg gagctaactc 2820

ctaagcggtg gggtgaatgt ggccgcctta gttctgaagc tactccagtt atgttctgtt 2880

tcttcaagct gtgatccaga aagatttttg tgcccccaga tgcctcttga taggagaggc 2940

aacatactcc aaatagttgg gttcttcagg gaagctatta gaaactcagg tgacttgtta 3000

gagcactaac ttggtcagag ccaaatcctg gcaaacgctg cctgaccttc actctgtggt 3060

tggggcggtg agaaccactg aggtccaatg atgagacttg gaggtctgga tccagtctct 3120

ctttgtttta atgtgactta ggtgctgtca acattagcaa gataatggaa atcacgacgc 3180

cagtgggtgc ttacctccct gctaggcatg caggggctgg cggttggcag gggaaggagg 3240

cccagtgagc cgggtccctt aggggaggga gagtttgtcc tctttgcccc acagtctacc 3300

cttcagggcc ttgtggcagt gccagtgttc ggggggtgtc tgggccactg agtacccact 3360

cggtcgtggt tgtgctggcc tcttgggtga gtgaacctgt gaagcccagg aggtggtgtt 3420

ggctgcaggg tacacaaata ctgagtggtg gtcttttgtt acaggcttag caacaaagct 3480

gtgccctggg catggggggc tgtagtgtag ctacagttgt gcgtttgtga aatggcttag 3540

ctttccatgt tgctgagagg aacctggaca tggtcccggg catctgaatg atctgtaggg 3600

gagggagttc aaataaagct ttattttgtt ca 3632

<210> 3
<211> 6845
<212> DNA
<213> Homo sapiens

<400> 3
agtccgcctg ctactcggtc ccggcgctgg gctgagggga ggggttgtct taaaagtctc 60

tccttccccc tgtaggggcg gccggcgagt cccagtgaga gcggagggtg ccagaggtag 120

ggggccgaga aacaaagttc ccggggcttc ctccggggcc gcggtcgggg ctgcgcgttt 180

gaccgccccc ctcctcgcga aggcaatggc ttccaaactc ctgcgcgcgg tcatcctcgg 240

gccgcccggc tcgggcaagg gcaccgtgtg ccagaggatc gcccagaact ttggtctcca 300

gcatctctcc agcggccact tcttgcggga gaacatcaag gccagcaccg aagttggtga 360

gatggcaaag cagtatatag agaaaagtct tttggttcca gaccatgtga tcacacgcct 420

aatgatgtcc gagttggaga acaggcgtgg ccagcactgg ctccttgatg gttttcctag 480

gacattagga caagccgaag ccctggacaa aatctgtgaa gtggatctag tgatcagttt 540

gaatattcca tttgaaacac ttaaagatcg tctcagccgc cgttggattc accctcctag 600

cggaagggta tataacctgg acttcaatcc acctcatgta catggtattg atgacgtcac 660

tggtgaaccg ttagtccagc aggaggatga taaacccgaa gcagttgctg ccaggctaag 720

acagtacaaa gacgtggcaa agccagtcat tgaattatac aagagccgag gagtgctcca 780

ccaattttcc ggaacggaga cgaacaaaat ctggccctac gtttacacac ttttctcaaa 840

caagatcaca cctattcagt ccaaagaagc atattgaccc tgcccaatgg aagaaccagg 900

aagatgtggt cattcattca atagtgtgtg tagtattggt gctgtgtcca aattagaagc 960

tagctgaggt agcttgcagc atcttttcta gttgaaatgg tgaactgata ggaaaacaaa 1020

tgagtagaaa gagttcatga agaggccctc ctctgccttt caaaaggctg gtcacctaca 1080

catgtttaag gtgtctctgc acatgtctca agcccatcac aagaaagcaa gtacagtgtg 1140

gatttcaaat ggtgtgtaac ttcagctcca gctggttttt gacagctgtt gctgtggtaa 1200

tatttttgac atgtgatggt gatagtctct ggttctcccc atccccacaa aggctgttga 1260

accacagcac caggaagcct gagaatgaat cctgagggct ctagcccagg ctttgtccca 1320

ggctttctgg tgtgtgccct cctggtaaca gtgaaattga agctacttac tcatagtggt 1380

tgtttctctg gtcttgagtg actgtgtcca cagttcattt ttttccggta ggaataactc 1440

cttttctaca tccacgctcc atagagtctc tccttttcag acatcctggg atgaaagaat 1500

ttggcttttt tttttctttt tttttttgga catctgtttt cactcttagg cttttaaaca 1560

atagttattg cttttatccc tctcagattc taataactga gagcgatggg gctatattga 1620

atctctgtat gcactgagaa ctgagctatg aagaggatct tattaaactg ctggtctgac 1680

tttatggatt gacactgttc ctttctttta ttgtgaaaaa aaaaaaaaac cctgaaagtc 1740

ttgggaaccc cctaaagtct tttgggaatc ctcaaaaagc atgggaagtt aagtatttag 1800

ctacataaat gttgtaagat catatcttat gtatagaagt aataagacca tttggaatta 1860

ctggactaat tgaatagtta aggtttctat tcgggacaat aaaatgtatt ttgaaagtgc 1920

tgctaactat tgatgctgac agtgtttcac tcctatgagt gacccaaaca tattataaat 1980

atgtggtaaa gggaatggag cctgtggggt tgagcagaat gttgtactag ctgtgcctgg 2040

actgagtata acagctttat gattatgaga aaacaaattc tttatttttt ttttctgttc 2100

caaagattca tcctatgggg tggccataaa gtctagaatt agatactaat attttgtcat 2160

tcattataac atatcaataa accatttgtt aaaagatttg cctggtttcc agacttggtg 2220

gccaccttga ataattcttg ctgtcttctg ggaaggatga tgaaatttat tcctgctgcc 2280

ttaaaaatat gtatcccttc ttcacccatc atgactgtcc ccagtgagtg tcctttacta 2340

ttcttgggag tgactcctgt ctaacttttc atactggcga gaagaaaaga agcctatttt 2400

aacactttag tggtgttgaa acacattact tactttctga agatgtccca gtgaatcctc 2460

tgtcaattca ctgccatatg taatctatat gataaggaat gcatcttcct tctaagtact 2520
gcccaaactc ttgccagctc ctctcccatt gtcccttcat gtgaatattt cttggctacc 2580
ttagtggaaa tatagatcag ttttctcccc atccatcctc tcaaacataa tgagattgtt 2640
tactttttag atttatgcag tgaaaatgcc cagtcaggtc tgaatcgtca gtgcattata 2700
ttgactctga gcactttaga atttagagtt gcaattgaat gccagctgtg gagatggggt 2760
gcatatcaga tatataaata aagctcaggt ttgctaggga accaggtata gagaaaaata 2820
agtctgatat gaggaaaatt gcacaattta gagtagttat gccgtagaga aaatttccac 2880
aaactaggaa atgtagagag ttattctata gaatactcaa aagaggaaag tatgtgattt 2940
ttggaaacag gaaaatcttc aaacttcttt cttcacttcc ctttgtgttt agctgaccct 3000
ccaatgtgat cattgccttt ggagtttggg agaggtacgg gaagtggcct gatccctgct 3060
tccatacttc actcctccat ccatccttcc ctccctcttc ccctccagct aaatggacaa 3120
ttctagccaa cattgagtca ctcaataagt ctcaacagtg ggtgtgtttg ctgagattgt 3180
ccagcggttg agcagtttgg tctcacctcc ctcgctagtt gagaccaaaa agagacaaat 3240
aactttttca tggtctttga aacataatgc ttatttcgtg gtcaatggct ttaaaaaaat 3300
ctgtttcttg ttttcttcaa caaactcact agttttccct taaatgatat tgtaaaaatt 3360
aaagtaatct tgaaaatgtt ttgacaaaag taaaattaaa gggacatctt ttcttgtttt 3420
gttttttttt tttctattgc cacacatgac cgttccttca cctttaagca aagagagtgg 3480
ttcagatggt ttctaagatg ccaacctgac ctcgcattct gtcattctac ccagctctta 3540
attcaatttg cttccattat cctaacaggc ttctttctta cttagaactt ggaaaggctg 3600
ctgtatttaa taccctccaa cactaacgca gacttaagat aggtactgtt tattgaaaac 3660
ctactgagtg aaatgtgcgg ttttaggacc ttcataaaca tctcatttaa tctttctagc 3720
atcctgtgaa acagccatga tttcacgttg ataaacaaag aagacagggg tcccagggat 3780
gtgaagcatc ttgcccaggc ttctgctgct ggtgaccagt gtagccagga ctccagccca 3840
ggttttcctg actcagaaga ctgagctttt tcctggatgt tattaatagc taattgtgtc 3900
caagcaacca agggccttga gtctgcttgg ttctgcttat ggcctcacat caagaaatgg 3960
agctagtcca tgtctgtagt cccaatgctt tgggaagcca tgatgggaag gttgtcggag 4020
gccaaaagtt caagaccagg ctgggcaata tcacaagact ccatctctac ggaaaagtaa 4080
aaaattagcc agtcatggtg gtgtacactt atggtcctag ttactcagga gacttaggca 4140
ggaggattgc ttgatcctag gaattcgagg ctgcagtgag ctatgattgc acctctgcac 4200
ccaagcctgg gcgacacagc gagaccctct ctcttaaaaa aaaaaaatag cagagctcac 4260
caaagtgatg ttcacctttt tatgacattc ctttttctta gcttaagaaa agaaagctgc 4320

tagatgagag tcttagtttt cctgcataag acctccttta tgaatagaat aaaagactgt 4380
caaagtaggc tgggcttggg cccaggctaa tctatgaagg aagcaagctc gtgttcctta 4440
cctatccttt tggtgtccat tggattgtgc cccgaagtgg cctttaccct tgagccgtcc 4500
ccagccatgg tgctcacaca taggcttttg agctccttgg agctatccag atcctgctca 4560
cttttccttc ctgagatcag aacaaatcac cccttactc ccactccaaa caaggccttg 4620
atgataaact aatccttcct aaaatgctgg taggtaaaca agcaatgatg aagcattgaa 4680
cacaggttaa ctcctgactt ttgtaccatt gtctattcca ttacacatta acatgactct 4740
gaatgccaga tccaaacctt tgcccaccat ctgcttgtcg tgcaacagtt gaggcagtaa 4800
ccaggggaga ttcacttcct gtcttgtcct tccccaggga tcacccccct gctgccctct 4860
agcagccaaa ctcagatgag ttccattgtt accctaggtg tgcccatctc tttggtaggg 4920
aaggagaaag gtaagaatag ccatcagtga ggaaggattc ttggagcgag gagccactgt 4980
ggtttttcct gctatttaag atgttgagac cggataactt tagaaagata cctgcacaaa 5040
cccataaata gtgcttttat aaagtttagt tcaccggaac ctgagttcag tatttgacat 5100
tagctttttg tccaaagagt tgaagcctgc tggaggtctt tgctcaaata ataaatacca 5160
catatttcca agtgtgttca ggtataggca ctaggtactg tctgtttact tcatgttagg 5220
cacattacat gcattggcta atcaaatcct catcaattac atatgtaata atctaaactt 5280
gcctccttgt attataaatg gaaataatcc tgtttattta aacgggtttt catgtacctg 5340
tagggattag gaaactcaaa tggccttttt aatacctttc cctagtttga gctccctgtt 5400
ctctttaaca gataaaacaa catatttgct tcagcctgga atctgttttt ggtgctttgg 5460
tgcagagaca ggaaatgggc actcagagtc acactggtag ttgcacactg tatctacaga 5520
gggcgtgtct catctgtact ctgctgggtt acaggatttc agtaggtatt tgtgtccacc 5580
tgagaattct gtttattacc tttcatttga cagtgtcttt cctttctgca gttgattttg 5640
ctagagaggc aattcataag gtgaggtcct gttcatagta tgacttgctt tctcaatatc 5700
tccttcaatt tttagtaact cttggtctat ttggtgtctt taaaaaaaat aacctagtaa 5760
taaagacttc ttttaatgtg gaaatgtggt ctggtagtaa gttatttctt tccacatgta 5820
actgacccaa tctggtttcc aaatgagaag tgtgcaggcc ccagaggttg agaagccata 5880
tttcaactgt gaaaaaaatc tgcttcctgc atctgttgaa atatagttgt tcatacttgc 5940
catcccttat ctttcttgta acaatttgca cagttcttgc cagaataaat gccattatct 6000
gtatgtttca gggagttccc caatttgatc atttttgtgt gtgtgtggtg tgtgtgtgag 6060
agagagagat actgcagtaa aacatttcta aaggatgaaa gctcttgtat ggcatagata 6120
tgaattcctt cctctggtaa taattaggtt attcccagaa gcacagtgtc attctttaaa 6180
taaaagcttt cctgtttaaa gcttttcaaa ggagcagacc accttgaaga ttccccctag 6240

ggttgatatg tgtctaattc attttataaa aattattctt gtcttcattt taaagctttg 6300

gctatatagt cagaaatgtc ctaaataaca aactattttg tatttaattt agggaagact 6360

aaagggaaga aaaatgaaaa ctcagtcttt atgtaagctc caaggatatt agggcttaaa 6420

gggcttttct agttttatga gaatttgtac tactgatttt tatatattcc tgtttttgag 6480

atgaacagat ctctggggaa attgttgagt tacaatggca tttcactgtg atccctctca 6540

agctcagatc agttctataa cccaatgaca acctgtctct ttggtttact gtcctgtgaa 6600

atgtcagctc aagtttccca gaagtcgtgt gtttatgatg agtcagagtg cttttcctcg 6660

gtgggacagt tgctggccct cttaattttg gtgtatgtgc ttccaagtat ctaaacctcc 6720

agtctgatct gtatatgcta tcctaactgt taattgtatt attgattatg ttgattatct 6780

tgcttgaagg ttcatacttt tcaatttgat agaaataaag ttttttttctg cttatagcta 6840

gcgaa 6845

<210> 4
<211> 1556
<212> DNA
<213> Homo sapiens

<400> 4
gtcgctgcgc ggctggccgg tgaggccgcg gcatggggcg agtgcagctc ttcgagatca 60

gcctgagcca cggccgcgtc gtctacagcc ccggggagcc gttggctggg accgtgcgcg 120

tgcgcctggg ggcaccgctg ccgttccgag ccatccgggt gacctgcata ggttcctgcg 180

gggtctccaa caaggctaat gacacagcgt gggtagtgga ggagggttac ttcaacagtt 240

ccctgtcgct ggcagacaag gggagcctgc ccgctggaga gcacagcttc cccttccagt 300

tcctgcttcc tgccactgca cccacgtcct ttgagggtcc tttcgggaag atcgtgcacc 360

aggtgagggc cgccatccac acgccacggt tttccaagga tcacaagtgc agcctcgtgt 420

tctatatctt gagccccttg aacctgaaca gcatcccaga cattgagcaa cccaacgtgg 480

cctctgccac caagaagttc tcctacaagc tggtgaagac gggcagcgtg gtcctcacag 540

ccagcactga tctccgcggc tatgtggtgg ggcaggcact gcagctgcat gccgacgttg 600

agaaccagtc aggcaaggac accagccctg tggtggccag tctgctgcag aaagtgtcct 660

ataaggccaa gcgctggatc cacgacgtac ggaccattgc ggaggtggag ggtgcgggcg 720

tcaaggcctg gcggcgggcg cagtggcacg agcagatcct ggtgcctgcc ttgccccagt 780

cggccctgcc gggctgcagc ctcatccaca tcgactacta cttacaggtc tctctgaagg 840

cgccggaagc tactgtgacc ctcccggtct tcattggcaa tattgctgtg aaccatgccc 900

cagtgagccc ccggccaggc ctggggctgc ctcctggggc cccacccctg gtggtgcctt 960

ccgcaccacc ccaggaggag gctgaggctg aggctgcggc tggcggcccc cacttcttgg 1020

```
accccgtctt cctctccacc aagagccatt cgcagcggca gcccctgctg gccaccttga      1080

gttctgtgcc tggtgcgccg gagccctgcc ctcaggatgg cagccctgcc tcacacccgc      1140

tgcaccctcc cttgtgcatt tcaacaggtg ccactgtccc ctactttgca gagggctccg      1200

gggggccagt gcccactacc agcaccttga ttcttcctcc agagtacagt tcttggggct      1260

accccctatga ggccccaccg tcttatgagc agagctgcgg cggcgtggaa cccagcctga      1320

cccctgagag ctgaccccgt gctgccttct ccaggcaggc ctggcctctg ccctgggact      1380

ggggcgccca gggcctcgtg ccttctctct tggcctagcc tggcccactc aggacctgcc      1440

cagcctctgc cagctcctct ggcatccgcc ctcttctccc tggggctggg gtgggggtgg      1500

cagggagctg ggacctggag agacaactcc tgtaaataaa acactttatt tgtaga          1556
```

<210> 5
<211> 1476
<212> DNA
<213> Homo sapiens

<400> 5

```
gccacccacc ctccggaccg cggcagctgc tgacccgcca tcgccatggc ccgcgggaaa        60

gccaaggagg agggcagctg gaagaaattc atctggaact cagagaagaa ggagtttctg       120

ggcaggaccg gtggcagttg gtttaagatc cttctattct acgtaatatt ttatggctgc       180

ctggctggca tcttcatcgg aaccatccaa gtgatgctgc tcaccatcag tgaatttaag       240

cccacatatc aggaccgagt ggccccgcca ggattaacac agattcctca gatccagaag       300

actgaaattt cctttcgtcc taatgatccc aagagctatg aggcatatgt actgaacata       360

gttaggttcc tggaaaagta caaagattca gcccagaggg atgacatgat tttgaagat       420

tgtggcgatg tgcccagtga accgaaagaa cgaggagact ttaatcatga acgaggagag       480

cgaaaggtct gcagattcaa gcttgaatgg ctgggaaatt gctctggatt aaatgatgaa       540

acttatggct acaaagaggg caaaccgtgc attattataa agctcaaccg agttctaggc       600

ttcaaaccta agcctcccaa gaatgagtcc ttggagactt acccagtgat gaagtataac       660

ccaaatgtcc ttcccgttca gtgcactggc aagcgagatg aagataagga taaagttgga       720

aatgtggagt attttggact gggcaactcc cctggttttc ctctgcagta ttatccgtac       780

tatggcaaac tcctgcagcc caaatacctg cagcccctgc tggccgtaca gttcaccaat       840

cttaccatgg acactgaaat tcgcatagag tgtaaggcgt acggtgagaa cattgggtac       900

agtgagaaag accgttttca gggacgtttt gatgtaaaaa ttaaatttta agtgacacta       960

cagaaaaaca caaaaaggtg atgggttgtg ttatgcttgt attgaatgct gtcttgacat      1020

ctcttgcctt gtcctccggt atgttctaaa gctgtgtctg agatctggat ctgcccatca      1080

ctttggctag tgacagggct aattaatttg ctttatacat tttcttttac tttccttttt      1140
```

tcctttctgg aggcatcaca tgctggtgct gtgtctttat gaatgtttta accattttca 1200

tggtggaaga attttatatt tatgcagttg tacaatttta ttttttctg caagaaaaag 1260

tgtaatgtat gaaataaacc aaagtcactt gtttgaaaat aaatctttat tttgaacttt 1320

ataaaaagca atgcagtacc ccatagactg gtgttaaatg ttgtctacag tgcaaaatcc 1380

atgttctaac atatgtaata attgccagga gtacagtgct cttgttgatc ttgtattcag 1440

tcaggttaaa acaacggtca ataaaagaat gaacac 1476

<210> 6
<211> 2574
<212> DNA
<213> Homo sapiens

<400> 6
ctcgatccgg gcgatggagg aggaagcaag cgaggggct ggttcctgag cttcgcaatt 60

cctgtgtcgc cttctgggct cccagcctgc cgggtcgcat gatccctccg gccggagctg 120

gtttttttgc cagccaccgc gaggccggct gagttaccgg catccccgca gccacctcct 180

ctcccgacct gtgatacaaa agatcttccg ggggctgcac ctgcctgcct ttgcctaagg 240

cggatttgaa tctctttctc tcccttcaga atcttatctt ggctttggat cttagaagag 300

aatcactaac cagagacgag actcagtgag tgagcaggtg ttttggacaa tggactggtt 360

gagcccatcc ctattataaa aatgtctcag agcaaccggg agctggtggt tgactttctc 420

tcctacaagc tttcccagaa aggatacagc tggagtcagt ttagtgatgt ggaagagaac 480

aggactgagg ccccagaagg gactgaatcg gagatggaga cccccagtgc catcaatggc 540

aacccatcct ggcacctggc agacagcccc gcggtgaatg gagccactgg ccacagcagc 600

agtttggatg cccgggaggt gatccccatg gcagcagtaa agcaagcgct gagggaggca 660

ggcgacgagt ttgaactgcg gtaccggcgg gcattcagtg acctgacatc ccagctccac 720

atcaccccag ggacagcata tcagagcttt gaacaggtag tgaatgaact cttccgggat 780

ggggtaaact ggggtcgcat tgtggccttt ttctccttcg gcggggcact gtgcgtggaa 840

agcgtagaca aggagatgca ggtattggtg agtcggatcg cagcttggat ggccacttac 900

ctgaatgacc acctagagcc ttggatccag gagaacggcg gctgggatac ttttgtggaa 960

ctctatggga acaatgcagc agccgagagc cgaaagggcc aggaacgctt caaccgctgg 1020

ttcctgacgg gcatgactgt ggccggcgtg gttctgctgg gctcactctt cagtcggaaa 1080

tgaccagaca ctgaccatcc actctaccct cccacccct tctctgctcc accacatcct 1140

ccgtccagcc gccattgcca ccaggagaac cactacatgc agcccatgcc cacctgccca 1200

tcacagggtt gggcccagat ctggtccctt gcagctagtt ttctagaatt tatcacactt 1260

ctgtgagacc cccacacctc agttcccttg gcctcagaat tcacaaaatt tccacaaaat 1320

ctgtccaaag gaggctggca ggtatggaag ggtttgtggc tgggggcagg agggccctac 1380

ctgattggtg caacccttac cccttagcct ccctgaaaat gtttttctgc cagggagctt 1440

gaaagttttc agaacctctt ccccagaaag gagactagat tgcctttgtt ttgatgtttg 1500

tggcctcaga attgatcatt ttcccccac tctccccaca ctaacctggg ttccctttcc 1560

ttccatccct accccctaag agccatttag gggccacttt tgactaggga ttcaggctgc 1620

ttgggataaa gatgcaagga ccaggactcc ctcctcacct ctggactggc tagagtcctc 1680

actcccagtc caaatgtcct ccagaagcct ctggctagag gccagcccca cccaggaggg 1740

agggggctat agctacagga agcaccccat gccaaagcta gggtggccct tgcagttcag 1800

caccacccta gtcccttccc ctccctggct cccatgacca tactgaggga ccaactgggc 1860

ccaagacaga tgccccagag ctgtttatgg cctcagctgc ctcacttcct acaagagcag 1920

cctgtggcat ctttgccttg ggctgctcct catggtgggt tcaggggact cagccctgag 1980

gtgaaaggga gctatcagga acagctatgg gagccccagg gtcttcccta cctcaggcag 2040

gaagggcagg aaggagagcc tgctgcatgg ggtggggtag ggctgactag aagggccagt 2100

cctgcctggc caggcagatc tgtgccccat gcctgtccag cctgggcagc caggctgcca 2160

aggccagagt ggcctggcca ggagctcttc aggcctccct ctctcttctg ctccaccctt 2220

ggcctgtctc atccccaggg gtcccagcca ccccgggctc tctgctgtac atatttgaga 2280

ctagttttta ttccttgtga agatgatata ctatttttgt taagcgtgtc tgtatttatg 2340

tgtgaggagc tgctggcttg cagtgcgcgt gcacgtggag agctggtgcc cggagattgg 2400

acggcctgat gctccctccc ctgccctggt ccagggaagc tggccgaggg tcctggctcc 2460

tgaggggcat ctgcccctcc cccaaccccc accccacact tgttccagct ctttgaaata 2520

gtctgtgtga aggtgaaagt gcagttcagt aataaactgt gtttactcag tgaa 2574

<210> 7
<211> 3306
<212> DNA
<213> Homo sapiens

<400> 7
agaactgggg ttcttagaag tggtgatgca agaagtttct aggaaaggcc ggacaccagg 60

ttttgagcaa aattttggac tgtgaagcaa ggcattggtg aagacaaaat ggcctcgccg 120

gctgacagct gtatccagtt cacccgccat gccagtgatg ttcttctcaa ccttaatcgt 180

ctccggagtc gagacatctt gactgatgtt gtcattgttg tgagccgtga gcagtttaga 240

gcccataaaa cggtcctcat ggcctgcagt ggcctgttct atagcatctt tacagaccag 300

ttgaaatgca accttagtgt gatcaatcta gatcctgaga tcaaccctga gggattctgc 360

atcctcctgg acttcatgta cacatctcgg ctcaatttgc gggagggcaa catcatggct 420

gtgatggcca cggctatgta cctgcagatg gagcatgttg tggacacttg ccggaagttt 480
attaaggcca gtgaagcaga gatggtttct gccatcaagc ctcctcgtga agagttcctc 540
aacagccgga tgctgatgcc ccaagacatc atggcctatc ggggtcgtga ggtggtggag 600
aacaacctgc cactgaggag cgcccctggg tgtgagagca gagcctttgc cccagcctg 660
tacagtggcc tgtccacacc gccagcctct tattccatgt acagccacct ccctgtcagc 720
agcctcctct tctccgatga ggagtttcgg gatgtccgga tgcctgtggc caacccccttc 780
cccaaggagc gggcactccc atgtgatagt gccaggccag tccctggtga gtacagccgg 840
ccgactttgg aggtgtcccc caatgtgtgc cacagcaata tctattcacc caaggaaaca 900
atcccagaag aggcacgaag tgatatgcac tacagtgtgg ctgagggcct caaacctgct 960
gcccccctcag cccgaaatgc cccctacttc ccttgtgaca aggccagcaa agaagaagag 1020
agaccctcct cggaagatga gattgccctg catttcgagc cccccaatgc accccctgaac 1080
cggaagggtc tggttagtcc acagagcccc cagaaatctg actgccagcc caactcgccc 1140
acagagtcct gcagcagtaa gaatgcctgc atcctccagg cttctggctc ccctccagcc 1200
aagagcccca ctgaccccaa agcctgcaac tggaagaaat acaagttcat cgtgctcaac 1260
agcctcaacc agaatgccaa accagaggggg cctgagcagg ctgagctggg ccgcctttcc 1320
ccacgagcct acacggcccc acctgcctgc cagccaccca tggagcctga gaaccttgac 1380
ctccagtccc caaccaagct gagtgccagc ggggaggact ccaccatccc acaagccagc 1440
cggctcaata acatcgttaa caggtccatg acgggctctc cccgcagcag cagcgagagc 1500
cactcaccac tctacatgca cccccccgaag tgcacgtcct gcggctctca gtccccacag 1560
catgcagaga tgtgcctcca caccgctggc cccacgttcc ctgaggagat gggagagacc 1620
cagtctgagt actcagattc tagctgtgag aacggggcct tcttctgcaa tgagtgtgac 1680
tgccgcttct ctgaggaggc ctcactcaag aggcacacgc tgcagaccca cagtgacaaa 1740
ccctacaagt gtgaccgctg ccaggcctcc ttccgctaca agggcaacct cgccagccac 1800
aagaccgtcc ataccggtga gaaaccctat cgttgcaaca tctgtggggc ccagttcaac 1860
cggccagcca acctgaaaac ccacactcga attcactctg gagagaagcc ctacaaatgc 1920
gaaacctgcg gagccagatt tgtacaggtg gcccacctcc gtgcccatgt gcttatccac 1980
actggtgaga agccctatcc ctgtgaaatc tgtggcaccc gtttccggca ccttcagact 2040
ctgaagagcc acctgcgaat ccacacagga gagaaacctt accattgtga gaagtgtaac 2100
ctgcatttcc gtcacaaaag ccagctgcga cttcacttgc gccagaagca tggcgccatc 2160
accaacacca aggtgcaata ccgcgtgtca gccactgacc tgcctccgga gctccccaaa 2220
gcctgctgaa gcatggagtg ttgatgcttt cgtctccagc cccttctcag aatctaccca 2280

aaggatactg taacacttta caatgttcat cccatgatgt agtgcctctt tcatccacta 2340

gtgcaaatca tagctggggg ttgggggtgg tgggggtcgg ggcctggggg actgggagcc 2400

gcagcagctc cccctccccc actgccataa aacattaaga aaatcatatt gcttcttctc 2460

ctatgtgtaa ggtgaaccat gtcagcaaaa agcaaaatca ttttatatgt caaagcaggg 2520

gagtatgcaa aagttctgac ttgactttag tctgcaaaat gaggaatgta tatgttttgt 2580

gggaacagat gtttcttttg tatgtaaatg tgcattcttt taaaagacaa gacttcagta 2640

tgttgtcaaa gagagggctt taattttttt aaccaaaggt gaaggaatat atggcagagt 2700

tgtaaatata taaatatata tatatataaa ataaatatat ataaacctaa aaaagatata 2760

ttaaaaatat aaaactgcgt taaaggctcg attttgtatc tgcaggcaga cacggatctg 2820

agaatcttta ttgagaaaga gcacttaaga gaatatttta agtattgcat ctgtataagt 2880

aagaaaatat tttgtctaaa atgcctcagt gtatttgtat ttttttgcaa gtgaaggttt 2940

acaatttaca aagtgtgtat taaaaaaaac aaaaagaaca aaaaaatctg cagaaggaaa 3000

aatgtgtaat tttgttctag ttttcagttt gtatataccc gtacaacgtg tcctcacggt 3060

gcctttttttc acggaagttt tcaatgatgg gcgagcgtgc accatccctt tttgaagtgt 3120

aggcagacac agggacttga agttgttact aactaaactc tctttgggaa tgtttgtctc 3180

atcccattct gcgtcatgct tgtgttataa ctactccgga gacagggttt ggctgtgtct 3240

aaactgcatt accgcgttgt aaaatatagc tgtacaaata taagaataaa atgttgaaaa 3300

gtcaaa 3306

<210> 8
<211> 6877
<212> DNA
<213> Homo sapiens

<400> 8
ggcacggaaa aggccaggcg acaggtgtcg cttgaaaaga ctgggcttgt ccttgctggt 60

gcatgcgtcg tcggcctctg ggcagcaggt ttacaaagga ggaaaacgac ttcttctaga 120

tttttttttc agtttcttct ataaatcaaa acatctcaaa atggagacct aaaatcctta 180

aagggactta gtctaatctc gggaggtagt tttgtgcatg ggtaaacaaa ttaagtatta 240

actggtgttt tactatccaa agaatgctaa ttttataaac atgatcgagt tatataaggt 300

ataccataat gagtttgatt ttgaatttga tttgtggaaa taaaggaaaa gtgattctag 360

ctggggcata ttgttaaagc atttttttca gagttggcca ggcagtctcc tactggcaca 420

ttctcccatt atgtagaata gaaatagtac ctgtgtttgg gaaagatttt aaaatgagtg 480

acagttattt ggaacaaaga gctaataatc aatccactgc aaattaaaga aacatgcaga 540

tgaaagtttt gacacattaa aatacttcta cagtgacaaa gaaaaatcaa gaacaaagct 600

ttttgatatg tgcaacaaat ttagaggaag taaaaagata aatgtgatga ttggtcaaga 660
aattatccag ttatttacaa ggccactgat attttaaacg tccaaaagtt tgtttaaatg 720
ggctgttacc gctgagaatg atgaggatga gaatgatggt tgaaggttac attttaggaa 780
atgaagaaac ttagaaaatt aatataaaga cagtgatgaa tacaaagaag atttttataa 840
caatgtgtaa aatttttggc cagggaaagg aatattgaag ttagatacaa ttacttacct 900
ttgagggaaa taattgttgg taatgagatg tgatgtttct cctgccacct ggaaacaaag 960
cattgaagtc tgcagttgaa aagcccaacg tctgtgagat ccaggaaacc atgcttgcaa 1020
accactggta aaaaaaaaaa aaaaaaaaaa aaaaagccac agtgacttgc ttattggtca 1080
ttgctagtat tatcgactca gaacctcttt actaatggct agtaaatcat aattgagaaa 1140
ttctgaattt tgacaaggtc tctgctgttg aaatggtaaa tttattattt tttttgtcat 1200
gataaattct ggttcaaggt atgctatcca tgaaataatt tctgaccaaa actaaattga 1260
tgcaatttga ttatccatct tagcctacag atggcatctg gtaacttttg actgttttaa 1320
aaaataaatc cactatcaga gtagatttga tgttggcttc agaaacattt agaaaaacaa 1380
aagttcaaaa atgttttcag gaggtgataa gttgaataac tctacaatgt tagttctttg 1440
agggggacaa aaaatttaaa atctttgaaa ggtcttattt tacagccata tctaaattat 1500
cttaagaaaa tttttaacaa agggaatgaa atatatatca tgattctgtt tttccaaaag 1560
taacctgaat atagcaatga agttcagttt tgttattggt agtttgggca gagtctcttt 1620
ttgcagcacc tgttgtctac cataattaca gaggacattt ccatgttcta gccaagtata 1680
ctattagaat aaaaaaactt aacattgagt tgcttcaaca gcatgaaact gagtccaaaa 1740
gaccaaatga acaaacacat taatctctga ttatttattt taaatagaat atttaattgt 1800
gtaagatcta atagtatcat tatacttaag caatcatatt cctgatgatc tatgggaaat 1860
aactattatt taattaatat tgaaaccagg ttttaagatg tgttagccag tcctgttact 1920
agtaaatctc tttatttgga gagaaatttt agattgtttt gttctcctta ttagaaggat 1980
tgtagaaaga aaaaaatgac taattggaga aaaattgggg atatatcata tttcactgaa 2040
ttcaaaatgt cttcagttgt aaatcttacc attattttac gtacctctaa gaaataaaag 2100
tgcttctaat taaaatatga tgtcattaat tatgaaatac ttcttgataa cagaagtttt 2160
aaaatagcca tcttagaatc agtgaaatat ggtaatgtat tattttcctc ctttgagtta 2220
ggtcttgtgc ttttttttcc tggccactaa atttcacaat ttccaaaaag caaaataaac 2280
atattctgaa tatttttgct gtgaaacact tgacagcaga gctttccacc atgaaaagaa 2340
gcttcatgag tcacacatta catctttggg ttgattgaat gccactgaaa cattctagta 2400
gcctggagaa gttgacctac ctgtggagat gcctgccatt aaatggcatc ctgatggctt 2460
aatacacatc actcttctgt gaagggtttt aattttcaac acagcttact ctgtagcatc 2520

atgtttacat tgtatgtata aagattatac aaaggtgcaa ttgtgtattt cttccttaaa 2580

atgtatcagt ataggattta gaatctccat gttgaaactc taaatgcata gaaataaaaa 2640

taataaaaaa tttttcattt tggcttttca gcctagtatt aaaactgata aaagcaaagc 2700

catgcacaaa actacctccc tagagaaagg ctagtccctt ttcttcccca ttcatttcat 2760

tatgaacata gtagaaaaca gcatattctt atcaaatttg atgaaaagcg ccaacacgtt 2820

tgaactgaaa tacgacttgt catgtgaact gtaccgaatg tctacgtatt ccacttttcc 2880

tgctggggtt cctgtctcag aaaggagtct tgctcgtgct ggtttctatt acactggtgt 2940

gaatgacaag gtcaaatgct tctgttgtgg cctgatgctg gataactgga aaagaggaga 3000

cagtcctact gaaaagcata aaaagttgta tcctagctgc agattcgttc agagtctaaa 3060

ttccgttaac aacttggaag ctacctctca gcctactttt ccttcttcag taacaaattc 3120

cacacactca ttacttccgg gtacagaaaa cagtggatat ttccgtggct cttattcaaa 3180

ctctccatca aatcctgtaa actccagagc aaatcaagat ttttctgcct tgatgagaag 3240

ttcctaccac tgtgcaatga ataacgaaaa tgccagatta cttacttttc agacatggcc 3300

attgactttt ctgtcgccaa cagatctggc aaaagcaggc ttttactaca taggacctgg 3360

agacagagtg gcttgctttg cctgtggtgg aaaattgagc aattgggaac cgaaggataa 3420

tgctatgtca gaacacctga gacattttcc caaatgccca tttatagaaa atcagcttca 3480

agacacttca agatacacag tttctaatct gagcatgcag acacatgcag cccgctttaa 3540

aacattcttt aactggccct ctagtgttct agttaatcct gagcagcttg caagtgcggg 3600

tttttattat gtgggtaaca gtgatgatgt caaatgcttt tgctgtgatg gtggactcag 3660

gtgttgggaa tctggagatg atccatgggt tcaacatgcc aagtggtttc caaggtgtga 3720

gtacttgata agaattaaag gacaggagtt catccgtcaa gttcaagcca gttaccctca 3780

tctacttgaa cagctgctat ccacatcaga cagcccagga gatgaaaatg cagagtcatc 3840

aattatccat tttgaacctg gagaagacca ttcagaagat gcaatcatga tgaatactcc 3900

tgtgattaat gctgccgtgg aaatgggctt tagtagaagc ctggtaaaac agacagttca 3960

gagaaaaatc ctagcaactg gagagaatta tagactagtc aatgatcttg tgttagactt 4020

actcaatgca gaagatgaaa taagggaaga ggagagagaa agagcaactg aggaaaaaga 4080

atcaaatgat ttattattaa tccggaagaa tagaatggca ctttttcaac atttgacttg 4140

tgtaattcca atcctggata gtctactaac tgccggaatt attaatgaac aagaacatga 4200

tgttattaaa cagaagacac agacgtcttt acaagcaaga gaactgattg atacgatttt 4260

agtaaaagga aatattgcag ccactgtatt cagaaactct ctgcaagaag ctgaagctgt 4320

gttatatgag catttatttg tgcaacagga cataaaatat attcccacag aagatgtttc 4380

agatctacca gtggaagaac aattgcggag actacaagaa gaaagaacat gtaaagtgtg 4440
tatggacaaa gaagtgtcca tagtgtttat tccttgtggt catctagtag tatgcaaaga 4500
ttgtgctcct tctttaagaa agtgtcctat ttgtaggagt acaatcaagg gtacagttcg 4560
tacatttctt tcatgaagaa gaaccaaaac atcgtctaaa ctttagaatt aatttattaa 4620
atgtattata actttaactt ttatcctaat ttggtttcct taaaattttt atttatttac 4680
aactcaaaaa acattgtttt gtgtaacata tttatatatg tatctaaacc atatgaacat 4740
atattttttta gaaactaaga gaatgatagg cttttgttct tatgaacgaa aaagaggtag 4800
cactacaaac acaatattca atcaaaattt cagcattatt gaaattgtaa gtgaagtaaa 4860
acttaagata tttgagttaa cctttaagaa ttttaaatat tttggcattg tactaatacc 4920
gggaacatga agccaggtgt ggtggtatgt gcctgtagtc ccaggctgag gcaagagaat 4980
tacttgagcc caggagtttg aatccatcct gggcagcata ctgagaccct gcctttaaaa 5040
acaaacagaa caaaaacaaa acaccaggga cacatttctc tgtctttttt gatcagtgtc 5100
ctatacatcg aaggtgtgca tatatgttga atgacatttt agggacatgg tgtttttata 5160
aagaattctg tgagaaaaaa tttaataaag caacaaaaat tactcttatt cttcattgct 5220
ttatttcaat gacattggat agtttagtca ctcccagact ctttccatac cttcttaaag 5280
cctctcaaat attgaactac agtttatact ccttcccata agatgcttct tcattgacac 5340
ttgtagaaca cggggtcaac acatcataaa atctattatg gaatgcctga gacaagaatc 5400
aaacagtccc tttagtaagt ttgtttattc acttctctat tgattcattc aagaagtctc 5460
atgccagccc cacctattgg aagaaggtct gagttttatt cttatctctt tggtattaat 5520
tctgaaactt agaaagtaca ctggttagca atgcttggga ccaacaggtt gttctggtaa 5580
ataaatctgt ttcatattgt cagtgcaaca aaatgtcccc ctctgcatta tgttattggt 5640
actcaacacg tccgagtcat aactctgtcc tttgcttctt atagaggtat taggtcttca 5700
agagcagaag taagactgta atagggaata ctcaggggaa ggcaggcaaa ggctagtcat 5760
ctaaaccagt tctagatgtc tgtatagggg cagatggctc tgtaagggca gaagggaaag 5820
acccttcat aagggtcaca gctgacaatc ctataacaaa agacaggtta acaagagaaa 5880
aacttaacaa atttatttaa tcacagattt acatcaccgg ggagccttcg taatgaagat 5940
ccaaaattac aggggaaact gtgcattttt atgcttaggt ttgataatga atggacagcc 6000
ctgaagaata gtgattggaa aaaaaggata tgatctaatg ggaatagaca caggttgggg 6060
acccagcaag gcctgtctgt tcagattatt cttggtctct gtgcagcatt ccttcctcct 6120
ggatataggg cagggcctgt atgggatggg gatattataa cctgctatca agcaaggtag 6180
gtcagagaat ttatttatgg ccagctctta catagttagg tgaggaaaga ttagagtact 6240
atctttaaga tgtaagtctg gcattgtgga aagatggttc cagtttctat gacctacctt 6300

gggggaagagg aattcaagtt tctgtggctt gccttcaggg agaatgaggc tgagacagga 6360

gggcaggata acatcagaga aaaactttgc ttctgaggcc ttcactttgg gttttctgag 6420

ccccaacatc tgctagtgtt gtaaagagaa caattaggga ccaagtgagg ggaggaaaga 6480

atccatctct gcattctgat gctgggagac ttatttcctt gaaatgcaat tgattttgcc 6540

tctgctaaga ggctctgctg gctacccatg tactagccag tgtcctgcat gggtgctagg 6600

ctgaattatt tgtaattgtg cttaggtgat ttgtaactca ggtatagggt atttaaatag 6660

taggcaccct ttttgcacca tgtgtttttt tttttatcta gttcttgtat actacagata 6720

atatttgaac tttgtcatct cactgtaaaa cttttgttca tttctcatta tggtaataaa 6780

tagctattat aaccaaccca tttattcaaa tatgttattt ccctaagtgt tattttgaca 6840

ttttgttttg gaaaaaataa atcaccatag ataataa 6877

<210> 9
<211> 4238
<212> DNA
<213> Homo sapiens

<400> 9
agagggctgt cggcgcagta gcagcgagca gcagagtccg cacgctccgg cgaggggcag 60

aagagcgcga gggagcgcgg ggcagcagaa gcgagagccg agcgcggacc cagccaggac 120

ccacagccct ccccagctgc ccaggaagag ccccagccat ggaacaccag ctcctgtgct 180

gcgaagtgga aaccatccgc cgcgcgtacc ccgatgccaa cctcctcaac gaccgggtgc 240

tgcgggccat gctgaaggcg gaggagacct gcgcgccctc ggtgtcctac ttcaaatgtg 300

tgcagaagga ggtcctgccg tccatgcgga agatcgtcgc cacctggatg ctggaggtct 360

gcgaggaaca gaagtgcgag gaggaggtct tcccgctggc catgaactac ctggaccgct 420

tcctgtcgct ggagcccgtg aaaaagagcc gcctgcagct gctgggggcc acttgcatgt 480

tcgtggcctc taagatgaag gagaccatcc ccctgacggc cgagaagctg tgcatctaca 540

ccgacaactc catccggccc gaggagctgc tgcaaatgga gctgctcctg gtgaacaagc 600

tcaagtggaa cctggccgca atgacccccgc acgatttcat tgaacacttc ctctccaaaa 660

tgccagaggc ggaggagaac aaacagatca tccgcaaaca cgcgcagacc ttcgttgccc 720

tctgtgccac agatgtgaag ttcatttcca atccgccctc catggtggca gcggggagcg 780

tggtggccgc agtgcaaggc ctgaacctga ggagccccaa caacttcctg tcctactacc 840

gcctcacacg cttcctctcc agagtgatca agtgtgaccc ggactgcctc cgggcctgcc 900

aggagcagat cgaagccctg ctggagtcaa gcctgcgcca ggcccagcag aacatggacc 960

ccaaggccgc cgaggaggag gaagaggagg aggaggaggt ggacctggct tgcacaccca 1020

ccgacgtgcg ggacgtggac atctgagggc gccaggcagg cgggcgccac cgccacccgc 1080

agcgagggcg gagccggccc caggtgctcc cctgacagtc cctcctctcc ggagcatttt 1140
gataccagaa gggaaagctt cattctcctt gttgttggtt gtttttttcct ttgctctttc 1200
ccccttccat ctctgactta agcaaaagaa aaagattacc caaaaactgt ctttaaaaga 1260
gagagagaga aaaaaaaaat agtatttgca taaccctgag cggtgggggga ggagggttgt 1320
gctacagatg atagaggatt ttatacccca ataatcaact cgtttttata ttaatgtact 1380
tgtttctctg ttgtaagaat aggcattaac acaaaggagg cgtctcggga gaggattagg 1440
ttccatcctt tacgtgttta aaaaaaagca taaaaacatt ttaaaaacat agaaaaattc 1500
agcaaaccat ttttaaagta gaagagggtt ttaggtagaa aaacatattc ttgtgctttt 1560
cctgataaag cacagctgta gtggggttct aggcatctct gtactttgct tgctcatatg 1620
catgtagtca ctttataagt cattgtatgt tattatattc cgtaggtaga tgtgtaacct 1680
cttcacctta ttcatggctg aagtcacctc ttggttacag tagcgtagcg tgcccgtgtg 1740
catgtccttt gcgcctgtga ccaccacccc aacaaaccat ccagtgacaa accatccagt 1800
ggaggtttgt cgggcaccag ccagcgtagc agggtcggga aaggccacct gtcccactcc 1860
tacgatacgc tactataaag agaagacgaa atagtgacat aatatattct atttttatac 1920
tcttcctatt tttgtagtga cctgtttatg agatgctggt tttctaccca acggccctgc 1980
agccagctca cgtccaggtt caacccacag ctacttggtt tgtgttcttc ttcatattct 2040
aaaaccattc catttccaag cactttcagt ccaataggtg taggaaatag cgctgttttt 2100
gttgtgtgtg cagggagggc agttttctaa tggaatggtt tgggaatatc catgtacttg 2160
tttgcaagca ggactttgag gcaagtgtgg gccactgtgg tggcagtgga ggtggggtgt 2220
ttgggaggct gcgtgccagt caagaagaaa aaggtttgca ttctcacatt gccaggatga 2280
taagttcctt tccttttctt taaagaagtt gaagtttagg aatcctttgg tgccaactgg 2340
tgtttgaaag tagggacctc agaggtttac ctagagaaca ggtggttttt aagggttatc 2400
ttagatgttt cacaccggaa ggtttttaaa cactaaaata tataatttat agttaaggct 2460
aaaaagtata tttattgcag aggatgttca taaggccagt atgatttata aatgcaatct 2520
ccccttgatt taaacacaca gatacacaca cacacacaca cacacacaaa ccttctgcct 2580
ttgatgttac agatttaata cagtttattt ttaaagatag atccttttat aggtgagaaa 2640
aaaacaatct ggaagaaaaa aaccacacaa agacattgat tcagcctgtt tggcgtttcc 2700
cagagtcatc tgattggaca ggcatgggtg caaggaaaat tagggtactc aacctaagtt 2760
cggttccgat gaattcttat cccctgcccc ttcctttaaa aaacttagtg acaaaataga 2820
caatttgcac atcttggcta tgtaattctt gtaattttta tttaggaagt gttgaaggga 2880
ggtggcaaga gtgtggaggc tgacgtgtga gggaggacag gcgggaggag gtgtgaggag 2940

gaggctcccg aggggaaggg gcggtgccca caccggggac aggccgcagc tccattttct 3000

tattgcgctg ctaccgttga cttccaggca cggtttggaa atattcacat cgcttctgtg 3060

tatctctttc acattgtttg ctgctattgg aggatcagtt ttttgtttta caatgtcata 3120

tactgccatg tactagtttt agttttctct tagaacattg tattacagat gccttttttg 3180

tagttttttt tttttttatg tgatcaattt tgacttaatg tgattactgc tctattccaa 3240

aaaggttgct gtttcacaat acctcatgct tcacttagcc atggtggacc cagcgggcag 3300

gttctgcctg ctttggcggg cagacacgcg ggcgcgatcc cacacaggct ggcgggggcc 3360

ggccccgagg ccgcgtgcgt gagaaccgcg ccggtgtccc cagagaccag gctgtgtccc 3420

tcttctcttc cctgcgcctg tgatgctggg cacttcatct gatcgggggc gtagcatcat 3480

agtagttttt acagctgtgt tattctttgc gtgtagctat ggaagttgca taattattat 3540

tattattatt ataacaagtg tgtcttacgt gccaccacgg cgttgtacct gtaggactct 3600

cattcgggat gattggaata gcttctggaa tttgttcaag ttttgggtat gtttaatctg 3660

ttatgtacta gtgttctgtt tgttattgtt ttgttaatta caccataatg ctaatttaaa 3720

gagactccaa atctcaatga agccagctca cagtgctgtg tgccccggtc acctagcaag 3780

ctgccgaacc aaaagaattt gcaccccgct gcgggcccac gtggttgggg ccctgccctg 3840

gcagggtcat cctgtgctcg gaggccatct cgggcacagg cccaccccgc cccacccctc 3900

cagaacacgg ctcacgctta cctcaaccat cctggctgcg gcgtctgtct gaaccacgcg 3960

ggggccttga gggacgcttt gtctgtcgtg atggggcaag ggcacaagtc ctggatgttg 4020

tgtgtatcga gaggccaaag gctggtggca agtgcacggg gcacagcgga gtctgtcctg 4080

tgacgcgcaa gtctgagggt ctgggcggcg ggcggctggg tctgtgcatt tctggttgca 4140

ccgcggcgct tcccagcacc aacatgtaac cggcatgttt ccagcagaag acaaaaagac 4200

aaacatgaaa gtctagaaat aaaactggta aaacccca 4238

<210> 10
<211> 1607
<212> DNA
<213> Homo sapiens

<400> 10
ccgactgagg cacgagcggg tgacgctggg cctgcagcgc ggagcagaaa gcagaacccg 60

cagagtcctc cctgctgctg tgtggacgac acgtgggcac aggcagaagt gggccctgtg 120

accagctgca ctggtttcgt ggaaggaagc tccaggactg gcgggatggg ctcagcctgt 180

atcaaagtca ccaaatactt tctcttcctc ttcaacttga tcttctttat cctgggcgca 240

gtgatcctgg gcttcggggt gtggatcctg gccgacaaga gcagtttcat ctctgtcctg 300

caaacctcct ccagctcgct taggatgggg gcctatgtct tcatcggcgt gggggcagtc 360

actatgctca tgggcttcct gggctgcatc ggcgccgtca acgaggtccg ctgcctgctg 420

gggctgtact ttgctttcct gctcctgatc ctcattgccc aggtgacggc cggggccctc 480

ttctacttca acatgggcaa gctgaagcag gagatgggcg gcatcgtgac tgagctcatt 540

cgagactaca acagcagtcg cgaggacagc ctgcaggatg cctgggacta cgtgcaggct 600

caggtgaagt gctgcggctg ggtcagcttc tacaactgga cagacaacgc tgagctcatg 660

aatcgccctg aggtcaccta cccctgttcc tgcgaagtca aggggggaaga ggacaacagc 720

ctttctgtga ggaagggctt ctgcgaggcc cccggcaaca ggacccagag tggcaaccac 780

cctgaggact ggcctgtgta ccaggagggc tgcatggaga aggtgcaggc gtggctgcag 840

gagaacctgg gcatcatcct cggcgtgggc gtgggtgtgg ccatcatcga gctcctgggg 900

atggtcctgt ccatctgctt gtgccggcac gtccattccg aagactacag caaggtcccc 960

aagtactgag gcagctgcta tccccatctc cctgcctggc cccaacctc agggctccca 1020

ggggtctccc tggctccctc ctccaggcct gcctcccact tcactgcgaa gaccctcttg 1080

cccaccctga ctgaaagtag ggggctttct ggggcctagc gatctctcct ggcctatccg 1140

ctgccagcct tgagccctgg ctgttctgtg gttcctctgc tcaccgccca tcagggttct 1200

cttatcaact cagagaaaaa tgctccccac agcgtccctg gcgcaggtgg gctggacttc 1260

tacctgccct caagggtgtg tatattgtat aggggcaact gtatgaaaaa ttggggagga 1320

gggggccggg cgcggtgctc acgcctgtaa tcccagcact ttgggaggcc gaggcgggtg 1380

gatcacgagg tcaggagatc gagaccatcc tggctaacat ggtgaaaccc cgtctctact 1440

aaaaatacaa aaaaatta gccgggcgcg gtggcgggca cctgtagtcc cagctacttg 1500

ggaggctgag gcaggagaat ggtgtgaacc cgggagcgga ggttgcagtg agctgagatc 1560

gtgctactgc actccagcct gggggacaga aagagactcc gtctcaa 1607

<210> 11
<211> 2122
<212> DNA
<213> Homo sapiens

<400> 11
ggtggctatt ttgtccttgg gctgcctgtt ttcagctgct gcaaccacag ggatttcttc 60

tgttcaggcg ccatgtcaga accggctggg gatgtccgtc agaacccatg cggcagcaag 120

gcctgccgcc gcctcttcgg cccagtggac agcgagcagc tgagccgcga ctgtgatgcg 180

ctaatggcgg gctgcatcca ggaggcccgt gagcgatgga acttcgactt tgtcaccgag 240

acaccactgg agggtgactt cgcctgggag cgtgtgcggg gccttggcct gcccaagctc 300

taccttccca cggggccccg gcgaggccgg gatgagttgg gaggaggcag gcggcctggc 360

acctcacctg ctctgctgca ggggacagca gaggaagacc atgtggacct gtcactgtct 420

```
tgtacccttg tgcctcgctc aggggagcag gctgaagggt ccccaggtgg acctggagac      480
tctcagggtc gaaaacggcg gcagaccagc atgacagatt tctaccactc caaacgccgg      540
ctgatcttct ccaagaggaa gccctaatcc gcccacagga agcctgcagt cctggaagcg      600
cgagggcctc aaaggcccgc tctacatctt ctgccttagt ctcagtttgt gtgtcttaat      660
tattatttgt gttttaattt aaacacctcc tcatgtacat accctggccg ccccctgccc      720
cccagcctct ggcattagaa ttatttaaac aaaaactagg cggttgaatg agaggttcct      780
aagagtgctg ggcattttta ttttatgaaa tactatttaa agcctcctca tcccgtgttc      840
tccttttcct ctctcccgga ggttgggtgg gccggcttca tgccagctac ttcctcctcc      900
ccacttgtcc gctgggtggt accctctgga ggggtgtggc tccttcccat cgctgtcaca      960
ggcggttatg aaattcaccc cctttcctgg acactcagac ctgaattctt tttcatttga     1020
gaagtaaaca gatggcactt tgaaggggcc tcaccgagtg ggggcatcat caaaaacttt     1080
ggagtcccct cacctcctct aaggttgggc agggtgaccc tgaagtgagc acagcctagg     1140
gctgagctgg ggacctggta ccctcctggc tcttgatacc cccctctgtc ttgtgaaggc     1200
agggggaagg tggggtcctg gagcagacca ccccgcctgc cctcatggcc cctctgacct     1260
gcactgggga gcccgtctca gtgttgagcc ttttccctct ttggctcccc tgtacctttt     1320
gaggagcccc agctaccctt cttctccagc tgggctctgc aattcccctc tgctgctgtc     1380
cctcccctt gtcctttccc ttcagtaccc tctcagctcc aggtggctct gaggtgcctg      1440
tcccaccccc acccccagct caatggactg gaaggggaag ggacacacaa gaagaagggc     1500
accctagttc tacctcaggc agctcaagca gcgaccgccc cctcctctag ctgtgggggt     1560
gagggtccca tgtggtggca caggccccct tgagtggggt tatctctgtg ttaggggtat     1620
atgatggggg agtagatctt tctaggaggg agacactggc ccctcaaatc gtccagcgac     1680
cttcctcatc caccccatcc ctccccagtt cattgcactt tgattagcag cggaacaagg     1740
agtcagacat tttaagatgg tggcagtaga ggctatggac agggcatgcc acgtgggctc     1800
atatggggct gggagtagtt gtctttcctg gcactaacgt tgagcccctg gaggcactga     1860
agtgcttagt gtacttggag tattggggtc tgaccccaaa caccttccag ctcctgtaac     1920
atactggcct ggactgtttt ctctcggctc cccatgtgtc ctggttcccg tttctccacc     1980
tagactgtaa acctctcgag ggcagggacc acaccctgta ctgttctgtg tctttcacag     2040
ctcctcccac aatgctgaat atacagcagg tgctcaataa atgattctta gtgactttac     2100
ttgtaaaaaa aaaaaaaaaa aa                                              2122
```

<210> 12
<211> 1744
<212> DNA
<213> Homo sapiens

<400> 12

```
gcagcaggcc aagggggagg tgcgagcgtg gacctgggac gggtctgggc ggctctcggt 60
ggttggcacg ggttcgcaca cccattcaag cggcaggacg cacttgtctt agcagttctc 120
gctgaccgcg ctagctgcgg cttctacgct ccggcactct gagttcatca gcaaacgccc 180
tggcgtctgt cctcaccatg cctagccttt gggaccgctt ctcgtcgtcg tccacctcct 240
cttcgccctc gtccttgccc cgaactccca ccccagatcg gccgccgcgc tcagcctggg 300
ggtcggcgac ccgggaggag gggtttgacc gctccacgag cctggagagc tcggactgcg 360
agtccctgga cagcagcaac agtggcttcg ggccggagga agacacggct tacctggatg 420
gggtgtcgtt gcccgacttc gagctgctca gtgaccctga ggatgaacac ttgtgtgcca 480
acctgatgca gctgctgcag gagagcctgg cccaggcgcg gctgggctct cgacgccctg 540
cgcgcctgct gatgcctagc cagttggtaa gccaggtggg caaagaacta ctgcgcctgg 600
cctacagcga gccgtgcggc ctgcgggggg cgctgctgga cgtctgcgtg gagcagggca 660
agagctgcca cagcgtgggc cagctggcac tcgaccccag cctggtgccc accttccagc 720
tgaccctcgt gctgcgcctg gactcacgac tctggcccaa gatccagggg ctgtttagct 780
ccgccaactc tcccttcctc cctggcttca gccagtccct gacgctgagc actggcttcc 840
gagtcatcaa gaagaagctg tacagctcgg aacagctgct cattgaggag tgttgaactt 900
caacctgagg gggccgacag tgccctccaa gacagagacg actgaacttt tggggtggag 960
actagaggca ggagctgagg gactgattcc tgtggttgga aaactgaggc agccacctaa 1020
ggtggaggtg ggggaatagt gtttcccagg aagctcattg agttgtgtgc gggtggctgt 1080
gcattgggga cacatacccc tcagtactgt agcatgaaac aaaggcttag gggccaacaa 1140
ggcttccagc tggatgtgtg tgtagcatgt accttattat ttttgttact gacagttaac 1200
agtggtgtga catccagaga gcagctgggc tgctcccgcc ccagcccggc ccagggtgaa 1260
ggaagaggca cgtgctcctc agagcagccg gagggagggg ggaggtcgga ggtcgtggag 1320
gtggtttgtg tatcttactg gtctgaaggg accaagtgtg tttgttgttt gttttgtatc 1380
ttgtttttct gatcggagca tcactactga cctgttgtag gcagctatct tacagacgca 1440
tgaatgtaag agtaggaagg ggtgggtgtc agggatcact tgggatcttt gacacttgaa 1500
aaattacacc tggcagctgc gtttaagcct tcccccatcg tgtactgcag agttgagctg 1560
gcaggggagg ggctgagagg gtgggggctg gaacccctcc ccgggaggag tgccatctgg 1620
gtcttccatc tagaactgtt tacatgaaga taagatactc actgttcatg aatacacttg 1680
atgttcaagt attaagacct atgcaatatt ttttactttt ctaataaaca tgtttgttaa 1740
aaca 1744
```

<210> 13
<211> 2690
<212> DNA
<213> Homo sapiens

<400> 13
gggactttgc aggcagcggc ggccgggggc ggagcgggat cgagccctcg ccgaggcctg 60

ccgccatggg cccgcgccgc cgccgccgcc tgtcacccgg gccgcgcggg ccgtgagcgt 120

catggccttg gccggggccc ctgcgggcgg cccatgcgcg ccggcgctgg aggccctgct 180

cggggccggc gcgctgcggc tgctcgactc ctcgcagatc gtcatcatct ccgccgcgca 240

ggacgccagc gccccgccgg ctcccaccgg ccccgcggcg cccgccgccg gcccctgcga 300

ccctgacctg ctgctcttcg ccacaccgca ggcgccccgg cccacaccca gtgcgccgcg 360

gcccgcgctc ggccgcccgc cggtgaagcg gaggctggac ctggaaactg accatcagta 420

cctggccgag agcagtgggc cagctcgggg cagaggccgc catccaggaa aaggtgtgaa 480

atccccgggg gagaagtcac gctatgagac ctcactgaat ctgaccacca agcgcttcct 540

ggagctgctg agccactcgg ctgacggtgt cgtcgacctg aactgggctg ccgaggtgct 600

gaaggtgcag aagcggcgca tctatgacat caccaacgtc cttgagggca tccagctcat 660

tgccaagaag tccaagaacc acatccagtg gctgggcagc cacaccacag tgggcgtcgg 720

cggacggctt gaggggttga cccaggacct ccgacagctg caggagagcg agcagcagct 780

ggaccacctg atgaatatct gtactacgca gctgcgcctg ctctccgagg acactgacag 840

ccagcgcctg gcctacgtga cgtgtcagga ccttcgtagc attgcagacc ctgcagagca 900

gatggttatg gtgatcaaag cccctcctga gacccagctc caagccgtgg actcttcgga 960

gaactttcag atctccctta agagcaaaca aggcccgatc gatgttttcc tgtgccctga 1020

ggagaccgta ggtgggatca gccctgggaa gaccccatcc caggaggtca cttctgagga 1080

ggagaacagg gccactgact ctgccaccat agtgtcacca ccaccatcat ctccccccctc 1140

atccctcacc acagatccca gccagtctct actcagcctg gagcaagaac cgctgttgtc 1200

ccggatgggc agcctgcggg ctcccgtgga cgaggaccgc ctgtccccgc tggtggcggc 1260

cgactcgctc ctggagcatg tgcgggagga cttctccggc ctcctccctg aggagttcat 1320

cagcctttcc ccaccccacg aggccctcga ctaccacttc ggcctcgagg agggcgaggg 1380

catcagagac ctcttcgact gtgactttgg ggacctcacc cccctggatt tctgacaggg 1440

cttggaggga ccagggtttc cagagatgct caccttgtct ctgcagccct ggagcccccct 1500

gtccctggcc gtcctcccag cctgtttgga aacatttaat ttatacccct ctcctctgtc 1560

tccagaagct tctagctctg ggtctggct accgctagga ggctgagcaa gccaggaagg 1620

gaaggagtct gtgtggtgtg tatgtgcatg cagcctacac ccacacgtgt gtaccggggg 1680

tgaatgtgtg tgagcatgtg tgtgtgcatg taccggggaa tgaaggtgaa catacacctc 1740

tgtgtgtgca ctgcagacac gccccagtgt gtccacatgt gtgtgcatga gtccatgtgt 1800

gcgcgtgggg gggctctaac tgcactttcg gcccttttgc tctgggggtc ccacaaggcc 1860

cagggcagtg cctgctccca gaatctggtg ctctgaccag gccaggtggg gaggctttgg 1920

ctggctgggc gtgtaggacg gtgagagcac ttctgtctta aaggtttttt ctgattgaag 1980

ctttaatgga gcgttattta tttatcgagg cctctttggt gagcctgggg aatcagcaaa 2040

ggggaggagg ggtgtggggt tgatacccca actccctcta cccttgagca agggcagggg 2100

tccctgagct gttcttctgc cccatactga aggaactgag gcctgggtga tttatttatt 2160

gggaaagtga gggagggaga cagactgact gacagccatg ggtggtcaga tggtggggtg 2220

ggccctctcc agggggccag ttcagggccc cagctgcccc ccaggatgga tatgagatgg 2280

gagaggtgag tgggggacct tcactgatgt gggcaggagg ggtggtgaag gcctccccca 2340

gcccagaccc tgtggtccct cctgcagtgt ctgaagcgcc tgcctcccca ctgctctgcc 2400

ccaccctcca atctgcactt tgatttgctt cctaacagct ctgttccctc ctgctttggt 2460

tttaataaat attttgatga cgtttgggcc gggttttggg actctgttgg gaacatttcg 2520

gggcgggaga ggccaaggtt gctggggaaa tgcccattct ccacttccct tctccctgtc 2580

cgtgcccgat ttgatttgag cctcataact cgaagaaagg tcagcttcct cgctgttttg 2640

gtcctaactc aaaagcagat ccagtaaagg tttttgttgt agaaagccaa 2690

<210> 14
<211> 2298
<212> DNA
<213> Homo sapiens

<400> 14
aagactgcga gctccccgca ccccctcgca ctccctctgg ccggcccagg gcgccttcag 60

cccaacctcc ccagccccac gggcgccacg gaacccgctc gatctcgccg ccaactggta 120

gacatggaga cccctgcctg gccccgggtc ccgcgccccg agaccgccgt cgctcggacg 180

ctcctgctcg gctgggtctt cgcccaggtg gccggcgctt caggcactac aaatactgtg 240

gcagcatata atttaacttg gaaatcaact aatttcaaga caattttgga gtgggaaccc 300

aaacccgtca atcaagtcta cactgttcaa ataagcacta agtcaggaga ttggaaaagc 360

aaatgctttt acacaacaga cacagagtgt gacctcaccg acgagattgt gaaggatgtg 420

aagcagacgt acttggcacg ggtcttctcc tacccggcag ggaatgtgga gagcaccggt 480

tctgctgggg agcctctgta tgagaactcc ccagagttca caccttacct ggagacaaac 540

ctcggacagc caacaattca gagttttgaa caggtgggaa caaaagtgaa tgtgaccgta 600

gaagatgaac ggactttagt cagaaggaac aacactttcc taagcctccg ggatgttttt 660

ggcaaggact taatttatac actttattat tggaaatctt caagttcagg aaagaaaaca 720

```
gccaaaacaa acactaatga gtttttgatt gatgtggata aaggagaaaa ctactgtttc      780
agtgttcaag cagtgattcc ctcccgaaca gttaaccgga agagtacaga cagcccggta      840
gagtgtatgg gccaggagaa aggggaattc agagaaatat tctacatcat tggagctgtg      900
gtatttgtgg tcatcatcct tgtcatcatc ctggctatat ctctacacaa gtgtagaaag      960
gcaggagtgg ggcagagctg gaaggagaac tccccactga atgtttcata aaggaagcac     1020
tgttggagct actgcaaatg ctatattgca ctgtgaccga gaacttttaa gaggatagaa     1080
tacatggaaa cgcaaatgag tatttcggag catgaagacc ctggagttca aaaaactctt     1140
gatatgacct gttattacca ttagcattct ggttttgaca tcagcattag tcactttgaa     1200
atgtaacgaa tggtactaca accaattcca agttttaatt tttaacacca tggcaccttt     1260
tgcacataac atgctttaga ttatatattc cgcactcaag gagtaaccag gtcgtccaag     1320
caaaaacaaa tgggaaaatg tcttaaaaaa tcctgggtgg acttttgaaa agcttttttt     1380
tttttttttt ttttttgag acggagtctt gctctgttgc ccaggctgga gtgcagtagc     1440
acgatctcgg ctcactgcac cctccgtctc tcgggttcaa gcaattgtct gcctcagcct     1500
cccgagtagc tgggattaca ggtgcgcact accacgccaa gctaattttt gtatttttta     1560
gtagagatgg ggtttcacca tcttggccag gctggtcttg aattcctgac ctcaggtgat     1620
ccacccacct tggcctccca aagtgctagt attatgggcg tgaaccacca tgcccagccg     1680
aaaagctttt gaggggctga cttcaatcca tgtaggaaag taaaatggaa ggaaattggg     1740
tgcatttcta ggacttttct aacatatgtc tataatatag tgtttaggtt cttttttttt     1800
tcaggaatac atttggaaat tcaaaacaat tggcaaactt tgtattaatg tgttaagtgc     1860
aggagacatt ggtattctgg gcaccttcct aatatgcttt acaatctgca ctttaactga     1920
cttaagtggc attaaacatt tgagagctaa ctatattttt ataagactac tatacaaact     1980
acagagttta tgatttaagg tacttaaagc ttctatggtt gacattgtat atataatttt     2040
ttaaaaaggt tttctatatg gggattttct atttatgtag gtaatattgt tctatttgta     2100
tatattgaga taatttattt aatatacttt aaataaaggt gactgggaat tgttactgtt     2160
gtacttattc tatcttccat ttattattta tgtacaattt ggtgtttgta ttagctctac     2220
tacagtaaat gactgtaaaa ttgtcagtgg cttacaacaa cgtatctttt tcgcttataa     2280
tacattttgg tgactgta                                                   2298
```

<210> 15
<211> 2291
<212> DNA
<213> Homo sapiens

<400> 15

```
gaacaatgaa gaaagcccca cagccactgt tgctgagcag ggagaggata ttacctccaa       60
```

```
aaaagacagg ggagtattaa agattgtcaa aagagtgggg aatggtgagg aaacgccgat      120
gattggagac aaagtttatg tccattacaa aggaaaattg tcaaatggaa agaagtttga      180
ttccagtcat gatagaaatg aaccatttgt ctttagtctt ggcaaaggcc aagtcatcaa      240
ggcatgggac attggggtgg ctaccatgaa gaaaggagag atatgccatt tactgtgcaa      300
accagaatat gcatatggct cggctggcag tctccctaaa attccctcga atgcaactct      360
ctttttgag attgagctcc ttgatttcaa aggagaggat ttatttgaag atggaggcat       420
tatccggaga accaaacgga aaggagaggg atattcaaat ccaaacgaag gagcaacagt      480
agaaatccac ctggaaggcc gctgtggtgg aaggatgttt gactgcagag atgtggcatt      540
cactgtgggc gaaggagaag accacgacat tccaattgga attgacaaag ctctggagaa      600
aatgcagcgg gaagaacaat gtattttata tcttggacca agatatggtt ttggagaggc      660
agggaagcct aaatttggca ttgaacctaa tgctgagctt atatatgaag ttacacttaa      720
gagcttcgaa aaggccaaag aatcctggga gatggatacc aaagaaaaat tggagcaggc      780
tgccattgtc aaagagaagg gaaccgtata cttcaaggga ggcaaataca tgcaggcggt      840
gattcagtat gggaagatag tgtcctggtt agagatggaa tatggtttat cagaaaagga      900
atcgaaagct tctgaatcat ttctccttgc tgcctttctg aacctggcca tgtgctacct      960
gaagcttaga gaatacacca aagctgttga atgctgtgac aaggcccttg gactggacag     1020
tgccaatgag aaaggcttgt ataggagggg tgaagcccag ctgctcatga acgagtttga     1080
gtcagccaag ggtgactttg agaaagtgct ggaagtaaac cccagaata aggctgcaag      1140
actgcagatc tccatgtgcc agaaaaaggc caaggagcac aacgagcggg accgcaggat     1200
atacgccaac atgttcaaga agtttgcaga gcaggatgcc aaggaagagg ccaataaagc     1260
aatgggcaag aagacttcag aaggggtcac taatgaaaaa ggaacagaca gtcaagcaat     1320
ggaagaagag aaacctgagg gccacgtatg acgccacgcc aaggagggaa gagtcccagt     1380
gaactcggcc cctcctcaat gggctttccc ccaactcagg acagaacagt gtttaatgta     1440
aagtttgtta tagtctatgt gattctggaa gcaaatggca aaaccagtag cttcccaaaa     1500
acagcccccc tgctgctgcc cggagggttc actgaggggt ggcacgggac cactccaggt     1560
ggaacaaaca gaaatgactg tggtgtggag ggagtgagcc agcagcttaa gtccagctca     1620
tttcagtttc tatcaacctt caagtatcca attcagggtc cctggagatc atcctaacaa     1680
tgtggggctg ttaggtttta cctttgaact ttcatagcac tgcagaaacc ttttaaaaaa     1740
aaatgcttca tgaatttctc ctttcctaca gttgggtagg gtaggggaag gaggataagc     1800
ttttgttttt taaatgactg aagtgctata aatgtagtct gttgcatttt taaccaacag     1860
aacccacagt agaggggtct catgtctccc cagttccaca gcagtgtcac agacgtgaaa     1920
```

```
gccagaacct cagaggccac ttgcttgctg acttagcctc ctcccaaagt ccccctcctc     1980

agccagcctc cttgtgagag tggctttcta ccacacacag cctgtccctg gggagtaat     2040

tctgtcattc ctaaaacacc cttcagcaat gataatgagc agatgagagt ttctggatta     2100

gcttttccta ttttcgatga agttctgaga tactgaaatg tgaaaagagc aatcagaatt     2160

gtgctttttc tcccctcctc tattcctttt agggaataat attcaataca cagtacttcc     2220

tcccagaaaa aaaaaaaaaa aaaaaaaaaa aaaaaaaaaa aaaaaaaaaa aaaaaaaaaa     2280

aaaaaaaaaa a                                                          2291


<210>  16
<211>  1978
<212>  DNA
<213>  Homo sapiens

<400>  16
agacctgacc tgccagctcc gggcgtgggg tgaaatctct tgattcctag tctctcgata       60

tggcacctcc gtcagtcttt gccgaggttc cgcaggccca gcctgtcctg gtcttcaagc      120

tcactgccga cttcagggag gatccggacc cccgcaaggt caacctggga gtgggagcat      180

atcgcacgga tgactgccat ccctgggttt tgccagtagt gaagaaagtg gagcagaaga      240

ttgctaatga caatagccta aatcacgagt atctgccaat cctgggcctg gctgagttcc      300

ggagctgtgc ttctcgtctt gcccttgggg atgacagccc agcactcaag gagaagcggg      360

taggaggtgt gcaatctttg gggggaacag gtgcacttcg aattggagct gatttcttag      420

cgcgttggta caatggaaca aacaacaaga acacacctgt ctatgtgtcc tcaccaacct      480

gggagaatca caatgctgtg ttttccgctg ctggttttaa agacattcgg tcctatcgct      540

actgggatgc agagaagaga ggattggacc tccagggctt cctgaatgat ctggagaatg      600

ctcctgagtt ctccattgtt gtcctccacg cctgtgcaca caacccaact gggattgacc      660

caactccgga gcagtggaag cagattgctt ctgtcatgaa gcaccggttt ctgttcccct      720

tctttgactc agcctatcag ggcttcgcat ctggaaacct ggagagagat gcctgggcca      780

ttcgctattt tgtgtctgaa ggcttcgagt tcttctgtgc ccagtccttc tccaagaact      840

tcgggctcta caatgagaga gtcgggaatc tgactgtggt tggaaaagaa cctgagagca      900

tcctgcaagt cctttcccag atggagaaga tcgtgcggat tacttggtcc aatcccccccg     960

cccagggagc acgaattgtg gccagcaccc tctctaaccc tgagctcttt gaggaatgga     1020

caggtaatgt gaagacaatg gctgaccgga ttctgaccat gagatctgaa ctcagggcac     1080

gactagaagc cctcaaaacc cctgggacct ggaaccacat cactgatcaa attggcatgt     1140

tcagcttcac tgggttgaac cccaagcagg ttgagtatct ggtcaatgaa aagcacatct     1200

acctgctgcc aagtggtcga atcaacgtga gtggcttaac caccaaaaat ctagattacg     1260
```

tggccacctc catccatgaa gcagtcacca aaatccagtg aagaaacacc acccgtccag 1320

taccaccaaa gtagttctct gtcatgtgtg ttccctgcct gcacaaacct acatgtacat 1380

accatggatt agagacactt gcaggactga aaggctgctc tggtgaggca gcctctgttt 1440

aaaccggccc cacatgaaga gaacatccct tgagacgaat ttggagactg ggattagagc 1500

ctttggaggt caaagcaaat taagattttt atttaagaat aaaagagtac tttgatcatg 1560

agacataggt atcttgtccc tctcactaaa aaggagtgtt gtgtgtggcg gccacgtgct 1620

tctatgtggt gtttgactct gtacaaattc tagtcccaaa gatcaagttg tctgaaggag 1680

ccaaagtgtg aatgtgggtg tcggctgcgg cattaaattc atcatctcaa cccagagtgt 1740

ctggtctccc tgctctttct gcatggttgt gtccctagtc ctaagctttg gttctttagg 1800

gtgactgtgg taagaaggat atttaatcat gacatgcacg gacacgtaca tatttaactg 1860

aaacaagttt taccaaacag tatttactcg tgatgtgcgt agtgcattct gatatttttg 1920

agccattcta ttgtgttcta cttcacctaa aaaaataaaa taaaaatgtt gatcaaga 1978

<210> 17
<211> 4562
<212> DNA
<213> Homo sapiens

<400> 17
aaatgtaatt tgaagaaggc agaaggaacc catggcttta gccggctgcc cagattcctt 60

tttgcaccat ccgtactacc aggacaaggt ggagcagaca cctcgcagtc aacaagaccc 120

ggcaggacca ggactccccg cacagtctga ccgacttgcg aatcaccagg aggatgatgt 180

ggacctggaa gccctggtga acgatatgaa tgcatccctg gagagcctgt actcggcctg 240

cagcatgcag tcagacacgg tgcccctcct gcagaatggc cagcatgccc gcagccagcc 300

tcgggcttca ggccctcctc ggtccatcca gccacaggtg tccccgaggc agagggtgca 360

gcgctcccag cctgtgcaca tcctcgctgt caggcgcctt caggaggaag accagcagtt 420

tagaacctca tctctgccgg ccatccccaa tccttttcct gaactctgtg gccctgggag 480

ccccccctgtg ctcacgccgg gttctttacc tccgagccag gccgccgcaa agcaggatgt 540

taaagtcttt agtgaagatg ggacaagcaa agtggtggag attctagcag acatgacagc 600

cagagacctg tgccaattgc tggtttacaa aagtcactgt gtggatgaca acagctggac 660

actagtggag caccacccgc acctaggatt agagaggtgc ttggaagacc atgagctggt 720

ggtccaggtg gagagtacca tggccagtga gagtaaattt ctattcagga agaattacgc 780

aaaatacgag ttctttaaaa atcccatgaa tttcttccca gaacagatgg ttacttggtg 840

ccagcagtca aatggcagtc aaacccagct tttgcaggaa cccagacacc tgcagctgct 900

ggccgacctg gaggacagca acatcttctc cctgatcgct ggcaggaagc agtacaacgc 960

ccctacagac cacgggctct gcataaagcc aaacaaagtc aggaatgaaa ctaaagagct 1020
gaggttgctc tgtgcagagg acgagcaaac caggacgtgc tggatgacag cgttcagact 1080
cctcaagtat ggaatgctcc tttaccagaa ttaccgaatc cctcagcaga ggaaggcctt 1140
gctgtccccg ttctcgacgc cagtgcgcag tgtctccgag aactccctcg tggcaatgga 1200
tttttctggg caaacaggac gcgtgataga gaatccggca gaggcccaga gcgcagccct 1260
ggaggagggc cacgcctgga ggaagcgaag cacacggatg aacatcctag gtagccaaag 1320
tcccctccac ccttctaccc taagtacagt gattcacagg acacagcact ggtttcacgg 1380
gaggatctcc agggaggaat cccacaggat cattaaacag caagggctcg tggatgggct 1440
ttttctcctc cgtgacagcc agagtaatcc aaaggcattt gtactcacac tgtgtcatca 1500
ccagaaaatt aaaaatttcc agatcttacc ttgcgaggac gacgggcaga cgttcttcag 1560
cctagatgac gggaacacca aattctctga cctgatccag ctggttgact tttaccagct 1620
gaacaaagga gtcctgcctt gcaaactcaa gcaccactgc atccgagtgg ccttatgacc 1680
gcagatgtcc tctcggctga agactggagg aagtgaacac tggagtgaag aagcggtctg 1740
tgcgttggtg aagaacacac atcgattctg cacctgggga cccagagcga gatgggtttg 1800
ttcggtgcca gccgaccaag attgactagt ttgttggact taaacgacga tttgctgctg 1860
tgaacccagc agggtcgcct ccctctgcat cggccaaatt ggggagggca tggaagatcc 1920
agcggaaagt tgaaaataaa ctggaatgat catcttggct tgggccgctt aggaacaaga 1980
accggagaga agtgattgga aatgaactct tgccctggaa taatcttgac aattaaaact 2040
gatatgttta ctttttttgt attgatcact tttttgcact ccttctttgt tttcaatatt 2100
gtattcagcc tattgtagga gggggatgtg gcgtttcaac tcatataata cagaaagagt 2160
tttgaatggg cagatttcaa actgaatatg ggtccccaaa tgttcccaga gggtcctcca 2220
caccctctgc cgactaccac ggtgtggatt cagctcccaa atgacaaacc cagcccttcc 2280
cagtatactt gaaaagcttt cttgttaaaa taaaaggtgt cactgtggta ggcatttggc 2340
atattttgtg gactcagtca agcaaccaca gtctgttaat catttctcta tgctcagatg 2400
tcagatcctc ttgttattag tgtgtcttgt tctgcacagt gcaggagact ttattccttt 2460
ggaaaattca ctgttccaca aacagcaggc tgaatggcct cgcctctaga ttgacgtggg 2520
ccagcctcct tgagacacac ctggcacccg tcatcggcca gcggtggatg ctgcataatc 2580
cacctgggta cttcagcctt gcgtttccac agccttcagc ctgttctaga acgatcactg 2640
ccttacccct gctgctgcag tggtgtgagt cgtttcacgg ctgatgtccc tcggggggatt 2700
aaaggatcta aagagaaaat ggcacctggt tgtcttcgtg ctgtgtctca tgggtttcca 2760
tagtgataaa gacaaggaaa cgctgcaggg gccacaggca caggctgata tttaaagatc 2820
tttgcttgca gccctccgtc ctgctgaaaa cccccataag ccagtgaaca cagagcagct 2880

agaggctcct cctctgctgg cttagggtca gaagtacctc acagtggttg tggacatgga 2940

agagttttgt caacacaaca ctttgtcccc gctccgggag atgagtcaga tggtggcttg 3000

agttgtcact tggtcccctc cgcccctcgg gtggccccct ttgccacgtc cccttagctt 3060

agtgatcagg tgtgagagtg gccatttcct tacctttgat ccctgtaaag cagaaaggac 3120

tcctttgaca ggcgacaaac tactgtggtg agcagaatga tttccttttt caagacaaca 3180

cctgcctggc ttctattaat gtgtgctggc catgatattg ccccaaatcc gccccactga 3240

agtgttccct aaggaacagc atttctctgc tcctcagtca acccccgtag cctagagcag 3300

tgtcacaagc ttcagtaagg ccagtcagct ggaagtcagt ctaccgtata gtaacactgt 3360

atgtcagtct acagaccaca ctctagttgt tttccatgaa aggtatacaa atgaagaatt 3420

ttctagcaaa acatgttttt aaccatcagt gctcaattgc attttcttcc tttcgcagcc 3480

agtcagtctt tcaaactatt gacagtaaga taattctcac gttcacacct ggtggcaggc 3540

ttcactgtag ggacggacat tgcagttaca ccacgattcc ttcctcttca ctggctcgag 3600

gtaaaccctt ttcaaggaaa aacaactcta ggatttcttt tttctgtgta cgtagaccag 3660

tcccatcagt gtataatctc tctctcacac gcctctctcc aatagacagc ttgtatttgc 3720

agtatttcat atttataaat atgcgtttat ttaaaaggag aacaaaagct tgactctgat 3780

tcacagtttt gtatgtagct ggtttgacgt agtcttttgt attttccctg ccgaagtgaa 3840

ttgttggaga atgtaaaccg cctccacgtg gcggcagact tcctaaggcc ccagctcgct 3900

ggcctcgcgc tgggcggctg ggaattccac ctgagaacaa gtcccgcaaa ccggggacgg 3960

aaggacattt gacttttatt tttgtattta attgacatga atgtaaaggg gacagctcag 4020

ggttgttttg gagcctgttg actttgtatc tctgcctgtg attttctttt ctaaatgaaa 4080

ctccatgtag caaccaggac gaagttgaga aggaaaacgc caaatgcttt ggttattaga 4140

gtttaatagg taagctctgt tacactaggt gttagagttc cagaatgttc ttttgtttgc 4200

taaaccttga agaaacatgt gcctcagcct agatgttttg tcttctcttt tctgcactta 4260

atacctgaca gtatgaccga tctctgcgcc tttctggggg cgggcaagct ggcggtagat 4320

ttgtgatgtc acagtgcaaa ctgcagtgac tgtaaattgg cctggcgtgt ataaacgttt 4380

tcagggaatg cagaaggtat taatgaagag acaaaacctt tattccatgt gctttgcttc 4440

attctgtaca tagctctttg gctcgtgaac ctaattgtaa actttcaggt atttttgtac 4500

aaataaggga ctgatgttct gtttcttgta attagaaata aacattaata cagtgttctt 4560

ca 4562

<210> 18
<211> 1749
<212> DNA

<213> Homo sapiens

<400> 18

```
aggcgggccg cggcggcggc gggcagcgga cgggcggact gacgggcgcc tccaccttgc      60
tccctccctg gctgccggct tccttttgtc tttctgggcg gcgatgagcg cagggccggc     120
gcagcagctg cgggcgcacg gaggcccgcg ctgctagtca ctcctcccgg cctcggcgcg     180
cttgtcccgg gcagcggccc gggcccgctg cagccgccgc cggcgccgaa cttgggctcg     240
ggaagccggc ggaccgcgtc ctgcgccgga gcaggggcat ggtctagtgg cccagtcagg     300
acgcggaaac actccctgga ggttctgacc cactccctct cagcctccgc ctggtctctg     360
gtgtagtcgc cgccgccagc cgccatgggc aaacagaaca gcaagctgcg gcccgaggtg     420
ctgcaggacc tgcgggagaa cacggagttc accgaccacg agctgcagga gtggtacaag     480
ggcttcctca aggactgccc caccggccac ctgaccgtgg acgagttcaa gaagatctac     540
gccaacttct tcccctacgg cgacgcttcc aagttcgccg agcacgtctt ccgcaccttc     600
gacaccaacg gcgacggcac catcgacttc cgggagttca tcattgcgct gagcgtgacc     660
tcgcggggca agctggagca gaagctcaag tgggccttca gcatgtacga cctggacggc     720
aacggctaca tcagccgcag cgagatgctg gagatcgtgc aggccatcta caagatggtg     780
tcgtctgtga tgaagatgcc ggaggatgag tccacccccg agaagcgcac agacaagatc     840
ttcaggcaga tggacaccaa caatgacggc aaactgtcct tggaagaatt catcagaggt     900
gccaagagcg acccctccat cgtccgcctg ctgcagtgcg accccagcag tgccagtcag     960
ttctgagcga gcggcccctg gacagttgca gagaaacaca ggcttgtcgt gccgtttaag    1020
ctttgcttgc aagagtggat gccccgcaat cgttcctgct ctcccgggcc ccgggcctgg    1080
ggcatgcgtt gcacctgccc agcccggtgg ctgcgcctcc ctcctccacc tgaccaacgc    1140
gacattcctc ccctcacgcc tggcccggtc ccttccaggg caactcccag ggatgtggtg    1200
acatgcaggg ttcaagtgtt cttggttcca ggcacctccc ggctcacggg gagctcagag    1260
gtccatgccg aggagaccag gcaggacctc ccgaggctgc gccccggccg gcccatgcgt    1320
tttgtgatcc caagtgactc tgtgggaagg gtggggacga ggcgtcggga gggtatacag    1380
ggagcccctc ccgtgcatgg ctgccccccc gttcattttc tccaccacag ccgcttgcac    1440
gtatagatac tgtggtcccc tttcttttaa tatataaatt atgtatggtg aagtggagtg    1500
tattgtgtag gtcccgtatt taatgcctct gactgccttt gaagcgcagc cctctgtggc    1560
ccgcagcccc ctgagcctgg ctgttgtgtg gtatttatgc tctctttgtc tgcctgtttc    1620
taaggaaatg catgtgtgcc ctgagccgtg atgatcctcc catccgtgtt gtgagcacag    1680
gcatttgtgt ctggtctgtc ctccctgttg attggtctgg catttccggt attaaaatga    1740
taaaataaa                                                            1749
```

<210> 19
<211> 1896
<212> DNA
<213> Homo sapiens

<400> 19
gaaagcgagt gtccctctcg cgccccaggc cggtgtaccc ccgcactccg cgccccggcc 60

tagaagctct ctctccccgc tccccggccc ggcccccgcc ccgccccgcc ccagcccgct 120

gggccgccat ggagcgctgg ccttggccgt cgggcggcgc ctggctgctc gtggctgccc 180

gcgcgctgct gcagctgctg cgctcagacc tgcgtctggg ccgcccgctg ctggcggcgc 240

tggcgctgct ggccgcgctc gactggctgt gccagcgcct gctgccccccg ccggccgcac 300

tcgccgtgct ggccgccgcc ggctggatcg cgttgtcccg cctggcgcgc ccgcagcgcc 360

tgccggtggc cactcgcgcg gtgctcatca ccggctgtga ctctggtttt ggcaaggaga 420

cggccaagaa actggactcc atgggcttca cggtgctggc caccgtattg gagttgaaca 480

gccccggtgc catcgagctg cgtacctgct gctcccctcg cctaaggctg ctgcagatgg 540

acctgaccaa accaggagac attagccgcg tgctagagtt caccaaggcc cacaccacca 600

gcaccggcct gtggggcctc gtcaacaacg caggccacaa tgaagtagtt gctgatgcgg 660

agctgtctcc agtggccact ttccgtagct gcatggaggt gaatttcttt ggcgcgctcg 720

agctgaccaa gggcctcctg cccctgctgc gcagctcaag gggccgcatc gtgactgtgg 780

ggagcccagc gggggacatg ccatatccgt gcttgggggc ctatggaacc tccaaagcgg 840

ccgtggcgct actcatggac acattcagct gtgaactcct tccctggggg gtcaaggtca 900

gcatcatcca gcctggctgc ttcaagacag agtcagtgag aaacgtgggt cagtgggaaa 960

agcgcaagca attgctgctg gccaacctgc ctcaagagct gctgcaggcc tacggcaagg 1020

actacatcga gcacttgcat gggcagttcc tgcactcgct acgcctggcc atgtccgacc 1080

tcaccccagt tgtagatgcc atcacagatg cgctgctggc agctcggccc cgccgccgct 1140

attaccccgg ccagggcctg gggctcatgt acttcatcca ctactacctg cctgaaggcc 1200

tgcggcgccg cttcctgcag gccttcttca tcagtcactg tctgcctcga gcactgcagc 1260

ctggccagcc tggcactacc ccaccacagg acgcagccca ggacccaaac ctgagccccg 1320

gcccttcccc agcagtggct cggtgagcca tgtgcaccta tggcccagcc actgcagcac 1380

aggaggctcc gtgagccctt ggttcctccc cgaaaacccc cagcattacg atcccccaag 1440

tgtcctggac cctggcctaa agaatcccac ccccacttca tgcccactgc cgatgcccaa 1500

tccaggcccg gtgaggccaa ggtttcccag tgagcctctg cgcctctcca ctgtttcatg 1560

agcccaaaca ccctcctggc acaacgctct accctgcagc ttggagaact ccgctggatg 1620

gggagtctca tgcaagactt cactgcagcc tttcacagga ctctgcagat agtgcctctg 1680

caaactaagg agtgactagg tgggttgggg accccctcag gattgtttct cggcaccagt 1740

gcctcagtgc tgcaattgag ggctaaatcc caagtgtctc ttgactggct caagaattag 1800

ggccccaact acacaccccc aagccacagg gaagcatgta ctgtacttcc caattgccac 1860

attttaaata aagacaaatt tttatttctt ctaaaa 1896

<210> 20
<211> 5213
<212> DNA
<213> Homo sapiens

<400> 20
acagctgtcg cccaccgcgg gtgagaggct tgctggtccg ccccggcttc cccgccccgg 60

cggcggccgt gcggttcggg ctttaattct ggcgcttgcc agccggggcc gactccgccc 120

cctgcgcccc gcccttcccc gagctccggg tccgcggcgg agcgagcgag cggccggcag 180

gttgggagga gcggccgaag gttgaatgcc tttgagaact tgatgcataa aatttgcatg 240

actcctcact cctttctgga tctctcattg gactcaagcc agcatggctc acaccacaaa 300

ggttaacggc agtgcctcag gaaaagcagg tgatattctc agtggagacc aggacaagga 360

acagaaagac ccttactttg tggagacccc ctatggttat caactagact tagatttcct 420

caaatatgtg gatgacatac agaagggaaa taccatcaaa agactgaaca tccagaagag 480

gcggaagccg tccgtgccat gcccagaacc caggaccaca tctggtcagc aaggtatatg 540

gacttccact gaatccctct catcctccaa cagtgatgac aacaagcagt gccccaactt 600

cctcatagcc agaagtcaag ttacatcaac tccaatctca aagccacctc cccctctgga 660

gacctcactc ccttttctta ccatcccaga aaatcgacag ctgccacctc cctcaccaca 720

actcccaaag cataaccttc atgtcaccaa gacactgatg gagacccgga gaagactgga 780

acaggagaga gccaccatgc agatgacacc gggtgagttc agaaggccca ggctggccag 840

ttttggaggc atgggcacca caagctccct cccttctttt gtgggttctg gaaaccacaa 900

tcctgccaag caccagcttc agaatggata ccaaggtaat ggggattatg gtagctatgc 960

cccagctgct cccaccactt cctccatggg gagctccatc cgccacagcc ccctgagctc 1020

agggatctcc accccagtga ccaacgtgag ccccatgcac ctgcagcaca tccgcgagca 1080

gatggccatt gctctgaaac gcctgaagga gctggaggag caggtgcgaa ccatccctgt 1140

gctccaggta aagatctctg tcttgcaaga agagaaaagg cagttggtct cacagctgaa 1200

aaaccaaagg gctgcatccc agatcaatgt ctgtggtgtg aggaagcggt cctatagtgc 1260

ggggaacgcc tcccagctgg aacagctctc ccgggcccga agaagtggcg gggaattata 1320

cattgactat gaggaggaag aaatggagac cgtagaacag agcacgcaga ggataaagga 1380

gttccggcaa cttacagcag acatgcaagc cctggagcag aagatccagg acagcagctg 1440

tgaggcctcc tcagagctca gggagaatgg agagtgccgg tctgtggctg tgggtgccga 1500

ggagaacatg aacgacatcg tcgtgtacca cagaggctcc aggtcctgta aggatgcagc 1560

tgtagggaca cttgttgaga tgagaaattg tggggtcagc gtgacagagg ccatgcttgg 1620

agtgatgact gaagctgaca aagaaattga gctgcaacag cagaccatag aatccttgaa 1680

ggaaaagatc tatcgcctag aagtacagct tagagaaacc acccatgacc gggagatgac 1740

taaactgaaa caagagctgc aggctgctgg atcgaggaaa aaggttgaca aagccacgat 1800

ggcccagccg cttgttttca gtaaggtggt ggaggcagtg gtgcagacca gagaccaaat 1860

ggtcggcagt cacatggacc tggtggacac gtgtgttggg acctccgtgg aaacaaacag 1920

tgtaggcatc tcctgccagc ctgaatgtaa gaataaagtc gtagggcctg agctgcctat 1980

gaattggtgg attgttaagg agagggtgga aatgcatgac cgatgtgctg ggaggtctgt 2040

ggaaatgtgt gacaagagtg tgagtgtgga agtcagcgtc tgcgaaacag gcagcaacac 2100

agaggagtct gtgaacgacc tcacactcct caagacaaac ttgaatctca aagaagtgcg 2160

gtctatcggt tgtggagatt gttctgttga cgtgaccgtc tgctctccaa aggagtgcgc 2220

ctcccggggc gtgaacactg aggctgttag ccaggtggaa gctgccgtca tggcagtgcc 2280

tcgtactgca gaccaggaca ctagcacaga tttggaacag gtgcaccagt tcaccaacac 2340

cgagacggcc accctcatag agtcctgcac caacacttgt ctaagcactt tggacaagca 2400

gaccagcacc cagactgtgg agacgcggac agtagctgta ggagaaggcc gtgtcaagga 2460

catcaactcc tccaccaaga cgcggtccat tggtgttgga acgttgcttt ctggccattc 2520

tgggtttgac aggccatcag ctgtgaagac caaagagtca ggtgtggggc agataaatat 2580

taacgacaac tatctggttg gtctcaaaat gaggactata gcttgtgggc caccacagtt 2640

gactgtgggg ctgacagcca gcagaaggag cgtgggggtt ggggatgacc ctgtagggga 2700

atctctggag aaccccccagc ctcaagctcc acttggaatg atgactggcc tggatcacta 2760

cattgagcgt atccagaagc tgctggcaga acagcagaca ctgctggctg agaactacag 2820

tgaactggca gaagctttcg gggaacctca ctcacagatg ggctccctca actctcagct 2880

catcagcacc ctgtcgtcta tcaactctgt catgaaatct gcaagcactg aagagctgag 2940

gaaccctgac ttccagaaaa ccagtctggg taaaatcaca ggcaattatt tgggatatac 3000

ctgtaagtgt gggggccttc agtcaggaag tcccttaagc tcccagacat cccagcctga 3060

gcaagaagtg gggacctcag aaggaaagcc aatcagcagc ctggatgcct tccccactca 3120

ggaaggtacg ctgtctccag tgaacctgac agacgaccag atcgccgctg gcctctatgc 3180

atgtacaaac aatgaaagta cactgaagtc catcatgaag aagaaagatg gtaacaaaga 3240

ttcaaatggc gcaaaaaaga atcttcagtt tgttggcatt aatggagggt atgaaacaac 3300

ttcaagtgat gattccagct cagatgaaag ctcttcttcc gagtcagatg acgagtgtga 3360

tgtcattgag tatcctcttg aagaagagga ggaggaggag gatgaagaca ctcggggaat 3420
ggcagaaggg caccatgcag ttaatattga aggtttgaag tctgccaggg tggaagatga 3480
aatgcaggtt caagaatgtg aacctgagaa ggtggaaatc agagagaggt atgaattaag 3540
tgaaaagatg ttgtctgcat gcaacttact gaaaaatact ataaatgacc ccaaagcttt 3600
gaccagcaaa gatatgaggt tctgtctgaa caccctccag cacgagtggt tccgcgtgtc 3660
cagtcagaag tcagccattc cagccatggt gggggactac atagctgctt ttgaggccat 3720
ttccccagat gtcctccgct atgtcatcaa cttggcagac ggcaacggca acacagccct 3780
ccattacagc gtgtcccact ccaacttcga gattgtgaag ctgctgttag atgccgatgt 3840
gtgtaatgtg gatcaccaga acaaggcagg ctacaccccc atcatgttgg cggccctcgc 3900
cgctgtggaa gcagagaagg acatgcggat tgtggaagaa ctcttcggct gtggggatgt 3960
gaatgccaaa gctagtcagg cgggacagac ggccctcatg ctggcggtca gtcacggacg 4020
gatagacatg gtgaagggcc ttctggcctg tggggctgat gtcaacatcc aggatgacga 4080
gggctccacg gccctcatgt gtgccagcga gcacgggcac gtggagattg tcaagctgct 4140
gctggcccag cccggctgca acggtcacct agaggacaac gatggcagca ctgcgctctc 4200
aatcgccctg gaagcaggac acaaggacat cgctgttctt ctgtatgccc atgtcaactt 4260
tgcaaaagcc cagtctccgg gcacccctag gcttggaagg aagacgtctc ctggccccac 4320
ccaccgaggt tcatttgatt gattgtatgc aaatagccct ttatttacat gccactatta 4380
agctgctaat tgttcctgtt ggggtgacag atactgaatg tatacgtatt gtgcctgagc 4440
tcaccagcaa acagaagcat caagcccagg ggtaaaggct gaagctttca cagtgcagag 4500
actgctagcc tgggcacaca cacctccttt ctggccgtct tctgtgtagg gcacacttta 4560
acccagtctc tgttgctgtt gagtctctgc tccgttttgt acagtcacag ggaattctga 4620
tctgaagggg caccttctgt tcactcccac aaagtggtgt ctggttctca ctgagacgtt 4680
ttaagatttt tccacaaata tttatatgta ctaaatgtgg aaccattaga aagttcttcc 4740
aaaatctcat tccagcatag ttttggattt ttcttttgtc ttattttaaa ataaggaagt 4800
cgagatgact ttgatcattg gtaacttggg cctgggccag acaaagtata aaacttacaa 4860
aagaatattc tcatttggtc ttaactaggt agatgtaata tatgactttt tataaaaagg 4920
gtatctatat gaacttgaca cagtattttc agcttttgta ttccatacta aagccatgaa 4980
gaactacacg taacatcatc atttgtatta attgcacaac tccaatgcta aaggttggat 5040
tgtgttagag gaatcggctc tgtatttgcc tctagagaaa cacagtgttc tctttgtatt 5100
tatggattcc tttttaccgt gtcacattta ctttggtcct ctatgtattt aaatgtttga 5160
agtgccttag actcttgcca tattttcaaa ataaaattcc attaagctct ttt 5213

<210> 21
<211> 2936
<212> DNA
<213> Homo sapiens

<400> 21
ggcagtttcc cgaccagaga gaacgaacgt gtctgcgggc gcgcggggag cagaggcggt 60
ggcgggcggc ggcggcaccg ggagccgccg agtgaccctc ccccgcccct ctggcccccc 120
accctcccac ccgcccgtgg cccgcgccca tggccgcgcg cgctccacac aactcaccgg 180
agtccgcgcc ttgcgccgcc gaccagttcg cagctccgcg ccacggcagc cagtctcacc 240
tggcggcacc gcccgcccac cgccccggcc acagcccctg cgcccacggc agcactcgag 300
gcgaccgcga cagtggtggg ggacgctgct gagtggaaga gagcgcagcc cggccaccgg 360
acctacttac tcgccttgct gattgtctat ttttgcgttt acaacttttc taagaacttt 420
tgtatacaaa ggaacttttt aaaaaagacg cttccaagtt atatttaatc caaagaagaa 480
ggatctcggc caatttgggg ttttgggttt tggcttcgtt tcttctcttc gttgactttg 540
gggttcaggt gccccagctg cttcgggctg ccgaggacct tctgggcccc cacattaatg 600
aggcagccac ctggcgagtc tgacatggct gtcagcgacg cgctgctccc atctttctcc 660
acgttcgcgt ctggcccggc gggaagggag aagacactgc gtcaagcagg tgccccgaat 720
aaccgctggc gggaggagct ctcccacatg aagcgacttc cccagtgct tcccggccgc 780
ccctatgacc tggcggcggc gaccgtggcc acagacctgg agagcggcgg agccggtgcg 840
gcttgcggcg gtagcaacct ggcgccccta cctcggagag agaccgagga gttcaacgat 900
ctcctggacc tggactttat tctctccaat tcgctgaccc atcctccgga gtcagtggcc 960
gccaccgtgt cctcgtcagc gtcagcctcc tcttcgtcgt cgccgtcgag cagcggccct 1020
gccagcgcgc cctccacctg cagcttcacc tatccgatcc gggccgggaa cgacccgggc 1080
gtggcgccgg gcggcacggg cggaggcctc ctctatggca gggagtccgc tccccctccg 1140
acggctccct tcaacctggc ggacatcaac gacgtgagcc cctcgggcgg cttcgtggcc 1200
gagctcctgc ggccagaatt ggacccggtg tacattccgc cgcagcagcc gcagccgcca 1260
ggtggcgggc tgatgggcaa gttcgtgctg aaggcgtcgc tgagcgcccc tggcagcgag 1320
tacggcagcc cgtcggtcat cagcgtcagc aaaggcagcc ctgacggcag ccacccggtg 1380
gtggtggcgc cctacaacgg cgggccgccg cgcacgtgcc ccaagatcaa gcaggaggcg 1440
gtctcttcgt gcacccactt gggcgctgga ccccctctca gcaatggcca ccggccggct 1500
gcacacgact tcccccctggg gcggcagctc cccagcagga ctaccccgac cctgggtctt 1560
gaggaagtgc tgagcagcag ggactgtcac cctgccctgc cgcttcctcc cggcttccat 1620
ccccacccgg ggcccaatta cccatccttc ctgcccgatc agatgcagcc gcaagtcccg 1680

ccgctccatt accaagagct catgccaccc ggttcctgca tgccagagga gcccaagcca 1740

aagaggggaa gacgatcgtg gccccggaaa aggaccgcca cccacacttg tgattacgcg 1800

ggctgcggca aaacctacac aaagagttcc catctcaagg cacacctgcg aacccacaca 1860

ggtgagaaac cttaccactg tgactgggac ggctgtggat ggaaattcgc ccgctcagat 1920

gaactgacca ggcactaccg taaacacacg gggcaccgcc cgttccagtg ccaaaaatgc 1980

gaccgagcat tttccaggtc ggaccacctc gccttacaca tgaagaggca tttttaaatc 2040

ccagacagtg gatatgaccc acactgccag aagagaattc agtatttttt acttttcaca 2100

ctgtcttccc gatgagggaa ggagcccagc cagaaagcac tacaatcatg gtcaagttcc 2160

caactgagtc atcttgtgag tggataatca ggaaaaatga ggaatccaaa agacaaaaat 2220

caaagaacag atggggtctg tgactggatc ttctatcatt ccaattctaa atccgacttg 2280

aatattcctg gacttacaaa atgccaaggg ggtgactgga agttgtggat atcagggtat 2340

aaattatatc cgtgagttgg gggagggaag accagaattc ccttgaattg tgtattgatg 2400

caatataagc ataaaagatc accttgtatt ctctttacct tctaaaagcc attattatga 2460

tgttagaaga agaggaagaa attcaggtac agaaaacatg tttaaatagc ctaaatgatg 2520

gtgcttggtg agtcttggtt ctaaaggtac caaacaagga agccaaagtt ttcaaactgc 2580

tgcatacttt gacaaggaaa atctatattt gtcttccgat caacatttat gacctaagtc 2640

aggtaatata cctggtttac ttctttagca tttttatgca gacagtctgt tatgcactgt 2700

ggtttcagat gtgcaataat ttgtacaatg gtttattccc aagtatgcct taagcagaac 2760

aaatgtgttt ttctatatag ttccttgcct taataaatat gtaatataaa tttaagcaaa 2820

cgtctatttt gtatatttgt aaactacaaa gtaaaatgaa cattttgtgg agtttgtatt 2880

ttgcatactc aaggtgagaa ttaagtttta aataaaccta taatatttta tctgaa 2936

<210> 22
<211> 2152
<212> DNA
<213> Homo sapiens

<400> 22
ggggagctac gtagggcaga gaagtcatgg cttctccgtc caaaggcaat gacttgtttt 60

cgccctacga ggagggccca gcagtggtgg ccggaccagg ccggggctgg ggcgccgagg 120

gggccgcgga ggagcgccgc gtcaaggtct ccagcctgcc cttcagcgtg gaggcgctca 180

tgtccgacaa gaagccgccc aaggaggcgt ccccgctgcc ggccgaaagc gcctcggccg 240

gggccacccct gcggccactg ctgctgtcgg ggcacggcgc tcgggaagcg cacagccccg 300

ggccgctggt gaagcccttc gagaccgcct cggtcaagtc ggaaaattca gaagatggag 360

cggcgtggat gcaggaaccc ggccgatatt cgccgccgcc aagacatacg agccctacca 420

```
cctgcaccct gaggaaacac aagaccaatc ggaagccgcg cacgcccttt accacatccc      480
agctcctcgc cctggagcgc aagttccgtc agaaacagta cctctccatt gcagagcgtg      540
cagagttctc cagctctctg aacctcacag agacccaggt caaaatctgg ttccagaacc      600
gaagggccaa ggcgaaaaga ctgcaggagg cagaactgga aaagctgaaa atggctgcaa      660
aacctatgct gccctccagc ttcagtctcc ctttccccat cagctcgccc ctgcaggcag      720
cgtccatata tggagcatcc tacccgttcc atagacctgt gcttcccatc ccgcctgtgg      780
gactctatgc cacgccagtg ggatatggca tgtaccacct gtggtaagga agaccagatc      840
aatagactcc atgatggatg cttgtttcaa agggtttcct ctccctctcc acgaaggcag      900
taccagccag tactcctgct ctgctaaccc tgcgtgcacc accctaagcg gctaggctga      960
cagggccaca ctacatagct gaaatttgtt ctgtaggcgg aggcaccaag ccctgttttc     1020
ttggtgtaat cttccagatg cccccttttc ctttcacaaa gattggctct gatggttttt     1080
atgtataaat atatatatat aataaaatat aatacatttt atacagcaga cgtaaaaatt     1140
caaattattt taaaaggcaa aatttatata catatgtgct ttttttctat atctcacctt     1200
cccaaaagac actgtgtaag tccatttgtt gtattttctt aaagagggag acaaattatt     1260
tgcaaaatgt gctaaagtca atgattttta cgggattatt gacttctgct tatggaaaac     1320
aaagaaacag acacagtgca cacagaaaat attagatatg gagagattat tcaaagtgaa     1380
ggggacacat catatttctg cattttactt gcattaaaag aaacctcttt atatactaca     1440
gttgttccta tctctccccc cgcccccccac cgccccacca cacacatatt tttaaagttt     1500
ttcctttttt aagaatattt ttgtaagacc aatacctggg atgagaagaa tcctgagact     1560
gcctggaggt gaggtagaaa attagaaata cttcctaatt cttctcaagg ctgttggtaa     1620
ctttatttca gataattgga gagtaaaatg ttaaaacctg ttgagaggaa ttgatggttt     1680
ctgagaaata ctaggtacat tcatcctcac agattgcaaa ggtgatttgg gtgggggttt     1740
agtaattttc tgcttaaaaa atgagtatct tgtaaccatt acctatatgc taaatattct     1800
tgaacaatta gtagatccag aaagaaaaaa aaaatatgct ttctctgtgt gtgtacctgt     1860
tgtatgtcct aaacttatta gaaaatttta tatacttttt tacatgttgg ggggcagaag     1920
gtaaagccat gttttgactt ggtgaaaatg gggttgtcaa acagcccatt aagctccctg     1980
gtatttcacc tcctgtccat ctctcccctc cctccggtat acctttatcc ctttgaaagg     2040
gtgcttgtac aatttgatat attttattga agagttatct cttattctga attaaattaa     2100
gcatttgttt tattgcagta aagtttgtcc aaactcaaaa aaaaaaaaaa aa             2152
```

<210> 23
<211> 1199
<212> DNA
<213> Homo sapiens

<400> 23
acctctcaag cagccagcgc ctgcctgaat ctgttctgcc ccctccccac ccatttcacc 60
accaccatga caccgggcac ccagtctcct ttcttcctgc tgctgctcct cacagtgctt 120
acagttgtta cgggttctgg tcatgcaagc tctaccccag gtggagaaaa ggagacttcg 180
gctacccaga gaagttcagt gcccagctct actgagaaga atgctttgtc tactggggtc 240
tctttctttt tcctgtcttt tcacatttca aacctccagt ttaattcctc tctggaagat 300
cccagcaccg actactacca agagctgcag agagacattt ctgaaatgtt tttgcagatt 360
tataaacaag gggttttct gggcctctcc aatattaagt tcaggccagg atctgtggtg 420
gtacaattga ctctggcctt ccgagaaggt accatcaatg tccacgacgt ggagacacag 480
ttcaatcagt ataaaacgga agcagcctct cgatataacc tgacgatctc agacgtcagc 540
gtgagtgatg tgccatttcc tttctctgcc cagtctgggg ctggggtgcc aggctggggc 600
atcgcgctgc tggtgctggt ctgtgttctg gttgcgctgg ccattgtcta tctcattgcc 660
ttggctgtct gtcagtgccg ccgaaagaac tacgggcagc tggacatctt tccagcccgg 720
gatacctacc atcctatgag cgagtacccc acctaccaca cccatgggcg ctatgtgccc 780
cctagcagta ccgatcgtag cccctatgag aaggtttctg caggtaatgg tggcagcagc 840
ctctcttaca caaacccagc agtggcagcc acttctgcca acttgtaggg gcacgtcgcc 900
cgctgagctg agtggccagc cagtgccatt ccactccact caggttcttc agggccagag 960
cccctgcacc ctgtttgggc tggtgagctg ggagttcagg tgggctgctc acagcctcct 1020
tcagaggccc caccaatttc tcggacactt ctcagtgtgt ggaagctcat gtgggcccct 1080
gagggctcat gcctgggaag tgttgtggtg gggctccca ggaggactgg cccagagagc 1140
cctgagatag cggggatcct gaactggact gaataaaacg tggtctccca ctgcgccaa 1199

<210> 24
<211> 4515
<212> DNA
<213> Homo sapiens

<400> 24
ggagtttatt cataacgcgc tctccaagta tacgtggcaa tgcgttgctg ggttatttta 60
atcattctag gcatcgtttt cctccttatg cctctatcat tcctccctat ctacactaac 120
atcccacgct ctgaacgcgc gcccattaat acccttcttt cctccactct ccctgggact 180
cttgatcaaa gcgcggccct ttccccagcc ttagcgaggc gccctgcagc ctggtacgcg 240
cgtggcgtgg cggtgggcgc gcagtgcgtt ctcggtgtgg agggcagctg ttccgcctgc 300
gatgatttat actcacagga caaggatgcg gtttgtcaaa cagtactgct acggaggagc 360
agcagagaaa gggagagggt ttgagaggga gcaaaagaaa atggtaggcg cgcgtagtta 420

attcatgcgg ctctcttact ctgtttacat cctagagcta gagtgctcgg ctgcccggct 480
gagtctcctc cccaccttcc ccaccctccc caccctcccc ataagcgccc ctcccgggtt 540
cccaaagcag agggcgtggg ggaaaagaaa aaagatcctc tctcgctaat ctccgcccac 600
cggcccttta taatgcgagg gtctggacgg ctgaggaccc ccgagctgtg ctgctcgcgg 660
ccgccaccgc cgggccccgg ccgtccctgg ctcccctcct gcctcgagaa gggcagggct 720
tctcagaggc ttggcgggaa aaagaacgga gggagggatc gcgctgagta taaaagccgg 780
ttttcggggc tttatctaac tcgctgtagt aattccagcg agaggcagag ggagcgagcg 840
ggcggccggc tagggtggaa gagccgggcg agcagagctg cgctgcgggc gtcctgggaa 900
gggagatccg gagcgaatag ggggcttcgc ctctggccca gccctcccgc tgatccccca 960
gccagcggtc cgcaaccctt gccgcatcca cgaaactttg cccatagcag cgggcgggca 1020
ctttgcactg gaacttacaa cacccgagca aggacgcgac tctcccgacg cggggaggct 1080
attctgccca tttggggaca cttccccgcc gctgccagga cccgcttctc tgaaaggctc 1140
tccttgcagc tgcttagacg ctggattttt ttcgggtagt ggaaaaccag cctcccgcga 1200
cgatgcccct caacgttagc ttcaccaaca ggaactatga cctcgactac gactcggtgc 1260
agccgtattt ctactgcgac gaggaggaga acttctacca gcagcagcag cagagcgagc 1320
tgcagccccc ggcgcccagc gaggatatct ggaagaaatt cgagctgctg cccaccccgc 1380
ccctgtcccc tagccgccgc tccgggctct gctcgccctc ctacgttgcg gtcacaccct 1440
tctcccttcg gggagacaac gacggcggtg gcgggagctt ctccacggcc gaccagctgg 1500
agatggtgac cgagctgctg ggaggagaca tggtgaacca gagtttcatc tgcgacccgg 1560
acgacgagac cttcatcaaa aacatcatca tccaggactg tatgtggagc ggcttctcgg 1620
ccgccgccaa gctcgtctca gagaagctgg cctcctacca ggctgcgcgc aaagacagcg 1680
gcagcccgaa ccccgcccgc ggccacagcg tctgctccac ctccagcttg tacctgcagg 1740
atctgagcgc cgccgcctca gagtgcatcg acccctcggt ggtcttcccc taccctctca 1800
acgacagcag ctcgcccaag tcctgcgcct cgcaagactc cagcgccttc tctccgtcct 1860
cggattctct gctctcctcg acggagtcct ccccgcaggg cagccccgag cccctggtgc 1920
tccatgagga gacaccgccc accaccagca gcgactctga ggaggaacaa gaagatgagg 1980
aagaaatcga tgttgtttct gtggaaaaga ggcaggctcc tggcaaaagg tcagagtctg 2040
gatcaccttc tgctggaggc cacagcaaac ctcctcacag cccactggtc ctcaagaggt 2100
gccacgtctc cacacatcag cacaactacg cagcgcctcc ctccactcgg aaggactatc 2160
ctgctgccaa gagggtcaag ttggacagtg tcagagtcct gagacagatc agcaacaacc 2220
gaaaatgcac cagccccagg tcctcggaca ccgaggagaa tgtcaagagg cgaacacaca 2280
acgtcttgga gcgccagagg aggaacgagc taaaacggag ctttttgcc ctgcgtgacc 2340

agatcccgga gttggaaaac aatgaaaagg cccccaaggt agttatcctt aaaaaagcca 2400
cagcatacat cctgtccgtc caagcagagg agcaaaagct catttctgaa gaggacttgt 2460
tgcggaaacg acgagaacag ttgaaacaca aacttgaaca gctacggaac tcttgtgcgt 2520
aaggaaaagt aaggaaaacg attccttcta acagaaatgt cctgagcaat cacctatgaa 2580
cttgtttcaa atgcatgatc aaatgcaacc tcacaacctt ggctgagtct tgagactgaa 2640
agatttagcc ataatgtaaa ctgcctcaaa ttggactttg ggcataaaag aacttttta 2700
tgcttaccat cttttttttt tctttaacag atttgtattt aagaattgtt tttaaaaaat 2760
tttaagattt acacaatgtt tctctgtaaa tattgccatt aaatgtaaat aactttaata 2820
aaacgtttat agcagttaca cagaatttca atcctagtat atagtaccta gtattatagg 2880
tactataaac cctaattttt tttatttaag tacattttgc tttttaaagt tgattttttt 2940
ctattgtttt tagaaaaaat aaaataactg gcaaatatat cattgagcca aatcttaagt 3000
tgtgaatgtt ttgtttcgtt tcttcccccct cccaaccacc accatccctg tttgttttca 3060
tcaattgccc cttcagaggg tggtcttaag aaaggcaaga gttttcctct gttgaaatgg 3120
gtctgggggc cttaaggtct ttaagttctt ggaggttcta agatgcttcc tggagactat 3180
gataacagcc agagttgaca gttagaagga atggcagaag gcaggtgaga aggtgagagg 3240
taggcaaagg agatacaaga ggtcaaaggt agcagttaag tacacaaaga ggcataagga 3300
ctggggagtt gggaggaagg tgaggaagaa actcctgtta ctttagttaa ccagtgccag 3360
tcccctgctc actccaaacc caggaattct gcccagttga tggggacacg gtgggaacca 3420
gcttctgctg ccttcacaac caggcgccag tcctgtccat gggttatctc gcaaacccca 3480
gaggatctct gggaggaatg ctactattaa ccctatttca caaacaagga aatagaagag 3540
ctcaaagagg ttatgtaact tatctgtagc cacgcagata atacaaagca gcaatctgga 3600
cccattctgt tcaaaacact taacccttcg ctatcatgcc ttggttcatc tgggtctaat 3660
gtgctgagat caagaaggtt taggacctaa tggacagact caagtcataa caatgctaag 3720
ctctatttgt gtcccaagca ctcctaagca ttttatccct aactctacat caaccccatg 3780
aaggagatac tgttgatttc cccatattag aagtagagag ggaagctgag gcacacaaag 3840
actcatccac atgcccaaga ttcactgata gggaaaagtg gaagcgagat ttgaacccag 3900
gctgtttact cctaacctgt ccaagccacc tctcagacga cggtaggaat cagctggctg 3960
cttgtgagta caggagttac agtccagtgg gttatgtttt ttaagtctca acatctaagc 4020
ctggtcaggc atcagttccc cttttttgt gatttatttt gtttttattt tgttgttcat 4080
tgtttaattt ttccttttac aatgagaagg tcaccatctt gactcctacc ttagccattt 4140
gttgaatcag actcatgacg gctcctggga agaagccagt tcagatcata aaataaaaca 4200

tatttattct ttgtcatggg agtcattatt ttagaaacta caaactctcc ttgcttccat 4260

cctttttttac atactcatga cacatgctca tcctgagtcc ttgaaaaggt atttttgaac 4320

atgtgtatta attataagcc tctgaaaacc tatggcccaa accagaaatg atgttgatta 4380

tataggtaaa tgaaggatgc tattgctgtt ctaattacct cattgtctca gtctcaaagt 4440

aggtcttcag ctccctgtac tttgggattt taatctacca ccacccataa atcaataaat 4500

aattactttc tttga 4515

<210> 25
<211> 4522
<212> DNA
<213> Homo sapiens

<400> 25
agagacttga gggaggcgct gcgactgaca agcggctctg cccgggacct tctcgctttc 60

atctagcgct gcactcaatg gaggggcggg caccgcagtg cttaatgctg tcttaactag 120

tgtaggaaaa cggctcaacc caccgctgcc gaaatgaagt ataagaatct tatggcaagg 180

gccttatatg acaatgtccc agagtgtgcc gaggaactgg cctttcgcaa gggagacatc 240

ctgaccgtca tagagcagaa cacaggggga ctggaaggat ggtggctgtg ctcattacac 300

ggtcggcaag gcattgtccc aggcaaccgg gtgaagcttc tgattggtcc catgcaggag 360

actgcctcca gtcacgagca gcctgcctct ggactgatgc agcagacctt tggccaacag 420

aagctctatc aagtgccaaa cccacaggct gctccccgag acaccatcta ccaagtgcca 480

ccttcctacc aaaatcaggg aatttaccaa gtccccactg gccacggcac ccaagaacaa 540

gaggtatatc aggtgccacc atcagtgcag agaagcattg gggaaccag tgggccccac 600

gtgggtaaaa aggtgataac cccgtgagg acaggccatg gctacgtata cgagtaccca 660

tccagatacc aaaaggacgt ctatgatatc cctccttctc ataccactca aggggtatac 720

gacatccctc cctcatcagc aaaaggccct gtgttttcag ttccagtggg agagataaaa 780

cctcaagggg tgtatgacat cccgcctaca aaaggggtat atgccattcc gccctctgct 840

tgccgggatg aagcagggct tagggaaaaa gactatgact tcccccctcc catgagacaa 900

gctggaaggc cggacctcag accggagggg gtttatgaca ttcctccaac ctgcaccaag 960

ccagcaggga aggaccttca tgtaaaatac aactgtgaca ttccaggagc tgcagaaccg 1020

gtggctcgaa ggcaccagag cctgtccccg aatcacccac ccccgcaact cggacagtca 1080

gtgggctctc agaacgacgc atatgatgtc ccccgaggcg ttcagtttct tgagccacca 1140

gcagaaacca gtgagaaagc aaacccccag gaaagggatg gtgtttatga tgtccctctg 1200

cataacccgc cagatgctaa aggctctcgg gacttggtgg atgggatcaa ccgattgtct 1260

ttctccagta caggcagcac ccggagtaac atgtccacgt cttccacctc ctccaaggag 1320

tcctcactgt cagcctcccc agctcaggac aaaaggctct tcctggatcc agacacagct 1380
attgagagac ttcagcggct ccagcaggcc cttgagatgg gtgtctccag cctaatggca 1440
ctggtcacta ccgactggcg gtgttacgga tatatggaaa gacacatcaa tgaaatacgc 1500
acagcagtgg acaaggtgga gctgttcctg aaggagtacc tccactttgt caagggagct 1560
gttgcaaatg ctgcctgcct cccggaactc atcctccaca acaagatgaa gcgggagctg 1620
caacgagttg aagactccca ccagatcctg agtcaaacca gccatgactt aaatgagtgc 1680
agctggtccc tgaatatctt ggccatcaac aagccccaga acaagtgtga cgatctggac 1740
cggtttgtga tggtggcaaa gacggtgccc gatgacgcca agcagctcac cacaaccatc 1800
aacaccaacg cagaggccct cttcagaccc ggccctggca gcttgcatct gaagaatggg 1860
ccggagagca tcatgaactc aacggagtac ccacacggtg gctcccaggg acagctgctg 1920
catcctggtg accacaaggc ccaggcccac aacaaggcac tgcccccagg cctgagcaag 1980
gagcaggccc ctgactgtag cagcagtgat ggttctgaga ggagctggat ggatgactac 2040
gattacgtcc acctacaggg taaggaggag tttgagaggc aacagaaaga gctattggaa 2100
aaagagaata tcatgaaaca gaacaagatg cagctggaac atcatcagct gagccagttc 2160
cagctgttgg aacaagagat tacaaagccc gtggagaatg acatctcgaa gtggaagccc 2220
tctcagagcc tacccaccac aaacagtggc gtgagtgctc aggatcggca gttgctgtgc 2280
ttctactatg accaatgtga gacccatttc atttcccttc tcaacgccat tgacgcactc 2340
ttcagttgtg tcagctcagc ccagcccccg cgaatcttcg tggcacacag caagtttgtc 2400
atcctcagtg cacacaaact ggtgttcatt ggagacacgc tgacacggca ggtgactgcc 2460
caggacattc gcaacaaagt catgaactcc agcaaccagc tctgcgagca gctcaagacc 2520
atagtcatgg caaccaagat ggccgccctc cattacccca gcaccacggc cctgcaggaa 2580
atggtgcacc aagtgacaga cctttctaga aatgcccagc tgttcaagcg ctctttgctg 2640
gagatggcaa cgttctgaga agaaaaaaaa gaggaagggg actgcgttaa cggttactaa 2700
ggaaaactgg aaatactgtc tggtttttgt aaatgttatc tatttttgta gatattttat 2760
ataaaaatga aatattttaa cattttatgg gtcagtcaac tttcagaaat tcagggagct 2820
ggagagggaa atcttttttt ttcccccctga gtggttctta tgtatacata gaagtatctg 2880
agacataaac tgtacagaaa acttgtccac gtgcttttgt atgcccatgt attcatgttt 2940
gtttgtagat gtttgtctga tgcatttcat taaaaaaaaa accatgaatt acgaagcacc 3000
ttagtaagca ccttctaatg ctgcattttt tttgttgttg ttaaaaacat accagctggt 3060
tataatattg ttctccacgt ccttgtgatg attctgagcc tggcactccc aaatctggga 3120
agcatagttt atttgcaagt gttcaccttc caaatcatga ggcatagcat gacttattct 3180
tgtttggaaa actcttttca aaactgacca tcttaaacac atgatggcca agtgcccaaa 3240

agccctcttg cggagcaaat ttcagaatat atatgtggat ccaagctctg atagttcagg 3300

tgctggaggg aagagagacc tgtgtgttta gaggccagga ccacagttag gattgggttg 3360

tttcaatact gagagacagc tacaataaaa ggagagcaat tgcctccctg gggctgttca 3420

atcttctgca tttgtgagtg gttcagtcat gaggttttcc aaaagatgtt tttagagttg 3480

taaaaaccat atttgcagca aagatttaca aaggcgtatc agactatgat tgttcaccaa 3540

aataggggaa tggtttgatc cgccagttgc aagtagaggc ctttctgact cttaatattc 3600

actttggtgc tactaccccc attacctgag ggaaactggc caggtccttg atcatggaac 3660

tatagagcta ccaggacata tcctgctctc taagggaatt tattgctatc ttgcaccttc 3720

tttaaaactc acatatgcag acctgacact caagagtggc tagctacaca gagtccatct 3780

aatttttgca acttcctgtg gccagtgtgt ataacccctt ccactatctc acagatagtc 3840

acagcgtcca ttccatagtc tgtctcctca catctgttag tattgacaca gcacagacac 3900

cacaagccat caggttcttc atggggcagg tgaaatactt ctaccccatg ggtaaatgta 3960

ttcacatatt accaagagaa gaagcacatt atctatgatc ttttggccca gttcttattt 4020

agcattttta ttccagccta cttggaaaca tgtttttatt tgcaatatat gcctgactga 4080

attaagcttg cttgttttaa acaaccaaat cattggaaca gaaaaggatt taaaaaacaa 4140

gaatgcatga tctcagagtg attaaaaaaa aatcagtgga aataaatgat catagaaggt 4200

gcttttcaaa acaactgcta ttataattct caaagtccta ctctgccaaa agaagattaa 4260

aagtcataca ttacattaca aggaaatgtt catgtgggaa gagggttgct gaaaatcaac 4320

aacgcttgaa gttaaaaagt gtgtctttgt agatttcatt gtataatgtg tatttcttag 4380

gagatggctg acttgattga tctacgctaa gtggagacat ttcacatttt taaaaccaaa 4440

tgttcaatct gtattactct ttgccgtctt gtatgtagag gctattttta aatcattaaa 4500

tttttagatc tctgttttca ta 4522

<210> 26
<211> 3848
<212> DNA
<213> Homo sapiens

<400> 26
agaagcctct gctccaccgc ggcgagaggc atgggcacgt ggctgccgag ggtggccgag 60

ctctgggaag aaaagcccgt gtgcctctgc atagcgtcgc tacagcgctg actcggtgtg 120

gattgattgg aaaggtttga gggagtactt gggaagcatg gtggcacatg atgagactgg 180

aggtctccta cctattaaaa ggaccatacg agtcctagat gtcaataacc agtccttcag 240

agaacaagag gagccaagca ataaaagagt tcgacctctg gctcgtgtca cgtccttggc 300

aaatttaatc tctcctgtaa gaaatggagc tgtcagacgt tttggtcaaa caatacagtc 360

atttaccctt cgtggtgacc acagatcccc agcctctgcc cagaagtttt ctagcaggtc 420
aacagtccca acacccgcca agagaaggag cagtgcactg tggtcagaga tgctggacat 480
caccatgaag gagtctctca ccaccaggga gatcagacgg caggaggcaa tatatgaaat 540
gtcccgaggt gaacaggatt taattgagga tctcaaactt gcaagaaagg cctatcatga 600
ccccatgtta aagttgtcca tcatgtcaga agaggaactc acacatatat ttggtgatct 660
ggactcttac atacctctgc atgaagattt gttgacaaga ataggagaag caaccaagcc 720
tgatggaaca gtggagcaga ttggtcacat tctcgtgagc tggttaccgc gcttgaatgc 780
ctacagaggt tactgtagta accagctggc agccaaagct cttcttgatc aaaagaaaca 840
ggatccaaga gtccaagact tcctccagcg atgtctcgag tctcccttca gtcgaaaact 900
agatctttgg agtttcctag atatccctcg aagtcgccta gtcaaatacc ctttactgtt 960
aaaagaaatt cttaaacaca ctccaaaaga gcaccctgat gttcagcttc tggaggatgc 1020
tatattgata atacagggag tcctctctga tatcaacttg aagaaaggtg aatccgagtg 1080
ccagtattac atcgacaagc tggagtacct ggatgaaaag cagagggacc ccagaatcga 1140
agcgagcaaa gtgctgctgt gccatgggga gctgcggagc aagagtggac ataaacttta 1200
cattttcctg tttcaagaca tcttggttct gactcggccc gtcacacgga acgaacggca 1260
ctcttaccag gtttaccggc agccaatccc agtccaagag ctagtcctag aagacctgca 1320
ggatggagat gtgagaatgg gaggctcctt tcgaggagct ttcagtaact cagagaaagc 1380
taaaaatatc tttagaattc gcttccatga cccctctcca gcccagtctc acactctgca 1440
agccaatgac gtgttccaca agcagcagtg gttcaactgt attcgagcgg ccattgcccc 1500
cttccagtcg gcaggcagtc cacctgagct gcagggcctg ccggagctgc acgaagagtg 1560
tgaggggaac cacccctctg cgaggaaact cacagcccag aggagggcat ccacagtttc 1620
cagtgttact caggtagaag ttgatgaaaa cgcttacaga tgtggctctg gcatgcagat 1680
ggcagaggac agcaagagct taaagacaca ccagacacag cccggcatcc gaagagcgag 1740
ggacaaagcc ctttctggtg gcaaacggaa agagactttg gtgtagagaa ggctctgtgt 1800
gttaactgat gggagagact gtttgtttat aaatgtgtac agttttgttt tctcgtaagg 1860
ggagcatcat agggttactt tataccagtt gtaacatttt cattgttttt ggttgttctt 1920
ttttcttttt ttaatggcag ctaaagatat acagattact gttaaattgc agtccttttt 1980
tttttaaaga tattttcttg aattatttag aacatggtaa gcctggtatt ttttaatcaa 2040
acaaaatatt tatgaaatgg gttttctctt aattctggat tcatcatggc tttctaatac 2100
caattgtaat atttacaata ttcaccaaaa cttagaattt tgcaaatgct ggaattctgc 2160
cagtgtttct ttgctaagcc ttgcatgcaa aatttgaaat tttaacattg gcacccaaaa 2220

cctacatgga atgtatgtct ggagtatttc aaactttaca ttgaaacata atttccttgg 2280
aaaacaaacc ataagcctga ggaggttttt atcaactgga atgctttata ttagtttgtt 2340
tttcactgta cattcctcat tttacattca tttaacctgc cgattattta atttttttat 2400
tgtaaagtag tttttagcat ttgcttttat ttttttactt tgatgccttt tcaaattggc 2460
atgtctttaa agtatttttc ttcctgatta aaaatgtgtg tgtatgtgtg tgtgtgtgtg 2520
tatatatata tatattttta aatcacatta attttaccaa gtgaaaccaa gccatactgt 2580
ttttgagcca attaagaaaa ttgccatttt taaagtgtag catttcaggg taaagaccca 2640
tgaaatggct tgatgtattc tagactactg aaagaaaacc acttcaaaga ttttgttgaa 2700
agttttagtg ttgtctgaaa tgcaagaggg aaggtgattg gtagtgagtt aaaagaaaaa 2760
gagaggaaaa gagagtagtt ttgtcttcaa gtaaaatgtc tggttgtgcc agacatttca 2820
caagtgtgaa aggagatagg agaagctcaa cttgagggcg tgtagtaagt tgtagaaggc 2880
tcgaggggac gtggacttat ttgccttggt ttgcaatacc tgcaaataat gagtttgaaa 2940
agaaacaatg aaatgtgtta aaaatttgac catattagat aaattttcgt ggatttagtc 3000
ataagatgga aaaagactgg tgaatctttt attacaaaat gtttctgtta aaatgggatc 3060
atcatctttg aaaggggga ggaggagtaa aagcccgatt ataatggtga tcaattcaag 3120
tcagtgttga ctattctgtg aaatatattt ggccagtgga aatgataatc agaaaagact 3180
gtaaatagat ccatccaaat gatttctctg tacaaatgaa tgatactatt aaaaaaaaaa 3240
aaagcacaca cataatcacc ctgaaggttt cttgcctggt ggctatgttg ggggcatttg 3300
gcaaaatatg ctggtgtggc aaaggaggta gtggtaagcc tcataaaagt tagtaaaaat 3360
tgtagttcaa cctttgaaat cattgatgaa tcagcgaaag gtaggtaagt cttgtgttag 3420
gaaatctttt aagaacatgg tgtaatcaga cacagcaatt gtattgaaaa taactgaaca 3480
cctgttactt tctggatagg atagtaaggg tagtagtggc aaaggatcct gattaatttc 3540
agattttaat gtaagtacct caggttcttg tctttggaaa ttcacattca taagtgtata 3600
ttacaccaat gattgtgatc tgcaacacca gaaagcaaga tttctaatca tttccagagg 3660
tgttgtatag aatagttatt ccagagttac cttgatcagc ttctgaagtt taagaagcaa 3720
gtaatagttc aagtgttgtt aatggaatta tgttttattt aaaaccgtat cttcttttgg 3780
ttccctcaaa ggtaaaaata agacccagaa tctgagtaaa tctattaaat gaatgttctt 3840
cagaaata 3848

<210> 27
<211> 1579
<212> DNA
<213> Homo sapiens

<400> 27

```
agcccacagc agtccgtgcc gccgtcccgc ccgccagcgc cccagcgagg aagcagcgcg      60
cagcccgcgg cccagcgcac ccgcagcagc gcccgcagct cgtccgcgcc atgttccagg     120
cggccgagcg cccccaggag tgggccatgg agggcccccg cgacgggctg aagaaggagc     180
ggctactgga cgaccgccac gacagcggcc tggactccat gaaagacgag gagtacgagc     240
agatggtcaa ggagctgcag gagatccgcc tcgagccgca ggaggtgccg cgcggctcgg     300
agccctggaa gcagcagctc accgaggacg gggactcgtt cctgcacttg gccatcatcc     360
atgaagaaaa ggcactgacc atggaagtga tccgccaggt gaagggagac ctggccttcc     420
tcaacttcca gaacaacctg cagcagactc cactccactt ggctgtgatc accaaccagc     480
cagaaattgc tgaggcactt ctgggagctg gctgtgatcc tgagctccga gactttcgag     540
gaaatacccc cctacacctt gcctgtgagc agggctgcct ggccagcgtg ggagtcctga     600
ctcagtcctg caccaccccg cacctccact ccatcctgaa ggctaccaac tacaatggcc     660
acacgtgtct acacttagcc tctatccatg gctacctggg catcgtggag cttttggtgt     720
ccttgggtgc tgatgtcaat gctcaggagc cctgtaatgg ccggactgcc cttcacctcg     780
cagtggacct gcaaaatcct gacctggtgt cactcctgtt gaagtgtggg gctgatgtca     840
acagagttac ctaccagggc tattctccct accagctcac ctggggccgc ccaagcaccc     900
ggatacagca gcagctgggc cagctgacac tagaaaacct tcagatgctg ccagagagtg     960
aggatgagga gagctatgac acagagtcag agttcacgga gttcacagag gacgagctgc    1020
cctatgatga ctgtgtgttt ggaggccagc gtctgacgtt atgagcgcaa aggggctgaa    1080
agaacatgga cttgtatatt tgtacaaaaa aaaagtttta tttttctaaa aaaagaaaaa    1140
agaagaaaaa atttaaaggg tgtacttata tccacactgc acactgcctg gcccaaaacg    1200
tcttattgtg gtaggatcag ccctcatttt gttgcttttg tgaacttttt gtaggggacg    1260
agaaagatca ttgaaattct gagaaaactt cttttaaacc tcacctttgt ggggtttttg    1320
gagaaggtta tcaaaaattt catggaagga ccacatttta tatttattgt gcttcgagtg    1380
actgacccca gtggtatcct gtgacatgta acagccagga gtgttaagcg ttcagtgatg    1440
tggggtgaaa agttactacc tgtcaaggtt tgtgttaccc tcctgtaaat ggtgtacata    1500
atgtattgtt ggtaattatt ttggtacttt tatgatgtat atttattaaa cagattttta    1560
caaatgaaaa aaaaaaaaa                                                 1579
```

```
<210>  28
<211>  3601
<212>  DNA
<213>  Homo sapiens

<400>  28
aagacttgaa cttgaatctc gaaccactgc atctccgact ctgcccagac tcttcactcc      60
```

gcggcaccct caaaccccag cccaggccgg ggcgcacaag ccagccagcg cacctgcagt 120
cctcgcccgg acgcgccgcg ccccctcgga accaggctct gctccgagca gccttcgccc 180
ctcaagccag ccacagtccc cgccaggccg ggtgggcgtc aagatgaagg cggcccgctt 240
cgtgctgcgc agcgctggct cgctcaacgg cgccggcctg gtgccccgag aggtggagca 300
tttctcgcgc tacagcccgt ccccgctgtc catgaagcag ctactggact ttggttcaga 360
aaatgcatgt gaaagaactt cttttgcatt tttgcgacaa gaattgcctg tgagactcgc 420
caacattctg aaggaaattg atatcctccc gacccaatta gtaaatacct cttcagtgca 480
attggttaaa agctggtata tacagagcct gatggatttg gtggaattcc atgagaaaag 540
cccagatgac cagaaagcat tatcagactt tgtagataca ctcatcaaag ttcgaaatag 600
acaccataat gtagtcccta caatggcaca aggaatcata gagtataaag atgcctgtac 660
agttgaccca gtcaccaatc aaaatcttca atatttcttg gatcgatttt acatgaaccg 720
tatttctact cggatgctga tgaaccagca cattcttata tttagtgact cacagacagg 780
aaacccaagc cacattggaa gcattgatcc taactgtgat gtggtagcag tggtccaaga 840
tgcctttgag tgttcaagga tgctctgtga tcagtattat ttatcatctc cagaattaaa 900
gcttacacaa gtgaatggaa aatttccaga ccaaccaatt cacatcgtgt atgttccttc 960
tcacctccat catatgctct ttgaactatt taagaatgca atgcgggcaa cagttgaaca 1020
ccaggaaaat cagccttccc ttacaccaat agaggttatt gttgtcttgg gaaaagaaga 1080
ccttaccatt aagatttcag acagaggagg tggtgttccc ctgagaatta ttgaccgcct 1140
ctttagttat acatactcca ctgcaccaac gcctgtgatg gataattccc ggaatgctcc 1200
tttggctggt tttggttacg gcttgccaat ttctcgtctg tatgcaaagt actttcaagg 1260
agatctgaat ctctactctt tatcaggata tggaacagat gctatcatct acttaaaggc 1320
tttgtcttct gagtctatag aaaaacttcc agtttttaac aagtcagcct tcaaacatta 1380
tcagatgagc tctgaggctg atgactggtg tatcccaagc agggaaccaa agaacctggc 1440
aaaagaagtg gccatgtgaa gagggacact caggacactt tacgggatca aagtgggtct 1500
acaccagtgc tgcttcctga atgtttgtgt gtgaaccctt gtttcctcca aaacaaacga 1560
cagcaacgaa aactccttaa tcagaacact gatccaatga ggaatggagc ttgtttctgt 1620
gacccaggag aacttagtgc aagactacag gagttaacag atggccagct ccttattttt 1680
taatgtagaa taactcctga gtttatatca aatcctgaag aaataagcct cagttttcca 1740
tctgtttttg ataagaataa gaaagggagt gagtgtgaag atggtggtta gcagtttcac 1800
taagactgat attttaggcc tcttgttcac atcaaaagat attggtgtca gaataccagc 1860
attttcctgc catgcaaagg attaaaactt agtttacact atgtggttac aaatatatgt 1920
caatgtacat tttgaacata tttatgtgct atggaaggaa atgctggtga ctaaaataag 1980

gtttactctg aaagaggagg aattttattc aaagcattca aacattttat tcaagtgttt 2040

caaaattcaa agcattgtat tcaaagttgc agtgaaggca tcaacttatg taaaaactca 2100

gaaggaaggc tcctctgata aaaacacagc tcctttatta tgctgctttt cttgttcact 2160

ttacacacta agtaaacact tattgtcagg tgcctagtct tgagtgaatt gttagatgtg 2220

cactgaactc gggatgttgg ggattggaga gagagaattg ccaaagtaac agcaaaaata 2280

tctcttactt tgctttgttt ataaataaat tagtagattg gaaaaactag tgttagggaa 2340

agaaatcaca tgttcagagc ctaattcagt aggaagggct tttctctacc ctgaaatgaa 2400

ggtaatccaa aggcatccat tttctaggct taaaagatat atttttgata tatttaatta 2460

tattctctac actccagcat taatatgtct gtttaaaaat tactaattct caaatggctc 2520

aagaacatta gaatttaagt accttttaga gtaattattt taagcaaata gcctggacgt 2580

aagagattct catgccagca tgctttcatt tgtcagttgt tgtgactgag agataatgaa 2640

tgacacctga aatgcatatg gtatttttgg gagagttaag gtataatttg aaggttggca 2700

gaccagttgc gctgattact cttagagaag aagaaatgga aaaatgaaag aaggcaggaa 2760

ggaaagaaag gatataggaa gagagggaag cagaaggcag gcatttttct attttcccca 2820

caaattattt caaaaaaaat ctgtattttc tgggatatgt cattggcaag aggaagaact 2880

ggtgttttga aagcagtatg gattctttaa atgcctctca ctcttacaag atagtaggct 2940

ttgagataat aaacttaccc gtgtcaatta acatttaaac tggcatatag aaaaaaagga 3000

ggatttttct gcattgtaaa ataatcagta tggtttatat gttgaatttg acatttgtgt 3060

gtaatttcat ggtggcctag tgttgtggtg cttctggtaa tggtaataga agctcaacta 3120

ttttttgtg gatttcagtt tttatcatca gaagtcctag acagtgacat ttcttaatgg 3180

tgggagtcca gctcatgcat ttctgattat acaaaacagt ttgcagtagg ttatttgtca 3240

tttcagtttt ttactgaaat ttgagctaaa catttttaca tgtaaatact tgtatttacc 3300

aaagatttaa atcagttgat taattaatta actcaaatac tgtgaactat ctctaaaaca 3360

ctagaaaaaa gaaatgttag tatctcaatt acaccaactg tgcaaatgaa ctttgataaa 3420

atagaaataa tctacattgg cctttgtgaa atctggggaa gagctttagg attctagtag 3480

atggatactg aatactcagg cccacttaaa ttattaatgt atacattgtg tttttgtctt 3540

tatgctatgt acagagaaat gtgataattt tttataataa atatttttta tgatgataaa 3600

a 3601

<210> 29
<211> 1897
<212> DNA
<213> Homo sapiens

<400> 29
gctttgaccg ggtcgtggca gccggagtcg tcttcgggac gcgcctgctc ttcgcctttc 60

gctgcagtcc gtcgatttct ttctccagga agaaaaatgg catccgttgc agttgatcca 120

caaccgagtg tggtgactcg ggtggtcaac ctgcccttgg tgagctccac gtatgacctc 180

atgtcctcag cctatctcag tacaaaggac cagtatccct acctgaagtc tgtgtgtgag 240

atggcagaga acggtgtgaa gaccatcacc tccgtggcca tgaccagtgc tctgcccatc 300

atccagaagc tagagccgca aattgcagtt gccaatacct atgcctgtaa ggggctagac 360

aggattgagg agagactgcc tattctgaat cagccatcaa ctcagattgt tgccaatgcc 420

aaaggcgctg tgactggggc aaaagatgct gtgacgacta ctgtgactgg ggccaaggat 480

tctgtggcca gcacgatcac aggggtgatg gacaagacca aaggggcagt gactggcagt 540

gtggagaaga ccaagtctgt ggtcagtggc agcattaaca cagtcttggg gagtcggatg 600

atgcagctcg tgagcagtgg cgtagaaaat gcactcacca aatcagagct gttggtagaa 660

cagtacctcc ctctcactga ggaagaacta gaaaaagaag caaaaaaagt tgaaggattt 720

gatctggttc agaagccaag ttattatgtt agactgggat ccctgtctac caagcttcac 780

tcccgtgcct accagcaggc tctcagcagg gttaaagaag ctaagcaaaa aagccaacag 840

accatttctc agctccattc tactgttcac ctgattgaat ttgccaggaa gaatgtgtat 900

agtgccaatc agaaaattca ggatgctcag gataagctct acctctcatg ggtagagtgg 960

aaaaggagca ttggatatga tgatactgat gagtcccact gtgctgagca cattgagtca 1020

cgtactcttg caattgcccg caacctgact cagcagctcc agaccacgtg ccacaccctc 1080

ctgtccaaca tccaaggtgt accacagaac atccaagatc aagccaagca catgggggtg 1140

atggcaggcg acatctactc agtgttccgc aatgctgcct cctttaaaga agtgtctgac 1200

agcctcctca cttctagcaa ggggcagctg cagaaaatga aggaatcttt agatgacgtg 1260

atggattatc ttgttaacaa cacgcccctc aactggctgg taggtccctt ttatcctcag 1320

ctgactgagt ctcagaatgc tcaggaccaa ggtgcagaga tggacaagag cagccaggag 1380

acccagcgat ctgagcataa aactcattaa acctgcccct atcactagtg catgctgtgg 1440

ccagacagat gacacctttt gttatgttga aattaacttg ctaggcaacc ctaaattggg 1500

aagcaagtag ctagtataaa ggccctcaat tgtagttgtt tccagctgaa ttaagagctt 1560

taaagtttct ggcattagca gatgatttct gttcacctgg taagaaaaga atgataggct 1620

tgtcagagcc tatagccaga actcagaaaa aattcaaatg cacttatgtt ctcattctat 1680

ggccattgtg ttgcctctgt tactgtttgt attgaataaa aacatcttca tgtgggctgg 1740

ggtagaaact ggtgtctgct ctggtgtgat ctgaaaaggc gtcttcactg ctttatctca 1800

tgatgcttgc ttgtaaaact tgattttagt ttttcatttc tcaaatagga atactacctt 1860

tgaattcaat aaaattcact gcaggataga ccagtta 1897

<210> 30
<211> 3910
<212> DNA
<213> Homo sapiens

<400> 30
ccggctgcgc gcgcagcggt ggtggtggcg gcgcgatcgg ccgggctgta accgtcgtct 60

gtccgggagc ggctggagcg gcagcggcgg ccgggcacgg cgcgaggtga cgccacaggg 120

cagcggcggc agcggaggca gcggcggcag caggagacgc agcggcggcc gcagcagcag 180

cagcaagacg gactcgtgga gacgcgccgc cgccgccgcc gccgggccgg gccgggtgtc 240

gcgcgccgag gctggggggg agtcgtcgcc gccgccgcca ccgctaccgc cgccgccgcc 300

gccgccgagg tgactgagga gagaggcgcc tcctcgctcc cgccaccgcc ggacttcaat 360

gcccagtccc cagctcgcca gcgtttttcg ttggaatata cgttgcacat ttatggcgat 420

tctgagtgtg agggcagact tctgccaggc tcagcacagc attttcgctg acaagtgagc 480

ttggaggttc tatgtgccat aattaacatt gccttgaaga ctcctggaca ccgagactgg 540

cctcagaaat agttggcttt tttttttttt aattgcaagc atatttcttt taatgactcc 600

agtaaaatta agcatcaagt aaacaagtgg aaagtgacct acacttttaa cttgtctcac 660

tagtgcctaa atgtagtaaa ggctgcttaa gttttgtatg tagttggatt ttttggagtc 720

cgaaggtatc catctgcaga aattgaggcc caaattgaat ttggattcaa gtggattcta 780

aatactttgc ttatcttgaa gagagaagct tcataaggaa taaacaagtt gaatagagaa 840

aacactgatt gataataggc attttagtgg tctttttaat gttttctgct gtgaaacatt 900

tcaagattta ttgatttttt tttttcactt tccccatcac actcacacgc acgctcacac 960

tttttatttg ccataatgaa ccgtccagcc cctgtggaga tctcctatga gaacatgcgt 1020

tttctgataa ctcacaaccc taccaatgct actctcaaca agttcacaga ggaacttaag 1080

aagtatggag tgacgacttt ggttcgagtt tgtgatgcta catatgataa agctccagtt 1140

gaaaaagaag gaatccacgt tctagattgg ccatttgatg atggagctcc accccctaat 1200

cagatagtag atgattggtt aaacctgtta aaaaccaaat ttcgtgaaga gccaggttgc 1260

tgtgttgcag tgcattgtgt tgcaggattg ggaagggcac ctgtgctggt tgcacttgct 1320

ttgattgaat gtggaatgaa gtacgaagat gcagttcagt ttataagaca aaaaagaagg 1380

ggagcgttca attccaaaca gctgctttat ttggagaaat accgacctaa gatgcgatta 1440

cgcttcagag ataccaatgg gcattgctgt gttcagtaga aggaaatgta aacgaaggct 1500

gacttgattg tgccatttag agggaactct tggtacctgg aaatgtgaat ctggaatatt 1560

acctgtgtca tcaaagtagt gatggattca gtactcctca accactctcc taatgattgg 1620

aacaaaagca aacaaaaaag aaatctctct ataaaatgaa taaaatgttt aagaaaagag 1680
aaagagaaaa ggaattaatt cagtgaagga tgattttgct cctagttttg gagtttgaat 1740
ttctgccagg attgaattat tttgaaatct cctgtctttt taaacttttt caaaataggt 1800
ctctaaggaa aaccagcaga acattagcct gtgcaaaacc atctgtttgg ggagcacact 1860
cttccattat gcttggcaca tagatctccc tgtggtggga tttttttttt cccttttttt 1920
gtgggggagg gttggtggta tatttttccc ctctttttttc cttcctctcc tacatctccc 1980
ttttcccccg atccaagttg tagatggaat agaagccctt gttgctgtag atgtgcgtgc 2040
agtctggcag ccttaagccc acctgggcac ttttagataa aaaaaaaaaa aaaacaaaaa 2100
acaacaccaa aaaaacagca gtgatatata tatatatata tatatatata tatatatata 2160
tatatatata tatatatata tatatatata atataatata tatatatata tatatatttt 2220
ccaggtggtt tttagtcttt actgatgaaa gggtgttcat gttagtttct tcaaaaccct 2280
atctaatact aggcaaagta gccaagagcc ttttgttttg tttttatttt gataaattag 2340
tggagaaatg gcattttaag aggagtctct tctcaactta cctgagagtc gaattcttct 2400
cttccctaac caatgaagct aagtggttat cccagaaact tgtcttctaa aagggaggac 2460
tccaggccat caataaagat gtccaggcag tgagcgtact ttttacaccc tgtagaattg 2520
tgggctgtag cgttactctg attttctgtc tagtatcaga gaatgctggt agcttaaaat 2580
ttttatttta ggacttgtac tctgaatttt caggaaccgt caaaggagca gcagcaaatt 2640
cacatatttt cgacttgaga aatgcttgtg gtatgtgttt tccaaactgc cccctatatg 2700
taaagttcag tttaaccact gattgccttg ttattactag gttttttgag attaaaaaaa 2760
aaaaatccct ggtttaaaac caacaatgat gcctagtgag tatgtgtcca caggccataa 2820
cagggtagaa gagagacatc gtgcaaccca atgagtagtg aagggactgt gttgcttgtg 2880
aagcggtgta gtagcatttt tgcagattct tggctgggtt tagtgtactg atctagaaaa 2940
gctgtttttc tgctcctttg tggaaggcag ttatgatcag gctgcatgga caaagcaggt 3000
agaggggcac catcaggggc tcttgcacta ttttcacctc taaatattac gtactcagta 3060
gtgccctgct tctagggctc tgaatacggg cttaaagtca tcttgtcctg ctggaatttg 3120
ctgtgcagag ccataagcct cccattttgt tagcgtcagc taggccaata ggaacagacc 3180
gggaccttgt ctcacactga tgatacctca catgttgacc ggctatgtga actgcctatt 3240
tcctatgctg gagttttgat ttttaactaa acgcaaatct gtagattctc tcctctccca 3300
tcccagaaaa caaaacaaaa taatgctttt cgaaattgtt tctaggactt taaaacataa 3360
tggtatatcc aaaattcttt atttcagaat gcaacaatag attccattaa tatagactca 3420
agatcaaaac agcatacctg ctaagctaag atagatggtg ttgattccac tgggttttga 3480
tcaatacaat aacaaacctt tttcctttga catactctga attttgttgt ttgggggggag 3540

ggggtgtgtg tgtgtgtgtg tgtgtgtgtg tgtgtatgtg tgtgtgtgtg tgtgcacgcg 3600

cagtgtccat cagtatcagt gcctgcctga gttaggaaaa ttacattcct ggttctgtat 3660

tgaggagaag gatgtataaa gcaacatgaa acattagccc tccttttatt ttaaagacta 3720

atgttaattg ttcttaaaac tggatttttt ttccttaaag caattttttt cttttcgatt 3780

taatgaagta ttgctagctg aagccagttt gacatagaga gatgtcagat tgatttgaaa 3840

ggtgtgcagc ctgatttaaa accaaaccct gaaccctttt aaagaacaat aaaacatatt 3900

ttacacgctc 3910

<210> 31
<211> 471
<212> DNA
<213> Homo sapiens

<400> 31
acattttctc ggccctgcca gcccccagga ggaaggtggg tctgaatcta gcaccatgac 60

ggaactagag acagccatgg gcatgatcat agacgtcttt tcccgatatt cgggcagcga 120

gggcagcacg cagaccctga ccaaggggga gctcaaggtg ctgatggaga aggagctacc 180

aggcttcctg cagagtggaa aagacaagga tgccgtggat aaattgctca aggacctgga 240

cgccaatgga gatgcccagg tggacttcag tgagttcata gtgttcgtgg ctgcaatcac 300

gtctgcctgt cacaagtact ttgagaaggc aggactcaaa tgatgccctg gagatgtcac 360

agattcctgg cagagccatg gtcccaggct tcccaaaagt gtttgttggc aattattccc 420

ctaggctgag cctgctcatg tacctctgat taataaatgc ttatgaaatg a 471

<210> 32
<211> 5718
<212> DNA
<213> Homo sapiens

<400> 32
ggccgagcgc gcggcctggc gcacgatacg ccgagccggt ctttgagcgc taacgtcttt 60

ctgtctcccc gcggtggtga tgacggtgaa aactgaggct gctaagggca ccctcactta 120

ctccaggatg aggggcatgg tggcaattct catcggtgag tgcaggaatc ttgcgggact 180

tctgctccag gagacgcaaa gtggaaattt tttgaaagtc ccggatcaga ttagtgtgtg 240

tggcgccggg acgttatgaa gccgtctaaa cgtttcttta tttctcctcc ttctatccac 300

agctttcatg aagcagagga ggatgggtct gaacgacttt attcagaaga ttgccaataa 360

ctcctatgca tgcaaacagt aagttcagac cggattgagg aaataactag tatagtttga 420

atttgccagc ggtaaacatt ctcatcacgg cgtttatcgg gaaggcgaag acttcttctg 480

gggtggggat ctcatttctc cttaaattct aatatatttg acacatttta aacattaaag 540

```
ttaatttgct gatttggctt gaactggaga tgtaagataa atggttcgtg ttggccgaat      600
tcacgctttc tccatgagca acaatcctta tttctgtatt taatggggtt tattattttc      660
tttaactgac taatgtattg ggtattttc agtttaaaca gtgaattatc gggtagaagt      720
cggtagagcc agaaactcac ttttgatgtt ggtgtgcccc ctagtggcga gctggattct      780
aaatcgtgcc ctttattccc tgcagccctg aagttcagtc catcttgaag atctcccaac      840
ctcaggagcc tgagcttatg aatgccaacc cttctcctcc agtaagtttt tgtatgtgcc      900
gtgcatctgt ggagaactgt aagggagtca gttagtattc ctacattaat ggattaaaat      960
agcatttcta gaaattagta tcaaggcagg aatgcttcat tatgcataac agtgatataa     1020
atatttaagt attgagtcag agtattattt ttattttttt cctgggcata ttttacctca     1080
agtggttatt ttaaaaggca tatttcataa aaaggtttta tctgtctgaa acaacatgac     1140
tgtgtgcagt ttccatactc atttgaaatg tgatgaaatg tagttttgaa tgtttataga     1200
tgtatggtca tttgcatcag tcatttgtag atgtaacatt ttctacatcg tttatgttat     1260
agatgtcttc ctttgaagca atggtattaa aagaaattct tttttttttt ttctagccaa     1320
gtccttctca gcaaatcaac cttggcccgt cgtccaatcc tcatgctaaa ccatctgact     1380
ttcacttctt gaaagtgatc ggaaagggca gttttggaaa ggtaatttca aatctgaaga     1440
tcttttggta cacttccttc atgtcctctt ttatattctc cctggatgag gatcgaaaaa     1500
tgattttttt aaattgaaat ttcaggttct tctagcaaga cacaaggcag aagaagtgtt     1560
ctatgcagtc aaagttttac agaagaaagc aatcctgaaa aagaaagagg tgagatgtgc     1620
ttgatggggc tggcattggc ggtagacact ccttgaataa tcttgattct ggaatgttgg     1680
tgccagttga acatgccact aaatctgaat cgtcattttc ctaggagaag catattatgt     1740
cggagcggaa tgttctgttg aagaatgtga agcacccttt cctggtgggc cttcacttct     1800
ctttccagac tgctgacaaa ttgtactttg tcctagacta cattaatggt ggagaggtga     1860
gcagggggga tagaagtcaa ctcttagtgt ctctgcacag cctgctttgt tttagtttga     1920
gaaaaaagtt ttcaaagatt tttggtgggg agaatgttac cagaattagc atttccttca     1980
acctgtcagg ttatagttaa tagattactt ggggccactt cctgcagttg ttcttttgct     2040
gtgtatgtca aaactaatta aattacattg cgcaacccag aatgactttg ttctgtctcc     2100
tgcagttgtt ctaccatctc cagagggaac gctgcttcct ggaaccacgg gctcgtttct     2160
atgctgctga aatagccagt gccttgggct acctgcattc actgaacatc gtttataggt     2220
aagcctgaga gctcttcagg ctaccagttt tggtataaag gagacgtagc actggctgtt     2280
tcatagggcc ttaaaataat ttgtgtttat ttgcaacttg gttcgctaaa accagatccc     2340
ctagcacgtg agctggcttg acttaagtgc caagggggaa cagccaagta ggattgtgcc     2400
taatccagaa tagatgagca gaacaagggc tcctttttc ttcactacac aactacagtg      2460
```

aacctaaatg cctctaatac cttagcaatt atctttaaga ggatatctta tgaagtgaaa 2520
ttaacttgtg caactacttt tctattcact tttttacaga gacttaaaac cagagaatat 2580
tttgctagat tcacagggac acattgtcct tactgatttc ggactctgca aggagaacat 2640
tgaacacaac agcacaacat ccaccttctg tggcacgccg gaggtaggcg ctgtcttggt 2700
ttggtgcctg gtttaccccc gccttccaag agagagatgt acaatcatgc acttaactac 2760
caaaaagagt aaactcctct cagagacttc ttaatacagt tcagtgcaaa taaaatacat 2820
ttgctgtttg atgtagcatg agaaatccca agtccttctg ttcctttact gaaaagtagc 2880
tgtttgtaag taagatctgc atcataaaaa ctttctaatc ctaagtaaga gatatcaagt 2940
gccagcagtt tcctaaatgt cagtacacat aggtagccag tcaccctcaa aaagtccagc 3000
agttttatca ggaaggaatc taaagatatc tatcttccaa gctggctctg ggtctctcag 3060
ctttttcaaa ctaaatgtgt ggtcgtggga ttgcttgctt tcgcaggttc taaacgctgt 3120
ttccctggtc tgtttttcag tatctcgcac ctgaggtgct tcataagcag ccttatgaca 3180
ggactgtgga ctggtggtgc ctgggagctg tcttgtatga gatgctgtat ggcctggtga 3240
gtggcacatt gggaaccact ggaacactgc ctgctcccta caatattgcc ttcacacagc 3300
aaaagcagct aagaggcata ttggttattt tatagttcat aagaataatc acttacctgg 3360
ttcttttgtg catttcacat tttactagat aggaccacat tgaacctgtg tggtggtgaa 3420
aaactaccac ttattaacat ctacccccta ccctccacac acacacacac aaacacacac 3480
acgggttgca aagtagacac ttaaatagca agggaaaaga aagcattgag gtggggagag 3540
tttctcaaat cgagcctaat atttattgcc gtttatatct ttttctctac tggtaatgtg 3600
tgccatatga aacttccaat taagtctaaa gtaattttcc ccttctttca gccgcctttt 3660
tatagccgaa acacagctga aatgtacgac aacattctga acaagcctct ccagctgaaa 3720
ccaaatatta caaattccgc aagacacctc ctggagggcc tcctgcagaa ggacaggaca 3780
aagcggctcg ggccaagga tgacttcgtg agtgatgttt tcctgtcctc ctgggccggc 3840
cgggacgtgc actagacctc cctgccctta ttgaatgcac ctgtctaaat taatcttggg 3900
tttcttatca acagatggag attaagagtc atgtcttctt ctccttaatt aactgggatg 3960
atctcattaa taagaagatt actcccctt ttaacccaaa tgtggtgagt atctgtctct 4020
cttctaagta tagagaagcc aagcgattta ttttaattca gaattgtctg ggggagggtt 4080
ggaaggaata cattggcaga tgttttctcc ataaacctgt tattttacct acatagacac 4140
atttatcaat tcgaagcacc aaaaggcaac aagtgaacat tattcttatg tttaactgtg 4200
tgtagccttt tgagattttg tgcttgaagt gggtgattat ggaagttgat ataagactta 4260
aacttggtat ttaaagcctg gtcaagattt ccctgtcctg tgtctagtgt gagttcttga 4320

caagagtgtt tttcccttcc cgtcacagag tgggcccaac gagctacggc actttgaccc 4380

cgagtttacc gaagagcctg tccccaactc cattggcaag tcccctgaca gcgtcctcgt 4440

cacagccagc gtcaaggaag ctgccgaggc tttcctaggc ttttcctatg cgcctcccac 4500

ggactctttc ctctgaaccc tgttagggct tggttttaaa ggattttatg tgtgtttccg 4560

aatgttttag ttagcctttt ggtggagccg ccagctgaca ggacatctta caagagaatt 4620

tgcacatctc tggaagctta gcaatcttat tgcacactgt tcgctggaat tttttgaaga 4680

gcacattctc ctcagtgagc tcatgaggtt ttcattttta ttcttccttc caacgtggtg 4740

ctatctctga aacgagcgtt agagtgccgc cttagacgga ggcaggagtt tcgttagaaa 4800

gcggacctgt tctaaaaaag gtctcctgca gatctgtctg ggctgtgatg acgaatatta 4860

tgaaatgtgc cttttctgaa gagattgtgt tagctccaaa gcttttccta tcgcagtgtt 4920

tcagttcttt attttccctt gtggatatgc tgtgtgaacc gtcgtgtgag tgtggtatgc 4980

ctgatcacag atggattttg ttataagcat caatgtgaca cttgcaggac actacaacgt 5040

gggacattgt ttgtttcttc catatttgga agataaattt atgtgtagac tttttttgtaa 5100

gatacggtta ataactaaaa tttattgaaa tggtcttgca atgactcgta ttcagatgcc 5160

taaagaaagc attgctgcta caaatatttc tatttttaga aagggttttt atggaccaat 5220

gccccagttg tcagtcagag ccgttggtgt tttcattgt ttaaaatgtc acctgtaaaa 5280

tgggcattat ttatgttttt ttttttgcat tcctgataat tgtatgtatt gtataaagaa 5340

cgtctgtaca ttgggttata acactagtat atttaaactt acaggcttat ttgtaatgta 5400

aaccaccatt ttaatgtact gtaattaaca tggttataat acgtacaatc cttccctcat 5460

cccatcacac aactttttt gtgtgtgata aactgatttt ggtttgcaat aaaaccttga 5520

aaaatattta catatattgt gtcatgtgtt attttgtata ttttggttaa gggggtaatc 5580

atgggttagt ttaaaattga aaaccatgaa aatcctgctg taatttcctg cttagtggtt 5640

tgctccaaca gcagtggttt ctgactccag ggagtatagg atggcttaag ccaccacgtc 5700

caggccttta gcagcatt 5718

<210> 33
<211> 1700
<212> DNA
<213> Homo sapiens

<400> 33
gccgaggctg cctgactgga atgagggtag ctgcggcgac tgcggcggct ggagcggggc 60

cggccatggc ggtgtggacg cgggccacca aagcggggct ggtggagctg ctcctgaggg 120

agcgctgggt ccgagtggtg gccgagctga gcggggagag cctgagcctg acgggcgacg 180

ccgccgcggc cgagctggag cccgctctgg gacccgcggc cgccgccttc aacggcctcc 240

caaacggcgg cggcgcgggc gactcgctgc ccgggagccc aagccgcggc ctggggcccc 300
cgagcccgcc ggcgccgcct cggggccccg cgggtgaggc gggcgcgtcg ccgcccgtgc 360
gccgggtgcg ggtggtgaag caagaggcgg gcggcctggg catcagcatc aagggcggcc 420
gcgagaaccg gatgccgatc ctcatctcca agatcttccc cgggctggct gccgaccaga 480
gccgggcgct gcggctgggc gacgccatcc tgtcggtgaa cggcaccgac ctgcgccagg 540
ccacccacga ccaggccgtg caggcgctga agcgcgcggg caaggaggtg ctgctggagg 600
tcaagttcat ccgagaagta acaccatata tcaagaagcc atcattagta tcagatctgc 660
cgtgggaagg tgcagccccc cagtcaccaa gctttagtgg cagtgaggac tctggttcgc 720
caaaacacca gaacagcacc aaggacagga agatcatccc tctcaaaatg tgctttgctg 780
ctagaaacct aagcatgccg gatctggaaa acagattgat agagctacat tctcctgata 840
gcaggaacac gttgatccta cgctgcaaag atacagccac agcacactcc tggttcgtag 900
ctatccacac caacataatg gctctcctcc cacaggtgtt ggctgaactc aacgccatgc 960
ttggggcaac cagtacagca ggaggcagta aagaggtgaa gcatattgcc tggctggcag 1020
aacaggcaaa actagatggt ggaagacagc aatggagacc tgtcctcatg gctgtgactg 1080
agaaggattt gctgctctat gactgtatgc cgtggacaag agatgcctgg gcgtcaccat 1140
gccacagcta cccacttgtt gccaccaggt tggttcattc tggctccgga tgtcgatccc 1200
cctcccttgg atctgacctt acatttgcta ccaggacagg ctctcgacag ggcattgaga 1260
tgcatctctt cagggtggag acacatcggg atctgtcatc ctggaccagg atacttgttc 1320
agggttgcca tgctgctgct gagctgatca aggaagtctc tctaggctgc atgttaaatg 1380
gccaagaggt gaggcttact attcactatg aaaatgggtt caccatctca agggaaaatg 1440
gaggctccag cagcatattg taccgctacc cctttgaaag gctgaagatg tctgctgatg 1500
atggcatccg aaatctatac ttggattttg gtggtcccga gggagaactg accatggacc 1560
tgcactcttg tccgaagccg attgtatttg tgttgcacac gtttttatcg gccaaagtca 1620
ctcgtatggg actgcttgta tgagcaacaa aaaatcagaa aagagccttg actgtcacaa 1680
gaaatatttc cacctccaaa 1700

<210> 34
<211> 4314
<212> DNA
<213> Homo sapiens

<400> 34
agacaggata ttcactgctg tggcaaggcc tgtagagagt ttcgaagtta ggaggactca 60
agacggtccc tccctggact tttctgaagg ggctcaaaag atgacacgcg ccagagctgg 120
aaggcgtcgc caattggtcc acttttccct cctccctttt tgcggatgag aaaactgagg 180

```
cccaggtttg ggatttccag agcccgggat ttcccggcaa cgcccgacaa ccacattccc      240
ccggctattc tgacccgccc cggttccggg acgctccctg ggagccgccg ccgagggcct      300
gctgggactc ccgggggacc ccgccgtcgg ggcagccccc acgcccggcg ccgcccgccg      360
ggaacggccg ccgctgttgc gcacttgcag gggagccggc gactgagggc gaggcaggga      420
gggagcaagc ggggctggga gggctgctgg cgcgggctcg cgcgctgtgt atggtctatc      480
gcaggcagct gacctttgag gaggaaatcg ctgctctccg ctccttcctg tagtaacagc      540
cgccgctgcc gccgccgcca ggaaccccgg ccgggagcga gagccgcggg gcgcagagcc      600
ggcccggctg ccggacggtg cggccccacc aggtgaacgg ccatggcggg ctggatccag      660
gcccagcagc tgcagggaga cgcgctgcgc cagatgcagg tgctgtacgg ccagcacttc      720
cccatcgagg tccggcacta cttggcccag tggattgaga gccagccatg ggatgccatt      780
gacttggaca atccccagga cagagcccaa gccacccagc tcctggaggg cctggtgcag      840
gagctgcaga agaaggcgga gcaccaggtg gggga agatg ggtttttact gaagatcaag      900
ctggggcact acgccacgca gctccagaaa acatatgacc gctgccccct ggagctggtc      960
cgctgcatcc ggcacattct gtacaatgaa cagaggctgg tccgagaagc caacaattgc     1020
agctctccgg ctgggatcct ggttgacgcc atgtcccaga agcaccttca gatcaaccag     1080
acatttgagg agctgcgact ggtcacgcag gacacagaga atgagctgaa gaaactgcag     1140
cagactcagg agtacttcat catccagtac caggagagcc tgaggatcca agctcagttt     1200
gcccagctgg cccagctgag cccccaggag cgtctgagcc gggagacggc cctccagcag     1260
aagcaggtgt ctctggaggc ctggttgcag cgtgaggcac agacactgca gcagtaccgc     1320
gtggagctgg ccgagaagca ccagaagacc ctgcagctgc tgcggaagca gcagaccatc     1380
atcctggatg acgagctgat ccagtggaag cggcggcagc agctggccgg gaacggcggg     1440
ccccccgagg gcagcctgga cgtgctacag tcctggtgtg agaagttggc cgagatcatc     1500
tggcagaacc ggcagcagat ccgcagggct gagcacctct gccagcagct gcccatcccc     1560
ggcccagtgg aggagatgct ggccgaggtc aacgccacca tcacggacat tatctcagcc     1620
ctggtgacca gcacattcat cattgagaag cagcctcctc aggtcctgaa gacccagacc     1680
aagtttgcag ccaccgtacg cctgctggtg ggcgggaagc tgaacgtgca catgaatccc     1740
ccccaggtga aggccaccat catcagtgag cagcaggcca agtctctgct taaaaatgag     1800
aacacccgca acgagtgcag tggtgagatc ctgaacaact gctgcgtgat ggagtaccac     1860
caagccacgg gcaccctcag tgcccacttc aggaacatgt cactgaagag gatcaagcgt     1920
gctgaccggc ggggtgcaga gtccgtgaca gaggagaagt tcacagtcct gtttgagtct     1980
cagttcagtg ttggcagcaa tgagcttgtg ttccaggtga agactctgtc cctacctgtg     2040
gttgtcatcg tccacggcag ccaggaccac aatgccacgg ctactgtgct gtgggacaat     2100
```

gcctttgctg agccgggcag ggtgccattt gccgtgcctg acaaagtgct gtggccgcag 2160

ctgtgtgagg cgctcaacat gaaattcaag gccgaagtgc agagcaaccg gggcctgacc 2220

aaggagaacc tcgtgttcct ggcgcagaaa ctgttcaaca acagcagcag ccacctggag 2280

gactacagtg gcctgtccgt gtcctggtcc cagttcaaca gggagaactt gccgggctgg 2340

aactacacct tctggcagtg gtttgacggg gtgatggagg tgttgaagaa gcaccacaag 2400

ccccactgga atgatggggc catcctaggt tttgtgaata agcaacaggc ccacgacctg 2460

ctcatcaaca agcccgacgg gaccttcttg ttgcgcttta gtgactcaga aatcgggggc 2520

atcaccatcg cctggaagtt tgactccccg gaacgcaacc tgtggaacct gaaaccattc 2580

accacgcggg atttctccat caggtccctg gctgaccggc tgggggacct gagctatctc 2640

atctatgtgt ttcctgaccg ccccaaggat gaggtcttct ccaagtacta cactcctgtg 2700

ctggctaaag ctgttgatgg atatgtgaaa ccacagatca agcaagtggt ccctgagttt 2760

gtgaatgcat ctgcagatgc tgggggcagc agcgccacgt acatggacca ggccccctcc 2820

ccagctgtgt gcccccaggc tccctataac atgtacccac agaaccctga ccatgtactc 2880

gatcaggatg gagaattcga cctggatgag accatggatg tggccaggca cgtggaggaa 2940

ctcttacgcc gaccaatgga cagtcttgac tcccgcctct cgccccctgc cggtcttttc 3000

acctctgcca gaggctccct ctcatgaatg tttgaatccc acgcttctct ttggaaacaa 3060

tatgcaatgt gaagcggtcg tgttgtgagt ttagtaaggc tgtgtacact gacacctttg 3120

caggcatgca tgtgcttgtg tgtgtgtgtg tgtgtgtgtc cttgtgcatg agctacgcct 3180

gcctcccctg tgcagtcctg ggatgtggct gcagcagcgg tggcctcttt tcagatcatg 3240

gcatccaaga gtgcgccgag tctgtctctg tcatggtaga gaccgagcct ctgtcactgc 3300

aggcactcaa tgcagccaga cctattcctc ctgggcccct catctgctca gcagctattt 3360

gaatgagatg attcagaagg ggaggggaga caggtaacgt ctgtaagctg aagtttcact 3420

ccggagtgag aagctttgcc ctcctaagag agagagacag agagacagag agagagaaag 3480

agagagtgtg tgggtctatg taaatgcatc tgtcctcatg tgttgatgta accgattcat 3540

ctctcagaag ggaggctggg gttcattttc gagtagtatt ttatacttta gtgaacgtgg 3600

actccagact ctctgtgaac cctatgagag cgcgtctggg cccggccatg tccttagcac 3660

agggggggccg ccggtttgag tgagggtttc tgagctgctc tgaattagtc cttgcttggc 3720

tgcttggcct tgggcttcat tcaagtctat gatgctgttg cccacgtttc ccgggatata 3780

tattctctcc cctccgttgg gccccagcct tctttgcttg cctctctgtt tgtaaccttg 3840

tcgacaaaga ggtagaaaag attgggtcta ggatatggtg ggtggacagg ggccccggga 3900

cttggagggt tggtcctctt gcctcctgga aaaaacaaaa acaaaaaact gcagtgaaag 3960

acaagctgca aatcagccat gtgctgcgtg cctgtggaat ctggagtgag ggtaaaagc 4020

tgatctggtt tgactccgct ggaggtgggg cctggagcag gccttgcgct gttgcgtaac 4080

tggctgtgtt ctggtgaggc cttgctccca accccacacg ctcctccctc tgaggctgta 4140

ggactcgcag tcaggggcag ctgaccatgg aagattgaga gcccaaggtt taaacttctc 4200

tgaagggagg tggggatgag aagaggggtt tttttgtact ttgtacaaag accacacatt 4260

tgtgtaaaca gtgttttgga ataaaatatt tttttcataa aaaaaaaaa aaaa 4314

<210> 35
<211> 2888
<212> DNA
<213> Homo sapiens

<400> 35
atttgtggag accagccctc tggcttggtg agtgaatctg gtttacaccg gctcctgccc 60

tgccttcact cttctcccct gattcaagac tcctctgctt tggactgaag cactgcagga 120

gtttgtgacc aagaacttca agagtcaaga cagaaggaag ccaagggagc agtgcaatgg 180

atttctcagt aaaggtagac atagagaagg aggtgacctg ccccatctgc ctggagctcc 240

tgacagaacc tctgagccta gattgtggcc acagcttctg ccaagcctgc atcactgcaa 300

agatcaagga gtcagtgatc atctcaagag gggaaagcag ctgtcctgtg tgtcagacca 360

gattccagcc tgggaacctc cgacctaatc ggcatctggc caacatagtt gagagagtca 420

aagaggtcaa gatgagccca caggaggggc agaagagaga tgtctgtgag caccatggaa 480

aaaaactcca gatcttctgt aaggaggatg gaaaagtcat ttgctgggtt tgtgaactgt 540

ctcaggaaca ccaaggtcac caaacattcc gcataaacga ggtggtcaag gaatgtcagg 600

aaaagctgca ggtagccctg cagaggctga taaaggagga tcaagaggct gagaagctgg 660

aagatgacat cagacaagag agaaccgcct ggaagaatta tatccagatc gagagacaga 720

agattctgaa agggttcaat gaaatgagag tcatcttgga caatgaggag cagagagagc 780

tgcaaaagct ggaggaaggt gaggtgaatg tgctggataa cctggcagca gctacagacc 840

agctggtcca gcagaggcag gatgccagca cgctcatctc agatctccag cggaggttga 900

ggggatcgtc agtagagatg ctgcaggatg tgattgacgt catgaaaagg agtgaaagct 960

ggacattgaa gaagccaaaa tctgtttcca agaaactaaa gagtgtattc cgagtaccag 1020

atctgagtgg gatgctgcaa gttcttaaag agctgacaga tgtccagtac tactgggtgg 1080

acgtgatgct gaatccaggc agtgccactt cgaatgttgc tatttctgtg gatcagagac 1140

aagtgaaaac tgtacgcacc tgcacattta agaattcaaa tccatgtgat ttttctgctt 1200

ttggtgtctt cggctgccaa tatttctctt cggggaaata ttactgggaa gtagatgtgt 1260

ctggaaagat tgcctggatc ctgggcgtac acagtaaaat aagtagtctg aataaaagga 1320

agagctctgg gtttgctttt gatccaagtg taaattattc aaaagtttac tccagatata 1380

gacctcaata tggctactgg gttataggat tacagaatac atgtgaatat aatgcttttg 1440

aggactcctc ctcttctgat cccaaggttt tgactctctt tatggctgtg cctccctgtc 1500

gtattggggt tttcctagac tatgaggcag gcattgtctc atttttcaat gtcacaaacc 1560

acggagcact catctacaag ttctctggat gtcgcttttc tcgacctgct tatccgtatt 1620

tcaatccttg gaactgccta gtccccatga ctgtgtgccc accgagctcc tgagtgttct 1680

cattccttta cccacttctg catagtagcc cttgtgctga gactcagatt ctgcacctga 1740

gttcatctct actgagacca tctcttcctt tctttcccct tcttttactt agaatgtctt 1800

tgtattcatt tgctagggct tccatagcaa agcatcatag attgctgatt taaactgtaa 1860

ttgtattgcc gtactgtggg ctggaaatcc caaatctaga ttccagcaga gttggttctt 1920

tctgaggtct gcaaggaagg gctctgttcc atgcctctct ccttggcttg tagaaggcat 1980

cttgtcccta tgactcttca cattgtcttt atgtacatct ctgtgcccaa gttttccctt 2040

tttattaaga caccagtcat actggctcag ggcccaccgc taatgcctta atgaaatcat 2100

tttaacatta tattctctac aaagacctta tttccaaata agataatatt tggaggtatt 2160

gggaataaaa actccaacat ataaatttga ggaaggcacg atttcactca taacaatctt 2220

accctttctt gcaagagatg cttgtacatt attttcctaa taccttggtt tcactagtag 2280

taaacattat tatttttttt atatttgcaa aggaaacata tctaatcctt cctatagaaa 2340

gaacagtatt gctgtaattc cttttctttt cttcctcatt tcctctgccc cttaaaagat 2400

tgaagaaaga gaaacttgtc aactcatatc cacgttatct agcaaagtac ataagaatct 2460

atcactaagt aatgtatcct tcagaatgtg ttggtttacc agtgacaccc catattcatc 2520

acaaaattaa agcaagaagt ccatagtaat ttatttgcta atagtggatt tttaatgctc 2580

agagtttctg aggtcaaatt ttatcttttc acttacaagc tctatgatct taaataattt 2640

acttaatgta ttttggtgta ttttcctcaa attaatattg gtgttcaaga ctatatctaa 2700

ttcctctgat cactttgaga aacaaacttt tattaaatgt aaggcacttt tctatgaatt 2760

ttaaatataa aaataaatat tgttctgatt attactgaaa agatgtcagc catttcaatg 2820

tcttgggaaa caatttttttg tttttgttct gttttctttt tgcttcaata aaacaatagc 2880

tggctcta 2888

<210> 36
<211> 2269
<212> DNA
<213> Homo sapiens

<400> 36
ctccacaggt ccgccccaat ccccgctcac acttgggaaa cttgggactg cgctggggcc 60

gcgtgtggca cctcaggggg gcggcccccg gcctcaagag gagggggagg agaaggagga 120

agaggaggaa gtgagcccga aggatccgct cggagctgtt tgtccagctg tttctattcg 180

cacccggagc agtacagcca gaagggggcc gagccgaagg tggctggctt taggcgctaa 240

tttccaactc ttttcctcac agcttgtctt ttccaggcac cctggagtcc cctcaggcca 300

gctcggtggg cgcgcacctg ccagccgccc ctgacctcgc aggccaggcg acctccgagc 360

ctgagaagat ggcccagtcc aagctcgatt gccgctcacc tgtcggcctc gactgctgca 420

actgctgcct ggacctggcc catcggagtg ggctccagcg aggcagcagc ggggagaaca 480

acaacccggg cagccctaca gtgagcaact ttcggcagct gcaggaaaag ctggtctttg 540

agaacctcaa taccgacaag ctcaacagca taatgcggca ggattcgcta gagccggtgc 600

tgcgggaccc ctgctacctg atcaacgagg gcatctgcaa ccgcaacatc gaccagacca 660

tgctctccat cctgctcttc ttccacagtg cctccggagc cagcgtggtg gccatagaca 720

acaagatcga acaggccatg gatctggtga agaatcatct gatgtatgct gtgagagagg 780

aggtggagat cctgaaggag cagatccgag agctggtgga gaagaactcc cagctagagc 840

gtgagaacac cctgttgaag accctggcaa gcccagagca gctggagaag ttccagtcct 900

gtctgagccc tgaagagcca gctcccgaat ccccacaagt gcccgaggcc cctggtggtt 960

ctgcggtgta agtggctctg tcctcagggt gggcagagcc actaaacttg ttttacctag 1020

ttctttccag tttgtttttg gctccccaag catcatctca cgaggagaac tttacaccta 1080

gcacagctgg tgccaagaga tgtcctaagg acatggccac ctgggtccac tccagcgaca 1140

gacccctgac aagagcaggt ctctggaggc tgagttgcat ggggcctagt aacaccaagc 1200

cagtgagcct ctaatgctac tgcgccctgg gggctcccag ggcctgggca acttagctgc 1260

aactggcaaa ggagaagggt agtttgaggt gtgacaccag tttgctccag aaagtttaag 1320

gggtctgttt ctcatctcca tggacatctt caacagcttc acctgacaac gactgttcct 1380

atgaagaagc cacttgtgtt ttaagcagag gcaacctctc tcttctcctc tgtttcgtga 1440

aggcagggga cacagatggg agagattgag ccaagtcagc cttctgttgg ttaatatggt 1500

ataatgcatg gctttgtgca cagcccagtg tgggattaca gctttgggat gaccgcttac 1560

aaagttctgt ttggttagta ttggcatagt ttttctatat agccataaat gcgtatatat 1620

acccataggg ctagatctgt atcttagtgt agcgatgtat acatatacac atccacctac 1680

atgttgaagg gcctaaccag ccttgggagt attgactggt cccttacctc ttatggctaa 1740

gtctttgact gtgttcattt accaagttga cccagtttgt cttttaggtt aagtaagact 1800

cgagagtaaa ggcaaggagg ggggccagcc tctgaatgcg gccacggatg ccttgctgct 1860

gcaacccttt ccccagctgt ccactgaaac gtgaagtcct gttttgaatg ccaaacccac 1920

cattcactgg tgctgactac atagaatggg gttgagagaa gatcagtttg ggcttcacag 1980

tgtcatttga aaacgttttt tgttttgttt tgtaattatt gtggaaaact ttcaagtgaa 2040

cagaaggatg gtgtcctact gtggatgagg gatgaacaag gggatggctt tgatccaatg 2100

gagcctggga ggtgtgccca gaaagcttgt ctgtagcggg ttttgtgaga gtgaacactt 2160

tccacttttt gacaccttat cctgatgtat ggttccagga tttggatttt gattttccaa 2220

atgtagcttg aaatttcaat aaactttgct ctgtttttct aaaaataaa 2269

<210> 37
<211> 2572
<212> DNA
<213> Homo sapiens

<400> 37
tcccccaaag tgcggatcgg gcaataccac agagcctcag gacaggggga agcgccgtcc 60

tcttgcccac ggacgggtga gctggttcag tggctgagct caagatgccg ttggtgaaaa 120

gaaacatcga ccctcggcac ttgtgccaca cagcactgcc caggggcatt aagaacgaac 180

tggaatgtgt aaccaacatt tccctggcaa atataattag acaactgagt agcctaagta 240

agtatgctga agacatattt ggagaattat tcaatgaagc acacagtttt tccttcagag 300

ttaactcgtt gcaagagcgc gtggaccgat tgtctgttag tgtcacacag cttgatccta 360

aggaggaaga actgtctttg caagatataa cgatgagaaa ggctttccgg agctccacaa 420

ttcaagatca gcagcttttc gaccgcaaga cgttgcctat tccattgcaa gaaacatacg 480

atgtgtgtga gcaacctccg cctctcaata tcctcacccc ttacagggat gatggtaagg 540

aagggctgaa gttttatacc aatccttcat atttctttga tctgtggaaa gaaaagatgt 600

tgcaagatac agaggacaag aggaaggaaa agagaaagca gaagcagaaa aatctagacc 660

gtcctcatga accagagaaa gtgccaagag cacctcatga caggcggcgg gaatggcaga 720

agctggccca aggtccagag ctggctgagg atgacgctga cctcctacac aagcatattg 780

aagttgccaa tggcccagcc tctcattatg agacaaggcc acagacatac gtggatcata 840

tggacggatc gtactcactc tctgccttgc cattcagtca gatgagtgaa cttctgacca 900

gagctgagga gagggtctta gtcagacccc atgaacctcc tccgcctcca ccaatgcatg 960

gagcaggaga tgcaaagccc acacccacct gtatcagttc tgctacaggt ttgatagaga 1020

atcgccccca gtccccagct gcaggcagaa cacctgtgtt tgtgagcccc actcccccac 1080

ctcctccccc acctcttccg tctgccttgt ccacttcttc actaagagct tcaatgacct 1140

caactcctcc cccaccagtc cctcctccgc ctcctccccc agccactgct ttgcaggccc 1200

cagcagtgcc cccacctccg gctcctcttc agattgcccc tggtgttctt cacccagctc 1260

ctcctccaat tgctcctcct ctagtgcagc cctctccccc agtagctcga gctgccccag 1320

tatgcgagac tgtgccagtt cacccactcc cgcaaggtga agtgcagggg ctgcctccac 1380

ccccaccacc gcctcctcta cctccgcctg gcatccggcc atcatctcct gtcgcagttg 1440

cagctcttgc tcatcctccc tctgggctcc atccagcacc atctactgcc ccgggtcccc 1500

atgctccatt aatgcctcca tctcctccat cacaagttct acctgcctct gagccaaagc 1560

gccatccatc caccctgcct gtaatcagtg acgcaaggag tgtgctgctg gaagccatcc 1620

gaaaaggtat tcagcttcgc aaagtggaag agcagcgtga acaggaagca aaacacgagc 1680

ggattgaaaa cgacgtggcc accatcctgt ctcgccgcat tgctgtggag tatagcgact 1740

cggaagatga ctcggagttc gatgaagtag actggctgga gtaagaaagc cacagtgact 1800

tggaagatga ctcggagttc gatgaagtag actggatgga gtaagacagc cgccttgctc 1860

tgtattgcaa ggctgaatgc acatgtccct cgtggcgctt gttctttgaa aatgttcggt 1920

cattctagtg ttttgcttcc tattcctcat aatgaataac ccttttcctc cattattttt 1980

gatttctatg aaaagtatta gcatatattt caaactaaat attttgtggc ttaccttaat 2040

ttttcccccct ggaaaaatat aatttggtcc aataaactac taagtattaa gcatggaaag 2100

ccgttattag agtagcagat tcagtttttg atattcctta attgtgcact ttgtgatttt 2160

tttttttttt tttaaatttg aagaacgcaa ctgagactgg aatcttgaag gcagctgtgt 2220

ctgggaacga gccttattcc gtttcttgat gttttaaatg aagaaacact gcctcgatta 2280

tatgaataca ctcagaagga acatgtagct tgtagtgttg aattctctta aaggaatggt 2340

tagctttggt tatcattttg ttgtttttga atacttgcct tatttgaacg tttagcagaa 2400

ccccttccca cttatatatt gtgtggtaca gttttgcttg cctatagaag tttaaatttt 2460

tccatgtgaa atactcagaa taagcatatg tgtgacttac ataatgtgag aattacaatc 2520

tgatgtaata aacggttcat tttaaaggct aaaaaaaaaa aaaaaaaaaa aa 2572

<210> 38
<211> 3660
<212> DNA
<213> Homo sapiens

<400> 38
gcggaggctt ggggcagccg ggtagctcgg aggtcgtggc gctgggggct agcaccagcg 60

ctctgtcggg aggcgcagcg gttaggtgga ccggtcagcg gactcaccgg ccagggcgct 120

cggtgctgga atttgatatt cattgatccg ggttttatcc ctcttctttt ttcttaaaca 180

ttttttttta aaactgtatt gtttctcgtt ttaatttatt tttgcttgcc attccccact 240

tgaatcgggc cgacggcttg gggagattgc tctacttccc caaatcactg tggattttgg 300

aaaccagcag aaagaggaaa gaggtagcaa gagctccaga gagaagtcga ggaagagaga 360

gacggggtca gagagagcgc gcgggcgtgc gagcagcgaa agcgacaggg gcaaagtgag 420

tgacctgctt ttgggggtga ccgccggagc gcggcgtgag ccctccccct tgggatcccg 480

cagctgacca gtcgcgctga cggacagaca gacagacacc gcccccagcc ccagctacca 540
cctcctcccc ggccggcggc ggacagtgga cgcggcggcg agccgcgggc aggggccgga 600
gcccgcgccc ggaggcgggg tggagggggt cggggctcgc ggcgtcgcac tgaaactttt 660
cgtccaactt ctgggctgtt ctcgcttcgg aggagccgtg gtccgcgcgg gggaagccga 720
gccgagcgga gccgcgagaa gtgctagctc gggccgggag gagccgcagc cggaggaggg 780
ggaggaggaa gaagagaagg aagaggagag gggccgcag tggcgactcg gcgctcggaa 840
gccgggctca tggacgggtg aggcggcggt gtgcgcagac agtgctccag ccgcgcgcgc 900
tccccaggcc ctggcccggg cctcgggccg gggaggaaga gtagctcgcc gaggcgccga 960
ggagagcggg ccgccccaca gcccgagccg gagagggagc gcgagccgcg ccggccccgg 1020
tcgggcctcc gaaaccatga actttctgct gtcttgggtg cattggagcc ttgccttgct 1080
gctctacctc caccatgcca agtggtccca ggctgcaccc atggcagaag gaggagggca 1140
gaatcatcac gaagtggtga agttcatgga tgtctatcag cgcagctact gccatccaat 1200
cgagaccctg gtggacatct tccaggagta ccctgatgag atcgagtaca tcttcaagcc 1260
atcctgtgtg cccctgatgc gatgcggggg ctgctgcaat gacgagggcc tggagtgtgt 1320
gcccactgag gagtccaaca tcaccatgca gattatgcgg atcaaacctc accaaggcca 1380
gcacatagga gagatgagct tcctacagca caacaaatgt gaatgcagac caaagaaaga 1440
tagagcaaga caagaaaaaa aatcagttcg aggaaaggga aaggggcaaa aacgaaagcg 1500
caagaaatcc cggtataagt cctggagcgt gtacgttggt gcccgctgct gtctaatgcc 1560
ctggagcctc cctggccccc atccctgtgg gccttgctca gagcggagaa agcatttgtt 1620
tgtacaagat ccgcagacgt gtaaatgttc ctgcaaaaac acagactcgc gttgcaaggc 1680
gaggcagctt gagttaaacg aacgtacttg cagatgtgac aagccgaggc ggtgagccgg 1740
gcaggaggaa ggagcctccc tcagggtttc gggaaccaga tctctcacca ggaaagactg 1800
atacagaacg atcgatacag aaaccacgct gccgccacca caccatcacc atcgacagaa 1860
cagtccttaa tccagaaacc tgaaatgaag gaagaggaga ctctgcgcag agcactttgg 1920
gtccggaggg cgagactccg gcggaagcat tcccgggcgg gtgacccagc acggtccctc 1980
ttggaattgg attcgccatt ttatttttct tgctgctaaa tcaccgagcc cggaagatta 2040
gagagtttta tttctgggat tcctgtagac acacccaccc acatacatac atttatatat 2100
atatatatta tatatatata aaaataaata tctctatttt atatatataa aatatatata 2160
ttcttttttt aaattaacag tgctaatgtt attggtgtct tcactggatg tatttgactg 2220
ctgtggactt gagttgggag gggaatgttc ccactcagat cctgacaggg aagaggagga 2280
gatgagagac tctggcatga tctttttttt gtcccacttg gtggggccag ggtcctctcc 2340

```
cctgcccagg aatgtgcaag gccagggcat gggggcaaat atgacccagt tttgggaaca     2400
ccgacaaacc cagccctggc gctgagcctc tctaccccag gtcagacgga cagaaagaca     2460
gatcacaggt acagggatga ggacaccggc tctgaccagg agtttgggga gcttcaggac     2520
attgctgtgc tttggggatt ccctccacat gctgcacgcg catctcgccc ccaggggcac     2580
tgcctggaag attcaggagc ctgggcggcc ttcgcttact ctcacctgct tctgagttgc     2640
ccaggagacc actggcagat gtcccggcga agagaagaga cacattgttg gaagaagcag     2700
cccatgacag ctccccttcc tgggactcgc cctcatcctc ttcctgctcc ccttcctggg     2760
gtgcagccta aaaggaccta tgtcctcaca ccattgaaac cactagttct gtccccccag     2820
gagacctggt tgtgtgtgtg tgagtggttg accttcctcc atcccctggt ccttcccttc     2880
ccttcccgag gcacagagag acagggcagg atccacgtgc ccattgtgga ggcagagaaa     2940
agagaaagtg ttttatatac ggtacttatt taatatccct ttttaattag aaattaaaac     3000
agttaattta attaaagagt agggtttttt ttcagtattc ttggttaata tttaatttca     3060
actatttatg agatgtatct tttgctctct cttgctctct tatttgtacc ggtttttgta     3120
tataaaattc atgtttccaa tctctctctc cctgatcggt gacagtcact agcttatctt     3180
gaacagatat ttaattttgc taacactcag ctctgccctc cccgatcccc tggctcccca     3240
gcacacattc ctttgaaata aggtttcaat atacatctac atactatata tatatttggc     3300
aacttgtatt tgtgtgtata tatatatata tatgtttatg tatatatgtg attctgataa     3360
aatagacatt gctattctgt tttttatatg taaaaacaaa acaagaaaaa atagagaatt     3420
ctacatacta aatctctctc ctttttttaat tttaatattt gttatcattt atttattggt     3480
gctactgttt atccgtaata attgtgggga aaagatatta acatcacgtc tttgtctcta     3540
gtgcagtttt tcgagatatt ccgtagtaca tatttatttt taaacaacga caaagaaata     3600
cagatatatc ttaaaaaaaa aaaagcattt tgtattaaag aatttaattc tgatctcaaa     3660
```

**Description**

FIELD OF THE INVENTION

The present invention generally relates to the field of bioinformatics, genomic processing, proteomic processing, and related arts. More particularly, the present invention relates to a computer-implemented method for inferring activity of a PR cellular signaling pathway in a subject performed by a digital processing device, wherein the inferring is based on expression levels of three or more target genes of the PR cellular signaling pathway measured in a sample of the subject. The present invention further relates to an apparatus for inferring activity of a PR cellular signaling pathway in a subject comprising a digital processor configured to perform the method, to a non-transitory storage medium for inferring activity of a PR cellular signaling pathway in a subject storing instructions that are executable by a digital processing device to perform the method, and to a computer program for inferring activity of a PR cellular signaling pathway in a subject comprising program code means for causing a digital processing device to perform the method, when the computer program is run on the digital processing device. The present invention further relates to a kit for measuring expression levels of three or more target genes of the PR cellular signaling pathway in a sample of a subject, to a kit for inferring activity of a PR cellular signaling pathway in a subject, and to uses of the kits in performing the method.

BACKGROUND OF THE INVENTION

Genomic and proteomic analyses have substantial realized and potential promise for clinical application in medical fields such as oncology, where various cancers are known to be associated with specific combinations of genomic mutations/variations and/or high or low expression levels for specific genes, which play a role in growth and evolution of cancer, e.g., cell proliferation and metastasis.

The progesterone receptor (PR) protein is a member of the nuclear receptor family of transcription factors. The main function of PR is to mediate the effects of progesterone, a progestogen sex hormone which concerns the regulation of events associated with pregnancy and embryogenesis, and it also plays a role in the development of the mammary glands. The PR protein has two main isoforms, PR alpha (PR-A) and PR beta (PR-B), which both originate from the same gene, the progesterone receptor gene (PGR). PR-A is shorter than PR-B, lacking 164 amino acids in the N-terminus (the B-upstream segment or BUS).

PR can modulate gene expression by various mechanisms. The classical mechanism is the canonical PR pathway, where PR is activated by the presence of progestogens in the cell environment. In the absence of a progestogen ligand, PR is in the cytoplasm bound to a complex of chaperone proteins in a conformation favorable for ligand binding. When a progestogen is present, it binds to PR initiating a sequence of transformations that result in PR dimerization, translocation to the nucleus, and initiating transcription of PR target genes by binding to Progesterone Responsive Elements (PRE) in the target gene promoter region. The specific target genes expressed by the canonical PR pathway depend on factors such as the presence of co-ligands (co-activators or co-repressors), PR post-translational modifications (such as SUMOylation and phosphorylation), and steroid receptor interaction (e.g., with estrogen receptor alpha (ER$\alpha$)) scaffolding (see Dressing, G.E. et al., "Progesterone Receptors Act as Sensors for Mitogenic Protein Kinases in Breast Cancer Models", Endocrine-Related Cancer, Vol. 16, No.2, 2009, pages 351-61; Hagan, C.R. and Lange, C.A., "Molecular Determinants of Context-Dependent Progesterone Receptor Action in Breast Cancer", BMC Medicine, 12:32, 2014). Furthermore, PR can also act through extra-nuclear mechanisms (see Obr, A.E. and Edwards D.P., "The Biology of Progesterone Receptor in the Normal Mammary Gland and in Breast Cancer", Molecular and Cellular Endocrinology, Vol. 357, No. 1-2, 2012, pages 4-17).

In breast cancer, PR molecular staining is currently used in the clinic to help stratification of breast cancer subtypes. A hormone positive breast cancer, i.e., an estrogen receptor (ER) and PR positive breast cancer, is eligible for anti-estrogen therapy. In this case, PR is merely used as a marker for ER pathway activity. Though there is currently no approved therapy that indicates the use of (anti-)progestogens in the treatment of breast cancer, a number of studies has been carried out to investigate this matter. For example, the anti-progestogen mifepristone has been suggested as a treatment course option for breast cancer patients with high PR-A/PR-B ratio (see Lanari, C. et al., "Antiprogestins in Breast Cancer Treatment: Are We Ready?", Endocrine-Related Cancer, Vol. 19, No.3, 2012, pages 35-50; Rojas, P.A. et al., "Progesterone Receptor Isoform Ratio: A Breast Cancer Prognostic and Predictive Factor for Antiprogestin Responsiveness", Journal of the National Cancer Institute, Vol. 109, No.7, 2017). Moreover, pre-clinical studies in xenograft models indicate that progesterone has an additive effect over tamoxifen regarding controlling tumor growth (see Mohammed, H. et al., "Progesterone Receptor Modulates ER$\alpha$ Action in Breast Cancer", Nature, Vol. 523, No. 7560, 2015, pages 313-17). In endometrial cancer, progestogens are commonly used in the treatment of recurrent or advanced endometrial cancer with variable positive response rates (from 26% to 89%) (see Emons, G. et al., "Phase II Study of Fulvestrant 250 Mg/Month in Patients with Recurrent or Metastatic Endometrial Cancer: A Study of the Arbeitsgemeinschaft Gynakologische Onkologie", Gynecologic Oncology, Vol. 129, No.3, 2013, pages 495-99).

Currently the only method used as an indication of a possibly active PR pathway is PR molecular staining (sometimes PGR expression is also used as a surrogate marker for PR protein expression). Establishment that the PR protein is present in a sample is a necessary condition for an active PR pathway. However, this does not indicate an active PR pathway. Lack of a better marker for PR pathway activity could be a reason for the different rates of response to progestogen treatment in endometrial cancer and for discordant results regarding the role of PR in breast cancer. Indeed, there is evidence that progesterone increases PR pathway activity, but at the same time induces PR ubiquitination that targets the PR protein for degradation (see Patel, B. et al., "Role of Nuclear Progesterone Receptor Isoforms in Uterine Pathophysiology", Human Reproduction Update, Vol. 21, No.2, 2015, pages 155-73), which may cause an inverse correlation between the PR protein and PR pathway activation and, thus, hindering the effectiveness of using the PR protein as a surrogate for PR activity. Furthermore, PR activity promoted by means of PR-A, PR-B, or PR-A&B activation could also be a cause for such discordant findings. The development of a PR pathway activation marker, as well as specific PR-A and PR-B activation markers, can be important for attaining a consistent picture of the role played by the PR pathway in e.g. breast cancer, endometrial cancer, and other types of cancers in which PR is involved. More specifically, such makers for PR pathway activation could then be used as companion diagnostics for the indication of (anti-)progestogens in the treatment of cancer and as a prognostic maker.

SUMMARY OF THE INVENTION

In accordance with a main aspect of the present invention, the above problem is solved by a computer-implemented method for inferring activity of a PR cellular signaling pathway in a subject performed by a digital processing device, wherein the inferring comprises:

receiving expression levels of three or more target genes of the PR cellular signaling pathway measured in a sample of the subject,
determining an activity level of a PR transcription factor (TF) element in the sample of the subject, the PR TF element controlling transcription of the three or more PR target genes, the determining being based on evaluating a calibrated mathematical pathway model relating the expression levels of the three or more PR target genes to the activity level of the PR TF element, and
inferring the activity of the PR cellular signaling pathway in the subject based on the determined activity level of the PR TF element in the sample of the subject,
wherein the calibrated mathematical pathway model is PR-A specific and the three or more, for example, three, four, five, six, seven or more target genes are selected from the group consisting of: BCL2L1, BIRC3, DDIT4, F3, MUC1, NEDD9, SGK1, and TRIM22, preferably, from the group consisting of: BCL2L1, DDIT4, NEDD9, and TRIM22, and/or
wherein the calibrated mathematical pathway model is PR-B specific and the three or more, for example, three, four, five, six, seven, eight, nine, ten, eleven, twelve, thirteen, fourteen, fiveteen, sixteen, seventeen, eighteen, nineteen, twenty, twentyone or more target genes are selected from the group consisting of: ARRDC1, ATP1B1, BIRC3, CCND1, CD82, DDIT4, E2F1, F3, FKBP5, GOT1, HSD11B2, KANK1, MSX2, MUC1, MYC, NET1, NFKBIA, PDK4, PLIN2, PTP4A2, SNTB2, and STAT5A, preferably, from the group consisting of: ARRDC1, ATP1B1, CCND1, CD82, E2F1, FKBP5, GOT1, HSD11B2, KANK1, MSX2, MYC, NET1, NFKBIA, PDK4, PLIN2, PTP4A2, SNTB2, and STAT5A, preferably, from the group consisting of: ARRDC1, ATP1B1, CCND1, E2F1, FKBP5, HSD11B2, KANK1, MSX2, MYC, NET1, NFKBIA, PDK4, and PLIN2, preferably, from the group consisting of: CCND1, FKBP5, and MYC, and/or
wherein the calibrated mathematical pathway model is PR-A&B specific and the three or more, for example, three, four, five, six, seven, eight, nine, ten, eleven, twelve, thirteen, fourteen, fiveteen, sixteen, seventeen, eighteen, nineteen, twenty, twentyone, twentytwo, twentythree, twentyfour, twentyfive, twentysix, twentyseven, twentyeight, twentynine, thirty, thirtyone, thirtytwo, thirtythree, thirtyfour, thirtyfive, thirtysix, thirtyseven or more target genes are selected from the group consisting of: ABCG2, ACSS1, AK4, ARRDC1, ATP1B1, BCL2L1, BCL6, BIRC3, CCND1, CD82, CDKN1A, DDIT4, E2F1, F3, FKBP5, GOT1, GRB10, HPCAL1, HSD11B2, KANK1, KLF4, MSX2, MUC1, MYC, NEDD9, NET1, NFKBIA, PDK4, PLIN2, PTP4A2, S100P, SGK1, SNTB2, STAT5A, TRIM22, TSC22D3, VASP, and VEGFA, preferably, from the group consisting of: AK4, ARRDC1, ATP1B1, BCL2L1, BCL6, BIRC3, CCND1, CD82, F3, FKBP5, GOT1, GRB10, HSD11B2, KLF4, MUC1, MYC, NEDD9, NET1, PDK4, PTP4A2, S100P, SGK1, SNTB2, STAT5A, TSC22D3, and VASP, or, preferably, from the group consisting of: ABCG2, ACSS1, AK4, ATP1B1, BCL6, CCND1, FKBP5, GRB10, HSD11B2, KANK1, KLF4, MYC, NFKBIA, PDK4, PLIN2, S100P, TSC22D3, and VASP, or, preferably, from the group consisting of: BCL6, CCND1, CDKN1A, FKBP5, MYC, SGK1, and VEGFA, or, preferably, from the group consisting of: BCL6, CCND1, FKBP5, and MYC.

Herein, the "activity level" of a TF element denotes the level of activity of the TF element regarding transcription of its target genes.

The present invention is based on the innovation of the inventors that a suitable way of identifying effects occurring in the PR cellular signaling pathway can be based on a measurement of the signaling output of the PR cellular signaling pathway, which is - amongst others - the transcription of the target genes, which is controlled by a PR transcription factor (TF) element that is controlled by the PR cellular signaling pathway. This innovation by the inventors assumes that the TF activity level is at a quasi-steady state in the sample, which can be detected by means of- amongst others - the expression values of the PR target genes. The PR cellular signaling pathway targeted herein is known to control many functions in many cell types in humans, such as proliferation, differentiation and wound healing. Regarding pathological disorders, such as cancer (e.g., breast, endometrial, ovarian, lung or acute lymphoblastic leukemia (ALL) cancer), the abnormal PR cellular signaling activity plays an important role, which is detectable in the expression profiles of the target genes and thus exploited by means of a calibrated mathematical pathway model.

The present invention makes it possible to determine the activity of the PR cellular signaling pathway in a subject by (i) determining an activity level of a PR TF element in the sample of the subject, wherein the determining is based on evaluating a calibrated mathematical model relating the expression levels of three or more target genes of the PR cellular signaling pathway, the transcription of which is controlled by the PR TF element, to the activity level of the PR TF element, and by (ii) inferring the activity of the PR cellular signaling pathway in the subject based on the determined activity level of the PR TF element in the sample of the subject. This preferably allows improving the possibilities of characterizing patients that have a disease, such as cancer, e.g., breast, endometrial, ovarian, lung or acute lymphoblastic leukemia (ALL) cancer, which is at least partially driven by an abnormal activity of the PR cellular signaling pathway, and that are therefore likely to respond to inhibitors of the PR cellular signaling pathway. Likewise, in cases where the PR cellular signaling pathway is acting as a protective (tumor suppressive) pathway, an abormally low activity of the pathway may preferably be identified using the methods of the present invention and a treatment that increases the activity of the PR cellular signaling pathway, for instance, by providing progesterone to the patient, may be given to the patient. The present invention also indicates that an imbalance of PR-A activity to PR-B activity seems to be an indication of a higher aggressiveness of a cancer. In particular embodiments, treatment determination can be based on a specific PR cellular signaling pathway activity. In a particular embodiment, the PR cellular signaling status can be set at a cutoff value of odds of the PR cellular signaling pathway being active of, for example, 10:1, 5:1, 4:1, 2:1, 1:1, 1:2, 1:4, 1:5, or 1:10.

Herein, the term "Progesterone receptor transcription factor element" or "PR TF element" or "TF element" is defined to be a protein complex containing at least the intracellular PR (PR-A, PR-B), with necessary co-factors, such as p300. Preferably, the term refers to either a protein or protein complex transcriptional factor, activated by binding a specific ligand such as progesterone or by an activating mutation in the PR gene.

The calibrated mathematical pathway model may be a probabilistic model, preferably a Bayesian network model, based on conditional probabilities relating the activity level of the PR TF element and the expression levels of the three or more PR target genes, or the calibrated mathematical pathway model may be based on one or more linear combination(s) of the expression levels of the three or more PR target genes. In particular, the inferring of the activity of the PR cellular signaling pathway may be performed as disclosed in the published international patent application WO 2013/011479 A2 ("Assessment of cellular signaling pathway activity using probabilistic modeling of target gene expression") or as described in the published international patent application WO 2014/102668 A2 ("Assessment of cellular signaling pathway activity using linear combination(s) of target gene expressions"), the contents of which are herewith incorporated in their entirety. Further details regarding the inferring of cellular signaling pathway activity using mathematical modeling of target gene expression can be found in Verhaegh W. et al., "Selection of personalized patient therapy through the use of knowledge-based computational models that identify tumor-driving signal transduction pathways", Cancer Research, Vol. 74, No. 11, 2014, pages 2936-2945.

The term "subject", as used herein, refers to any living being. In some embodiments, the subject is an animal, preferably a mammal. In certain embodiments, the subject is a human being, preferably a medical subject. In still other embodiments, the subject is a cell line.

The term "target gene" as used herein, means a gene whose transcription is directly or indirectly controlled by a PR transcription factor element. The "target gene" may be a "direct target gene" and/or an "indirect target gene" (as described herein). Moreover, the "target genes" may be "direct target genes" and/or "indirect target genes" (as described herein).

Particularly suitable PR target genes are described in the following text passages as well as the examples below (see, e.g., Tables 6 to 8 below).

Thus, according to a preferred embodiment the PR target genes are selected from the group consisting of the PR target genes listed in Table 6, Table 7, or Table 8 below.

Other suitable PR target genes are listed in Table 9, Table 10, Table 11, Table 12, Table 13, Table 14, Table 15, or Table 16 below.

Another aspect of the present invention relates to a method (as described herein), further comprising:

determining whether the PR cellular signaling pathway is operating abnormally in the subject based on the inferred activity of the PR cellular signaling pathway in the subject.

Another aspect of the present invention relates to a method (as described herein), further comprising: determining a prognostic cancer marker based on a combination of inferred activities of the PR cellular signaling pathway in the subject using two or more of the PR-A specific calibrated mathematical pathway model, the PR-B specific calibrated mathematical pathway model and the PR-A&B specific calibrated mathematical pathway model.

The present invention also relates to a method (as described herein), wherein the combination is a ratio between the inferred activity of the PR cellular signaling pathway in the subject using the PR-A&B specific calibrated mathematical pathway model and the inferred activity of the PR cellular signaling pathway in the subject using the PR-B specific calibrated mathematical pathway model.

The present invention also relates to a method (as described herein), further comprising:

recommending prescribing a drug for the subject that corrects for the abnormal operation of the PR cellular signaling pathway,
wherein the recommending is performed if the PR cellular signaling pathway is determined to be operating abnormally in the subject based on the inferred activity of the PR cellular signaling pathway.

The phrase "the cellular signaling pathway is operating abnormally" refers to the case where the "activity" of the pathway is not as expected, wherein the term "activity" may refer to the activity of the transcription factor complex in driving the target genes to expression, i.e., the speed by which the target genes are transcribed. "Normal" may be when it is inactive in tissue where it is expected to be inactive and active where it is expected to be active. Furthermore, there may be a certain level of activity that is considered "normal", and anything higher or lower maybe considered "abnormal".

The present invention also relates to a method (as described herein), wherein the abnormal operation of the PR cellular signaling pathway is an operation in which the PR cellular signaling pathway operates as a tumor promoter in the subject.

The sample(s) to be used in accordance with the present invention can be an extracted sample, that is, a sample that has been extracted from the subject. Examples of the sample include, but are not limited to, a tissue, cells, blood and/or a body fluid of a subject. If the subject is a medical subject that has or may have cancer, it can be, e.g., a sample obtained from a cancer lesion, or from a lesion suspected for cancer, or from a metastatic tumor, or from a body cavity in which fluid is present which is contaminated with cancer cells (e.g., pleural or abdominal cavity or bladder cavity), or from other body fluids containing cancer cells, and so forth, preferably via a biopsy procedure or other sample extraction procedure. The cells of which a sample is extracted may also be tumorous cells from hematologic malignancies (such as leukemia or lymphoma). In some cases, the cell sample may also be circulating tumor cells, that is, tumor cells that have entered the bloodstream and may be extracted using suitable isolation techniques, e.g., apheresis or conventional venous blood withdrawal. Aside from blood, a body fluid of which a sample is extracted may be urine, gastrointestinal contents, or an extravasate. The term "sample", as used herein, also encompasses the case where e.g. a tissue and/or cells and/or a body fluid of the subject have been taken from the subject and, e.g., have been put on a microscope slide, and where for performing the claimed method a portion of this sample is extracted, e.g., by means of Laser Capture Microdissection (LCM), or by scraping off the cells of interest from the slide, or by fluorescence-activated cell sorting techniques. In addition, the term "sample", as used herein, also encompasses the case where e.g. a tissue and/or cells and/or a body fluid of the subject have been taken from the subject and have been put on a microscope slide, and the claimed method is performed on the slide. In addition, the term "sample", as used herein, also encompasses the case where e.g. a cell line and/or cell culture has been generated based on the cells/tissue/body fluid that have been taken from the subject.

In accordance with another disclosed aspect, an apparatus for inferring activity of a PR cellular signaling pathway in a subject comprises a digital processor configured to perform the method of the present invention as described herein.

In accordance with another disclosed aspect, a non-transitory storage medium for inferring activity of a PR cellular signaling pathway in a subject stores instructions that are executable by a digital processing device to perform the method of the present invention as described herein. The non-transitory storage medium may be a computer-readable storage medium, such as a hard drive or other magnetic storage medium, an optical disk or other optical storage medium, a random access memory (RAM), read only memory (ROM), flash memory, or other electronic storage medium, a network server, or so forth. The digital processing device may be a handheld device (e.g., a personal data assistant or smartphone), a notebook computer, a desktop computer, a tablet computer or device, a remote network server, or so forth.

In accordance with another disclosed aspect, a computer program for inferring activity of a PR cellular signaling pathway in a subject comprises program code means for causing a digital processing device to perform the method of the present invention as described herein, when the computer program is run on the digital processing device. The digital processing device may be a handheld device (e.g., a personal data assistant or smartphone), a notebook computer, a desktop computer, a tablet computer or device, a remote network server, or so forth.

In accordance with another disclosed aspect, a kit for measuring expression levels of three or more target genes of the PR cellular signaling pathway in a sample of a subject comprises:

one or more components for determining the expression levels of the three or more PR target genes in the sample of the subject,
wherein the calibrated mathematical pathway model is PR-A specific and the three or more, for example, three, four, five, six, seven or more target genes are selected from the group consisting of: BCL2L1, BIRC3, DDIT4, F3, MUC1, NEDD9, SGK1, and TRIM22, preferably, from the group consisting of: BCL2L1, DDIT4, NEDD9, and TRIM22, and/or
wherein the calibrated mathematical pathway model is PR-B specific and the three or more, for example, three, four, five, six, seven, eight, nine, ten, eleven, twelve, thirteen, fourteen, fiveteen, sixteen, seventeen, eighteen, nineteen, twenty, twentyone or more target genes are selected from the group consisting of: ARRDC1, ATP1B1, BIRC3, CCND1, CD82, DDIT4, E2F1, F3, FKBP5, GOT1, HSD11B2, KANK1, MSX2, MUC1, MYC, NET1, NFKBIA, PDK4, PLIN2, PTP4A2, SNTB2, and STAT5A, preferably, from the group consisting of: ARRDC1, ATP1B1, CCND1, CD82, E2F1, FKBP5, GOT1, HSD11B2, KANK1, MSX2, MYC, NET1, NFKBIA, PDK4, PLIN2, PTP4A2, SNTB2, and STAT5A, preferably, from the group consisting of: ARRDC1, ATP1B1, CCND1, E2F1, FKBP5, HSD11B2, KANK1, MSX2, MYC, NET1, NFKBIA, PDK4, and PLIN2, preferably, from the group consisting of: CCND1, FKBP5, and MYC, and/or
wherein the calibrated mathematical pathway model is PR-A&B specific and the three or more, for example, three, four, five, six, seven, eight, nine, ten, eleven, twelve, thirteen, fourteen, fiveteen, sixteen, seventeen, eighteen, nineteen, twenty, twentyone, twentytwo, twentythree, twenty four, twentyfive, twentysix, twentyseven, twentyeight, twentynine, thirty, thirtyone, thirtytwo, thirtythree, thirtyfour, thirtyfive, thirtysix, thirtyseven or more target genes are selected from the group consisting of: ABCG2, ACSS1, AK4, ARRDC1, ATP1B1, BCL2L1, BCL6, BIRC3, CCND1, CD82, CDKN1A, DDIT4, E2F1, F3, FKBP5, GOT1, GRB10, HPCAL1, HSD11B2, KANK1, KLF4, MSX2, MUC1, MYC, NEDD9, NET1, NFKBIA, PDK4, PLIN2, PTP4A2, S100P, SGK1, SNTB2, STAT5A, TRIM22, TSC22D3, VASP, and VEGFA, preferably, from the group consisting of: AK4, ARRDC1, ATP1B1, BCL2L1, BCL6, BIRC3, CCND1, CD82, F3, FKBP5, GOT1, GRB10, HSD11B2, KLF4, MUC1, MYC, NEDD9, NET1, PDK4, PTP4A2, S100P, SGK1, SNTB2, STAT5A, TSC22D3, and VASP, or, preferably, from the group consisting of: ABCG2, ACSS1, AK4, ATP1B1, BCL6, CCND1, FKBP5, GRB10, HSD11B2, KANK1, KLF4, MYC, NFKBIA, PDK4, PLIN2, S100P, TSC22D3, and VASP, or, preferably, from the group consisting of: BCL6, CCND1, CDKN1A, FKBP5, MYC, SGK1, and VEGFA, or, preferably, from the group consisting of: BCL6, CCND1, FKBP5, and MYC.

The one or more components or means for measuring the expression levels of the three or more PR target genes can be selected from the group consisting of: a DNA array chip, an oligonucleotide array chip, a protein array chip, an antibody, a plurality of probes, for example, labeled probes, a set of RNA reverse-transcriptase sequencing components, and/or RNA or DNA, including cDNA, amplification primers. In an embodiment, the kit includes a set of labeled probes directed to a portion of an mRNA or cDNA sequence of the three or more PR target genes as described herein. In an embodiment, the kit includes a set of primers and probes directed to a portion of an mRNA or cDNA sequence of the three or more PR target genes. In an embodiment, the labeled probes are contained in a standardized 96-well plate. In an embodiment, the kit further includes primers or probes directed to a set of reference genes. Such reference genes can be, for example, constitutively expressed genes useful in normalizing or standardizing expression levels of the target gene expression levels described herein.

In an embodiment, the kit for measuring the expression levels of three or more target genes of the PR cellular signaling pathway in a sample of a subject comprises:

polymerase chain reaction primers directed to the three or more PR target genes,
probes directed to the three or more PR target genes,
wherein the calibrated mathematical pathway model is PR-A specific and the three or more, for example, three, four, five, six, seven or more target genes are selected from the group consisting of: BCL2L1, BIRC3, DDIT4, F3, MUC1, NEDD9, SGK1, and TRIM22, preferably, from the group consisting of: BCL2L1, DDIT4, NEDD9, and TRIM22, and/or
wherein the calibrated mathematical pathway model is PR-B specific and the three or more, for example, three, four, five, six, seven, eight, nine, ten, eleven, twelve, thirteen, fourteen, fiveteen, sixteen, seventeen, eighteen, nineteen, twenty, twentyone or more target genes are selected from the group consisting of: ARRDC1, ATP1B1, BIRC3, CCND1, CD82, DDIT4, E2F1, F3, FKBP5, GOT1, HSD11B2, KANK1, MSX2, MUC1, MYC, NET1, NFKBIA, PDK4, PLIN2, PTP4A2, SNTB2, and STAT5A, preferably, from the group consisting of: ARRDC1, ATP1B1, CCND1, CD82, E2F1, FKBP5, GOT1, HSD11B2, KANK1, MSX2, MYC, NET1, NFKBIA, PDK4, PLIN2, PTP4A2, SNTB2, and STAT5A, preferably, from the group consisting of: ARRDC1, ATP1B1, CCND1, E2F1, FKBP5, HSD11B2,

KANK1, MSX2, MYC, NET1, NFKBIA, PDK4, and PLIN2, preferably, from the group consisting of: CCND1, FKBP5, and MYC, and/or

wherein the calibrated mathematical pathway model is PR-A&B specific and the three or more, for example, three, four, five, six, seven, eight, nine, ten, eleven, twelve, thirteen, fourteen, fiveteen, sixteen, seventeen, eighteen, nineteen, twenty, twentyone, twentytwo, twentythree, twentyfour, twentyfive, twentysix, twentyseven, twentyeight, twentynine, thirty, thirtyone, thirtytwo, thirtythree, thirtyfour, thirtyfive, thirtysix, thirtyseven or more target genes are selected from the group consisting of: ABCG2, ACSS1, AK4, ARRDC1, ATP1B1, BCL2L1, BCL6, BIRC3, CCND1, CD82, CDKN1A, DDIT4, E2F1, F3, FKBP5, GOT1, GRB10, HPCAL1, HSD11B2, KANK1, KLF4, MSX2, MUC1, MYC, NEDD9, NET1, NFKBIA, PDK4, PLIN2, PTP4A2, S100P, SGK1, SNTB2, STAT5A, TRIM22, TSC22D3, VASP, and VEGFA, preferably, from the group consisting of: AK4, ARRDC1, ATP1B1, BCL2L1, BCL6, BIRC3, CCND1, CD82, F3, FKBP5, GOT1, GRB10, HSD11B2, KLF4, MUC1, MYC, NEDD9, NET1, PDK4, PTP4A2, S100P, SGK1, SNTB2, STAT5A, TSC22D3, and VASP, or, preferably, from the group consisting of: ABCG2, ACSS1, AK4, ATP1B1, BCL6, CCND1, FKBP5, GRB10, HSD11B2, KANK1, KLF4, MYC, NFKBIA, PDK4, PLIN2, S100P, TSC22D3, and VASP, or, preferably, from the group consisting of: BCL6, CCND1, CDKN1A, FKBP5, MYC, SGK1, and VEGFA, or, preferably, from the group consisting of: BCL6, CCND1, FKBP5, and MYC.

In accordance with another disclosed aspect, a kit for inferring activity of a PR cellular signaling pathway in a subject comprises:

the kit of the present invention as described herein, and

the apparatus of the present invention as described herein, the non-transitory storage medium of the present invention as described herein, or the computer program of the present invention as described herein.

In accordance with another disclosed aspect, the kits of the present invention as described herein are used in performing the method of the present invention as described herein.

The present invention as described herein can, e.g., also advantageously be used in at least one of the following activities:

diagnosis based on the inferred activity of the PR cellular signaling pathway in the subject;

prognosis based on the inferred activity of the PR cellular signaling pathway in the subject;

drug prescription based on the inferred activity of the PR cellular signaling pathway in the subject;

prediction of drug efficacy based on the inferred activity of the PR cellular signaling pathway in the subject;

prediction of adverse effects based on the inferred activity of the PR cellular signaling pathway in the subject;

monitoring of drug efficacy;

drug development;

assay development;

pathway research;

cancer staging;

enrollment of the subject in a clinical trial based on the inferred activity of the PR cellular signaling pathway in the subject;

selection of subsequent test to be performed; and

selection of companion diagnostics tests.

Further advantages will be apparent to those of ordinary skill in the art upon reading and understanding the attached figures, the following description and, in particular, upon reading the detailed examples provided herein below.

It shall be understood that the method of claim 1, the apparatus of claim 9, the non-transitory storage medium of claim 10, the computer program of claim 11, the kits of claims 12 to 14, and the use of the kits of claim 15 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

Fig. 1 shows schematically and exemplarily a mathematical model, herein, a Bayesian network model, used to model the transcriptional program of the PR cellular signaling pathway.

Fig. 2 shows a flow chart exemplarily illustrating a process for inferring activity of the PR cellular signaling pathway in a subject based on expression levels of target genes of the PR cellular signaling pathway measured in a sample of a subject.

Fig. 3 shows a flow chart exemplarily illustrating a process for obtaining a calibrated mathematical pathway model as described herein.

Fig. 4 shows a flow chart exemplarily illustrating a process for determining an activity level of a PR transcription factor (TF) element in a sample of a subject as described herein.

Fig. 5 shows a flow chart exemplarily illustrating a process for inferring activity of a PR cellular signaling pathway in a subject using discretized observables.

Fig. 6 shows a flow chart exemplarily illustrating a process for inferring activity of a PR cellular signaling pathway in a subject using continuous observables.

Fig. 7 shows a flow chart exemplarily illustrating a process for determining Cq values from RT-qPCR analysis of the target genes of the PR cellular signaling pathway.

Fig. 8 shows PR cellular signaling pathway activity predictions of trained Bayesian network models on QC pass normal endometrium samples from GSE29921.

Figs. 9 and 10 show PR cellular signaling pathway activity predictions of trained Bayesian network models on QC pass normal endometrium samples from GSE6364 and GSE51981, respectively.

Fig. 11 shows the progression of the menstrual cycle and the different hormones contributing to it.

Fig. 12 shows PR cellular signaling pathway activity predictions of trained Bayesian network models on fallopian tube samples from GSE10971.

Fig. 13 shows PR cellular signaling pathway activity predictions of trained Bayesian network models on QC pass menopausal normal endometrium samples from GSE12446.

Fig. 14 shows PR cellular sugnaling pathway activity predictions of trained Bayesian network models on QC pass term decidual cells (from normal placentae) samples from GSE65835.

Fig. 15 shows PR cellular signaling pathway activity predictions of trained Bayesian network models on QC pass PR-A and PR-B expressing ishikawa IKPR-AB36 endometrium cell line samples from GSE29435.

Fig. 16 shows PR cellular signaling pathway activity predictions of trained Bayesian network models on QC pass PR-B expressing hMEC breast cell line samples from GSE24468.

Fig. 17 shows PR cellular signaling pathway activity predictions of trained Bayesian network models on QC pass paired normal vs. breast cancer samples from GSE10810.

Fig. 18 shows PR cellular signaling pathway activity predictions of trained Bayesian network models on normal vs. endometrial cancer samples from GSE17025.

Fig. 19 shows PR cellular signaling pathway activity predictions of trained Bayesian network models on paired normal vs. tumor lung cancer samples. Left: Samples from non-smoking female lung cancer in Taiwan from GSE19804; right: Samples from stage I lung adenocarcinoma from GSE27626.

Fig. 20 shows PR cellular signaling pathway activity predictions of trained Bayesian network models on normal and tumor lung cancer samples from GSE30219.

Fig. 21 shows PR cellular signaling pathway activity predictions of trained Bayesian network models on normal vs. idiopathic pulmonary fibrosis (IPF) samples from GSE24206.

Fig. 22 shows PR-B cellular signaling pathway activity predictions of trained Bayesian network models on QC pass paired pre/post letrozole treatment breast cancer samples from GSE10281.

Fig. 23 shows PR cellular signaling pathway activity predictions of trained Bayesian network models as a prognostic risk factor for the progression of lung cancer on QC pass samples from GSE30219.

Fig. 24 shows combined PR cellular signaling pathway activities predictions of trained Bayesian network models as a prognostic risk factor for the progression of lung cancer on QC pass samples from GSE30219.

Fig. 25 shows PR cellular signaling pathway activity predictions of trained Bayesian network models as a prognostic risk factor for the progression of breast cancer on QC pass samples from GSE6532, GSE9195, GSE21653, GSE20685, GSE58812, and EMTAB365.

Fig. 26 shows PR cellular signaling pathway activity predictions of trained Bayesian network models as a prognostic risk factor for the progression of pediatric acute lymphoblastic leukemia (ALL) on QC pass samples from GSE13576.

Fig. 27 shows PR cellular signaling pathway activity predictions of trained Bayesian network models as a prognostic risk factor in breast cancer on primary normal breast cell cultures samples from GSE13671.

Fig. 28 shows PR cellular signaling pathway activity predictions of linear models as a predictor for progestogen therapy response in endometrial cancer.

Figs. 29 and 30 show PR cellular signaling pathway activity predictions of trained Bayesian network models calibrated with the alternative calibration set IKAB01M (IPR-AB36 endometrial cell line treated with vehicle control or InM MPA; see Table 4) for the preferred PR-A, PR-B and PR-A&B target gene lists.

Figs. 31 and 32 show PR cellular pathway activity predictions of trained Bayesian network models calibrated with various alternative target gene lists and the preferred calibration set EmLPxHS (see Table 4).

DETAILED DESCRIPTION OF EMBODIMENTS

The following examples merely illustrate particularly preferred methods and selected aspects in connection therewith. The teaching provided therein may be used for constructing several tests and/or kits, e.g., to detect, predict and/or diagnose the abnormal activity of the PR cellular signaling pathway. Furthermore, upon using methods as described herein drug prescription can advantageously be guided, drug response prediction and monitoring of drug efficacy (and/or adverse effects) can be made, drug resistance can be predicted and monitored, e.g., to select subsequent test(s) to be performed (like a companion diagnostic test). The following examples are not to be construed as limiting the scope of the present invention.

**1. Mathematical model construction**

As described in detail in the published international patent application WO 2013/011479 A2 ("Assessment of cellular signaling pathway activity using probabilistic modeling of target gene expression"), by constructing a probabilistic model, e.g., a Bayesian network model, and incorporating conditional probabilistic relationships between the expression levels of three or more target genes of a cellular signaling pathway, herein, the PR cellular signaling pathway, and the activity level of a transcription factor (TF) element, herein, the PR TF element, the TF element controlling transcription of the three or more target genes of the cellular signaling pathway, such a model may be used to determine the activity of the cellular signaling pathway with a high degree of accuracy. Moreover, the probabilistic model can be readily updated to incorporate additional knowledge obtained by later clinical studies, by adjusting the conditional probabilities and/or adding new nodes to the model to represent additional information sources. In this way, the probabilistic model can be updated as appropriate to embody the most recent medical knowledge.

In another easy to comprehend and interpret approach described in detail in the published international patent application WO 2014/102668 A2 ("Assessment of cellular signaling pathway activity using linear combination(s) of target gene expressions"), the activity of a cellular signaling pathway, herein, the PR cellular signaling pathway, may be determined by constructing and evaluating a linear or (pseudo-)linear model incorporating relationships between expression levels of three or more target genes of the cellular signaling pathway and the level of a transcription factor (TF) element, herein, the PR TF element, the TF element controlling transcription of the three or more target genes of the cellular signaling pathway, the model being based on one or more linear combination(s) of expression levels of the three or more target genes.

In both approaches, the expression levels of the three or more target genes may preferably be measurements of the level of mRNA, which can be the result of, e.g., (RT)-PCR and microarray techniques using probes associated with the target genes mRNA sequences, and of RNA-sequencing. In another embodiment, the expression levels of the three or more target genes can be measured by protein levels, e.g., the concentrations and/or activity of the protein(s) encoded by the target genes.

The aforementioned expression levels may optionally be converted in many ways that might or might not suit the application better. For example, four different transformations of the expression levels, e.g., microarray-based mRNA levels, may be:

- "continuous data", i.e., expression levels as obtained after preprocessing of microarrays using well known algorithms such as MAS5.0 and fRMA,
- "z-score", i.e., continuous expression levels scaled such that the average across all samples is 0 and the standard deviation is 1,
- "discrete", i.e., every expression above a certain threshold is set to 1 and below it to 0 (e.g., the threshold for a probeset may be chosen as the (weighted) median of its value in a set of a number of positive and the same number of negative clinical samples),
- "fuzzy", i.e., the continuous expression levels are converted to values between 0 and 1 using a sigmoid function of the following format: 1 / (1 + *exp*((*thr* - *expr*) / *se*)), with *expr* being the continuous expression levels, *thr* being the threshold as mentioned before and *se* being a softening parameter influencing the difference between 0 and 1.

One of the simplest linear models that can be constructed is a model having a node representing the transcription factor (TF) element, herein, the PR TF element, in a first layer and weighted nodes representing direct measurements of the target genes expression levels, e.g., by one probeset that is particularly highly correlated with the particular target gene, e.g., in microarray or (q)PCR experiments, in a second layer. The weights can be based either on calculations from a training data set or based on expert knowledge. This approach of using, in the case where possibly multiple expression levels are measured per target gene (e.g., in the case of microarray experiments, where one target gene can be measured with multiple probesets), only one expression level per target gene is particularly simple. A specific way of selecting the one expression level that is used for a particular target gene is to use the expression level from the probeset that is able to separate active and passive samples of a training data set the best. One method to determine this probeset is to perform a statistical test, e.g., the t-test, and select the probeset with the lowest p-value. The training data set's expression levels of the probeset with the lowest p-value is by definition the probeset with the least likely probability that the expression levels of the (known) active and passive samples overlap. Another selection method is based on odds-ratios. In such a model, one or more expression level(s) are provided for each of the three or more target genes and the one or more linear combination(s) comprise a linear combination including for each of the three or more target genes a weighted term, each weighted term being based on only one expression level of the one or more expression level(s) provided for the respective target gene. If only one expression level is chosen per target gene as described above, the model may be called a "most discriminant probesets" model.

In an alternative to the "most discriminant probesets" model, it is possible, in the case where possibly multiple expression levels are measured per target gene, to make use of all the expression levels that are provided per target gene. In such a model, one or more expression level(s) are provided for each of the three or more target genes and the one or more linear combination(s) comprise a linear combination of all expression levels of the one or more expression level(s) provided for the three or more target genes. In other words, for each of the three or more target genes, each of the one or more expression level(s) provided for the respective target gene may be weighted in the linear combination by its own (individual) weight. This variant may be called an "all probesets" model. It has an advantage of being relatively simple while making use of all the provided expression levels.

Both models as described above have in common that they are what may be regarded as "single-layer" models, in which the activity level of the TF element is calculated based on a linear combination of expression levels of the one or more probeset of the three or more target genes.

After the activity level of the TF element, herein, the PR TF element, has been determined by evaluating the respective model, the determined TF element activity level can be thresholded in order to infer the activity of the cellular signaling pathway, herein, the PR cellular signaling pathway. A preferred method to calculate such an appropriate threshold is by comparing the determined TF element activity levels *wlc* (weighted linear combination) of training samples known to have a passive cellular signaling pathway and training samples with an active cellular signaling pathway. A method that does so and also takes into account the variance in these groups is given by using a threshold

$$thr = \frac{\sigma_{wlc_{\mathrm{pas}}}\,\mu_{wlc_{\mathrm{act}}} + \sigma_{wlc_{\mathrm{act}}}\mu_{wlc_{\mathrm{pas}}}}{\sigma_{wlc_{\mathrm{pas}}} + \sigma_{wlc_{\mathrm{act}}}} \qquad (1)$$

where $\sigma$ and $\mu$ are the standard deviation and the mean of the determined TF element activity levels w*lc* for the training samples. In case only a small number of samples are available in the active and/or passive training samples, a pseudo count may be added to the calculated variances based on the average of the variances of the two groups:

$$\begin{aligned} \tilde{v} &= \frac{v_{wlc_{act}} + v_{wlc_{pas}}}{2} \\ \tilde{v}_{wlc_{act}} &= \frac{x\,\tilde{v} + (n_{act} - 1)v_{wlc_{act}}}{x + n_{act} - 1} \\ \tilde{v}_{wlc_{pas}} &= \frac{x\,\tilde{v} + (n_{pas} - 1)v_{wlc_{pas}}}{x + n_{pas} - 1} \end{aligned} \qquad (2)$$

where $v$ is the variance of the determined TF element activity levels *wlc* of the groups, $x$ is a positive pseudo count, e.g., 1 or 10, and $n_{act}$ and $n_{pas}$ are the number of active and passive samples, respectively. The standard deviation $\sigma$ can next be obtained by taking the square root of the variance $v$.

The threshold can be subtracted from the determined TF element activity levels *wlc* for ease of interpretation, resulting in a cellular signaling pathway's activity score in which negative values correspond to a passive cellular signaling pathway and positive values correspond to an active cellular signaling pathway.

As an alternative to the above-described "single-layer" models, a "two-layer" may also be used in an example. In such a model, a summary value is calculated for every target gene using a linear combination based on the measured intensities of its associated probesets ("first (bottom) layer"). The calculated summary value is subsequently combined with the summary values of the other target genes of the cellular signaling pathway using a further linear combination ("second (upper) layer"). Again, the weights can be either learned from a training data set or based on expert knowledge or a combination thereof. Phrased differently, in the "two-layer" model, one or more expression level(s) are provided for each of the three or more target genes and the one or more linear combination(s) comprise for each of the three or more target genes a first linear combination of all expression levels of the one or more expression level(s) provided for the respective target gene ("first (bottom) layer"). The model is further based on a further linear combination including for each of the three or more target genes a weighted term, each weighted term being based on the first linear combination for the respective target gene ("second (upper) layer").

The calculation of the summary values can, in a preferred version of the "two-layer" model, include defining a threshold for each target gene using the training data and subtracting the threshold from the calculated linear combination, yielding the target gene summary. Here the threshold may be chosen such that a negative target gene summary value corresponds to a down-regulated target gene and that a positive target gene summary value corresponds to an up-regulated target gene. Also, it is possible that the target gene summary values are transformed using, e.g., one of the above-described transformations (fuzzy, discrete, etc.), before they are combined in the "second (upper) layer".

After the activity level of the TF element has been determined by evaluating the "two-layer" model, the determined TF element activity level can be thresholded in order to infer the activity of the cellular signaling pathway, as described above.

In the following, the models described above are collectively denoted as "(pseudo-)linear" models. A more detailed description of the training and use of probabilistic models, e.g., a Bayesian network model, is provided in section 3 below.

## 2. Selection of target genes

A transcription factor (TF) is a protein complex (i.e., a combination of proteins bound together in a specific structure) or a protein that is able to regulate transcription from target genes by binding to specific DNA sequences, thereby controlling the transcription of genetic information from DNA to mRNA. The mRNA directly produced due to this action of the TF complex is herein referred to as a "direct target gene" (of the transcription factor). Cellular signaling pathway activation may also result in more secondary gene transcription, referred to as "indirect target genes". In the following, (pseudo-)linear models or Bayesian network models (as exemplary mathematical models) comprising or consisting of direct target genes as direct links between cellular signaling pathway activity and mRNA level, are preferred, however the distinction between direct and indirect target genes is not always evident. Herein, a method to select direct target genes using a scoring function based on available scientific literature data is presented. Nonetheless, an accidental selection of indirect target genes cannot be ruled out due to limited information as well as biological variations and uncertainties.

Here we propose a list of PR target genes (specifically PR-A specific, PR-B specific, or PR-A&B specific target genes) which are found to be transcribed upon binding of a dimeric protein complex consisting of PR to cellular DNA. The list was generated by manually curating scientific literature found using Pubmed (www.ncbi.nlm.nih.gov/pubmed/) and ScienceDirect (www.sciencedirect.com/). Collected evidence was classified into three categories: 1) PR binds to regulatory region; 2) Presence of a progesterone response element (PRE) in the regulatory region; 3) Gene is differentially regulated by progesterone. When possible, we also annotated which PR isoform was implicated in order to create isoform specific models.

An overall evidence score was computed by adding a normalized literature score (NLscore) and a normalized differential expression score (NDscore) as follows:

1. Normalized Literature score (NLscore): We computed literature scores (Lscores) for each literature evidence category using a weighted sum (see Tables 1 to 3). Per literature source, only the strongest evidence in each category (per gene) was used. The weight given to evidence produced in certain specific settings was corrected as indicated in Tables 1 to 3. We then computed the final Lscore by summing the Lscores obtained for each evidence category plus an extra point for genes with evidence in all three categories (complete evidence), or half a point for genes with evidence in two categories. The normalized literature score (NLscore) was then computed by dividing the Lscore of each gene by the maximum Lscore.
2. Normalized Differential expression score (NDscore): We estimated the differential gene expression score (Dscore) based on the magnitude and significance of the differential expression of the genes in question on a selection of Affymetrix HG1133Plus2 data sets (see Table 4). For each dataset, we calibrated a PR cellular signaling pathway model and used the calibration summary results to estimate differential expression magnitude. Only probesets that were significantly differentially expressed were taken into account. For each calibration set, we computed the proportion of significantly differentially expressed probesets, %sig, the average of odds ratios for the significantly differentially expressed probesets, av(OR), and the average differences in mean expression between PR cellular signaling pathway active and inactive calibration samples (of significantly differentially expressed probesets),

$$\text{av(diff)}=\text{av}_{\text{ps}}((\overline{on}_{ps,set}) - (\overline{off}_{ps,set})), \quad (3)$$

where ($\overline{on}_{ps,set}$) is the average expression of active samples for a given probeset *ps,* on calibration set *set* and ($\overline{off}_{ps,set}$) is the average expression of inactive samples for a given probeset *ps,* on calibration set *set.*

We then computed a differential expression score for each data set as:

$$\text{Dscore} = \%\text{sig} * \text{av(diff)} * \log_2(\text{av(OR)}) * \text{sign(av(diff))}, \quad (4)$$

and computed an overall score by adding the mean score obtained with the two normal endometrium datasets (GSE6364, GSE11352) to the scores of two other datasets (GSE24468, GSE29435).The normalized score was computed by dividing the absolute value of the Dscore of each gene by the maximum absolute value of the Dscores.

Table 1: Weights given depending on evidence strength for category "PR binds to regulatory region" evidence.

| Evidence type | Strength rank | Weight |
|---|---|---|
| ChIP/PCR | 1 | 1/1 |
| Luciferase assay, CAT assay, EMSA assay, ChIP/CHIP, ChIP/reChIP, ChIP/seq | 2 | 1/2 |
| H3K4me ChIP | 4 | 1/4 |
| Literature | 8 | 1/8 |
| **Extra weight** | | |
| PR binds near PR motif | | + half weight |
| Binding is weaker in mutated PR motif | | + half weight |

Table 2: Weights given depending on evidence strength for category "PRE motif in the regulatory region" evidence.

| Evidence type | Strength rank | Weight |
|---|---|---|
| Palindromic/perfect PRE (with sequence) | 1 | 1/1 |
| 1-2 mismatches/non specified PRE (with sequence) | 2 | 1/2 |
| putative PRE/Half site PRE (with sequence) | 3 | 1/3 |
| Perfect PRE (no sequence) | 3 | 1/3 |
| PRE (no sequence) | 4 | 1/4 |
| Half site PRE | 5 | 1/5 |
| putative PRE | 6 | 1/6 |
| Literature | 8 | 1/8 |
| **Extra weight** | | |
| PR binds near motif differentially | | + half weight |

Table 3: Weights given depending on evidence strength for category "differential mRNA transcription" evidence.

| Evidence type | Strength rank | Weight |
|---|---|---|
| PCR/Northern blot in CHX | 1 | 1 |
| PCR/Northern blot | 2 | 1/2 |
| Microarray in CHX | 2 | 1/2 |
| Microarray | 3 | 1/3 |
| RNAseq | 3 | 1/3 |
| Western blot/immune fluorescence | 4 | 1/4 |
| RNA PolII ChIP/PCR | 4 | 1/4 |

(continued)

| Evidence type | Strength rank | Weight |
|---|---|---|
| RNA PolII ChIP/CHIP | 5 | 1/5 |
| literature | 8 | 1/8 |
| **Extra weight** | | |
| diff. expr. by nuclear PR | | + half weight |
| down regulated by anti-progestogen | | + half weight |

Table 4: Affymetrix hg u133 Plus 2.0 calibration sets used in differential expression analysis for computing Dscore and selecting PR isoform specific models. R5020: promegestone, synthetic progestin; MPA: medroxyprogesterone acetate, synthetic progestin; E2: estradiol.

| Name | Datasets | Description | Active samples | Inactive samples |
|---|---|---|---|---|
| HMB10R | GSE24468 | hMEC normal breast cell line with a PR-B construct treated with vehicle control (inactive) or 10nM R5020 (active) | GSM602697<br>GSM602698<br>GSM602699<br>GSM602700<br>GSM602701<br>GSM602702<br>GSM602703<br>GSM602704<br>GSM602705<br>GSM602706 | GSM602707<br>GSM602708<br>GSM602709<br>GSM602710<br>GSM602711<br>GSM602712<br>GSM602713<br>GSM602714<br>GSM602715<br>GSM602716 |
| IKAB01M | GSE29435 | IKPR-AB36 endometrial cell line treated with vehicle control (inactive) or InM MPA (active) | GSM728708<br>GSM728709<br>GSM728710 | GSM728705<br>GSM728706<br>GSM728707 |
| EmPxS* | GSE6364 | Normal endometrium during proliferative (inactive; low progesterone) or mid secretory phase (active; high progesterone) of the menstrual cycle | GSM150221<br>GSM150222<br>GSM150223<br>GSM150224<br>GSM150225 | GSM150196<br>GSM150197<br>GSM150198<br>GSM150199<br>GSM150201 |
| EmExEM* | GSE12446 | Normal endometrium of postmenopausal women treated with E2 (inactive) or E2+MPA (active) | GSM312568<br>GSM312667<br>GSM312668<br>GSM312669<br>GSM312671<br>GSM312673 | GSM312560<br>GSM312561<br>GSM312563<br>GSM312564<br>GSM312565<br>GSM312566 |
| EmPxMS** | GSE6364 | Healthy endometrium during proliferative (inactive; low progesterone) or mid secretory (active; high progesterone) phase of the menstrual cycle | GSM150223<br>GSM150224<br>GSM150225<br>GSM150226<br>GSM150227 | GSM150198<br>GSM150199<br>GSM150201 |
| EmLPxHS** | GSE6364 and GSE29981 | Healthy endometrium during proliferative/low progesterone phase (active) or mid secretory/high progesterone phase (active) of the menstrual cycle | GSM150223<br>GSM150224<br>GSM150225<br>GSM150226<br>GSM150227<br>GSM742061<br>GSM742073 | GSM150198<br>GSM150199<br>GSM150201<br>GSM742055<br>GSM742057<br>GSM742065<br>GSM742069 |

(continued)

| Name | Datasets | Description | Active samples | Inactive samples |
|---|---|---|---|---|
| | | | GSM742077 | GSM742079 |
| *used only for Dscore; **used only for isoform specific target list selection | | | | |

Table 5. Selection of PR target genes to determine PR transcription activation, PR isoform specificity, associated Affymetrix probesets and evidence scores. In bold are genes with evidence in all categories, in italics are genes with only regulation evidence. The genes with rank 9, 10, 15 to 17, 19, 25, 28, 29, 34, 36 and 38 are down-regulated genes. The "*"

sign indicates that regulation is in the opposite direction in at least one calibration set. Isoform specificity is defined as follows: "B>A" means PR-B is a stronger transactivator than PR-A; "A&B" means PR-A and PR-B have comparable activation strength; "B<>A" means PR-A regulation is in the opposite direction to PR-B; "B" means PR-B specific; "A" means PR-A specific.

| rank | Gene Symbol | PR isoform | Regul. | Affymetric hg u133 Plus 2.0 probesets | Lscore | NLscore | Dscore | NDscore | Total score |
|---|---|---|---|---|---|---|---|---|---|
| **1** | **FKBP5** | **B>A** | **Up** | **204560_at; 224840_at; 224856_at** | **15.40** | **1.00** | **45.80** | **1.00** | **2.00** |
| 2 | SGK1 | A>B | Up | 201739_at | 5.46 | 0.35 | 31.56 | 0.69 | 1.04 |
| 3 | F3 | B<>A | Up | 204363_at | 7.13 | 0.46 | 16.14 | 0.35 | 0.82 |
| **4** | **BIRC3** | **B<>A** | **Up** | **210538_s_at; 230499_at** | **5.33** | **0.35** | **21.44** | **0.47** | **0.81** |
| 5 | BCL6 | A&B | Up | 203140_at, 215990_s_at | 4.33 | 0.28 | 23.83 | 0.52 | 0.80 |
| 6 | NET1 | B | Up* | 201829_at; 201830_s_at | 4.25 | 0.28 | 20.72 | 0.45 | 0.73 |
| **7** | **S100P** | **A&B** | **Up** | **204351_at** | **5.50** | **0.36** | **16.05** | **0.35** | **0.71** |
| 8 | PDK4 | B>A | Up | 225207_at; 205960_at; 1562321_at | 3.50 | 0.23 | 21.45 | 0.47 | 0.70 |
| **9** | **MYC** | **B>A** | **Down*** | **202431_s_at** | **9.83** | **0.64** | **-0.93** | **-0.02** | **0.66** |
| **10** | **CCND1** | **B>A** | **Down** | **208711_s_at; 208712_at; 214019_at** | **8.12** | **0.53** | **-4.58** | **-0.10** | **0.63** |
| **11** | **HSD11B2** | **B>A** | **Up*** | **204130_at** | **7.46** | **0.48** | **5.84** | **0.13** | **0.61** |
| 12 | TSC22D3 | A&B | Up | 208763_s_at, 207001_x_at | 4.09 | 0.27 | 13.86 | 0.30 | 0.57 |
| **13** | **MUC1** | **B<>A** | **Up** | **213693_s_at; 207847_s_at; 211695_x_at** | **5.58** | **0.36** | **7.76** | **0.17** | **0.53** |
| 14 | KLF4 | A&B | Up | 220266_s_at, 221841_s_at | 3.67 | 0.24 | 12.40 | 0.27 | 0.51 |
| **15** | **MSX2** | **B** | **Down*** | **205556_a; 205555_s_at; 210319_x_at** | **3.63** | **0.24** | **-10.19** | **-0.22** | **0.46** |
| **16** | **ATP1B1** | **B>A** | **Down** | **201242_s_at; 201243_s_at** | **4.67** | **0.30** | **-7.05** | **-0.15** | **0.46** |
| **17** | **BCL2L1** | **A** | **Down*** | **212312_at; 206665_s_at; 215037_s_at** | **6.38** | **0.41** | **-1.13** | **-0.02** | **0.44** |
| **18** | **ABCG2** | **A&B** | **Up*** | **209735_at** | **3.13** | **0.20** | **10.70** | **0.23** | **0.44** |
| **19** | **CDKN1A** | **A&B** | **Down*** | **1555186_at; 202284_s_at** | **5.96** | **0.39** | **-1.31** | **-0.03** | **0.42** |
| 20 | NFKBIA | B>A | Up* | 201502_s_at | 3.58 | 0.23 | 7.78 | 0.17 | 0.40 |
| **21** | **VEGFA** | **A&B** | **Up*** | **212171_x_at; 210512_s_at; 211527_x_at; 210513_s_at** | **5.77** | **0.37** | **0.12** | **0.00** | **0.38** |
| **22** | **TRIM22** | **A>B** | **Up*** | **213293_s_at** | **3.84** | **0.25** | **5.37** | **0.12** | **0.37** |
| **23** | **ARRDC1** | **B** | **Up** | **226405_s_at** | **2.03** | **0.13** | **8.57** | **0.19** | **0.32** |
| 24 | GOT1 | B>A | Up | 208813_at | 2.83 | 0.18 | 6.13 | 0.13 | 0.32 |
| **25** | **DDIT4** | **B<>A** | **Down*** | **202887_s_at** | **2.42** | **0.16** | **-7.04** | **-0.15** | **0.31** |
| 26 | *AK4* | *A&B* | *Up* | *204347_at, 204348_s_at, 225342_at, 230630_at* | *1.83* | *0.12* | *8.72* | *0.19* | *0.31* |
| 27 | STAT5A | B>A | Up | 201502_s_at | 4.50 | 0.29 | 0.42 | 0.01 | 0.30 |
| 28 | *NEDD9* | *A* | *NEDD9* | *202149_at; 202150_s_at; 1569020_at* | *2.00* | *0.13* | *-5.75* | *-0.13* | *0.26* |
| **29** | **E2F1** | **B** | **E2F1** | **204947_at; 2028_s_at** | **3.42** | **0.22** | **-0.60** | **-0.01** | **0.23** |
| 30 | KANK1 | B>A | KANK1 | 213005_s_at; 237162_at; 203010_at | 3.00 | 0.19 | 1.29 | 0.03 | 0.22 |
| 31 | SNTB2 | B | SNTB2 | 205315_s_at; 226685_at; 227312_at; 238925_at | 2.96 | 0.19 | 0.81 | 0.02 | 0.21 |
| 32 | GRB10 | A&B | GRB10 | 209409_at, 209410_s_at, 210999_s_at, 215248_at | 2.83 | 0.18 | 0.60 | 0.01 | 0.20 |
| 33 | PLIN2 | B>A | PLIN2 | 209122_at | 1.37 | 0.09 | 4.30 | 0.09 | 0.18 |
| 34 | CD82 | B | CD82 | 203904_x_at | 2.25 | 0.15 | -1.23 | -0.03 | 0.17 |
| 35 | VASP | A&B | VASP | 202205_at | 2.00 | 0.13 | 0.78 | 0.02 | 0.15 |
| 36 | *HPCAL1* | *A&B* | *HPCAL1* | *205462_s_at, 212552_at* | *1.46* | *0.09* | *-1.75* | *-0.04* | *0.13* |
| 37 | *PTP4A2* | *B>A* | *PTP4A2* | *208615_s_at, 208616_s_at, 208617_s_at, 216988_s_at* | *1.17* | *0.08* | *1.44* | *0.03* | *0.11* |
| 38 | ACSS1 | A&B | ACSS1 | 224882_at; 234801_s_at | 1.58 | 0.10 | -0.12 | 0.00 | 0.11 |

Based on the target gene ranking and PR isoform specificity (see Table 5) we calibrated a series of candidate PR models (using the HMB10R, IKAB01M, EmPxMS, and EmLPxHS calibration sets, see Table 4) based on the following preferred PR-A specific, PR-B specific and PR-A&B target gene lists.

Table 6. Preferred "PR-A specific list" of four target genes of the PR cellular signaling pathway.

| **Target gene** |
|---|
| BCL2L1 |
| DDIT4 |
| NEDD9 |
| TRIM22 |

Table 7. Preferred "PR-B specific list" of thirteen target genes of the PR cellular signaling pathway.

| **Target gene** |
|---|
| ARRDC 1 |
| ATP1B1 |
| CCND1 |
| E2F1 |
| FKBP5 |
| HSD11B2 |
| KANK1 |
| MSX2 |
| MYC |
| NET1 |
| NFKBIA |
| PDK4 |
| PLIN2 |

Table 8. Preferred "PR-A&B specific list" of eighteen target genes of the PR cellular signaling pathway.

| **Target gene** |
|---|
| ABCG2 |
| ACSS1 |
| AK4 |
| ATP1B1 |
| BCL6 |
| CCND1 |
| FKBP5 |
| GRB10 |
| HSD11B2 |
| KANK1 |
| KLF4 |

(continued)

| Target gene |
|---|
| MYC |
| NFKBIA |
| PDK4 |
| PLIN2 |
| S100P |
| TSC22D3 |
| VASP |

These preferred target gene lists were selected based on their capacity of separating a series of expected active samples from expected inactive samples from the calibration sets of Table 4 according to the following criteria:

(1) best AUC,
(2) best balanced accuracy,
(3) largest difference in activity between expected active and expected inactive samples, and
(4) smallest standard deviation of the average differences between inferred PR cellular signaling pathway activity of active and inactive samples (ground truth) from individual data sets. (The rationale behind this is that average difference in inferred PR cellular signaling pathway activity for active and inactive samples within a data set is preferably similar.)

Other suitable target gene lists include:

Table 9. "PR-A specific list" of eight target genes of the PR cellular signaling pathway.

| Target gene |
|---|
| BCL2L1 |
| BIRC3 |
| DDIT4 |
| F3 |
| MUC1 |
| NEDD9 |
| SGK1 |
| TRIM22 |

Table 10. "PR-B specific list" of three target genes of the PR cellular signaling pathway.

| Target gene |
|---|
| CCND1 |
| FKBP5 |
| MYC |

Table 11. "PR-B specific list" of eightteen target genes of the PR cellular signaling pathway.

| Target gene |
|---|
| ARRDC 1 |

(continued)

| Target gene |
|---|
| ATP1B1 |
| CCND1 |
| CD82 |
| E2F1 |
| FKBP5 |
| GOT1 |
| HSD11B2 |
| KANK1 |
| MSX2 |
| MYC |
| NET1 |
| NFKBIA |
| PDK4 |
| PLIN2 |
| PTP4A2 |
| SNTB2 |
| STAT5A |

Table 12. "PR-B specific list" of twentytwo target genes of the PR cellular signaling pathway.

| Target gene |
|---|
| ARRDC 1 |
| ATP1B1 |
| BIRC3 |
| CCND1 |
| CD82 |
| DDIT4 |
| E2F1 |
| F3 |
| FKBP5 |
| GOT1 |
| HSD11B2 |
| KANK1 |
| MSX2 |
| MUC1 |
| MYC |
| NET1 |
| NFKBIA |

(continued)

| Target gene |
|---|
| PDK4 |
| PLIN2 |
| PTP4A2 |
| SNTB2 |
| STAT5A |

Table 13. "PR-A&B specific list" of four target genes of the PR cellular signaling pathway.

| Target gene |
|---|
| BCL6 |
| CCND1 |
| FKBP5 |
| MYC |

Table 14. "PR-A&B specific list" of seven target genes of the PR cellular signaling pathway.

| Target gene |
|---|
| BCL6 |
| CCND1 |
| CDKN1A |
| FKBP5 |
| MYC |
| SGK1 |
| VEGFA |

Table 15. "PR-A&B specific list" of twentysix target genes of the PR cellular signaling pathway.

| Target gene |
|---|
| AK4 |
| ARRDC 1 |
| ATP1B1 |
| BCL2L1 |
| BCL6 |
| BIRC3 |
| CCND1 |
| CD82 |
| F3 |
| FKBP5 |
| GOT1 |
| GRB10 |

(continued)

| Target gene |
|---|
| HSD11B2 |
| KLF4 |
| MUC1 |
| MYC |
| NEDD9 |
| NET1 |
| PDK4 |
| PTP4A2 |
| S100P |
| SGK1 |
| SNTB2 |
| STAT5A |
| TSC22D3 |
| VASP |

Table 16. "PR-A&B specific list" of thirtyeight target genes of the PR cellular signaling pathway.

| Target gene |
|---|
| ABCG2 |
| ACSS1 |
| AK4 |
| ARRDC 1 |
| ATP1B1 |
| BCL2L1 |
| BCL6 |
| BIRC3 |
| CCND1 |
| CD82 |
| CDKN1A |
| DDIT4 |
| E2F1 |
| F3 |
| FKBP5 |
| GOT1 |
| GRB10 |
| HPCAL1 |
| HSD11B2 |
| KANK1 |

(continued)

| Target gene |
|---|
| KLF4 |
| MSX2 |
| MUC1 |
| MYC |
| NEDD9 |
| NET1 |
| NFKBIA |
| PDK4 |
| PLIN2 |
| PTP4A2 |
| S100P |
| SGK1 |
| SNTB2 |
| STAT5A |
| TRIM22 |
| TSC22D3 |

### 3. Training and using the mathematical model

Before the mathematical model can be used to infer the activity of the cellular signaling pathway, herein, the PR cellular signaling pathway, in a subject, the model must be appropriately trained.

If the mathematical pathway model is a probabilistic model, e.g., a Bayesian network model, based on conditional probabilities relating the activity level of the PR TF element and expression levels of three or more target genes of the PR cellular signaling pathway measured in the sample of the subject, the training may preferably be performed as described in detail in the published international patent application WO 2013/011479 A2 ("Assessment of cellular signaling pathway activity using probabilistic modeling of target gene expression").

If the mathematical pathway model is based on one or more linear combination(s) of expression levels of three or more target genes of the PR cellular signaling pathway measured in the sample of the subject, the training may preferably be performed as described in detail in the published international patent application WO 2014/102668 A2 ("Assessment of cellular signaling pathway activity using linear combination(s) of target gene expressions").

Herein, an exemplary Bayesian network model as shown in Fig. 1 was used to model the transcriptional program of the PR cellular signaling pathway in a simple manner. The model consists of three types of nodes: (a) a transcription factor (TF) element (with states "absent" and "present") in a first layer 1; (b) target genes $TG_1$, $TG_2$, $TG_n$ (with states "down" and "up") in a second layer 2, and; (c) measurement nodes linked to the expression levels of the target genes in a third layer 3. These can be microarray probesets $PS_{1,1}$, $PS_{1,2}$, $PS_{1,3}$, $PS_{2,1}$, $PS_{n,1}$, $PS_{n,m}$ (with states "low" and "high"), as preferably used herein, but could also be other gene expression measurements such as RNAseq or RT-qPCR.

A suitable implementation of the mathematical model, herein, the exemplary Bayesian network model, is based on microarray data. The model describes (i) how the expression levels of the target genes depend on the activation of the TF element, and (ii) how probeset intensities, in turn, depend on the expression levels of the respective target genes. For the latter, probeset intensities may be taken from fRMA pre-processed Affymetrix HG-U133Plus2.0 microarrays, which are widely available from the Gene Expression Omnibus (GEO, www.ncbi.nlm.nih.gov/geo) and ArrayExpress (www.ebi.ac.uk/arrayexpress).

As the exemplary Bayesian network model is a simplification of the biology of a cellular signaling pathway, herein, the PR cellular signaling pathway, and as biological measurements are typically noisy, a probabilistic approach was opted for, i.e., the relationships between (i) the TF element and the target genes, and (ii) the target genes and their respective probesets, are described in probabilistic terms. Furthermore, it was assumed that the activity of the oncogenic cellular signaling pathway which drives tumor growth is not transiently and dynamically altered, but long term or even irreversibly altered. Therefore the exemplary Bayesian network model was developed for interpretation of a static cellular condition. For this reason complex dynamic cellular signaling pathway features were not incorporated into the model.

Once the exemplary Bayesian network model is built and calibrated (see below), the model can be used on microarray data of a new sample by entering the probeset measurements as observations in the third layer 3, and inferring backwards in the model what the probability must have been for the TF element to be "present". Here, "present" is considered to be the phenomenon that the TF element is bound to the DNA and is controlling transcription of the cellular signaling pathway's target genes, and "absent" the case that the TF element is not controlling transcription. This probability is hence the primary read-out that may be used to indicate activity of the cellular signaling pathway, herein, the PR cellular signaling pathway, which can next be translated into the odds of the cellular signaling pathway being active by taking the ratio of the probability of it being active vs. it being passive (i.e., the odds are given by p/(1-p), where p is the predicted probability of the cellular signaling pathway being active).

In the exemplary Bayesian network model, the probabilistic relations have been made quantitative to allow for a quantitative probabilistic reasoning. In order to improve the generalization behavior across tissue types, the parameters describing the probabilistic relationships between (i) the TF element and the target genes have been carefully hand-picked. If the TF element is "absent", it is most likely that the target gene is "down", hence a probability of 0.95 is chosen for this, and a probability of 0.05 is chosen for the target gene being "up". The latter (non-zero) probability is to account for the (rare) possibility that the target gene is regulated by other factors or that it is accidentally observed as being "up" (e.g. because of measurement noise). If the TF element is "present", then with a probability of 0.70 the target gene is considered "up", and with a probability of 0.30 the target gene is considered "down". The latter values are chosen this way, because there can be several causes why a target gene is not highly expressed even though the TF element is present, e.g., because the gene's promoter region is methylated. In the case that a target gene is not up-regulated by the TF element, but down-regulated, the probabilities are chosen in a similar way, but reflecting the down-regulation upon presence of the TF element. The parameters describing the relationships between (ii) the target genes and their respective probesets have been calibrated on experimental data. For the latter, in this example, microarray data was used from patients samples which are known to have an active PR cellular signaling pathway whereas normal, healthy samples from a different data set were used as passive PR cellular signaling pathway samples, but this could also be performed using cell line experiments or other patient samples with known cellular signaling pathway activity status. The resulting conditional probability tables are given by:

*A: for upregulated target genes*

| | $PS_{i,j}$ = low | $PS_{i,j}$ = high |
|---|---|---|
| $TG_i$ = down | $\frac{AL_{i,j}+1}{AL_{i,j}+AH_{i,j}+2}$ | $\frac{AH_{i,j}+1}{AL_{i,j}+AH_{i,j}+2}$ |
| $TG_i$ = up | $\frac{PL_{i,j}+1}{PL_{i,j}+PH_{i,j}+2}$ | $\frac{PH_{i,j}+1}{PL_{i,j}+PH_{i,j}+2}$ |

*B: for downregulated target genes*

| | $PS_{i,j}$ = low | $PS_{i,j}$ = high |
|---|---|---|
| $TG_i$ = down | $\frac{PL_{i,j}+1}{PL_{i,j}+PH_{i,j}+2}$ | $\frac{PH_{i,j}+1}{PL_{i,j}+PH_{i,j}+2}$ |
| $TG_i$ = up | $\frac{AL_{i,j}+1}{AL_{i,j}+AH_{i,j}+2}$ | $\frac{AH_{i,j}+1}{AL_{i,j}+AH_{i,j}+2}$ |

In these tables, the variables $AL_{i,j}$, $AH_{i,j}$, $PL_{i,j}$, and $PH_{i,j}$ indicate the number of calibration samples with an "absent" (A) or "present" (P) transcription complex that have a "low" (L) or "high" (H) probeset intensity, respectively. Dummy counts have been added to avoid extreme probabilities of 0 and 1.

To discretize the observed probeset intensities, for each probeset $PS_{i,j}$ a threshold $t_{i,j}$ was used, below which the observation is called "low", and above which it is called "high". This threshold has been chosen to be the (weighted) median intensity of the probeset in the used calibration data set. Due to the noisiness of microarray data, a fuzzy method was used when comparing an observed probeset intensity to its threshold, by assuming a normal distribution with a standard deviation of 0.25 (on a log2 scale) around the reported intensity, and determining the probability mass below and above the threshold.

If instead of the exemplary Bayesian network described above, a (pseudo-)linear model as described in section 1 above was employed, the weights indicating the sign and magnitude of the correlation between the nodes and a threshold to call whether a node is either "absent" or "present" would need to be determined before the model could be used to infer cellular signaling pathway activity in a test sample. One could use expert knowledge to fill in the weights and the threshold a priori, but typically the model would be trained using a representative set of training samples, of which preferably the ground truth is known, e.g., expression data of probesets in samples with a known "present" transcription factor complex (= active cellular signaling pathway) or "absent" transcription factor complex (= passive cellular signaling pathway).

Known in the field are a multitude of training algorithms (e.g., regression) that take into account the model topology and changes the model parameters, here, the weights and the threshold, such that the model output, here, a weighted linear score, is optimized. Alternatively, it is also possible to calculate the weights directly from the observed expression levels without the need of an optimization algorithm.

A first method, named "black and white"-method herein, boils down to a ternary system, in which each weight is an element of the set {-1, 0, 1}. If this is put in a biological context, the -1 and 1 correspond to target genes or probesets that are down- and up-regulated in case of cellular signaling pathway activity, respectively. In case a probeset or target gene cannot be statistically proven to be either up- or down-regulated, it receives a weight of 0. In one example, a left-sided and right-sided, two sample t-test of the expression levels of the active cellular signaling pathway samples versus the expression levels of the samples with a passive cellular signaling pathway can be used to determine whether a probe or gene is up- or down-regulated given the used training data. In cases where the average of the active samples is statistically larger than the passive samples, i.e., the p-value is below a certain threshold, e.g., 0.3, the target gene or probeset is determined to be up-regulated. Conversely, in cases where the average of the active samples is statistically lower than the passive samples, the target gene or probeset is determined to be down-regulated upon activation of the cellular signaling pathway. In case the lowest p-value (left- or right-sided) exceeds the aforementioned threshold, the weight of the target gene or probeset can be defined to be 0.

A second method, named "log odds"-weights herein, is based on the logarithm (e.g., base e) of the odds ratio. The odds ratio for each target gene or probeset is calculated based on the number of positive and negative training samples for which the probeset/target gene level is above and below a corresponding threshold, e.g., the (weighted) median of all training samples. A pseudo-count can be added to circumvent divisions by zero. A further refinement is to count the samples above/below the threshold in a somewhat more probabilistic manner, by assuming that the probeset/target gene levels are e.g. normally distributed around its observed value with a certain specified standard deviation (e.g., 0.25 on a 2-log scale), and counting the probability mass above and below the threshold. Herein, an odds ratio calculated in combination with a pseudo-count and using probability masses instead of deterministic measurement values is called a "soft" odds ratio.

Further details regarding the inferring of cellular signaling pathway activity using mathematical modeling of target gene expression can be found in Verhaegh W. et al., "Selection of personalized patient therapy through the use of knowledge-based computational models that identify tumor-driving signal transduction pathways", Cancer Research, Vol. 74, No. 11, 2014, pages 2936 to 2945.

## 4. Experimental results

To demonstrate the PR models utility, here we present results obtained by applying various calibrated PR models to a series of Affymetrix HGU133Plus2.0 data sets. The models are identified by their target gene list and calibration set. For example, PR-A_EmLPxHS is the PR-A specific model calibrated with the EmLPxHS calibration set (see Table 4) that uses the PR-A specific target gene list. Activity read-outs are either presented as odds on:off (the odds of being active vs. being inactive), represented in a base 2 log scale, or as a normalized activity obtained by normalizing the log2(odds on:off) to values between 0 and 100. The later normalized activity scale is useful when comparing activities of two different models that use a different number of target genes, target probesets, or calibration set, since the range of the log2(odds on:off) of a model is highly dependent on those values.

Fig. 8 shows PR cellular signaling pathway activity predictions of trained Bayesian network models on QC pass normal endometrium samples from GSE29921. Samples were taken at different days of the menstrual cycle and the progesterone level is available. Left: PR-A (PR-A EmLPxHS) model; middle: PR-B (PR-B_EmLPxHS) model; right: PR-A&B (PR-A&B_EmLPxHS) model. PR cellular signaling pathway activity levels (shown as odds PR on:off) are significantly lower in samples with a low progesterone ("1") concentration (av = 0.16 ng/mL, sd = 0.11 ng/mL, n=6) than in samples with a high progesterone ("h") concentration (av = 10.2 ng/mL, sd = 3.5 ng/mL, n=3). Correlations between progesterone levels and PR cellular signaling pathway activity are 0.97, 0.97, and 0.96 for the PR-A, PR-B and PR-A&B models, respectively. (All samples in the EmLPxHS calibration set.)

Figs. 9 and 10 show PR cellular signaling pathway activity predictions of trained Bayesian network models on QC pass normal endometrium samples from GSE6364 and GSE51981, respectively. The samples were taken at different phases of the menstrual cycle. Left: PR-A (PR-A_EmLPxHS) model; middle PR-B (PR-B_EmLPxHS) model; right: PR-A&B (PR-A&B_EmLPxHS) model. PR cellular signaling pathway activity levels (shown as odds PR on:off) are significantly higher in the mid secretory phase ("ms") than in the proliferative phase ("p"). Except for PR-A, PR cellular signaling pathway activity levels are also significantly higher in the early secretory phase ("es") than in the proliferative phase. (The triangles show samples in the EmLPxHS calibration set.)

Fig. 11 shows the progression of the menstrual cycle and the different hormones contributing to it. The menstrual cycle consists of a proliferative (1-2 weeks) and a secretory phase (2 weeks) which is followed by menstruation. During the proliferative phase the estradiol level increases and then peaks high, just prior to ovulation. Then the luteal or secretory phase starts, during which progesterone levels increases, and estradiol levels are lower. As can be seen, the ovarian cycle phases consist of the follicular phase ("FP") and the luteal phase ("LP"). The cycle is required for the production of oocytes, and for the preparation of the uterus for pregnancy. The menstrual cycle occurs due to the rise and fall of hormones. This cycle results in the thickening of the lining of the uterus, and the growth of an egg, (which is required for pregnancy). The egg is released from an ovary around day fourteen in the cycle; the thickened lining of the uterus provides nutrients to an embryo after implantation. If pregnancy does not occur, the lining is released in what is known as menstruation. Abbreviations used in the figure: Selected tertiary follicle ("STF"); Ovluation ("O"); Corpus luteuni ("CL"); Corpus ablicans ("CA"); Degrading corpus ("DC"); Menses ("M"); Proliferative phase ("PP"); Secretory phase ("SP"). The two plots at the bottom of the figure show the hormone levels during the different days of the menstrual cycle. Upper plot: Pituitary hormon levels (FSH; LH). Lower plot: Ovarian hormon levels (estrogen ("E"); progesterone ("P")).

Fig. 12 shows PR cellular signaling pathway activity predictions of trained Bayesian network models on fallopian tube samples from GSE10971. Samples were taken at different phases of the ovulation cycle. Left: PR-A (PR-A_EmLPxHS) model; middle: PR-B (PR-B_EmLPxHS) model; right: PR-A&B (PR-A&B_EmLPxHS) model. Mean PR cellular signaling pathway activity levels (shown as odds PR on:off) are higher in the luteal phase ("1") compared to the follicular phase ("f').

Fig. 13 shows PR cellular signaling pathway activity predictions of trained Bayesian network models on QC pass menopausal normal endometrium samples from GSE12446. Patients were treated with estradiol ("e") or estradiol + MPA ("e + MPA") for 21 days prior to surgery. Left: PR-B (PR-B_EmLPxHS) model; right: PR-A&B (PR-A&B_EmLPxHS) model. PR cellular signaling activity levels (shown as odds PR on:off) are significantly higher in estradiol + MPA treated samples than in estradiol only treated samples.

Fig. 14 shows PR cellular signaling pathway activity predictions of trained Bayesian network models on QC pass term decidual cells (from normal placentae) samples from GSE65835. Cells were cultured with estradiol ("e") or estradiol + MPA ("e+MPA"). Left: PR-B (PR-B_EmLPxHS) model; right: PR-A&B (PR-A&B_EmLPxHS) model. PR cellular signaling pathway activity levels (shown as odds PR on:off) are significantly higher in estradiol + MPA treated samples than in estradiol only treated samples.

Fig. 15 shows PR cellular signaling pathway activity predictions of trained Bayesian network models on QC pass PR-A and PR-B expressing ishikawa IKPR-AB36 endometrium cell line samples from GSE29435. Cells were cultured for 48h in the presence or absence of 1 nM MPA. Left: PR-A (PR-A_EmLPxHS) model; middle: PR-B (PR-B_EmLPxHS) model; right: PR-A&B (PR-A&B_EmLPxHS) model. PR cellular signaling pathway activity levels (shown as odds PR on:off) are significantly higher in MPA treated samples than in control ("c") treated samples.

Fig. 16 shows PR cellular signaling pathway activity predictions of trained Bayesian network models on QC pass PR-B expressing hMEC breast cell line samples from GSE24468. Cells were cultured for 16h in the presence or absence of 10 nM R5020. Left: PR-A (PR-A_EmLPxHS) model; middle: PR-B (PR-B_EmLPxHS) model; right: PR-A&B (PR-A&B_EmLPxHS) model. PR cellular signaling pathway activity levels are significantly higher in R5020 treated samples than in control ("c") treated samples.

Fig. 17 shows PR cellular signaling pathway activity predictions of trained Bayesian network models on QC pass paired normal vs. breast cancer samples from GSE10810. Left: Difference between PR-A and PR-B cellular signaling pathway activity in adjacent normal ("S") and tumor ("T") samples. Right: Difference in PR-A and PR-B cellular signaling pathway activity between paired adjacent normal ("S") and tumor ("T") samples. Normalized activities are used for a better comparison of the PR-A and PR-B models. Compared to normal tissue, PR-A and PR-B cellular signaling pathway activity levels are disrupted in breast cancer tissue. While normalized PR-A and PR-B cellular signaling pathway activities are similar or PR-B is higher than PR-A in normal issue (see "a"), normalized PR-B cellular signaling pathway activity is generally lower than normalized PR-A cellular signaling pathway activity in tumor tissue (see "b"). While normalized PR-A cellular signaling pathway activity is generally higher in tumor than in normal samples (see "c"), PR-B is generally lower in tumor than in normal samples (see "d").

Fig. 18 shows PR cellular signaling pathway activity predictions of trained Bayesian network models on normal vs. endometrial cancer samples from GSE17025. From left to right: Normal samples ("NL"), Endometrioid cancer grades 1 to 3 ("EE 1-2-3"), Papillary serous cancer grades 2 and 3 ("PS 2-3"). Normalized PR-A cellular signaling pathway activity is increased in endometrial cancer samples compared to normal samples and normalized PR-B cellular signaling pathway activity is similar or decreased in endometrial cancer samples compared to normal samples. The difference between PR-A and PR-B increases with cancer grade.

Fig. 19 shows PR cellular signaling pathway activity predictions of trained Bayesian network models on paired normal vs. tumor lung cancer samples. Left: Samples from non-smoking female lung cancer in Taiwan from GSE19804; right: Samples from stage I lung adenocarcinoma from GSE27626. Compared to normal lung ("N"), normalized PR-A and PR-B cellular signaling pathway activity levels are disrupted in cancer ("T"). While normalized PR-A cellular signaling pathway activity levels increase significantly in cancer compared to normal tissue, normalized PR-B and PR-A&B cellular signaling pathway activity levels are significantly reduced from normal to cancer. Cancer samples are grouped by tumor stage. Disruption in normalized PR-A and PR-B cellular signaling pathway activity levels are larger on more severe stages.

Fig. 20 shows PR cellular signaling pathway activity predictions of trained Bayesian network models on normal and tumor lung cancer samples from GSE30219. Left: PR-A (PR-A_EmLPxHS) model; middle: PR-B (PR-B_EmLPxHS) model; right: PR-A&B (PR-A&B_EmLPxHS). Cancer samples are grouped by TNM tumor stage from top to bottom. Compared to normal lung ("NTL"), normalized PR-A and PR-B cellular signaling pathway activity levels are disrupted in cancer ("N0" to "N3"). While normalized PR-A cellular signaling pathway activity levels increase significantly in cancer compared to normal tissue, normalized PR-B and PR-A&B cellular signaling pathway activity levels are significantly reduced from normal to cancer. Disruption in normalized PR-A and PR-B cellular signaling pathway activity levels are larger on more severe stages (mostly due to further increase in PR-A cellular signaling pathway activity levels).

Fig. 21 shows PR cellular signaling pathway activity predictions of trained Bayesian network models on normal vs. idiopathic pulmonary fibrosis (IPF) samples from GSE24206. Compared to normal lung ("H"), normalized PR-A and PR-B cellular signaling pathway activity levels are disrupted in IPF. While normalized PR-A cellular signaling pathway activity levels remain similar in normal and IPF, normalized PR-B and PR-A&B cellular signaling pathway activity levels are significantly reduced from normal to IPF.

Fig. 22 shows PR-B cellular signaling pathway activity predictions of trained Bayesian network models on QC pass paired pre/post letrozole treatment breast cancer samples from GSE10281. ER positive breast cancer patients were treated for 3 months with the aromase inhibitor letrozole in a neoadjuvant setting. Left: Normalized PR-B cellular signaling pathway activity difference in samples from non-responsive ("NR") vs. responsive ("R") patients in pre-treatment samples ("pre") and post-treatment samples ("post"); right: Normalized PR-B cellular signaling pathway activity difference in paired pre vs. post treatetment samples in non-responders and in responders. After treatment, mean normalized PR-B cellular signaling pathway activity is higher in responders than in non-responders. Mean normalized PR-B cellular signaling pathway activity goes up in responders with activity levels approaching PR-B activity in normal samples (see Fig. 17). Normalized PR-B cellular signaling pathway activity levels remain similar to pre-treatment in non-responders.

Fig. 23 shows PR cellular signaling pathway activity predictions of trained Bayesian network models as a prognostic risk factor for the progression of lung cancer on QC pass samples from GSE30219. Left: PR-A, PR-B and PR-A&B were discretized between "on" and "off' (probability of being active ≥ 50% and <50%, respectively); right: Ratios between (1) normalized PR-A and normalized PR-B (ABratio), (2) normalized PR-A&B and normalized PR-B (A&BBratio) and (3) normalized A&BBratio and normalized PR-B (A&BBBratio) were computed and discretized between "ge.1" and "lt. 1" (ratio is ≥ 1 and <1, respectively). Survival curves show (in time to relapse) that, besides for the PR-A&B model (PR-A&B_EmLHxHS), the PR cellular signaling pathway activity predictions are significant prognostic factors for the progression of lung cancer.

Fig. 24 shows combined PR cellular signaling pathway activities predictions of trained Bayesian network models as a prognostic risk factor for the progression of lung cancer on QC pass samples from GSE30219. PR-A, PR-B and PR-A&B were discretized between "on" and "off" (probability of being active ≥ 50% and <50%, respectively). Ratios between (1) normalized PR-A&B and normalized PR-B (A&BBratio) and (2) normalized A&BBratio and normalized PR-B (A&BBBratio) were computed and discretized between "ge.1" and "lt.1" (ratio is ≥ 1 and <1, respectively). ">1" and "<1" (ratio is ≥ 1 and <1, respectively). Left: combination of PR-A and PR-B. Right: combination of A&BBratio and A&BBBratio. Survival curves show (in survival probability) that both combinations are significant prognostic factors in progression of lung cancer. The PR-B off group has the shortest time to progression independent of PR-A. PR-B on/PR-A off has the longest time to progression and PR-B on/PR-A on has an intermediate time to progression. A&BBratio>1/A&BBBratio>1 group have the shortest time to progression, while A&BBratio<1/A&BBBratio<1 groups have the longest time to progression.

Fig. 25 shows PR cellular signaling pathway activity predictions of trained Bayesian network models as a prognostic risk factor for the progression of breast cancer on QC pass samples from GSE6532, GSE9195, GSE21653, GSE20685, GSE58812, and EMTAB365. ER (ER_E01MCF7g28 previously described), PR-B (PR-B_EmLHxHS) and PR-A&B (PR-A&B_EmLHxHS) were discretized between "on" and "off (probability of being active ≥ 50% and <50%, respectively). A&BBratio (ratio between normalized PR-A&B and normalized PR-B) was discretized between "A&B>B" and "B>A&B" (ratio is ≥ 1 and <1, respectively). A1 to A3: survival curves of discretized ER, PR-B, and ER+PR-B, respectively. A4: summary of multivariate cox proportional hazard regression on the continuous ER+PR-B model. B1 to B3: survival curves of discretized ER, A&BBratio, and ER+A&BBratio, respectively. B4: summary of multivariate cox proportional hazard regression on the continuous ER+A&BBratio. A&BBratio in the survival curves and cox proportional hazard regression exemplifies the added value adding PR-B or A&BBratio to ER in a prognostic cancer model.

Fig. 26 shows PR cellular signaling pathway activity predictions of trained Bayesian network models as a prognostic risk factor for the progression of pediatric acute lymphoblastic leukemia (ALL) on QC pass samples from GSE13576. Ratios between (1) normalized PR-A and normalized PR-B (ABratio), (2) normalized PR-A&B and normalized PR-B (A&BBratio) and (3) normalized A&BBratio and normalized PR-B (A&BBBratio) were computed and normalized. Left: normalized PR-A, PR-B and PR-A&B pathway activities. Right: PR-B and all 3 ratios are significantly higher in early relapse ("ER") compared to controls ("NR"), A&BBratio is also significantly higher in early relapse compared to late relapse ("LR").

Fig. 27 shows PR cellular signaling pathway activity predictions of trained Bayesian network models as a prognostic risk factor in breast cancer on primary normal breast cell cultures samples from GSE13671. Compared to cultures form normal non-mutated breast tissue ("NC"), PR-A and PR-B cellular signaling pathway activity levels are disrupted in cultures from BCRA1 mutated tissue ("BCRA1 M"). While PR-A cellular signaling pathway activity levels are significantly lower in mutated cultures, PR-B and PR-A&B cellular signaling pathway activity levels are significantly higher in mutated cultures. Different indicators represent different donors.

Fig. 28 shows PR cellular signaling pathway activity predictions of trained linear models as a predictor for progestogen therapy response in endometrial cancer. As an example, a linear PR-B model using only a shortlist of three target genes CCND1, FKBP5, and MYC (see also Table 10) based on PCR data was used. Normalized RT-qPCR of genes from the target gene shortlist were added and normalized to values between 0 and 10. PR cellular signaling pathway activity scores of the linear model were computed for pre-treatment endometrium samples of 19 patients that underwent subsequent progestogen hormone therapy. The linear model was discretized between high and low using the median as threshold and the graph shows the survival probability for low PR-B cellular signaling pathway activity (upper curve) and high PR-B cellular signaling pathway activity (lower curve). A) Survival curves for the discretized PR-B pathway activity. The numbers below the graphs show the number at risk and the cumulative number of events for the low PR-B cellular signaling pathway activity and the high PR-B cellular signaling pathway activity. B) Summary of univariate cox proportional hazard regression (number at risk) on the linear PR-B model using only the target genes CCND1, FKBP5, and MYC (number of samples = 19; number of events = 9, Hazard Ratio (HR) = 3.8; Wald test p value = 0.006). C) Summary of multivariate cox proportional hazard regression (cumulative number of events) on the linear PR-B model using only the target genes CCND1, FKBP5 and MYC and ER IHC (immunohistochemistry staining of nuclear ER, values normalized between 0 and 10) (number of samples = 19 for both PR-B and ER IHC; number of events = 9 for both PR-B and ER IHC, Hazard Ratio (HR) = 1.12 for PR-B and 0.99 for ER IHC; Wald test p value = 0.03 for PR-B and 0.3 for ER IHC).

Figs. 29 and 30 show PR cellular signaling pathway activity predictions of trained Bayesian network models calibrated with the alternative calibration set IKAB01M (IPR-AB36 endometrial cell line treated with vehicle control or InM MPA; see Table 4) for the preferred PR-A, PR-B and PR-A&B target gene lists. Fig. 29: Inferred PR cellular signaling pathway activity levels on IKAB01M calibration samples from GSE29435 (see Fig. 15). Fig. 30: Normal endometrium during menstrual cycle QC pass samples from GSE51981 (see Fig. 10). The resulting PR cellular signaling pathway activity levels (shown as odds PR on:off) are similar to the results obtained with the preferred calibration set.

Fig. 31 shows PR cellular pathway activity predictions of trained Bayesian network models calibrated with two alternative target gene lists and the preferred calibration set EmLPxHS (see Table 4). The activities were inferred for normal endometrium during menstrual cycle QP pass samples from GSE51981 (see Fig. 10). Left: PR-B model using only a shortlist of three target genes CCND1, FKBP5, and MYC (PR-B_3_EmLPxHS; see also Table 10). Middle: PR-A&B model using only a shortlist of four target genes BCL6, CCND1, FKBP5, and MYC (PR-A&B_4_EmLPxHS; see also Table 13). Right: PR-A&B model using only a shortlist of seven target genes BCL6, CCND1, CDKN1A, FKBP5, MYC, SBK1, and VEGFA (PR-A&B _7EmLPxHS; see also Table 14). The resulting PR cellular signaling pathway activities are substantially comparable to the results obtained with the preferred target gene lists (ses Tables 6 to 8).

Fig. 31 shows PR cellular pathway activity predictions of trained Bayesian network models calibrated with five further alternative target gene lists and the preferred calibration set EmLPxHS (see Table 4). The activities were inferred for normal endometrium during menstrual cycle QP pass samples from GSE51981 (see Fig. 10). Left: PR-A model using a longlist of eight target genes BCL2L1, BIRC3, DDIT4, F3, MUC1, NEDD9, SGK1, and TRIM22 (PR-A_8_EmLPxHS; see also Table 9). Next, PR-B model using a longlist of eightteen target genes ARRDC1, ATP1B1, CCND1, CD82, E2F1,

FKBP5, GOT1, HSD11B2, KANK1, MSX2, MYC, NET1, NFKBIA, PDK4, PLIN2, PTP4A2, SNTB2, and STAT5A (PR-B_18_EmLPxHS; see also Table 11). Next, PR-B model using a longlist of twentytwo target genes ARRDC1, ATP1B1, BIRC3, CCND1, CD82, DDIT4, E2F1, F3, FKBP5, GOT1, HSD11B2, KANK1, MSX2, MUC1, MYC, NET1, NFKBIA, PDK4, PLIN2, PTP4A2, SNTB2, and STAT5A (PR-B_22_EmLPxHS; see also Table 12). Next, PR-A&B model using a longlist of twentysix target genes AK4, ARRDC1, ATP1B1, BCL2L1, BCL6, BIRC3, CCND1, CD82, F3, FKBP5, GOT1, GRB10, HSD11B2, KLF4, MUC1, MYC, NEDD9, NET1, PDK4, PTP4A2, S100P, SGK1, SNTB2, STAT5A, TSC22D3, and VASP (PR-A&B_26_EmLPxHS; see also Table 15). Right, PR-A&B model using a longlist of thirtyeight target genes ABCG2, ACSS1, AK4, ARRDC1, ATP1B1, BCL2L1, BCL6, BIRC3, CCND1, CD82, CDKN1A, DDIT4, E2F1, F3, FKBP5, GOT1, GRB10, HPCAL1, HSD11B2, KANK1, KLF4, MSX2, MUC1, MYC, NEDD9, NET1, NFKBIA, PDK4, PLIN2, PTP4A2, S100P, SGK1, SNTB2, STAT5A, TRIM22, TSC22D3, VASP, and VEGFA (PR-A&B_38_EmLPxHS; see also Table 16). The resulting PR cellular signaling pathway activities are substantially comparable to the results obtained with the preferred target gene lists (ses Tables 6 to 8).

The experimental results can be summarized as follows:

(1) PR cellular signaling pathway models correctly infer increase in PR cellular signaling pathway activity in progesterone sensitive tissue and cell lines exposed to progestogens.

(a) Normal reproductive tissue samples during menstrual cycle: The concentration of progesterone (P4) in the endometrium varies considerably during the menstrual cycle from near absent levels in the proliferative phase of the endometrium cycle to high levels of the secretory phase of the endometrium cycle (with mean peak values in the order of 13 ng/mL). Fig. 8 shows that all preferred PR-A, PR-B and PR-A&B target gene lists correctly infer an increase in PR cellular signaling pathway activity levels with an increase of the progesterone level and that PR cellular signaling pathway activity is highly correlated with progesterone levels. Figs. 9 and 10 show that all preferred PR-A, PR-B and PR-A&B target gene lists correctly infer an increase in PR cellular signaling pathway activity levels as the endometrium goes from the proliferative phase to the secretory phase. The follicular and luteal phases of the ovarian cycle corresponds to the proliferative phase and secretory phases of the endometrium cycle, where progesterone levels are, respectively, the lowest and highest (see Fig. 11). Fig. 12 shows that all preferred PR-A, PR-B and PR-A&B target gene lists correctly infer an increase in PR cellular signaling pathway activity levels in fallopian tube tissue as the cycle moves from the follicular to the luteal phase. However, in this case, only the PR-B and PR-A&B models achieve statistical significance.

(b) Other progesterone sensitive tissue and cell lines exposed to progestogens: Figs. 13 to 16 illustrate other exemplary cases where the preferred PR-A, PR-B and PR-A&B target gene lists correctly infer an increase in PR cellular signaling pathway activity when progesterone sensitive tissues or cell lines are stimulated with either the synthetic progestin MPA or the synthetic progestin R5020.

(2) In cancer and other morbidities, similarly to PR-A and PR-B protein levels, PR-A and PR-B cellular signaling pathway inferred activity levels are frequently disrupted.

(a) PR is disrupted in breast cancer: Mote, P.A. et al., "Progesterone Receptor A Predominance Is a Discriminator of Benefit from Endocrine Therapy in the ATAC Trial", Breast Cancer Research and Treatment, Vol. 151, No. 2, 2015, pages 309-318 have shown that in breast cancer PR-A and PR-B protein levels are disrupted. PR-A is generally predominant but PR-B may also be predominant. Accordingly, Fig. 17 shows that, compared to normal tissue, PR-A and PR-B cellular signaling pathway activity levels are disrupted in breast cancer tissue. While normalized PR-A and PR-B cellular signaling pathway activity levels are similar or PR-B is higher than PR-A in normal issue (see Fig. 17 "a"), normalized PR-B cellular signaling pathway activity levels are generally lower than normalized PR-A cellular signaling pathway activity levels in tumor tissue (see Fig. 17 "b"). In most cases, normalized PR-A cellular signaling pathway activity levels are higher in tumor than in normal samples (see Fig. 17 "c") and normalized PR-B cellular signaling pathway activity levels are lower in tumor than in normal samples (see Fig. 17 "d").

(b) PR is disrupted in endometrial cancer: In endometrial cancer, Arnett-Mansfield, R.L. et al., "Subnuclear Distribution of Progesterone Receptors A and B in Normal and Malignant Endometrium", The Journal of Clinical Endocrinology & Metabolism Vol. 89, No. 3, 2004, pages 1429-1442 also showed a disruption between PR-A and PR-B protein isoform expression in cancer compared to the normal endometrium. Fig. 18 shows that PR-A cellular signaling pathway activity levels are increased in endometrial cancer samples compared to normal samples and PR-B cellular signaling pathway activity levels are similar or decreased in endometrial cancer samples compared to normal samples, wherein the difference between PR-A and PR-B increases with cancer grade.

(c) PR is also disrupted in other cancer types as exemplified in Figs. 19 and 20: In lung cancer, while PR-A cellular signaling pathway activity levels increase significantly in cancer compared to normal tissue, PR-B and PR-A&B levels are significantly reduced. Disruption in PR-A and PR-B cellular signaling pathway activity levels are larger on more severe stages of lung cancer.

(d) PR is also disrupted in other morbidities as exemplified in Figs. 21 and 27.

(3) PR activity as a monitor of therapy response: Fig. 22 shows that in ER positive breast cancer patients treated with neoadjuvant aromatase inhibitor letrozole, mean PR-B cellular signaling pathway activity levels are higher in responders than in non-responders. Mean PR-B cellular signaling pathway activity levels go up in responders with activity levels approaching PR-B cellular signaling pathway activity in normal samples (see Fig. 16). Activity levels remain similar to pre-treatment in non-responders.

(4) PR activity as prognostic risk marker: The survival curves of Fig. 23 exemplify PR-A, PR-B and ratios computed from normalized PR-A, PR-B and PR-A&B cellular signaling pathway activity levels as significant prognostic factors for the progression of lung cancer. The survival curves of Fig. 24 exemplify combinations of PR cellular signaling pathway activity levels and PR cellular signaling pathway activity level ratios that are significant prognostic factors for the progression of lung cancer. Fig. 25 exemplifies combinations of PR cellular signaling pathway activity levels and PR cellular signaling pathway activity level ratios that are significant prognostic factors for the progression of breast cancer. Fig. 26 exemplifies PR cellular signaling pathway activity levels as a prognostic risk factor for the progression of pediatric acute lymphoblastic leukemia (ALL).

(5) PR cellular signaling pathway activity levels as a risk factor: Fig. 27 exemplifies PR cellular signaling pathway activity levels as a prognostic risk factor in breast cancer.

(6) PR cellular signaling pathway activity levels as response predictor: The survival curves in Fig. 28 and the cox proportional hazard regression summary exemplify the use of PR cellular signaling pathway models as a predictor to progestogen therapy response in endometrial cancer and its added value over use of ER immunohistochemistry (IHC) only.

(7) Variations of the PR cellular signaling pathway model: PR cellular signaling pathway models can also be constructed using alternative calibration sets as exemplified in Figs. 29 and 30 and using alternative target gene lists as exemplified in Figs. 31 and 32. Fig. 28 exemplifies results obtained by inferring activities by simply adding the normalized expressions of target genes (i.e., using a linear combination) obtained using RT-qPCR.

Instead of applying the calibrated mathematical model, e.g., the exemplary Bayesian network model, on mRNA input data coming from microarrays or RNA sequencing, it may be beneficial in clinical applications to develop dedicated assays to perform the sample measurements, for instance on an integrated platform using qPCR to determine mRNA levels of target genes. The RNA/DNA sequences of the disclosed target genes can then be used to determine which primers and probes to select on such a platform.

Validation of such a dedicated assay can be done by using the microarray-based mathematical model as a reference model, and verifying whether the developed assay gives similar results on a set of validation samples. Next to a dedicated assay, this can also be done to build and calibrate similar mathematical models using RNA sequencing data as input measurements.

The set of target genes which are found to best indicate specific cellular signaling pathway activity based on microarray/RNA sequencing based investigation using the calibrated mathematical model, e.g., the exemplary Bayesian network model, can be translated into a multiplex quantitative PCR assay to be performed on a sample of the subject and/or a computer to interpret the expression measurements and/or to infer the activity of the PR cellular signaling pathway. To develop such a test (e.g., FDA-approved or a CLIA waived test in a central service lab or a laboratory developed test for research use only) for cellular signaling pathway activity, development of a standardized test kit is required, which needs to be clinically validated in clinical trials to obtain regulatory approval.

The present invention relates to a computer-implemented method for inferring activity of a PR cellular signaling pathway in a subject performed by a digital processing device, wherein the inferring is based on expression levels of three or more target genes of the PR cellular signaling pathway measured in a sample of the subject. The present invention further relates to an apparatus for inferring activity of a PR cellular signaling pathway in a subject comprising a digital processor configured to perform the method, to a non-transitory storage medium for inferring activity of a PR cellular signaling pathway in a subject storing instructions that are executable by a digital processing device to perform the method, and to a computer program for inferring activity of a PR cellular signaling pathway in a subject comprising program code means for causing a digital processing device to perform the method, when the computer program is run on the digital processing device.

The method may be used, for instance, in diagnosing an (abnormal) activity of the PR cellular signaling pathway, in prognosis based on the inferred activity of the PR cellular signaling pathway, in the enrollment of a subject in a clinical trial based on the inferred activity of the PR cellular signaling pathway, in the selection of subsequent test(s) to be performed, in the selection of companion diagnostics tests, in clinical decision support systems, or the like. In this regard, reference is made to the published international patent application WO 2013/011479 A2 ("Assessment of cellular signaling pathway activity using probabilistic modeling of target gene expression"), to the published international patent application WO 2014/102668 A2 ("Assessment of cellular signaling pathway activity using linear combination(s) of target gene expressions"), and to Verhaegh W. et al., "Selection of personalized patient therapy through the use of knowledge-based computational models that identify tumor-driving signal transduction pathways", Cancer Research, Vol. 74, No. 11, 2014, pages 2936 to 2945, which describe these applications in more detail.

**5. Further information for illustrating the present invention**

(1) Measuring Levels of gene expression

Data derived from the unique set of target genes described herein is further utilized to infer an activity of the PR cellular signaling pathway using the methods described herein.

Methods for analyzing gene expression levels in extracted samples are generally known. For example, methods such as Northern blotting, the use of PCR, nested PCR, quantitative real-time PCR (qPCR), RNA-seq, or microarrays can all be used to derive gene expression level data. All methods known in the art for analyzing gene expression of the target genes are contemplated herein.

Methods of determining the expression product of a gene using PCR based methods may be of particular use. In order to quantify the level of gene expression using PCR, the amount of each PCR product of interest is typically estimated using conventional quantitative real-time PCR (qPCR) to measure the accumulation of PCR products in real time after each cycle of amplification. This typically utilizes a detectible reporter such as an intercalating dye, minor groove binding dye, or fluorogenic probe whereby the application of light excites the reporter to fluoresce and the resulting fluorescence is typically detected using a CCD camera or photomultiplier detection system, such as that disclosed in U.S. Pat. No. 6,713,297 which is hereby incorporated by reference.

In some embodiments, the probes used in the detection of PCR products in the quantitative real-time PCR (qPCR) assay can include a fluorescent marker. Numerous fluorescent markers are commercially available. For example, Molecular Probes, Inc. (Eugene, Oreg.) sells a wide variety of fluorescent dyes. Non-limiting examples include Cy5, Cy3, TAMRA, R6G, R110, ROX, JOE, FAM, Texas Red™, and Oregon Green™. Additional fluorescent markers can include IDT ZEN Double-Quenched Probes with traditional 5' hydrolysis probes in qPCR assays. These probes can contain, for example, a 5' FAM dye with either a 3' TAMRA Quencher, a 3' Black Hole Quencher (BHQ, Biosearch Technologies), or an internal ZEN Quencher and 3' Iowa Black Fluorescent Quencher (IBFQ).

Fluorescent dyes useful according to the invention can be attached to oligonucleotide primers using methods well known in the art. For example, one common way to add a fluorescent label to an oligonucleotide is to react an N-Hydroxysuccinimide (NHS) ester of the dye with a reactive amino group on the target. Nucleotides can be modified to carry a reactive amino group by, for example, inclusion of an allyl amine group on the nucleobase. Labeling via allyl amine is described, for example, in U.S. Pat. Nos. 5,476,928 and 5,958,691, which are incorporated herein by reference. Other means of fluorescently labeling nucleotides, oligonucleotides and polynucleotides are well known to those of skill in the art.

Other fluorogenic approaches include the use of generic detection systems such as SYBR-green dye, which fluoresces when intercalated with the amplified DNA from any gene expression product as disclosed in U.S. Pat. Nos. 5,436,134 and 5,658,751 which are hereby incorporated by reference.

Another useful method for determining target gene expression levels includes RNA-seq, a powerful analytical tool used for transcriptome analyses, including gene expression level difference between different physiological conditions, or changes that occur during development or over the course of disease progression.

Another approach to determine gene expression levels includes the use of microarrays for example RNA and DNA microarray, which are well known in the art. Microarrays can be used to quantify the expression of a large number of genes simultaneously.

(2) Generalized workflow for determining the activity of PR cellular signaling

A flowchart exemplarily illustrating a process for inferring the activity of PR cellular signaling from a sample isolated from a subject is shown in Fig. 2. First, the mRNA from a sample is isolated (11). Second, the mRNA expression levels of a unique set of at least three or more PR target genes, as described herein, are measured (12) using methods for measuring gene expression that are known in the art. Next, an activity level of a PR transcription factor (TF) element (13) is determined using a calibrated mathematical pathway model (14) relating the expression levels of the three or more PR target genes to the activity level of the PR TF element. Finally, the activity of the PR cellular signaling pathway in the subject is inferred (15) based on the determined activity level of the PR TF element in the sample of the subject. For example, the PR cellular signaling pathway is determined to be active if the activity is above a certain threshold, and can be categorized as passive if the activity falls below a certain threshold.

(3) Calibrated mathematical pathway model

As contemplated herein, the expression levels of the unique set of three or more PR target genes described herein are used to determine an activity level of a PR TF element using a calibrated mathematical pathway model as further described herein. The calibrated mathematical pathway model relates the expression levels of the three or more PR target genes to the activity level of the PR TF element.

As contemplated herein, the calibrated mathematical pathway model is based on the application of a mathematical pathway model. For example, the calibrated mathematical pathway model can be based on a probabilistic model, for example, a Bayesian network model, or a linear or pseudo-linear model.

In an embodiment, the calibrated mathematical pathway model is a probabilistic model incorporating conditional probabilistic relationships relating the PR TF element and the expression levels of the three or more PR target genes. In an embodiment, the probabilistic model is a Bayesian network model.

In an alternative embodiment, the calibrated pathway mathematical model can be a linear or pseudo-linear model. In an embodiment, the linear or pseudo-linear model is a linear or pseudo-linear combination model as further described herein.

A flowchart exemplarily illustrating a process for generating a calibrated mathematical pathway model is shown in Fig. 3. As an initial step, the training data for the mRNA expression levels is collected and normalized. The data can be collected using, for example, microarray probeset intensities (101), real-time PCR Cq values (102), raw RNAseq reads (103), or alternative measurement modalities (104) known in the art. The raw expression level data can then be normalized for each method, respectively, by normalization using a normalization algorithm, for example, frozen robust multiarray analysis (fRMA) or MAS5.0 (111), normalization to average Cq of reference genes (112), normalization of reads into reads/fragments per kilobase of transcript per million mapped reads (RPKM/FPKM) (113), or normalization w.r.t. reference genes/proteins (114). This normalization procedure leads to a normalized probeset intensity (121), normalized Cq values (122), normalized RPKM/FPKM (123), or normalized measurement (124) for each method, respectively, which indicate target gene expression levels within the training samples.

Once the training data has been normalized, a training sample ID or IDs (131) is obtained and the training data of these specific samples is obtained from one of the methods for determining gene expression (132). The final gene expression results from the training sample are output as training data (133). All of the data from various training samples are incorporated to calibrate the model (including for example, thresholds, CPTs, for example in the case of the probabilistic or Bayesian network, weights, for example, in the case of the linear or pseudo-linear model, etc) (144). In addition, the pathway's target genes and measurement nodes (141) are used to generate the model structure for example, as described in Fig. 1 (142). The resulting model structure (143) of the pathway is then incorporated with the training data (133) to calibrate the model (144), wherein the gene expression levels of the target genes is indicative of the transcription factor element activity. As a result of the TF element determination in the training samples, a calibrated pathway model (145) is generated, which assigns the PR cellular signaling pathway activity for a subsequently examined sample of interest, for example from a subject with a cancer, based on the target gene expression levels in the training samples.

(4) TF element determination

A flowchart exemplarily illustrating a process for determining an activity level of a TF element is shown in Fig. 4. The expression level data (test data) (163) from a sample extracted from a subject is input into the calibrated mathematical pathway model (145). The mathematical pathway model may be a probabilistic model, for example, a Bayesian network model, a linear model, or a pseudo-linear model.

The mathematical pathway model may be a probabilistic model, for example, a Bayesian network model, based on conditional probabilities relating the PR TF element and expression levels of the three or more target genes of the PR cellular signaling pathway measured in the sample of the subject, or the mathematical model may be based on one or more linear combination(s) of expression levels of the three or more target genes of the PR cellular signaling pathway measured in the sample of the subject. In particular, the determining of the activity of the PR cellular signaling pathway may be performed as disclosed in the published international patent application WO 2013/011479 A2 ("Assessment of cellular signaling pathway activity using probabilistic modeling of target gene expression"), the contents of which are herewith incorporated in their entirety. Briefly, the data is entered into a Bayesian network (BN) inference engine call (for example, a BNT toolbox) (154). This leads to a set of values for the calculated marginal BN probabilities of all the nodes in the BN (155). From these probabilities, the transcription factor (TF) node's probability (156) is determined and establishes the TF element's activity level (157).

Alternatively, the mathematical model may be a linear model. For example, a linear model can be used as described in the published international patent application WO 2014/102668 A2 ("Assessment of cellular signaling pathway activity using linear combination(s) of target gene expressions"), the contents of which are herewith incorporated in their entirety. Further details regarding the calculating/determining of cellular signaling pathway activity using mathematical modeling of target gene expression can also be found in Verhaegh W. et al., "Selection of personalized patient therapy through the use of knowledge-based computational models that identify tumor-driving signal transduction pathways", Cancer Research, Vol. 74, No. 11, 2014, pages 2936-2945. Briefly, the data is entered into a calculated weighted linear combination score (w/c) (151). This leads to a set of values for the calculated weighted linear combination score (152). From these weighted linear combination scores, the transcription factor (TF) node's weighted linear combination score (153) is determined and establishes the TF's element activity level (157).

(5) Procedure for discretized observables

A flowchart exemplarily illustrating a process for inferring activity of a PR cellular signaling pathway in a subject as a discretized observable is shown in Fig. 5. First, the test sample is extracted and given a test sample ID (161). Next, the test data for the mRNA expression levels is collected and normalized (162). The test data can be collected using the same methods as discussed for the training samples in Fig. 4, using microarray probeset intensities (101), real-time PCR Cq values (102), raw RNAseq reads (103), or an alternative measurement modalities (104). The raw expression level data can then be normalized for each method, respectively, by normalization using an algorithm, for example fRMA or MAS5.0 (111), normalization to average Cq of reference genes (112), normalization of reads into RPKM/FPKM (113), and normalization w.r.t. reference genes/proteins (114). This normalization procedure leads to a normalized probeset intensity (121), normalized Cq values (122), normalized RPKM/FPKM (123), or normalized measurement (124) for each method, respectively.

Once the test data has been normalized, the resulting test data (163) is analyzed in a thresholding step (164) based on the calibrated mathematical pathway model (145), resulting in the thresholded test data (165). In using discrete observables, in one non-limiting example, every expression above a certain threshold is, for example, given a value of 1 and values below the threshold are given a value of 0, or in an alternative embodiment, the probability mass above the threshold as described herein is used as a thresholded value. Based on the calibrated mathematical pathway model, this value represents the TF element's activity level (157), which is then used to calculate the cellular signaling pathway's activity (171). The final output gives the cellular signaling pathway's activity (172) in the subject.

(6) Procedure for continuous observables

A flowchart exemplarily illustrating a process for inferring activity of a PR cellular signaling pathway in a subject as a continuous observable is shown in Fig. 6. First, the test sample is extracted and given a test sample ID (161). Next, the test data for the mRNA expression levels is collected and normalized (162). The test data can be collected using the same methods as discussed for the training samples in Figure 5, using microarray probeset intensities (101), real-time PCR Cq values (102), raw RNAseq reads (103), or an alternative measurement modalities (104). The raw expression level data can then be normalized for each method, respectively, by normalization using an algorithm, for example fRMA (111), normalization to average Cq of reference genes (112), normalization of reads into RPKM/FPKM (113), and normalization w.r.t. reference genes/proteins (114). This normalization procedure leads to a normalized probeset intensity (121), normalized Cq values (122), normalized RPKM/FPKM (123), or normalized measurement (124) for each method, respectively.

Once the test data has been normalized, the resulting test data (163) is analyzed in the calibrated mathematical pathway model (145). In using continuous observables, as one non-limiting example, the expression levels are converted to values between 0 and 1 using a sigmoid function as described in further detail herein. The TF element determination as described herein is used to interpret the test data in combination with the calibrated mathematical pathway model, the resulting value represents the TF element's activity level (157), which is then used to calculate the cellular signaling pathway's activity (171). The final output gives the cellular signaling pathway's activity (172) in the subject.

(7) Target gene expression level determination procedure

A flowchart exemplary illustrating a process for deriving target gene expression levels from a sample extracted from a subject is shown in Fig. 7. In an exemplary embodiment, samples are received and registered in a laboratory. Samples can include, for example, Formalin-Fixed, Paraffin-Embedded (FFPE) samples (181) or fresh frozen (FF) samples (180). FF samples can be directly lysed (183). For FFPE samples, the paraffin can be removed with a heated incubation step upon addition of Proteinase K (182). Cells are then lysed (183), which destroys the cell and nuclear membranes which makes the nucleic acid (NA) available for further processing. The nucleic acid is bound to a solid phase (184) which could for example, be beads or a filter. The nucleic acid is then washed with washing buffers to remove all the cell debris which is present after lysis (185). The clean nucleic acid is then detached from the solid phase with an elution buffer (186). The DNA is removed by DNAse treatment to ensure that only RNA is present in the sample (187). The nucleic acid sample can then be directly used in the RT-qPCR sample mix (188). The RT-qPCR sample mixes contains the RNA sample, the RT enzyme to prepare cDNA from the RNA sample and a PCR enzyme to amplify the cDNA, a buffer solution to ensure functioning of the enzymes and can potentially contain molecular grade water to set a fixed volume of concentration. The sample mix can then be added to a multiwell plate (i.e., 96 well or 384 well plate) which contains dried RT-qPCR assays (189). The RT-qPCR can then be run in a PCR machine according to a specified protocol (190). An example PCR protocol includes i) 30 minutes at 50°C; ii) 5 minutes at 95°C; iii) 15 seconds at 95°C; iv) 45 seconds at 60°C; v) 50 cycles repeating steps iii and iv. The Cq values are then determined with the raw data by using the second derivative method (191). The Cq values are exported for analysis (192).

(8) PR mediated diseases and disorders and methods of treatment

As contemplated herein, the methods and apparatuses of the present invention can be utilized to assess PR cellular signaling pathway activity in a subject, for example, a subject suspected of having, or having, a disease or disorder wherein the status of the PR signaling pathway is probative, either wholly or partially, of disease presence or progression. In an embodiment, provided herein is a method of treating a subject comprising receiving information regarding the activity status of a PR cellular signaling pathway derived from a sample extracted from the subject using the methods described herein and administering to the subject a PR inhibitor if the information regarding the activity of the PR cellular signaling pathway is indicative of an active PR signaling pathway. In a particular embodiment, the PR cellular signaling pathway activity indication is set at a cutoff value of odds of the PR cellular signaling pathway being active of 10:1, 5:1, 4:1, 2:1, 1:1, 1:2, 1:4, 1:5, 1:10.

PR inhibitors that may be used in the present invention are well known. Examples of PR inhibitors include, but are not limited to, mifepristone (MFP; RU-486), Bisphenol A: (BPA), Asoprisnil. Likewise, PR agonists that may be used in the present invention are well-known. Examples of PR agonists include, but are not limited to, Progesterone (P4), Org2058, promegestone (R5020), medroxyprogesterone acetate (MPA).

In a particular embodiment, the subject is suffering, or suspected to be suffering from, a breast cancer, an endometrial cancer, an ovarian cancer, a lung cancer or an acute lymphoblastic leukemia (ALL) cancer. In a particular embodiment, the subject is suffering from, or suspected to be suffering from, a breast cancer.

This application describes several preferred embodiments. Modifications and alterations may occur to others upon reading and understanding the preceding detailed description. It is intended that the application is construed as including all such modifications and alterations insofar as they come within the scope of the appended claims or the equivalents thereof.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Calculations like the determination of the risk score performed by one or several units or devices can be performed by any other number of units or devices.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

**6. Sequence Listings Used in Application**

SEQUENCE LISTING:

| Seq. No. | Gene: |
|---|---|
| Seq. 1 | ABCG2 |
| Seq. 2 | ACSS1 |
| Seq. 3 | AK4 |
| Seq. 4 | ARRDC1 |
| Seq. 5 | ATP1B1 |
| Seq. 6 | BCL2L1 |

(continued)

| Seq. No. | Gene: |
|---|---|
| Seq. 7 | BCL6 |
| Seq. 8 | BIRC3 |
| Seq. 9 | CCND1 |
| Seq. 10 | CD82 |
| Seq. 11 | CDKN1A |
| Seq. 12 | DDIT4 |
| Seq. 13 | E2F1 |
| Seq. 14 | F3 |
| Seq. 15 | FKBP5 |
| Seq. 16 | GOT1 |
| Seq. 17 | GRB10 |
| Seq. 18 | HPCAL1 |
| Seq. 19 | HSD11B2 |
| Seq. 20 | KANK1 |
| Seq. 21 | KLF4 |
| Seq. 22 | MSX2 |
| Seq. 23 | MUC1 |
| Seq. 24 | MYC |
| Seq. 25 | NEDD9 |
| Seq. 26 | NET1 |
| Seq. 27 | NFKBIA |
| Seq. 28 | PDK4 |
| Seq. 29 | PLIN2 |
| Seq. 30 | PTP4A2 |
| Seq. 31 | S100P |
| Seq. 32 | SGK1 |
| Seq. 33 | SNTB2 |
| Seq. 34 | STAT5A |
| Seq. 35 | TRIM22 |
| Seq. 36 | TSC22D3 |
| Seq. 37 | VASP |
| Seq. 38 | VEGFA |

## Claims

1. A computer-implemented method for inferring activity of a PR cellular signaling pathway in a subject performed by a digital processing device, wherein the inferring comprises:

   receiving expression levels of three or more target genes of the PR cellular signaling pathway measured in a sample of the subject,
   determining an activity level of a PR transcription factor (TF) element in the sample of the subject, the PR TF element controlling transcription of the three or more PR target genes, the determining being based on evaluating a calibrated mathematical pathway model relating the expression levels of the three or more PR target genes to the activity level of the PR TF element, and
   inferring the activity of the PR cellular signaling pathway in the subject based on the determined activity level of the PR TF element in the sample of the subject,
   wherein the calibrated mathematical pathway model is PR-A specific and the three or more target genes are selected from the group consisting of: BCL2L1, BIRC3, DDIT4, F3, MUC1, NEDD9, SGK1, and TRIM22, preferably, from the group consisting of: BCL2L1, DDIT4, NEDD9, and TRIM22, and/or
wherein the calibrated mathematical pathway model is PR-B specific and the three or more target genes are selected from the group consisting of: ARRDC1, ATP1B1, BIRC3, CCND1, CD82, DDIT4, E2F1, F3, FKBP5, GOT1, HSD11B2, KANK1, MSX2, MUC1, MYC, NET1, NFKBIA, PDK4, PLIN2, PTP4A2, SNTB2, and STAT5A, preferably, from the group consisting of: ARRDC1, ATP1B1, CCND1, CD82, E2F1, FKBP5, GOT1, HSD11B2, KANK1, MSX2, MYC, NET1, NFKBIA, PDK4, PLIN2, PTP4A2, SNTB2, and STAT5A, preferably, from the group consisting of: ARRDC1, ATP1B1, CCND1, E2F1, FKBP5, HSD11B2, KANK1, MSX2, MYC, NET1, NFKBIA, PDK4, and PLIN2, preferably, from the group consisting of: CCND1, FKBP5, and MYC, and/or
wherein the calibrated mathematical pathway model is PR-A&B specific and the three or more target genes are selected from the group consisting of: ABCG2, ACSS1, AK4, ARRDC1, ATP1B1, BCL2L1, BCL6, BIRC3, CCND1, CD82, CDKN1A, DDIT4, E2F1, F3, FKBP5, GOT1, GRB10, HPCAL1, HSD11B2, KANK1, KLF4, MSX2, MUC1, MYC, NEDD9, NET1, NFKBIA, PDK4, PLIN2, PTP4A2, S100P, SGK1, SNTB2, STAT5A, TRIM22, TSC22D3, VASP, and VEGFA, preferably, from the group consisting of: AK4, ARRDC1, ATP1B1, BCL2L1, BCL6, BIRC3, CCND1, CD82, F3, FKBP5, GOT1, GRB10, HSD11B2, KLF4, MUC1, MYC, NEDD9, NET1, PDK4, PTP4A2, S100P, SGK1, SNTB2, STAT5A, TSC22D3, and VASP, or, preferably, from the group consisting of: ABCG2, ACSS1, AK4, ATP1B1, BCL6, CCND1, FKBP5, GRB10, HSD11B2, KANK1, KLF4, MYC, NFKBIA, PDK4, PLIN2, S100P, TSC22D3, and VASP, or, preferably, from the group consisting of: BCL6, CCND1, CDKN1A, FKBP5, MYC, SGK1, and VEGFA, or, preferably, from the group consisting of: BCL6, CCND1, FKBP5, and MYC.

**2.** The method claim 1, further comprising:
determining whether the PR cellular signaling pathway is operating abnormally in the subject based on the inferred activity of the PR cellular signaling pathway in the subject.

**3.** The method of claim 1 or 2, further comprising:
determining a prognostic cancer marker based on a combination of inferred activities of the PR cellular signaling pathway in the subject using two or more of the PR-A specific calibrated mathematical pathway model, the PR-B specific calibrated mathematical pathway model and the PR-A&B specific calibrated mathematical pathway model.

**4.** The method of claim 3, wherein the combination is a ratio between the inferred activity of the PR cellular signaling pathway in the subject using the PR-A&B specific calibrated mathematical pathway model and the inferred activity of the PR cellular signaling pathway in the subject using the PR-B specific calibrated mathematical pathway model.

**5.** The method of claim 4, further comprising:

recommending prescribing a drug for the subject that corrects for abnormal operation of the PR cellular signaling pathway,
wherein the recommending is performed if the PR cellular signaling pathway is determined to be operating abnormally in the subject based on the inferred activity of the PR cellular signaling pathway.

**6.** The method of claim 4 or 5, wherein the abnormal operation of the PR cellular signaling pathway is an operation in which the PR cellular signaling pathway operates as a tumor promoter in the subject.

**7.** The method of any of claims 1 to 6, wherein the method is used in at least one of the following activities:

diagnosis based on the inferred activity of the PR cellular signaling pathway in the subject;
prognosis based on the inferred activity of the PR cellular signaling pathway in the subject;
drug prescription based on the inferred activity of the PR cellular signaling pathway in the subject;
prediction of drug efficacy based on the inferred activity of the PR cellular signaling pathway in the subject;
prediction of adverse effects based on the inferred activity of the PR cellular signaling pathway in the subject;
monitoring of drug efficacy;
drug development;
assay development;
pathway research;
cancer staging;
enrollment of the subject in a clinical trial based on the inferred activity of the PR cellular signaling pathway in the subject;
selection of subsequent test to be performed; and

selection of companion diagnostics tests.

8. The method of any of claims 1 to 7, wherein the calibrated mathematical pathway model is a probabilistic model, preferably a Bayesian network model, based on conditional probabilities relating the activity level of the PR TF element and the expression levels of the three or more PR target genes, or wherein the mathematical pathway model is based on one or more linear combination(s) of the expression levels of the three or more PR target genes.

9. An apparatus for inferring activity of a PR cellular signaling pathway in a subject comprising a digital processor configured to perform the method of any of claims 1 to 8.

10. A non-transitory storage medium for inferring activity of a PR cellular signaling pathway in a subject storing instructions that are executable by a digital processing device to perform the method of any of claims 1 to 8.

11. A computer program for inferring activity of a PR cellular signaling pathway in a subject comprising program code means for causing a digital processing device to perform the method of any of claims 1 to 8, when the computer program is run on the digital processing device.

12. A kit for measuring expression levels of three or more target genes of the PR cellular signaling pathway in a sample of a subject, comprising:

polymerase chain reaction primers directed to the three or more PR target genes,
probes directed to the three or more PR target genes,
wherein the calibrated mathematical pathway model is PR-A specific and the three or more target genes are selected from the group consisting of: BCL2L1, BIRC3, DDIT4, F3, MUC1, NEDD9, SGK1, and TRIM22, preferably, from the group consisting of: BCL2L1, DDIT4, NEDD9, and TRIM22, and/or
wherein the calibrated mathematical pathway model is PR-B specific and the three or more target genes are selected from the group consisting of: ARRDC1, ATP1B1, BIRC3, CCND1, CD82, DDIT4, E2F1, F3, FKBP5, GOT1, HSD11B2, KANK1, MSX2, MUC1, MYC, NET1, NFKBIA, PDK4, PLIN2, PTP4A2, SNTB2, and STAT5A, preferably, from the group consisting of: ARRDC1, ATP1B1, CCND1, CD82, E2F1, FKBP5, GOT1, HSD11B2, KANK1, MSX2, MYC, NET1, NFKBIA, PDK4, PLIN2, PTP4A2, SNTB2, and STAT5A, preferably, from the group consisting of: ARRDC1, ATP1B1, CCND1, E2F1, FKBP5, HSD11B2, KANK1, MSX2, MYC, NET1, NFKBIA, PDK4, and PLIN2, preferably, from the group consisting of: CCND1, FKBP5, and MYC, and/or
wherein the calibrated mathematical pathway model is PR-A&B specific and the three or more target genes are selected from the group consisting of: ABCG2, ACSS1, AK4, ARRDC1, ATP1B1, BCL2L1, BCL6, BIRC3, CCND1, CD82, CDKN1A, DDIT4, E2F1, F3, FKBP5, GOT1, GRB10, HPCAL1, HSD11B2, KANK1, KLF4, MSX2, MUC1, MYC, NEDD9, NET1, NFKBIA, PDK4, PLIN2, PTP4A2, S100P, SGK1, SNTB2, STAT5A, TRIM22, TSC22D3, VASP, and VEGFA, preferably, from the group consisting of: AK4, ARRDC1, ATP1B1, BCL2L1, BCL6, BIRC3, CCND1, CD82, F3, FKBP5, GOT1, GRB10, HSD11B2, KLF4, MUC1, MYC, NEDD9, NET1, PDK4, PTP4A2, S100P, SGK1, SNTB2, STAT5A, TSC22D3, and VASP, or, preferably, from the group consisting of: ABCG2, ACSS1, AK4, ATP1B1, BCL6, CCND1, FKBP5, GRB10, HSD11B2, KANK1, KLF4, MYC, NFKBIA, PDK4, PLIN2, S100P, TSC22D3, and VASP, or, preferably, from the group consisting of: BCL6, CCND1, CDKN1A, FKBP5, MYC, SGK1, and VEGFA, or, preferably, from the group consisting of: BCL6, CCND1, FKBP5, and MYC.

13. A kit for inferring activity of a PR cellular signaling pathway in a subject, comprising:

the kit of claim 12, and
the apparatus of claim 9, the non-transitory storage medium of claim 10, or the computer program of claim 11.

14. A kit for inferring activity of a PR cellular signaling pathway in a subject, comprising:

one or more components for determining expression levels of three or more target genes of the PR cellular signaling pathway in a sample of the subject,
wherein the one or more components are preferably selected from the group consisting of: a DNA array chip, an oligonucleotide array chip, a protein array chip, an antibody, a plurality of probes, for example, labeled probes, a set of RNA reverse-transcriptase sequencing components, and/or RNA or DNA, including cDNA, amplification primers,
wherein the calibrated mathematical pathway model is PR-A specific and the three or more target genes are selected from the group consisting of: BCL2L1, BIRC3, DDIT4, F3, MUC1, NEDD9, SGK1, and TRIM22, preferably, from the group consisting of: BCL2L1, DDIT4, NEDD9, and TRIM22, and/or
wherein the calibrated mathematical pathway model is PR-B specific and the three or more target genes are selected from the group consisting of: ARRDC1, ATP1B1, BIRC3, CCND1, CD82, DDIT4, E2F1, F3, FKBP5, GOT1, HSD11B2, KANK1, MSX2, MUC1, MYC, NET1, NFKBIA, PDK4, PLIN2, PTP4A2, SNTB2, and STAT5A, preferably, from the group consisting of: ARRDC1, ATP1B1, CCND1, CD82, E2F1, FKBP5, GOT1, HSD11B2, KANK1, MSX2, MYC, NET1, NFKBIA, PDK4, PLIN2, PTP4A2, SNTB2, and STAT5A, preferably, from the group consisting of: ARRDC1, ATP1B1, CCND1, E2F1, FKBP5, HSD11B2, KANK1, MSX2, MYC, NET1, NFKBIA, PDK4, and PLIN2, preferably, from the group consisting of: CCND1, CDKN1A, FKBP5, and MYC, and/or
wherein the calibrated mathematical pathway model is PR-A&B specific and the three or more target genes are selected from the group consisting of: ABCG2, ACSS1, AK4, ARRDC1, ATP1B1, BCL2L1, BCL6, BIRC3, CCND1, CD82, CDKN1A, DDIT4, E2F1, F3, FKBP5, GOT1, GRB10, HPCAL1, HSD11B2, KANK1, KLF4, MSX2, MUC1, MYC, NEDD9, NET1, NFKBIA, PDK4, PLIN2, PTP4A2, S100P, SGK1, SNTB2, STAT5A, TRIM22, TSC22D3, VASP, and VEGFA, preferably, from the group consisting of: AK4, ARRDC1, ATP1B1, BCL2L1, BCL6, BIRC3, CCND1, CD82, F3, FKBP5, GOT1, GRB10, HSD11B2, KLF4, MUC1, MYC, NEDD9, NET1, PDK4, PTP4A2, S100P, SGK1, SNTB2, STAT5A, TSC22D3, and VASP, or, preferably, from the group consisting of: ABCG2, ACSS1, AK4, ATP1B1, BCL6, CCND1, FKBP5, GRB10, HSD11B2, KANK1, KLF4, MYC, NFKBIA, PDK4, PLIN2, S100P, TSC22D3, and VASP, or, preferably, from the group consisting of: BCL6, CCND1, CDKN1A, FKBP5, MYC, SGK1, and VEGFA, or, preferably, from the group consisting of: BCL6, CCND1, FKBP5, and MYC.

**15.** Use of the kit of any of claims 12 to 14 in performing the method of any of claims 1 to 8.

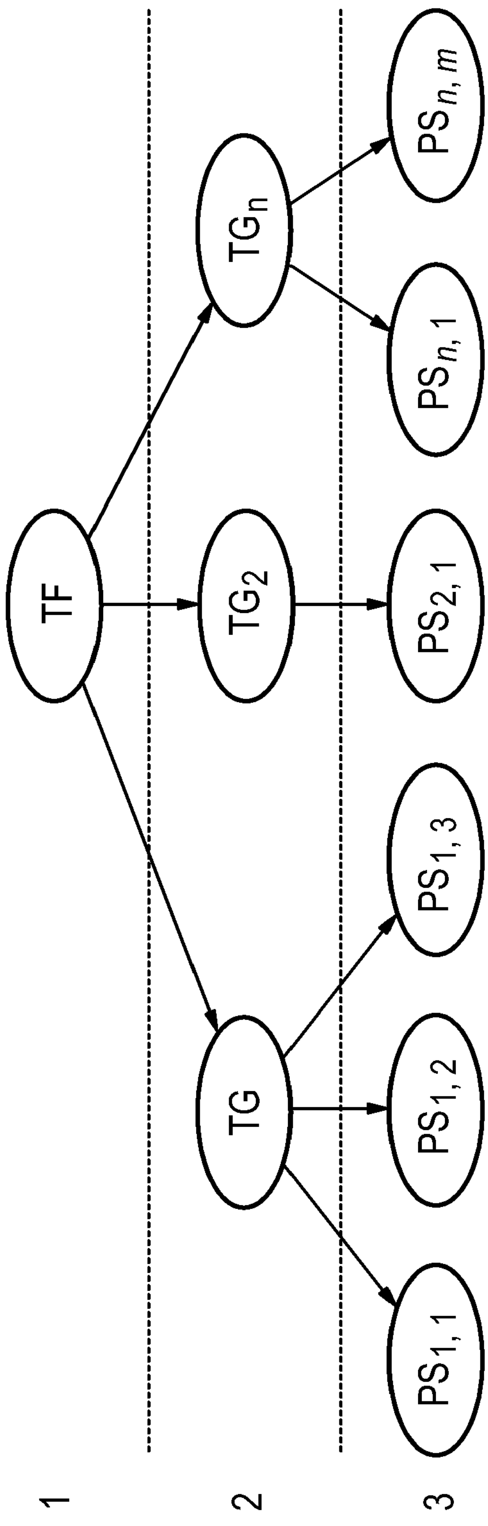


FIG. 1

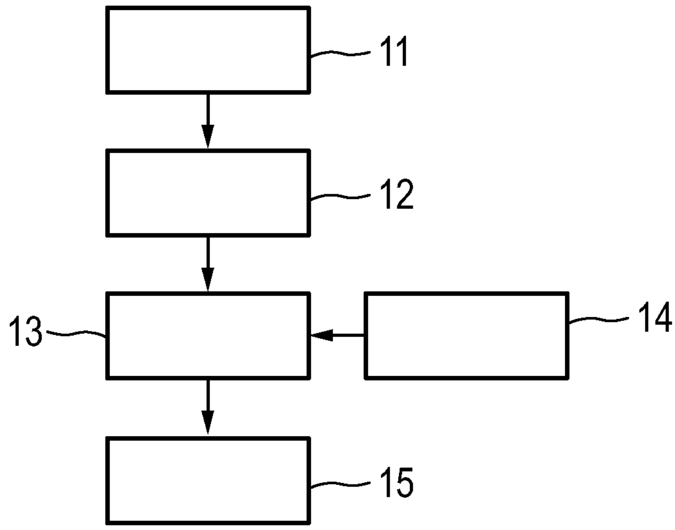


FIG. 2

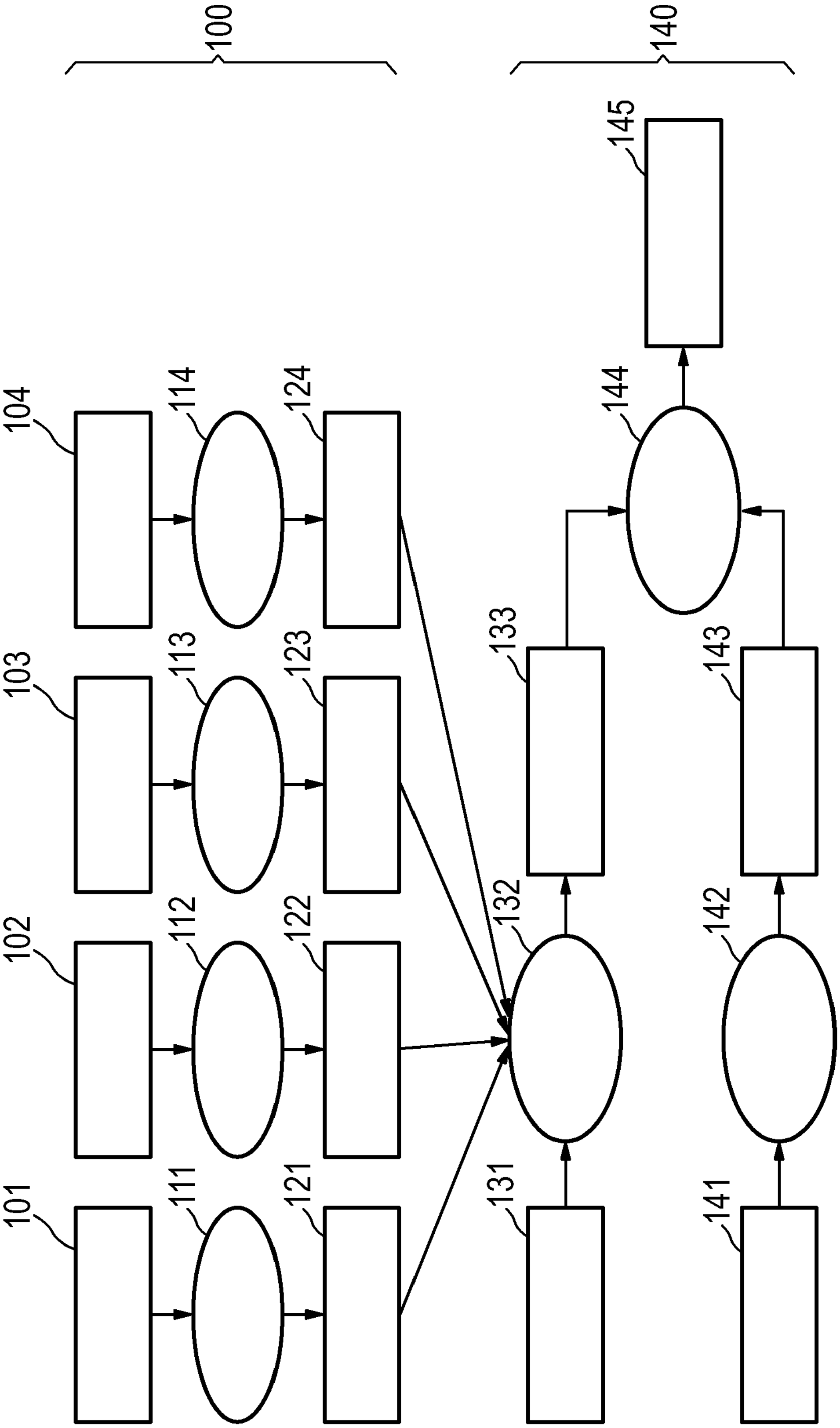
100
140
101
102
103
104
111
112
113
114
121
122
123
124
131
132
133
141
142
143
144
145

FIG. 3

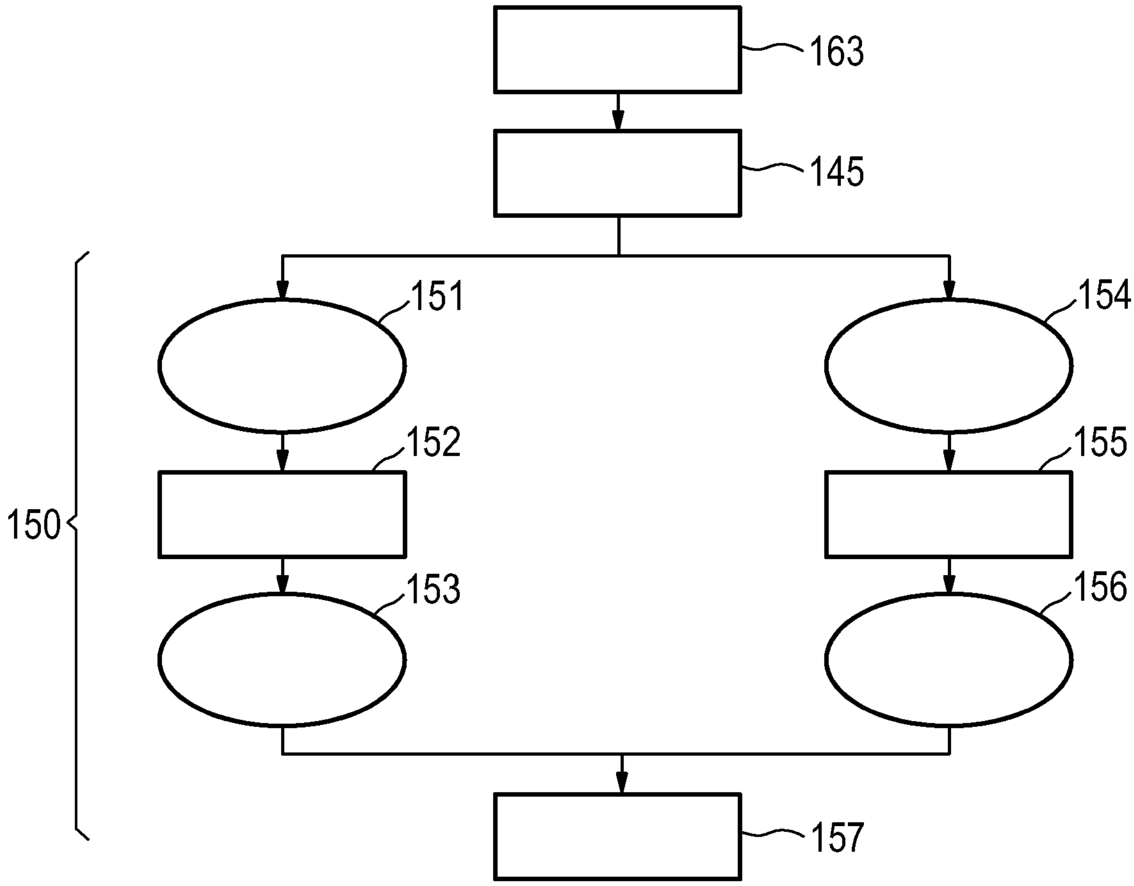


FIG. 4

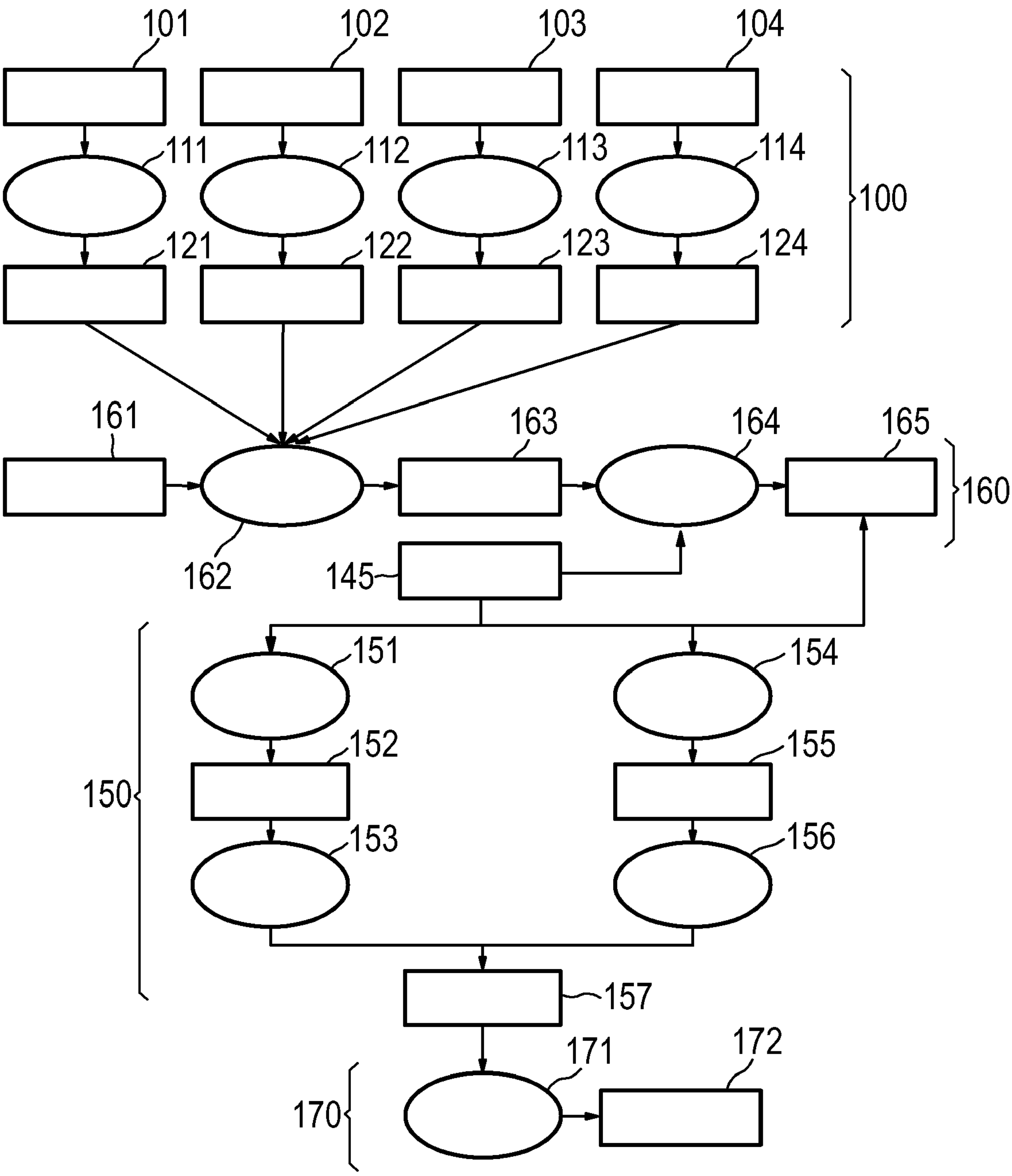
101
102
103
104
111
112
113
114
100
121
122
123
124
161
163
164
165
160
162
145
151
154
152
155
150
153
156
157
171
172
170

FIG. 5

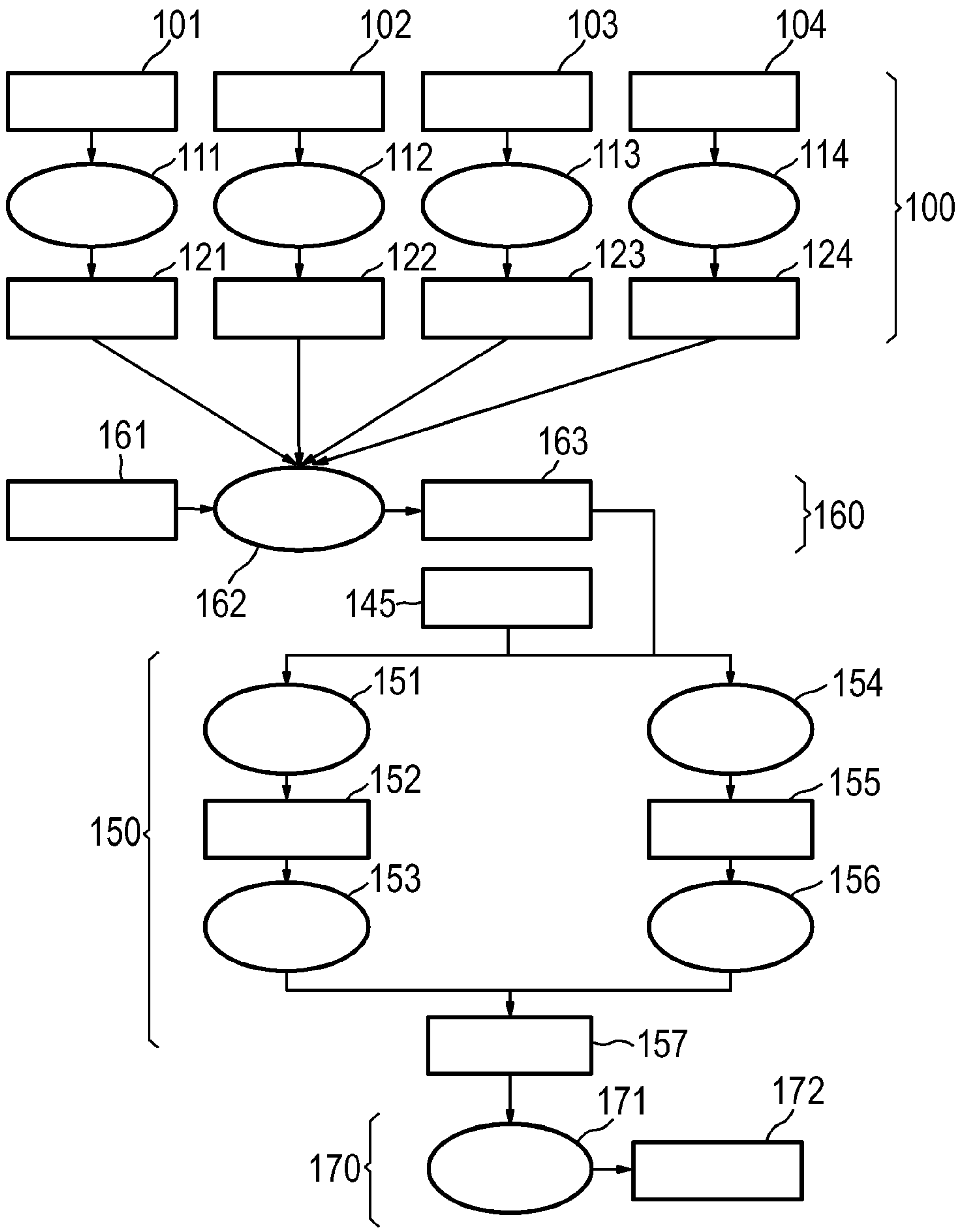
101
102
103
104
111
112
113
114
100
121
122
123
124
161
163
160
162
145
151
154
152
155
150
153
156
157
171
172
170

FIG. 6

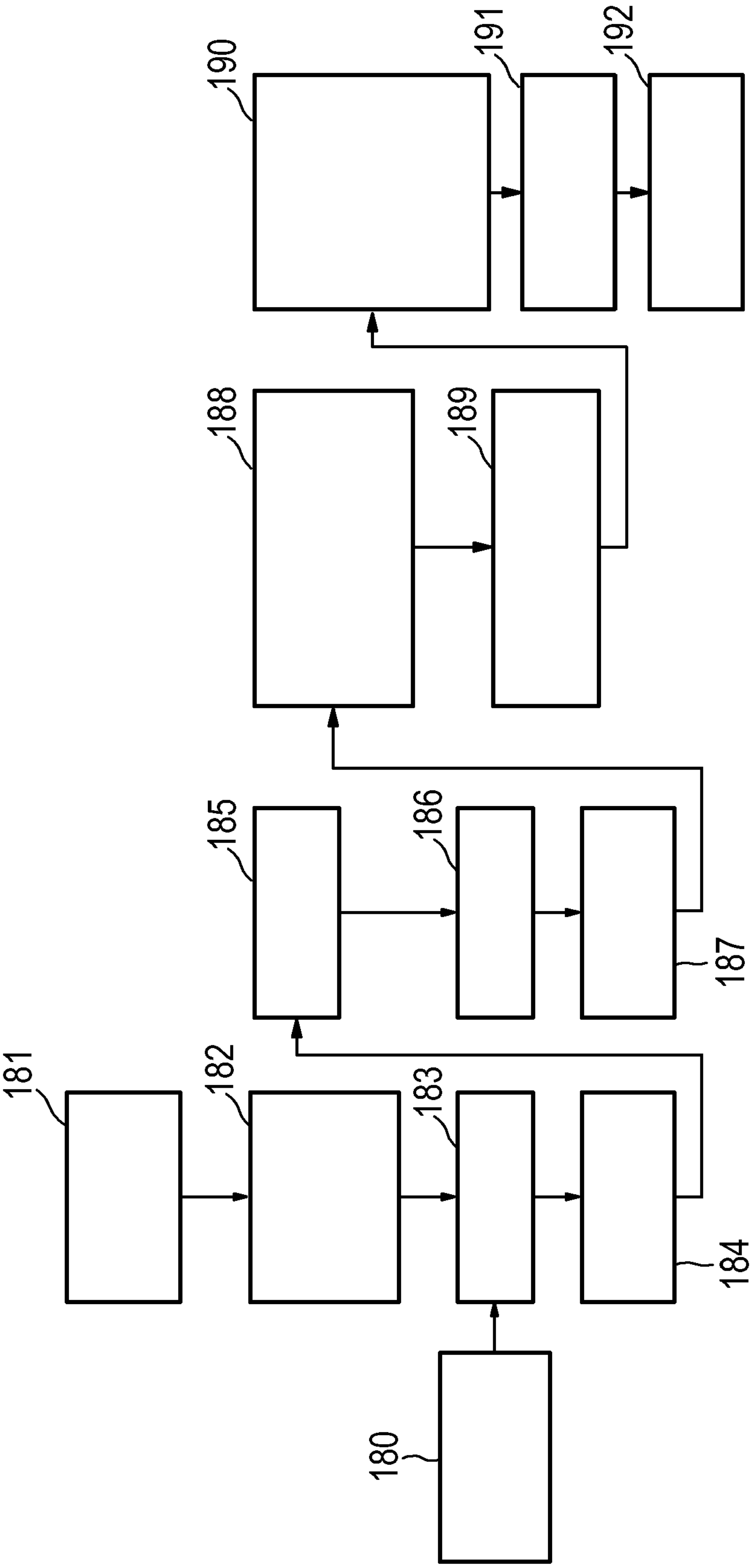


FIG. 7

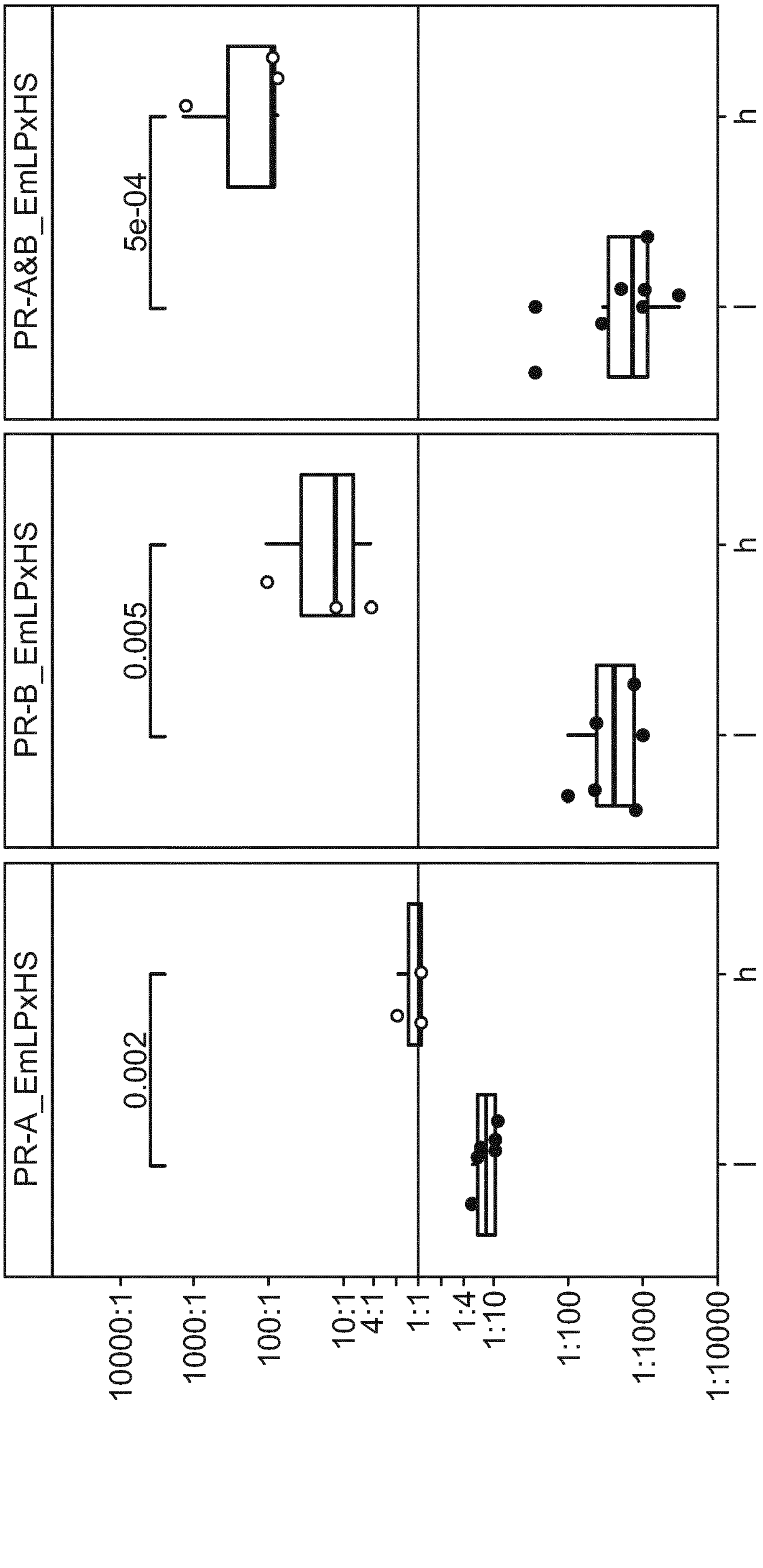
PR-A_EmLPxHS
0.002
PR-B_EmLPxHS
0.005
PR-A&B_EmLPxHS
5e-04
10000:1
1000:1
100:1
10:1
4:1
1:1
1:4
1:10
1:100
1:1000
1:10000
l
h
l
h
l
h

FIG. 8

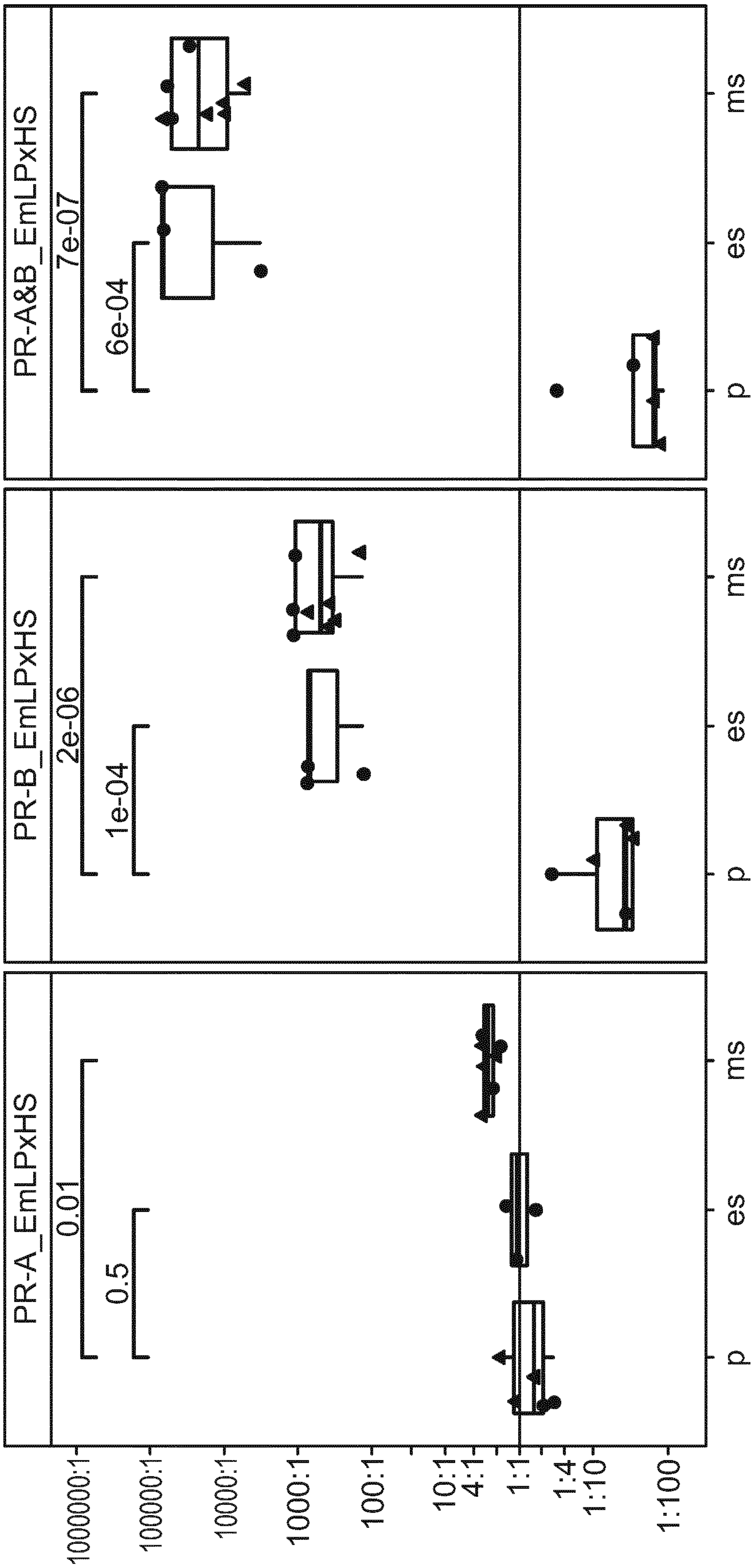
PR-A_EmLPxHS
0.01
0.5
PR-B_EmLPxHS
2e-06
1e-04
PR-A&B_EmLPxHS
7e-07
6e-04
1000000:1
100000:1
10000:1
1000:1
100:1
10:1
4:1
1:1
1:4
1:10
1:100
p
es
ms

FIG. 9

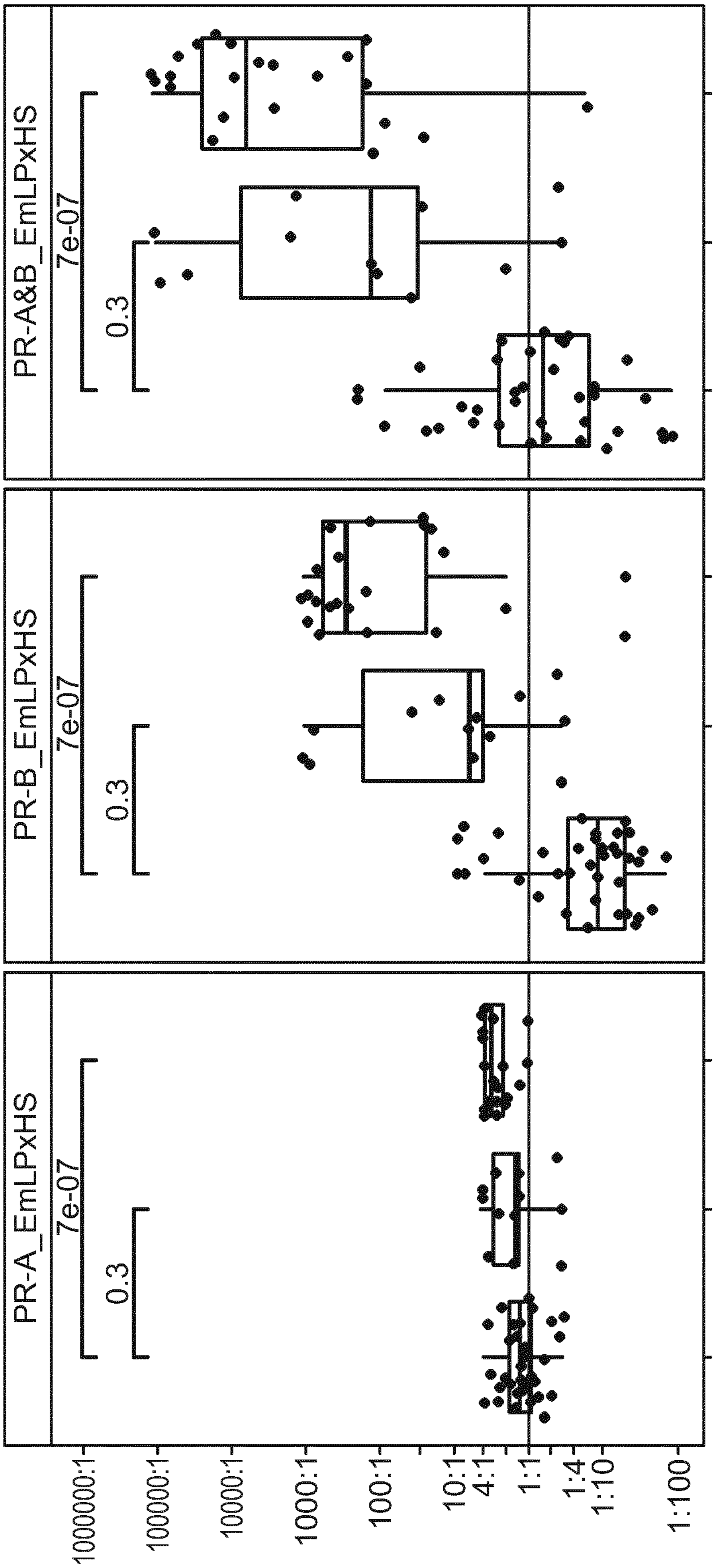
PR-A_EmLPxHS
PR-B_EmLPxHS
PR-A&B_EmLPxHS
7e-07
0.3
1000000:1
100000:1
10000:1
1000:1
100:1
10:1
4:1
1:1
1:4
1:10
1:100
p
es
ms

FIG. 10

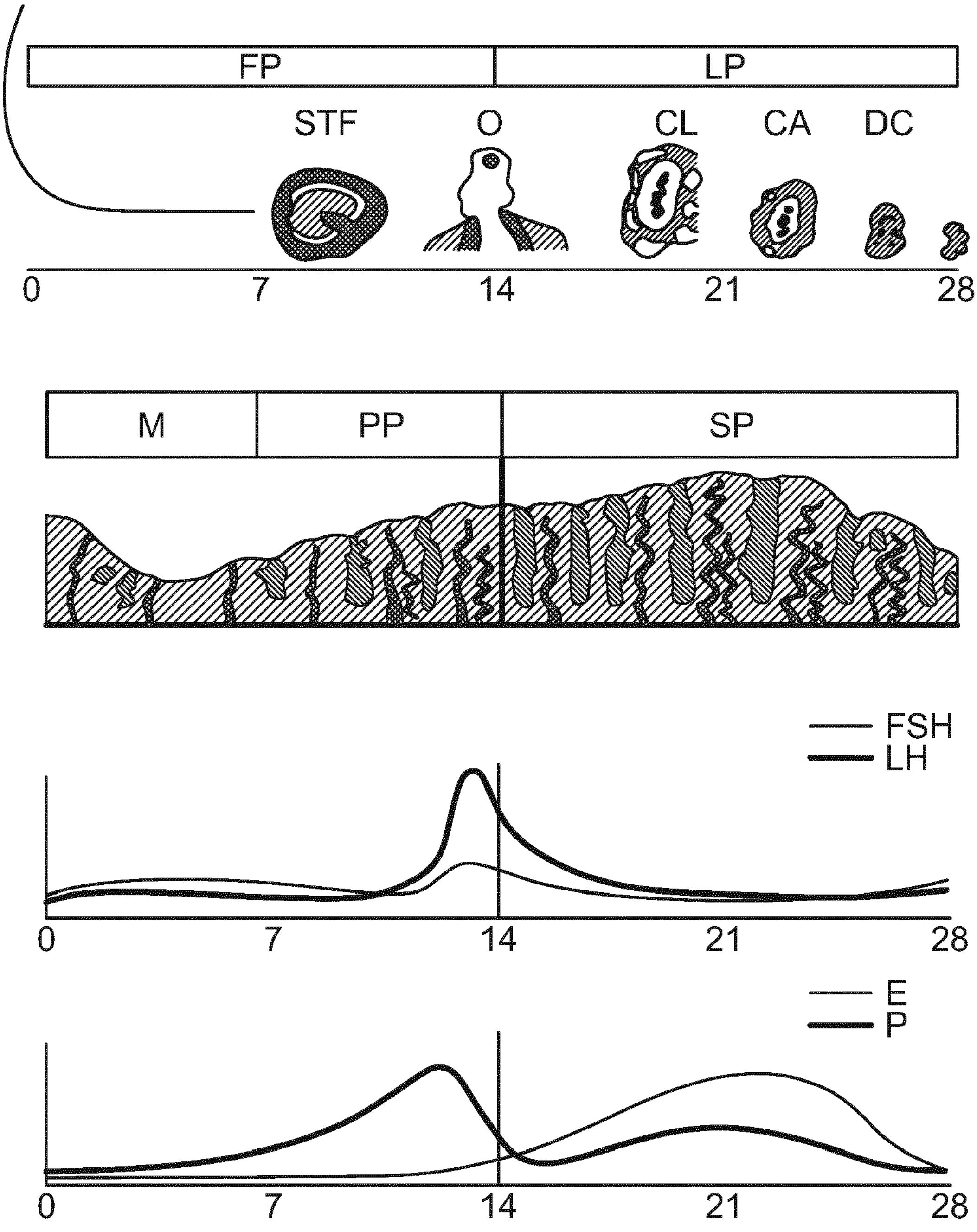
FP
LP
STF
O
CL
CA
DC
0
7
14
21
28
M
PP
SP
FSH
LH
0
7
14
21
28
E
P
0
7
14
21
28

FIG. 11

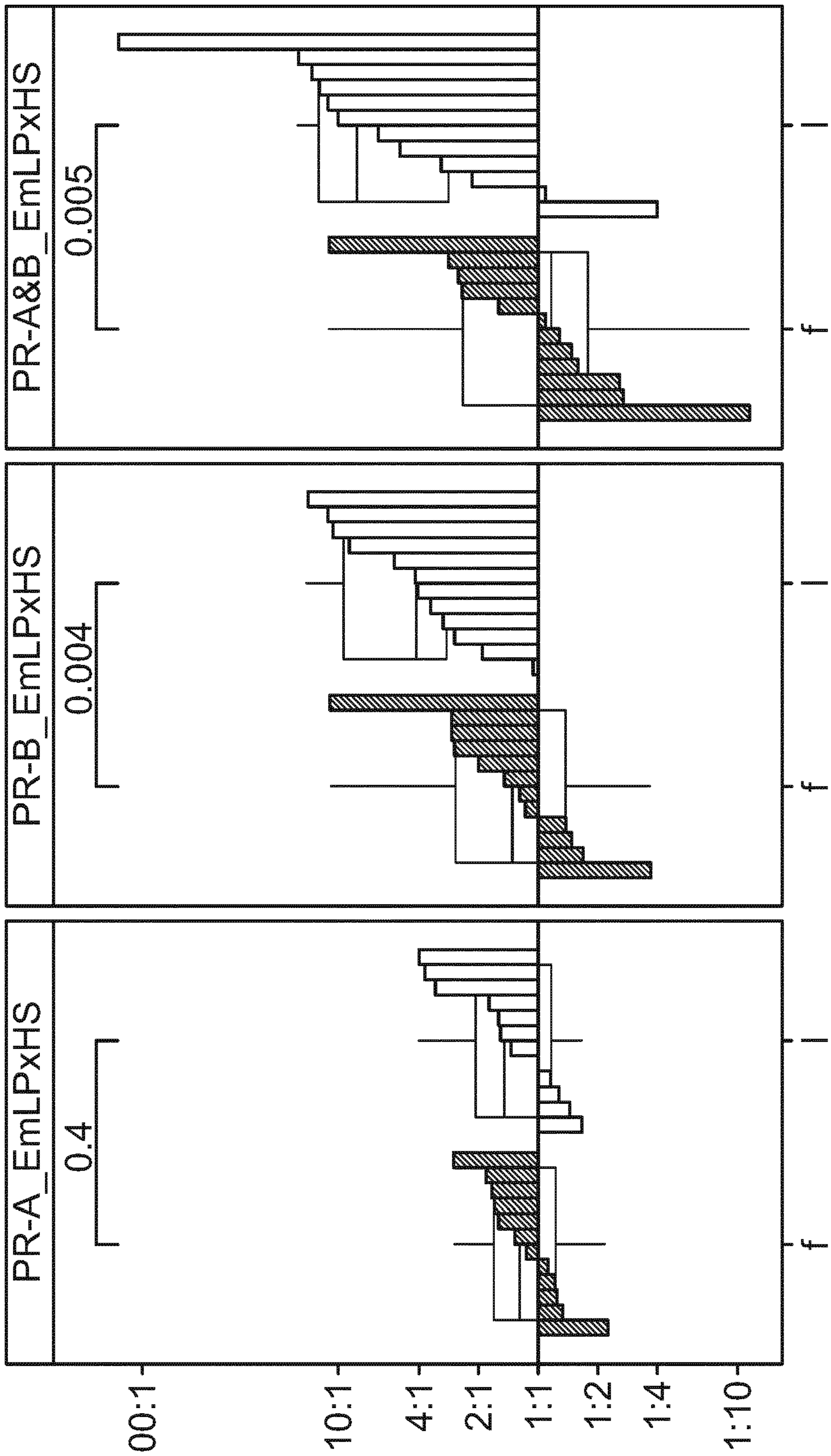
PR-A_EmLPxHS
0.4
PR-B_EmLPxHS
0.004
PR-A&B_EmLPxHS
0.005
f
l
100:1
10:1
4:1
2:1
1:1
1:2
1:4
1:10

FIG. 12

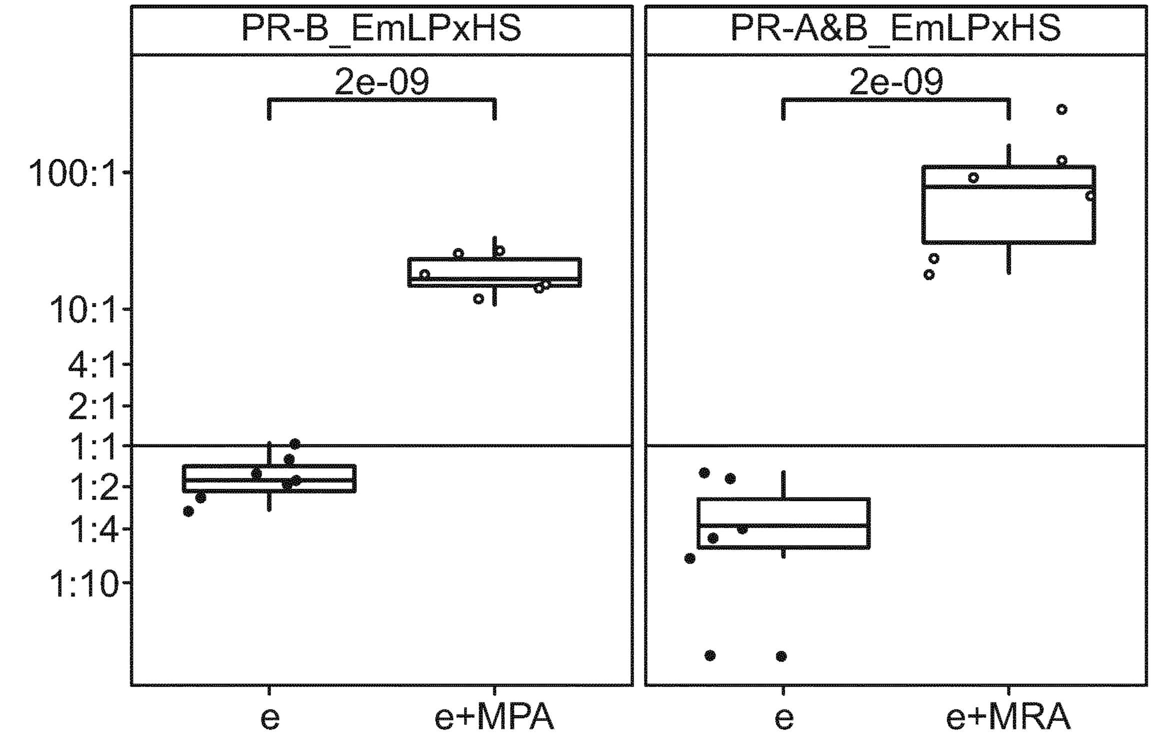

PR-B_EmLPxHS
PR-A&B_EmLPxHS
2e-09
2e-09
100:1
10:1
4:1
2:1
1:1
1:2
1:4
1:10
e
e+MPA
e
e+MRA


FIG. 13

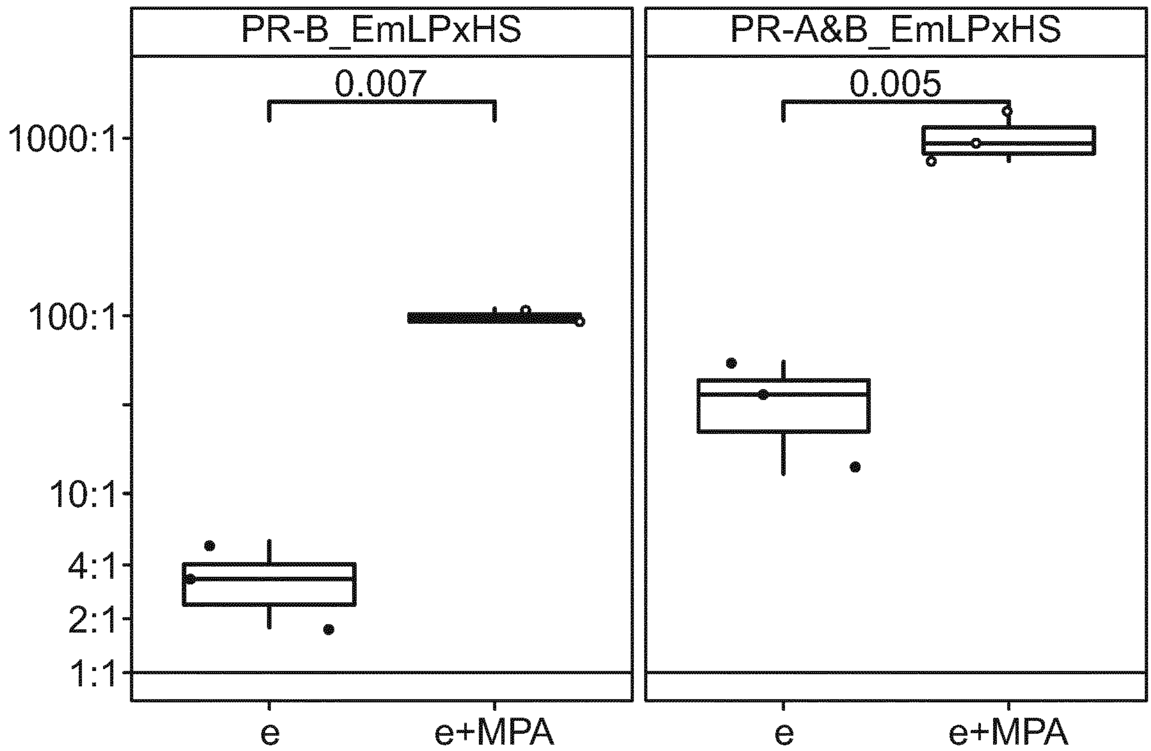

PR-B_EmLPxHS
PR-A&B_EmLPxHS
0.007
0.005
1000:1
100:1
10:1
4:1
2:1
1:1
e
e+MPA
e
e+MPA


FIG. 14

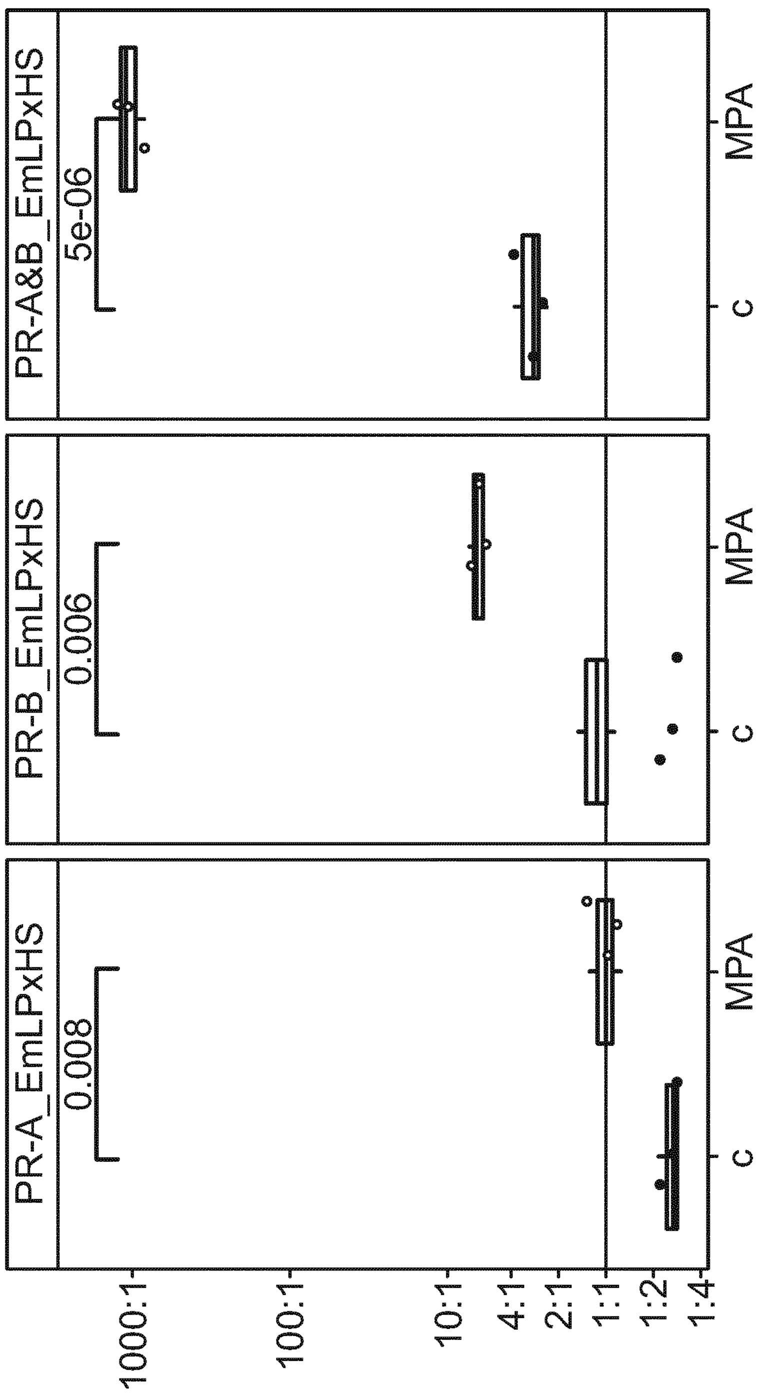
PR-A_EmLPxHS
0.008
PR-B_EmLPxHS
0.006
PR-A&B_EmLPxHS
5e-06
1000:1
100:1
10:1
4:1
2:1
1:1
1:2
1:4
c
MPA

FIG. 15

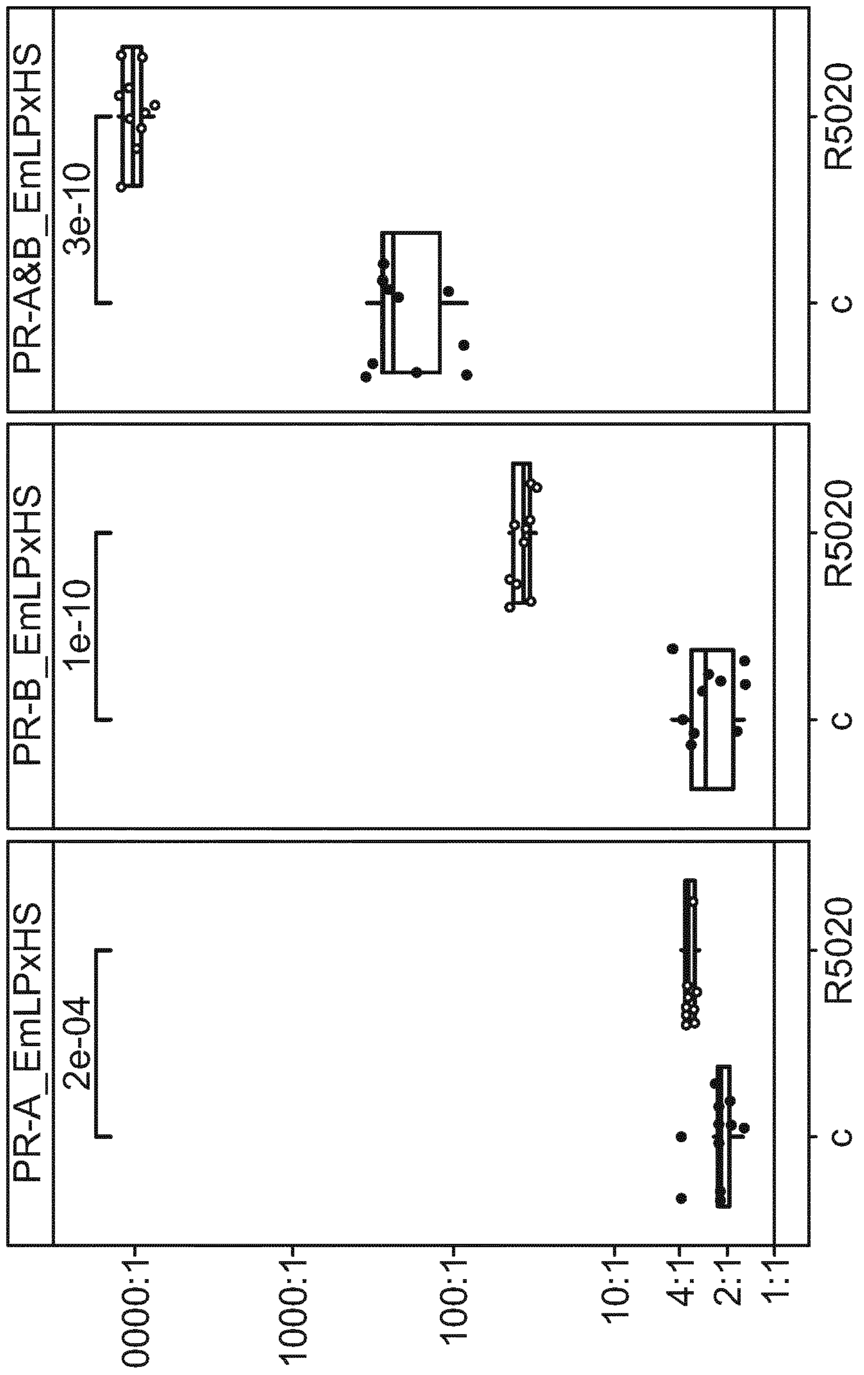
PR-A_EmLPxHS
2e-04
PR-B_EmLPxHS
1e-10
PR-A&B_EmLPxHS
3e-10
10000:1
1000:1
100:1
10:1
4:1
2:1
1:1
c
R5020

FIG. 16

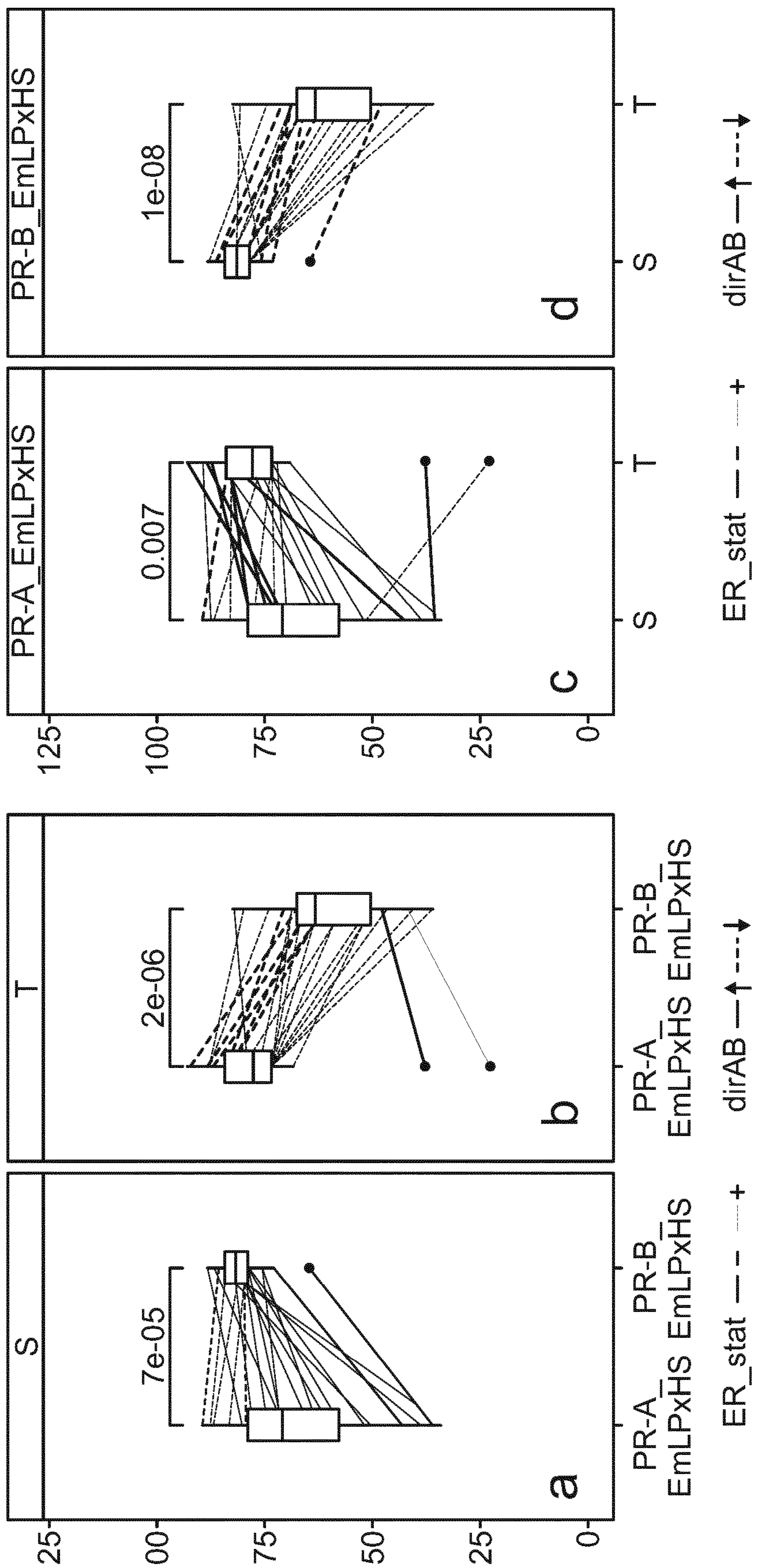
S
T
PR-A_EmLPxHS
PR-B_EmLPxHS
7e-05
2e-06
0.007
1e-08
a
b
c
d
0
25
50
75
100
125
PR-A_ EmLPxHS
PR-B_ EmLPxHS
S
T
ER_stat
dirAB
+

FIG. 17

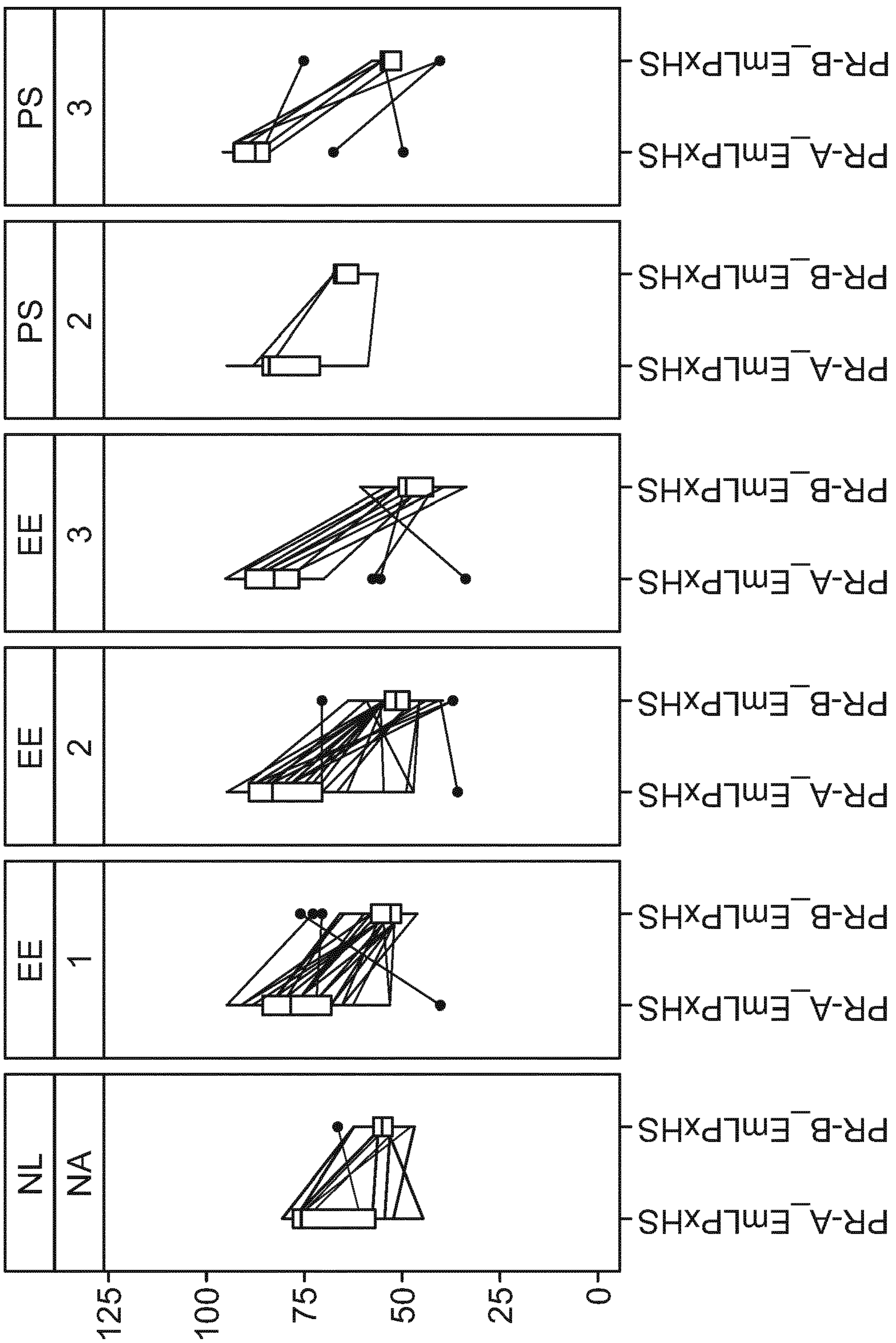
NL
NA
EE
1
EE
2
EE
3
PS
2
PS
3
125
100
75
50
25
0
PR-A_EmLPxHS
PR-B_EmLPxHS

FIG. 18

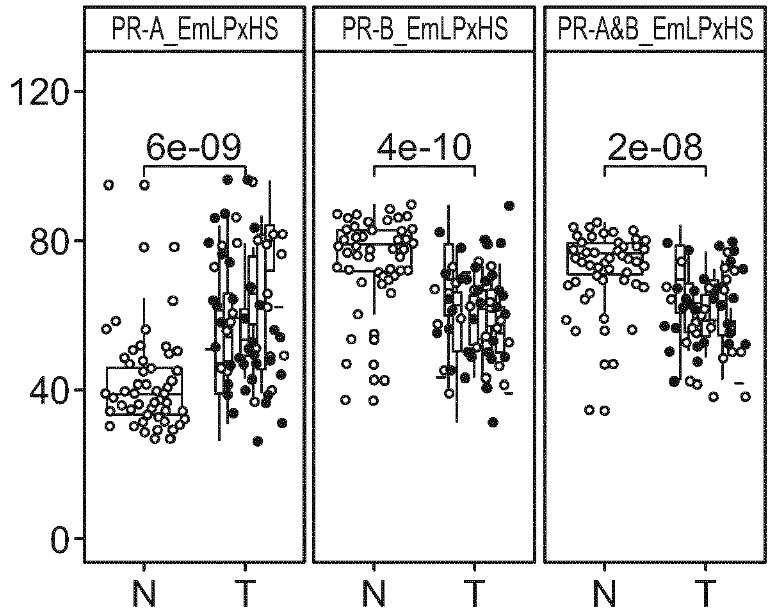
PR-A_EmLPxHS
PR-B_EmLPxHS
PR-A&B_EmLPxHS
120
80
40
0
6e-09
4e-10
2e-08
N
T
N
T
N
T

(a)

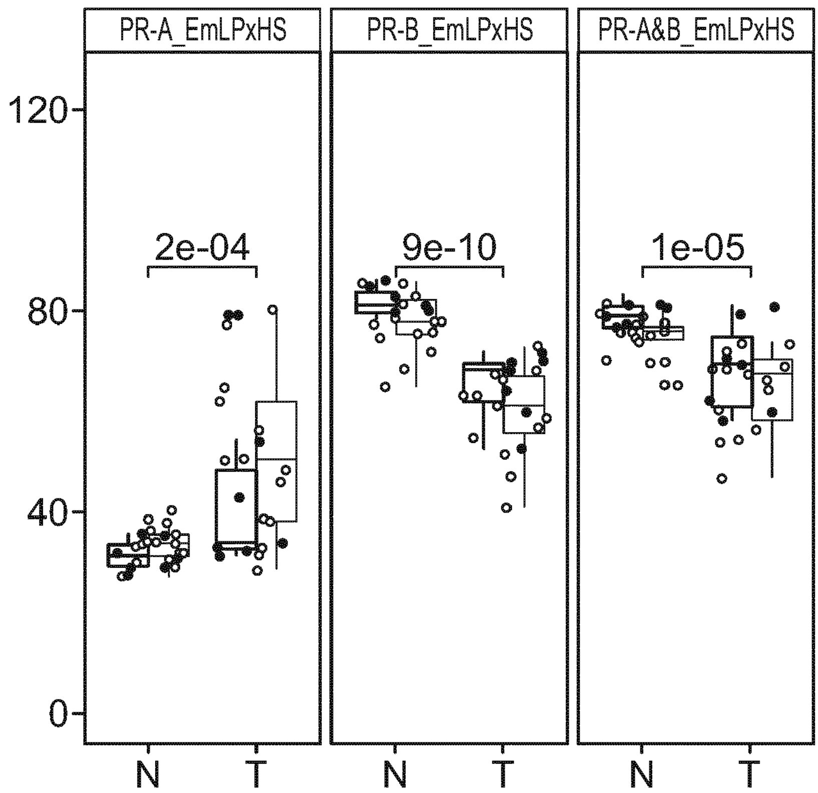
PR-A_EmLPxHS
PR-B_EmLPxHS
PR-A&B_EmLPxHS
120
80
40
0
2e-04
9e-10
1e-05
N
T
N
T
N
T

(b)

FIG. 19

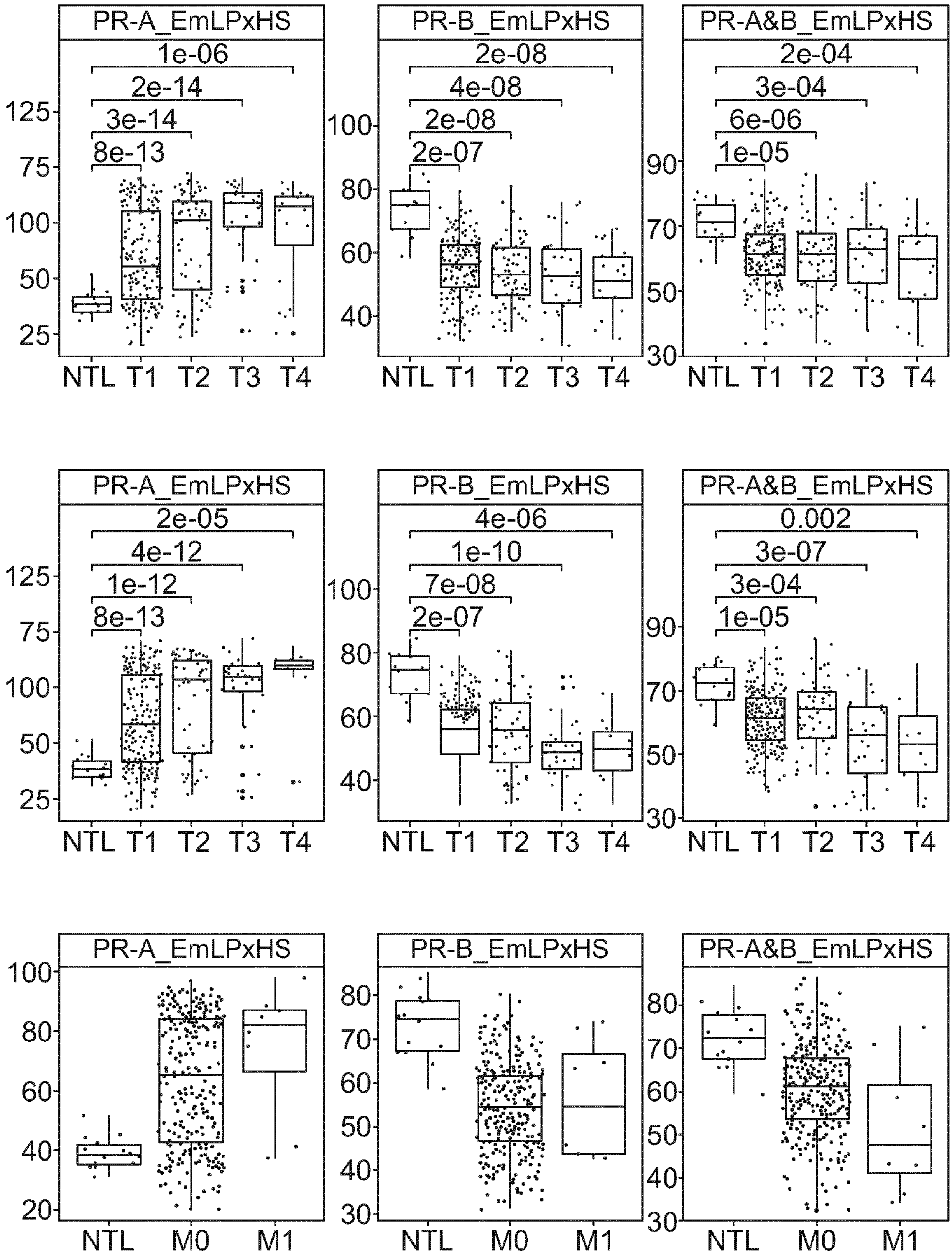
PR-A_EmLPxHS
PR-B_EmLPxHS
PR-A&B_EmLPxHS
1e-06
2e-14
3e-14
8e-13
2e-08
4e-08
2e-08
2e-07
2e-04
3e-04
6e-06
1e-05
2e-05
4e-12
1e-12
8e-13
4e-06
1e-10
7e-08
2e-07
0.002
3e-07
3e-04
1e-05
NTL T1 T2 T3 T4
NTL M0 M1

FIG. 20

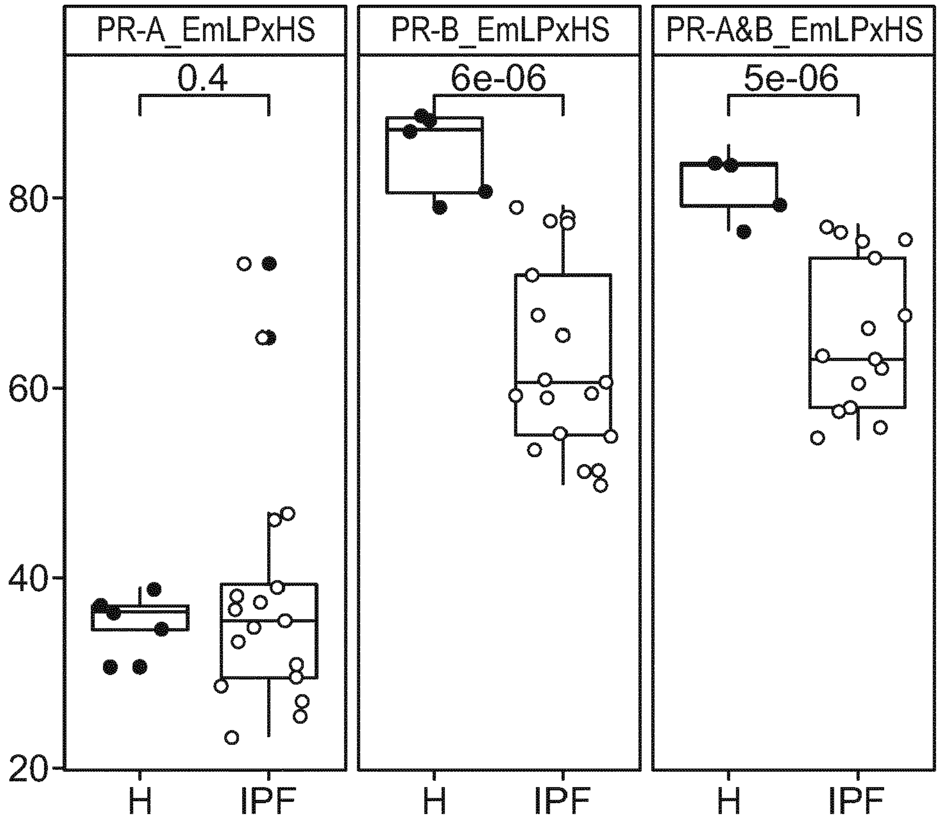
PR-A_EmLPxHS
PR-B_EmLPxHS
PR-A&B_EmLPxHS
0.4
6e-06
5e-06
80
60
40
20
H
IPF
H
IPF
H
IPF

FIG. 21

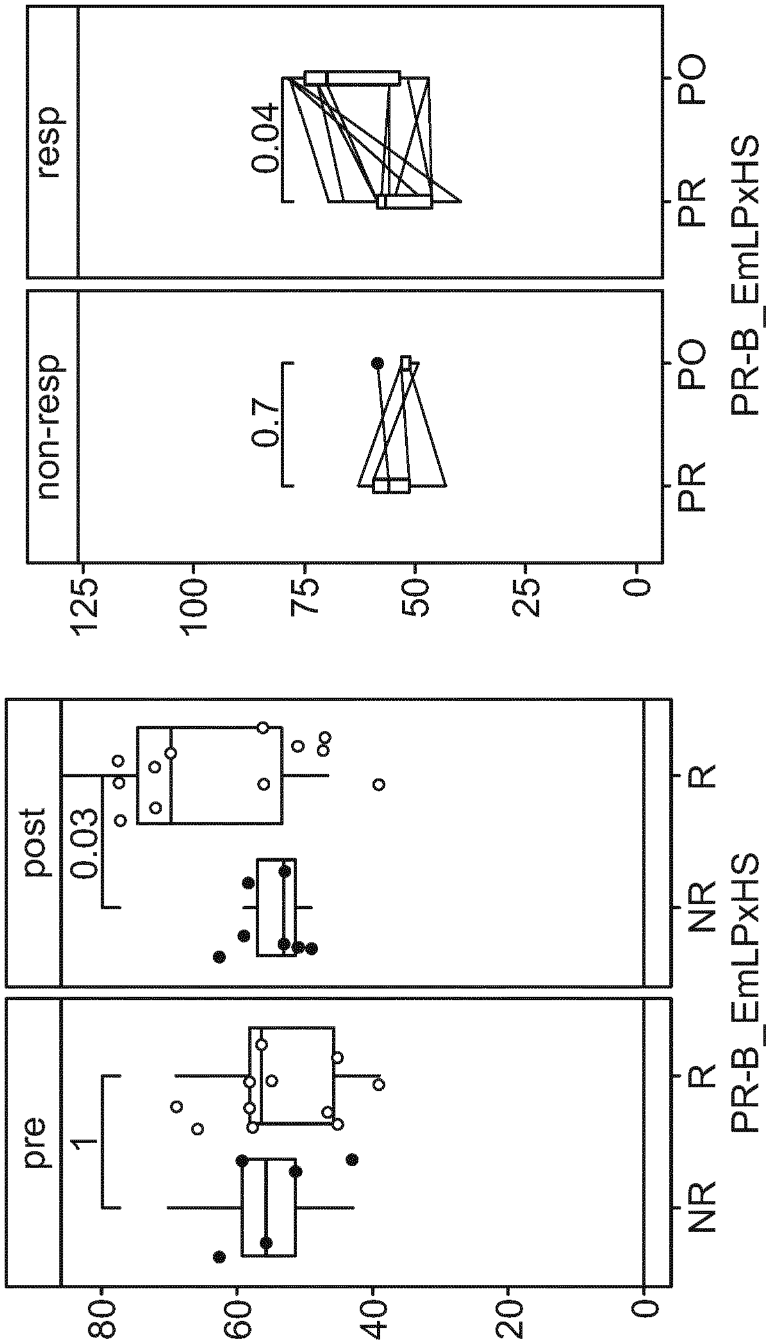
pre
post
1
0.03
80
60
40
20
0
NR
R
PR-B_EmLPxHS
non-resp
resp
0.7
0.04
125
100
75
50
25
0
PR
PO
PR-B_EmLPxHS

FIG. 22

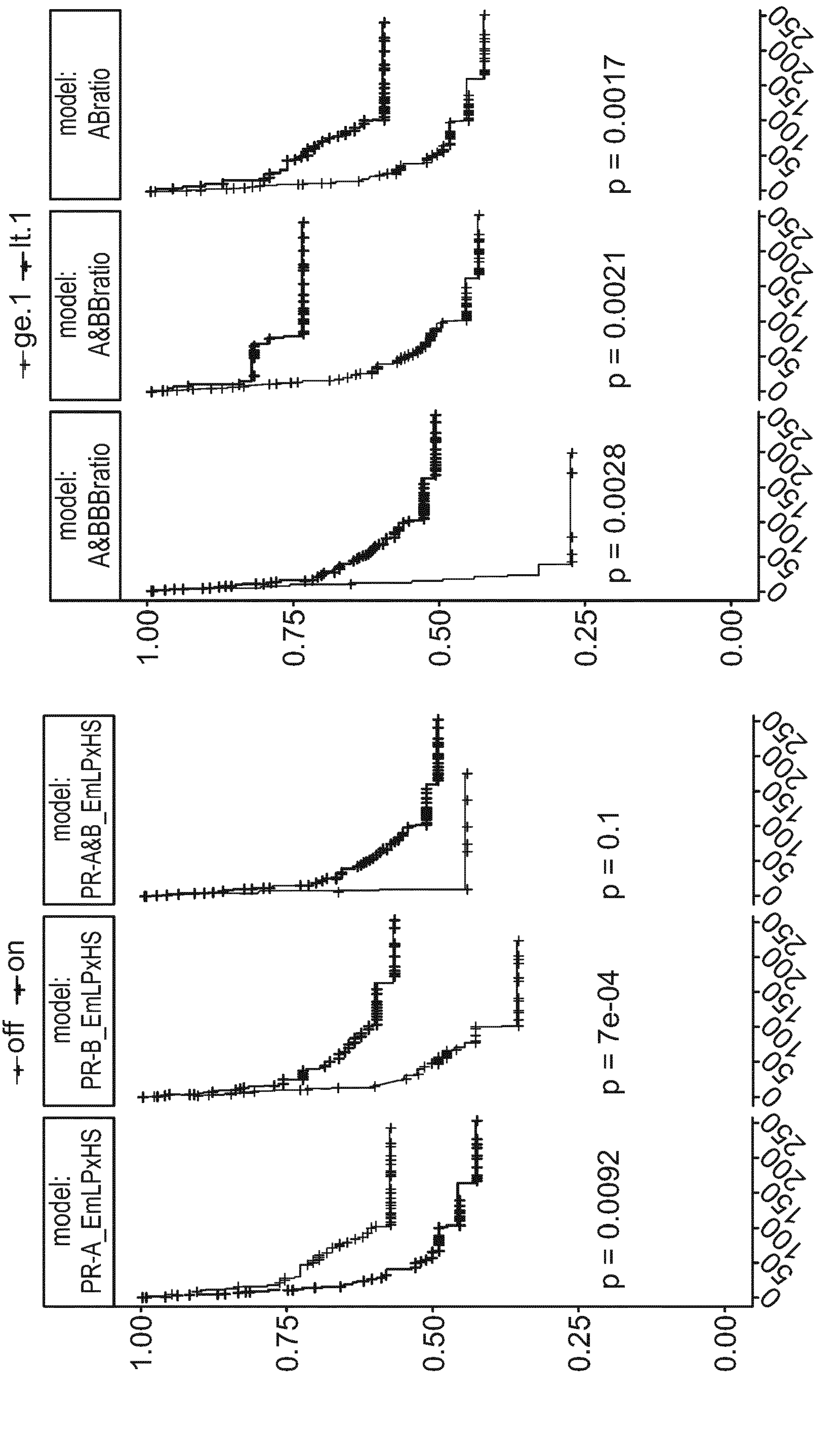
off
on
model: PR-A_EmLPxHS
p = 0.0092
model: PR-B_EmLPxHS
p = 7e-04
model: PR-A&B_EmLPxHS
p = 0.1
ge.1
lt.1
model: A&BBBratio
p = 0.0028
model: A&BBratio
p = 0.0021
model: ABratio
p = 0.0017
1.00
0.75
0.50
0.25
0.00
0
50
100
150
200
250

FIG. 23

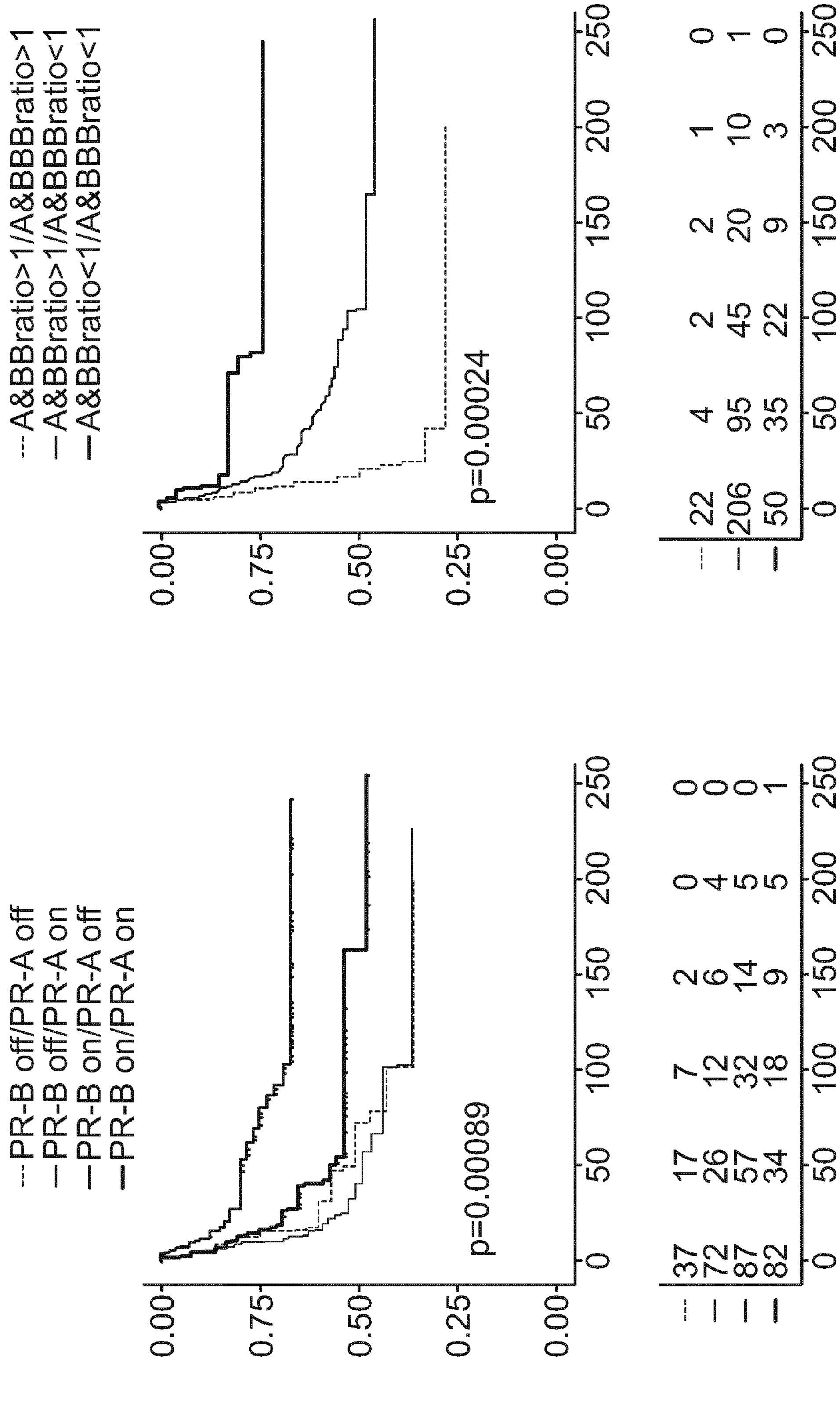
PR-B off/PR-A off
PR-B off/PR-A on
PR-B on/PR-A off
PR-B on/PR-A on
p=0.00089
0.00
0.75
0.50
0.25
0.00
0
50
100
150
200
250
37 17 7 2 0 0
72 26 12 6 4 0
87 57 32 14 5 0
82 34 18 9 5 1
A&BBratio>1/A&BBBratio>1
A&BBratio>1/A&BBBratio<1
A&BBratio<1/A&BBBratio<1
p=0.00024
22 4 2 2 1 0
206 95 45 20 10 1
50 35 22 9 3 0

FIG. 24

FIG. 25

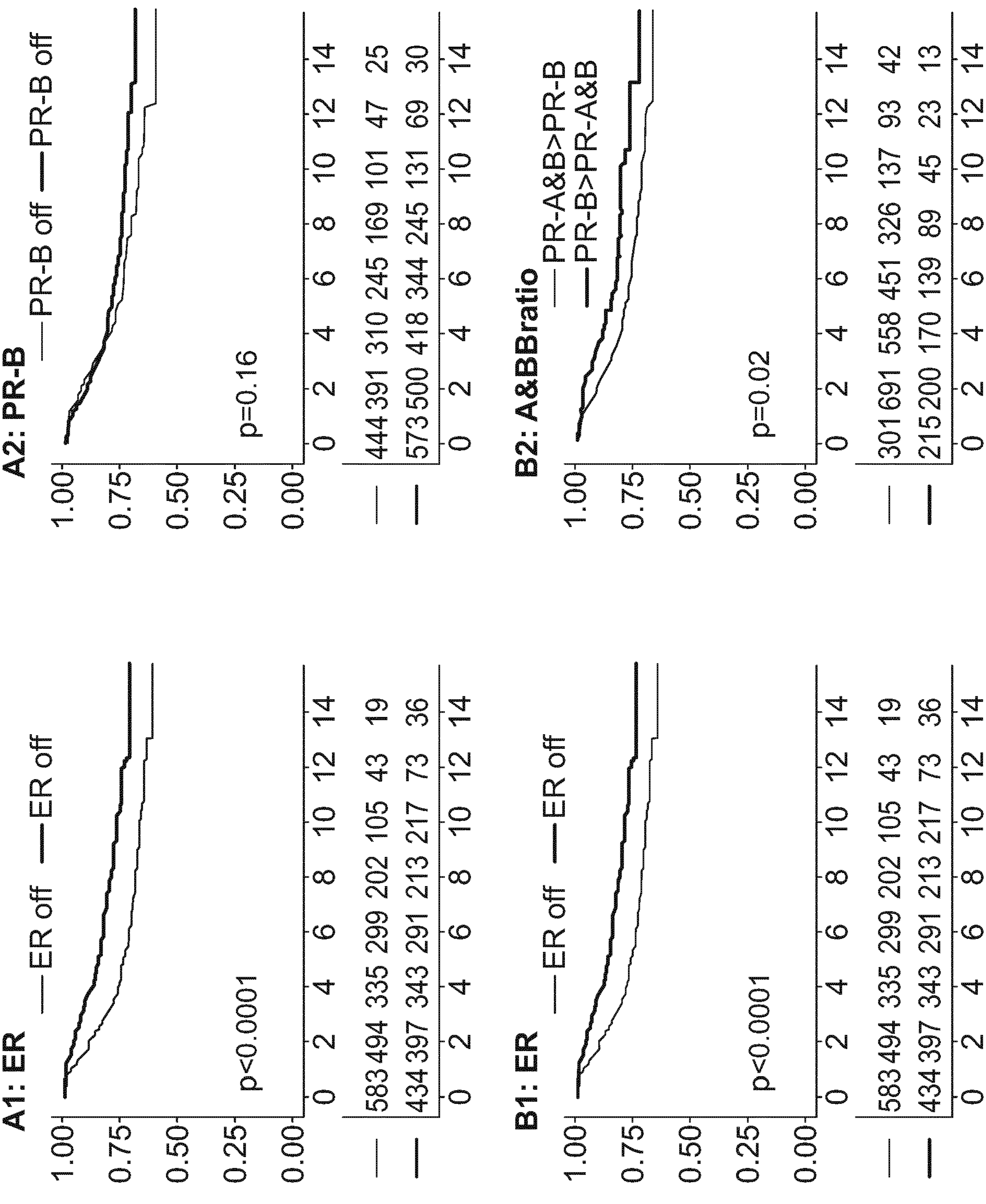

A1: ER
ER off
ER off
p<0.0001
583 494 335 299 202 105 43 19
434 397 343 291 213 217 73 36
A2: PR-B
PR-B off
PR-B off
p=0.16
444 391 310 245 169 101 47 25
573 500 418 344 245 131 69 30
B1: ER
ER off
ER off
p<0.0001
583 494 335 299 202 105 43 19
434 397 343 291 213 217 73 36
B2: A&BBratio
PR-A&B>PR-B
PR-B>PR-A&B
p=0.02
301 691 558 451 326 137 93 42
215 200 170 139 89 45 23 13
1.00
0.75
0.50
0.25
0.00
0 2 4 6 8 10 12 14

FIG. 25 (cont.)

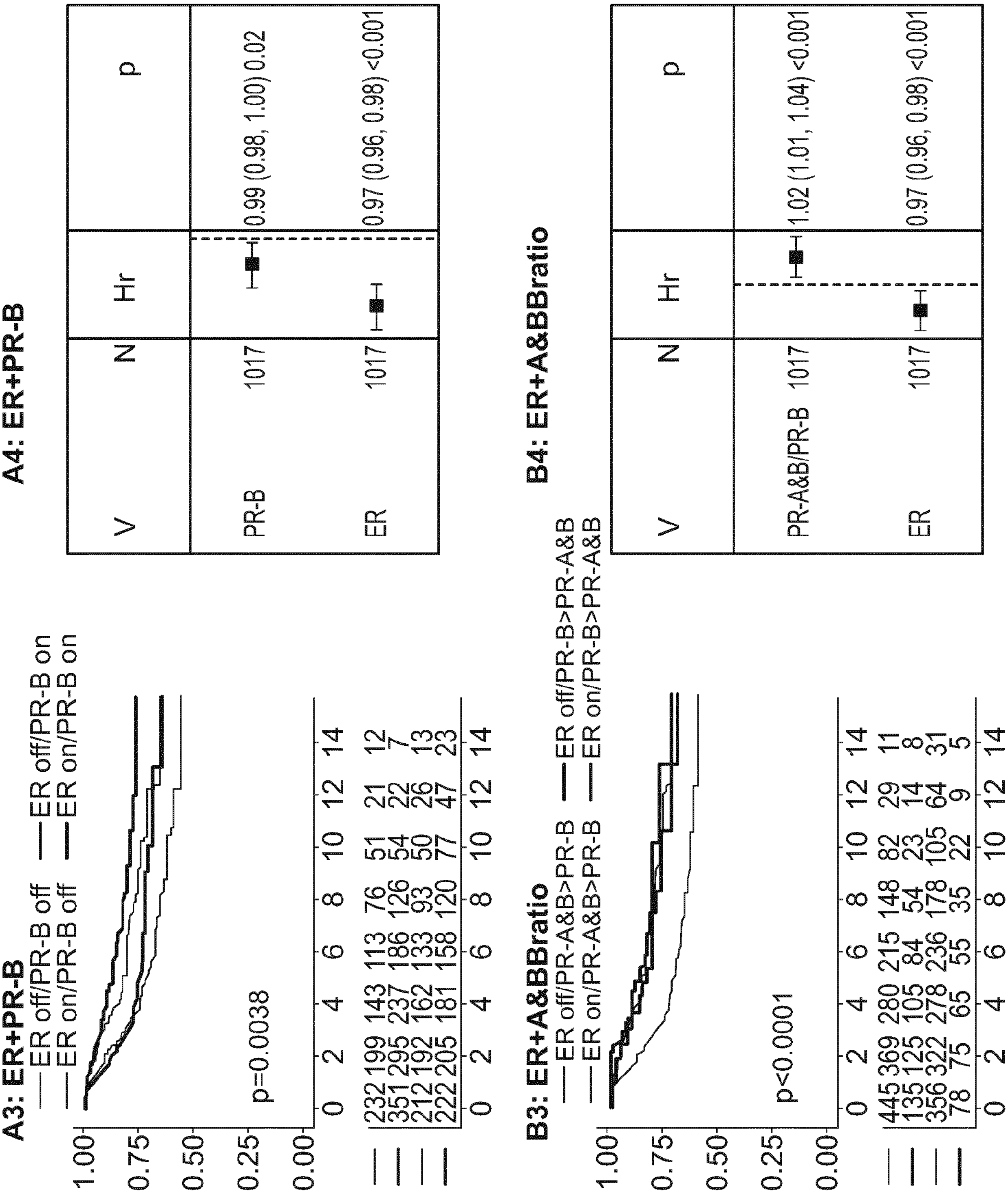
A3: ER+PR-B
ER off/PR-B off
ER on/PR-B off
ER off/PR-B on
ER on/PR-B on
1.00
0.75
0.50
0.25
0.00
p=0.0038
0 2 4 6 8 10 12 14
232 199 143 113 76 51 21 12
351 295 237 186 126 54 22 7
212 192 162 133 93 50 26 13
222 205 181 158 120 77 47 23
A4: ER+PR-B
V N Hr p
PR-B 1017 0.99 (0.98, 1.00) 0.02
ER 1017 0.97 (0.96, 0.98) <0.001
B3: ER+A&BBratio
ER off/PR-A&B>PR-B
ER on/PR-A&B>PR-B
ER off/PR-B>PR-A&B
ER on/PR-B>PR-A&B
p<0.0001
445 369 280 215 148 82 29 11
135 125 105 84 54 23 14 8
356 322 278 236 178 105 64 31
78 75 65 55 35 22 9 5
B4: ER+A&BBratio
V N Hr p
PR-A&B/PR-B 1017 1.02 (1.01, 1.04) <0.001
ER 1017 0.97 (0.96, 0.98) <0.001

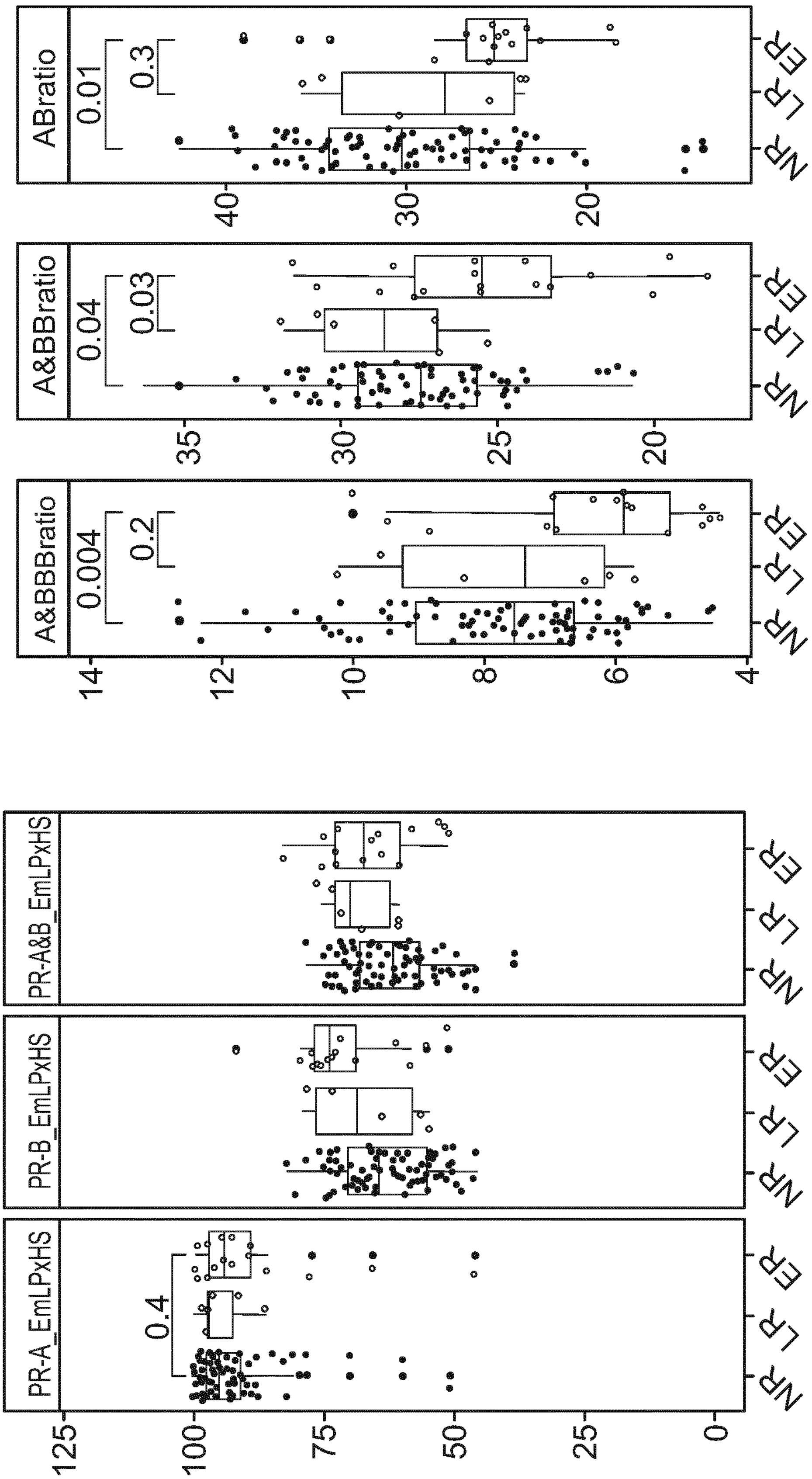
PR-A_EmLPxHS
0.4
PR-B_EmLPxHS
PR-A&B_EmLPxHS
A&BBBratio
0.004
0.2
A&BBratio
0.04
0.03
ABratio
0.01
0.3
NR
LR
ER

FIG. 26

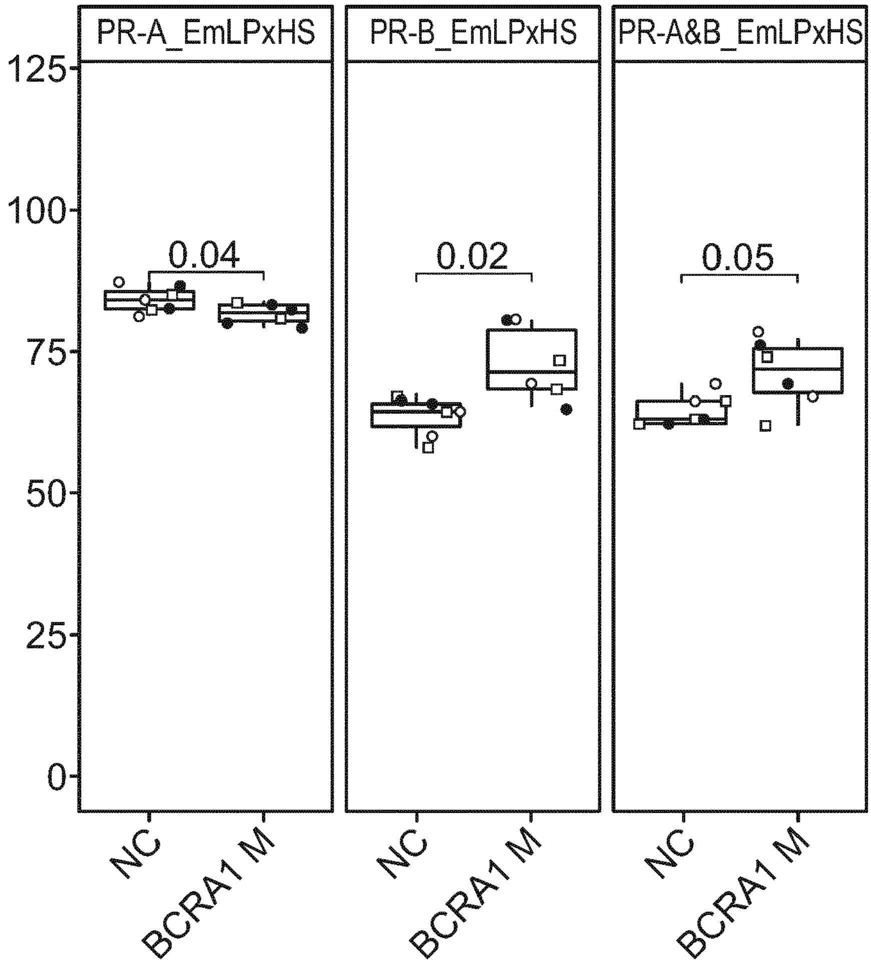
PR-A_EmLPxHS
PR-B_EmLPxHS
PR-A&B_EmLPxHS
125
100
75
50
25
0
0.04
0.02
0.05
NC
BCRA1 M

FIG. 27

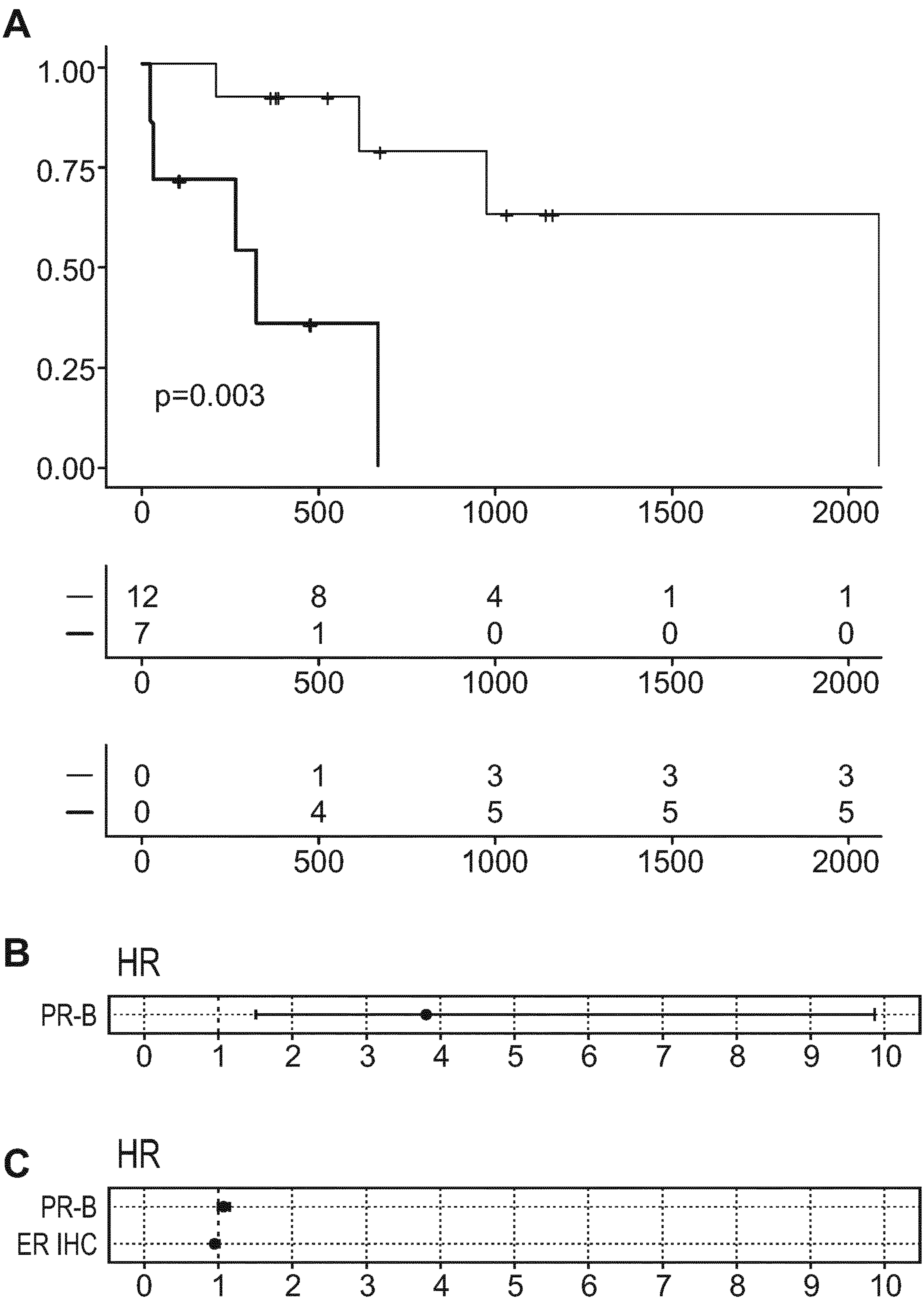
A
1.00
0.75
0.50
0.25
0.00
p=0.003
0
500
1000
1500
2000
12 8 4 1 1
7 1 0 0 0
0 1 3 3 3
0 4 5 5 5
B
HR
PR-B
0 1 2 3 4 5 6 7 8 9 10
C
HR
PR-B
ER IHC
0 1 2 3 4 5 6 7 8 9 10

FIG. 28

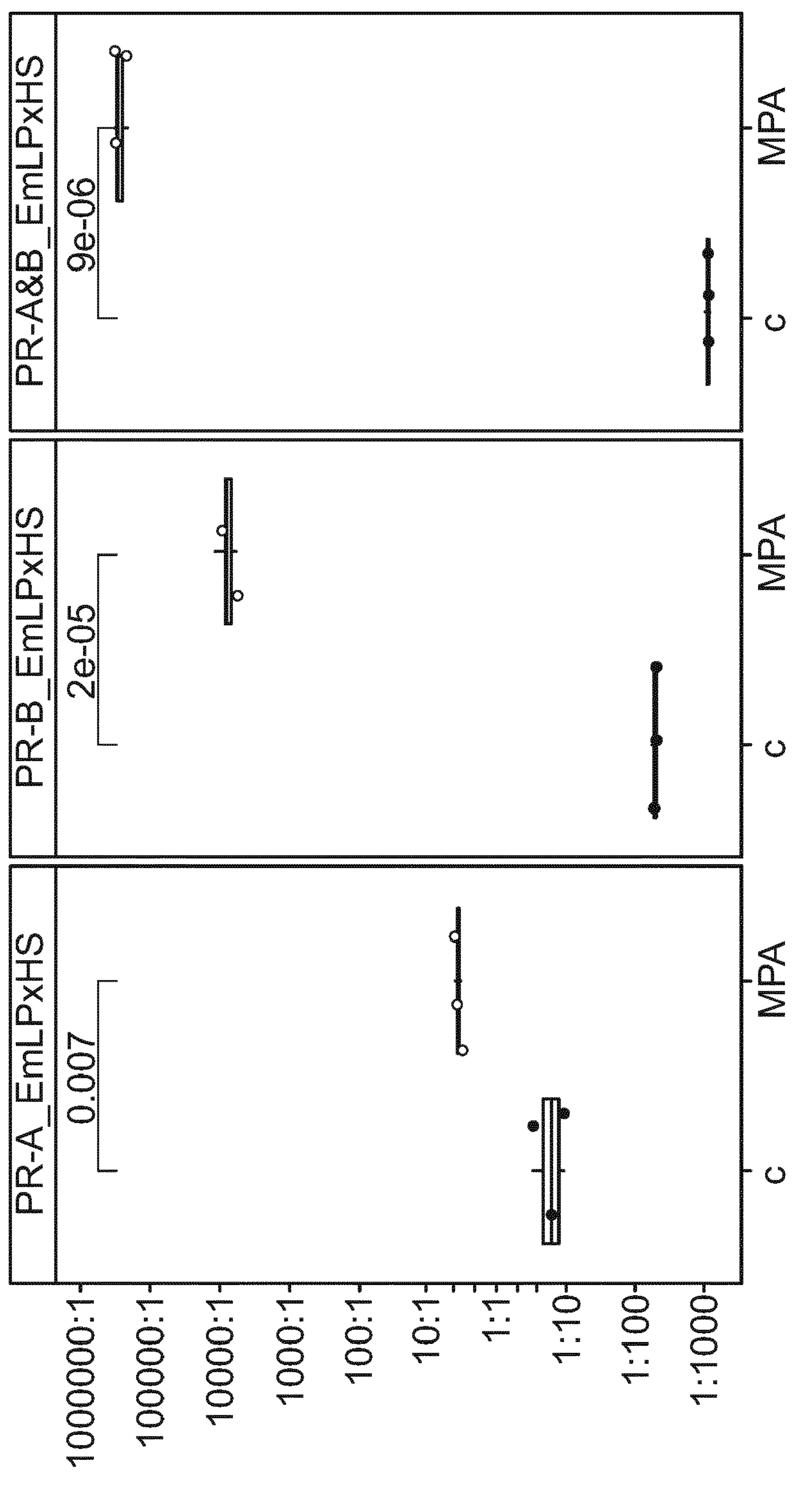
PR-A_EmLPxHS
0.007
PR-B_EmLPxHS
2e-05
PR-A&B_EmLPxHS
9e-06
1000000:1
100000:1
10000:1
1000:1
100:1
10:1
1:1
1:10
1:100
1:1000
c
MPA

FIG. 29

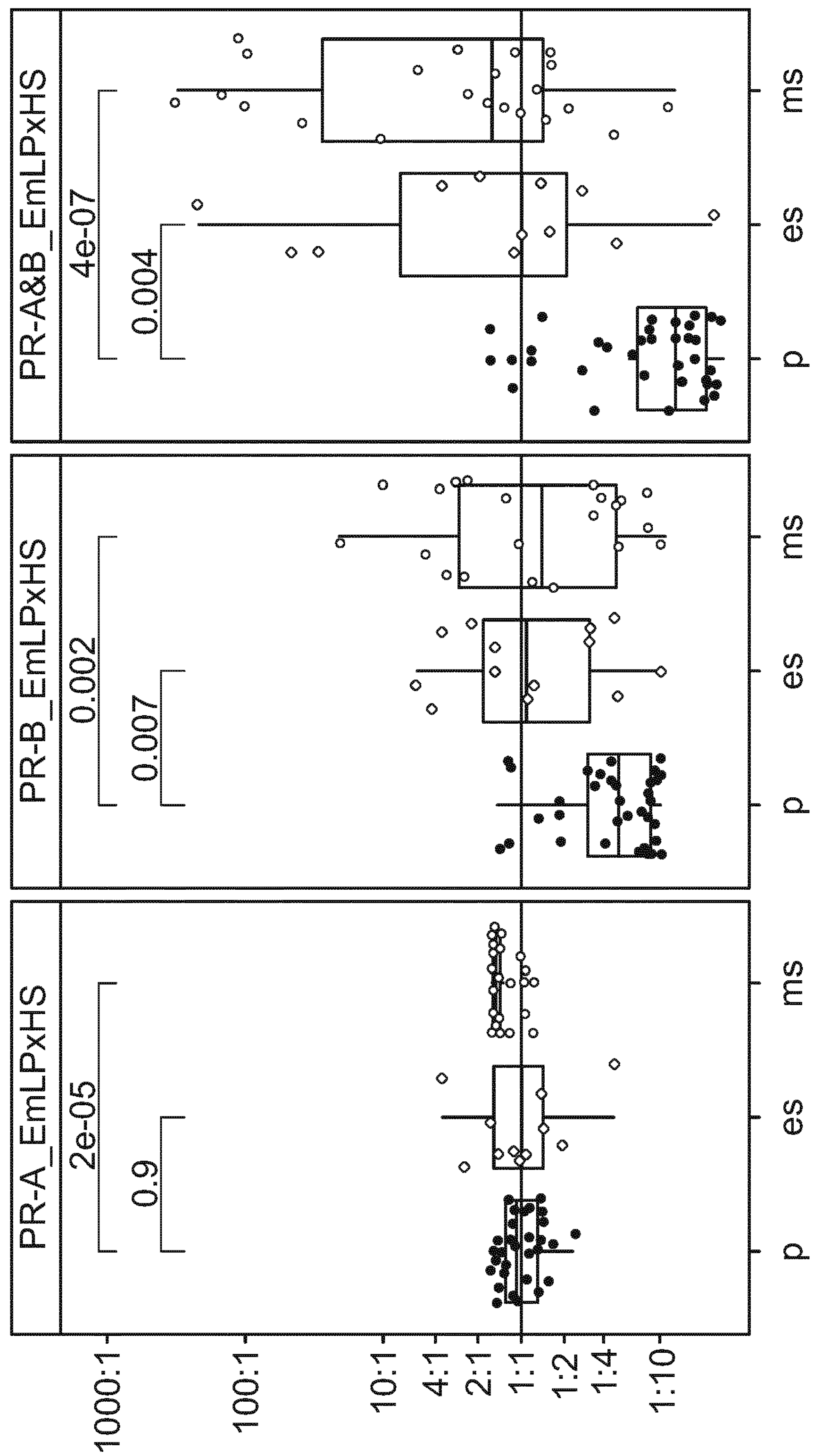
PR-A_EmLPxHS
2e-05
0.9
PR-B_EmLPxHS
0.002
0.007
PR-A&B_EmLPxHS
4e-07
0.004
p
es
ms
1000:1
100:1
10:1
4:1
2:1
1:1
1:2
1:4
1:10

FIG. 30

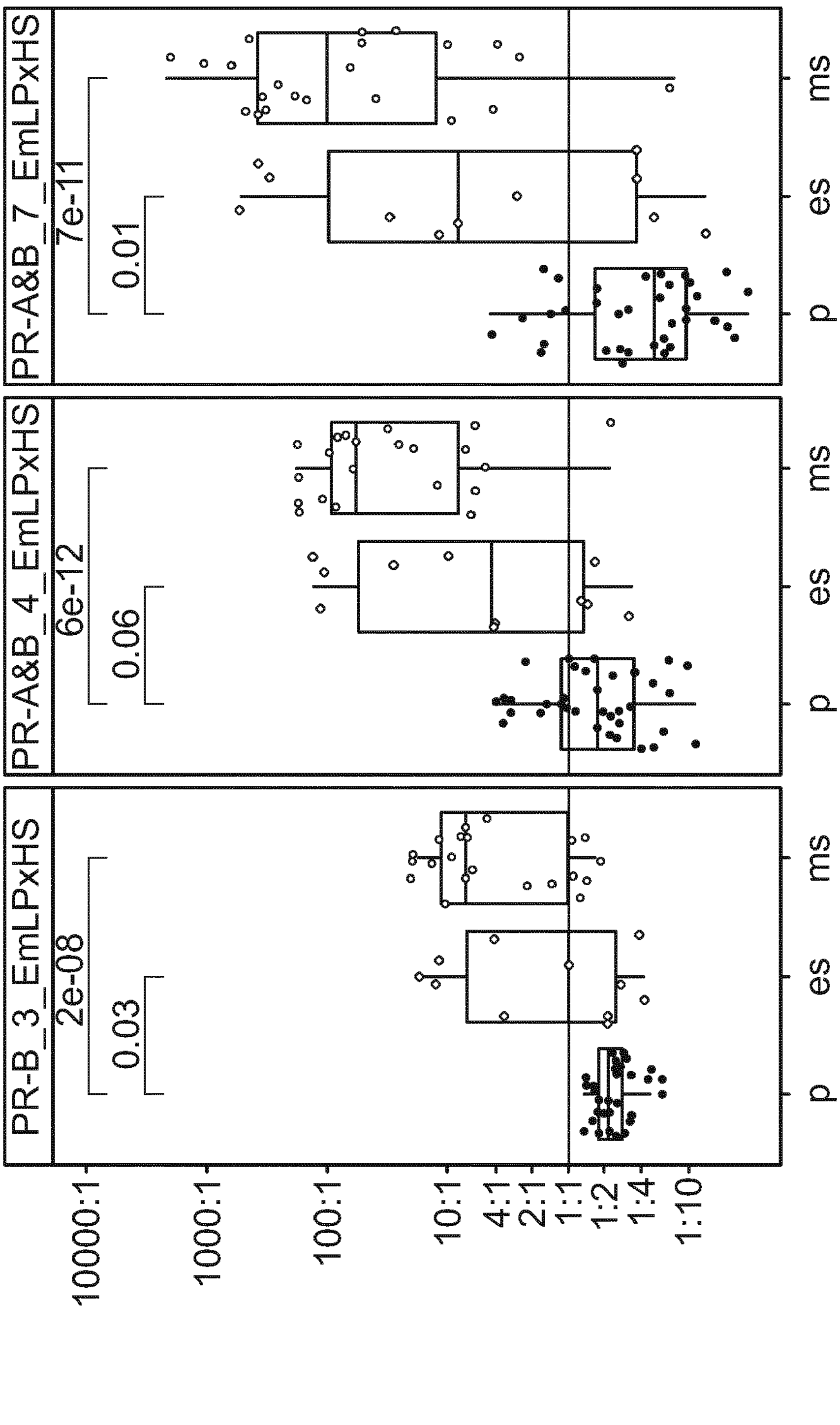
PR-B_3_EmLPxHS
2e-08
0.03
PR-A&B_4_EmLPxHS
6e-12
0.06
PR-A&B_7_EmLPxHS
7e-11
0.01
10000:1
1000:1
100:1
10:1
4:1
2:1
1:1
1:2
1:4
1:10
p
es
ms

FIG. 31

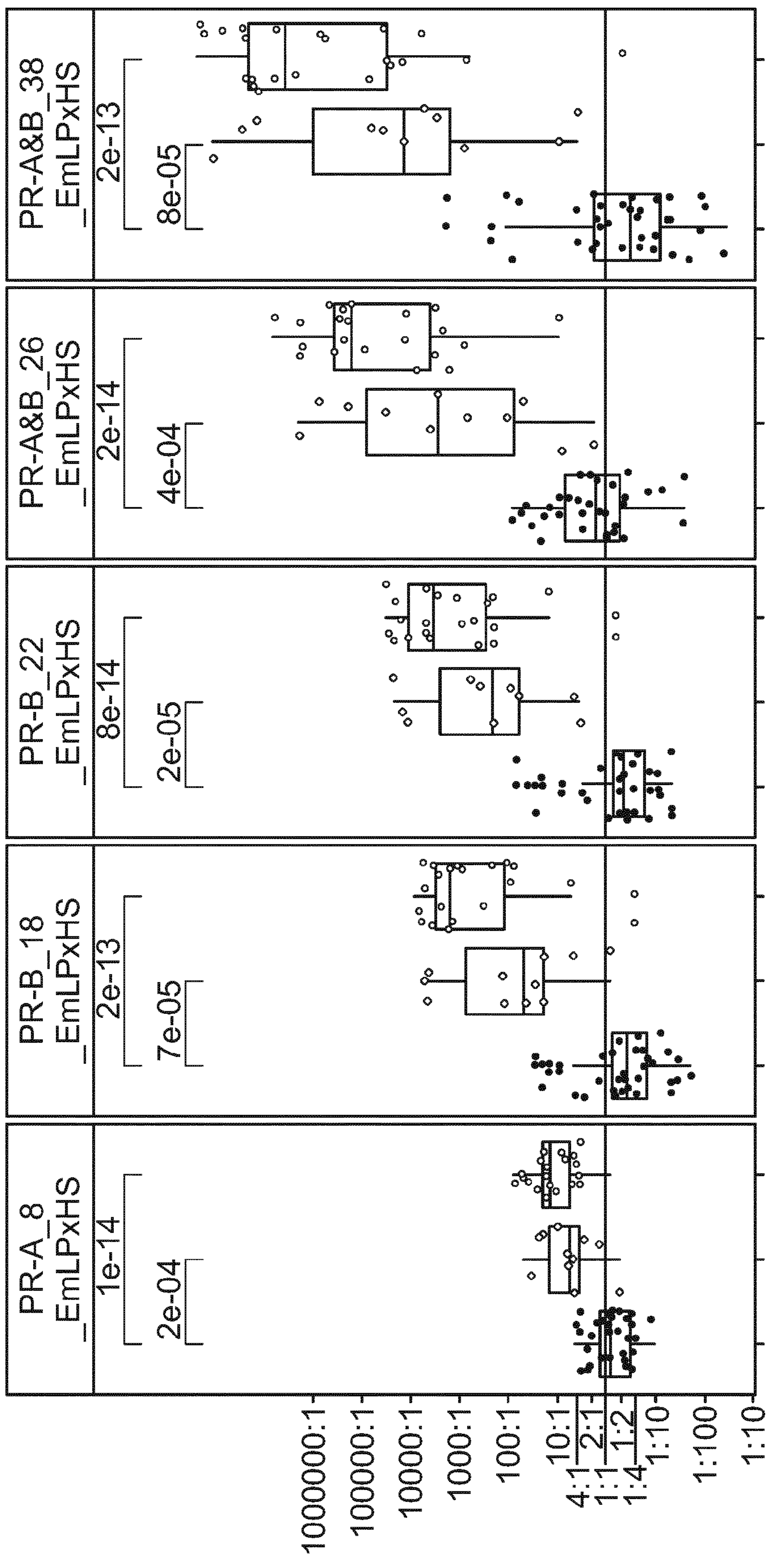
PR-A_8 _EmLPxHS
1e-14
2e-04
PR-B_18 _EmLPxHS
2e-13
7e-05
PR-B_22 _EmLPxHS
8e-14
2e-05
PR-A&B_26 _EmLPxHS
2e-14
4e-04
PR-A&B_38 _EmLPxHS
2e-13
8e-05
p
es
ms
1000000:1
100000:1
10000:1
1000:1
100:1
10:1
4:1
2:1
1:1
1:2
1:4
1:10
1:100
1:10

FIG. 32

Europäisches Patentamt
European Patent Office
Office européen des brevets

# EUROPEAN SEARCH REPORT

Application Number
EP 19 20 9115

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2013/162776 A1 (LANGE CAROL [US]; KNUTSON TODD [US]; NIKAS JASON BASIL [US]) 31 October 2013 (2013-10-31)<br>* page 43, line 17 - line 24; claims 1, 26 *<br>----- | 1-15 | INV.<br>C12Q1/6886 |
| A | WO 02/00618 A2 (UNIV TECHNOLOGY CORP [US]; HORWITZ KATHRYN B [US] ET AL.) 3 January 2002 (2002-01-03)<br>* tables 9-14 *<br>----- | 1-15 | |
| A | TREVIÑO LINDSEY S ET AL: "The requirement for p42/p44 MAPK activity in progesterone receptor-mediated gene regulation is target gene-specific", STEROIDS, ELSEVIER SCIENCE PUBLISHERS, NEW YORK, NY, US, vol. 78, no. 6, 1 February 2013 (2013-02-01), pages 542-547, XP028586371, ISSN: 0039-128X, DOI: 10.1016/J.STEROIDS.2012.12.014<br>* page 543, right-hand column, paragraph 2; figure 3 *<br>----- | 1-15 | TECHNICAL FIELDS SEARCHED (IPC)<br>C12Q |
| A | US 2014/363425 A1 (GRAHAM J DINNY [AU] ET AL) 11 December 2014 (2014-12-11)<br>* paragraph [0059]; claim 1 *<br>----- | 1-15 | |
| A,D | WO 2014/102668 A2 (KONINKL PHILIPS NV [NL]) 3 July 2014 (2014-07-03)<br>* the whole document *<br>-----<br>-/-- | 1-15 | |

The present search report has been drawn up for all claims

1

| Place of search | Date of completion of the search | Examiner |
|---|---|---|
| The Hague | 8 May 2020 | Santagati, Fabio |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 19 20 9115

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | "GeneChip Human Genome U133 Set", INTERNET CITATION, 26 February 2003 (2003-02-26), XP002232760, Retrieved from the Internet: URL:http://www.affymetrix.com/support/technical/datasheets/hgu133_datasheet.pdf [retrieved on 2003-02-26] * the whole document * ----- | 14 | |

TECHNICAL FIELDS SEARCHED (IPC)

The present search report has been drawn up for all claims

| Place of search | Date of completion of the search | Examiner |
|---|---|---|
| The Hague | 8 May 2020 | Santagati, Fabio |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

1

EPO FORM 1503 03.82 (P04C01)

## ANNEX TO THE EUROPEAN SEARCH REPORT ON EUROPEAN PATENT APPLICATION NO. EP 19 20 9115

This annex lists the patent family members relating to the patent documents cited in the above-mentioned European search report.
The members are as contained in the European Patent Office EDP file on
The European Patent Office is in no way liable for these particulars which are merely given for the purpose of information.

08-05-2020

| Patent document cited in search report | Publication date | Patent family member(s) | Publication date |
|---|---|---|---|
| WO 2013162776 A1 | 31-10-2013 | AU 2013252913 A1 | 27-11-2014 |
| | | CA 2871590 A1 | 31-10-2013 |
| | | EP 2841594 A1 | 04-03-2015 |
| | | HK 1208057 A1 | 19-02-2016 |
| | | JP 2015516155 A | 11-06-2015 |
| | | KR 20150028232 A | 13-03-2015 |
| | | NZ 701652 A | 30-09-2016 |
| | | PH 12014502410 A1 | 12-01-2015 |
| | | RU 2014147625 A | 20-06-2016 |
| | | US 2013316992 A1 | 28-11-2013 |
| | | WO 2013162776 A1 | 31-10-2013 |
| | | ZA 201407853 B | 23-12-2015 |
| WO 0200618 A2 | 03-01-2002 | AU 7157901 A | 08-01-2002 |
| | | WO 0200618 A2 | 03-01-2002 |
| US 2014363425 A1 | 11-12-2014 | NONE | |
| WO 2014102668 A2 | 03-07-2014 | AU 2013368945 A1 | 13-08-2015 |
| | | BR 112015015131 A2 | 11-07-2017 |
| | | CA 2896414 A1 | 03-07-2014 |
| | | CN 105074005 A | 18-11-2015 |
| | | EP 2938745 A2 | 04-11-2015 |
| | | JP 6449779 B2 | 09-01-2019 |
| | | JP 2016509472 A | 31-03-2016 |
| | | JP 2019076096 A | 23-05-2019 |
| | | KR 20150100896 A | 02-09-2015 |
| | | RU 2015131009 A | 30-01-2017 |
| | | US 2015347672 A1 | 03-12-2015 |
| | | WO 2014102668 A2 | 03-07-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013011479 A2
- WO 2014102668 A2
- US 6713297 B
- US 5476928 A
- US 5958691 A
- US 5436134 A
- US 5658751 A


### Non-patent literature cited in the description


- **DRESSING, G.E. et al.** Progesterone Receptors Act as Sensors for Mitogenic Protein Kinases in Breast Cancer Models. *Endocrine-Related Cancer,* 2009, vol. 16 (2), 351-61
- **HAGAN, C.R. ; LANGE, C.A.** Molecular Determinants of Context-Dependent Progesterone Receptor Action in Breast Cancer. *BMC Medicine,* 2014, vol. 12, 32
- **OBR, A.E. ; EDWARDS D.P.** The Biology of Progesterone Receptor in the Normal Mammary Gland and in Breast Cancer. *Molecular and Cellular Endocrinology,* 2012, vol. 357 (1-2), 4-17
- **LANARI, C. et al.** Antiprogestins in Breast Cancer Treatment: Are We Ready. *Endocrine-Related Cancer,* 2012, vol. 19 (3), 35-50
- **ROJAS, P.A. et al.** Progesterone Receptor Isoform Ratio: A Breast Cancer Prognostic and Predictive Factor for Antiprogestin Responsiveness. *Journal of the National Cancer Institute,* 2017, vol. 109 (7
- **MOHAMMED, H. et al.** Progesterone Receptor Modulates ERα Action in Breast Cancer. *Nature,* 2015, vol. 523 (7560), 313-17
- **EMONS, G. et al.** Phase II Study of Fulvestrant 250 Mg/Month in Patients with Recurrent or Metastatic Endometrial Cancer: A Study of the Arbeitsgemeinschaft Gynakologische Onkologie. *Gynecologic Oncology,* 2013, vol. 129 (3), 495-99
- **PATEL, B. et al.** Role of Nuclear Progesterone Receptor Isoforms in Uterine Pathophysiology. *Human Reproduction Update,* 2015, vol. 21 (2), 155-73
- **VERHAEGH W. et al.** Selection of personalized patient therapy through the use of knowledge-based computational models that identify tumor-driving signal transduction pathways. *Cancer Research,* 2014, vol. 74 (11), 2936-2945
- **MOTE, P.A. et al.** Progesterone Receptor A Predominance Is a Discriminator of Benefit from Endocrine Therapy in the ATAC Trial. *Breast Cancer Research and Treatment,* 2015, vol. 151 (2), 309-318
- **ARNETT-MANSFIELD, R.L. et al.** Subnuclear Distribution of Progesterone Receptors A and B in Normal and Malignant Endometrium. *The Journal of Clinical Endocrinology & Metabolism,* 2004, vol. 89 (3), 1429-1442